# EUROPEAN PATENT APPLICATION

(11) **EP 4 442 690 A1**
(43) Date of publication of application: **09.10.2024**
(21) Application number: 22900534.3
(22) Date of filing: 30.11.2022
(51) Int. Cl.: C07D 498/22, C07D 519/00, A61K 31/496, A61K 31/4545, A61K 31/5377, A61P 35/00

(54) **COMPOUND CONTAINING CYCLOALKYL OR HALOALKYL**

(30) Priority: 30.11.2021 CN 202111441498; 29.12.2021 CN 202111638379; 23.05.2022 CN 202210564004; 27.07.2022 CN 202210889925; 25.11.2022 CN 202211492694
(71) Applicant: Chia Tai Tianqing Pharmaceutical Group Co., Ltd., Lianyungang, Jiangsu 222062 (CN)
(72) Inventor: LIU, Fei, Lianyungang Jiangsu 222062 (CN); PENG, Yan, Lianyungang Jiangsu 222062 (CN); XU, Hongjiang, Lianyungang Jiangsu 222062 (CN); FENG, Taotao, Lianyungang Jiangsu 222062 (CN); ZHANG, Xiquan, Lianyungang Jiangsu 222062 (CN); REN, Cheng, Lianyungang Jiangsu 222062 (CN); SHI, Wei, Lianyungang Jiangsu 222062 (CN)
(74) Representative: Potter Clarkson
(86) International application number: PCT/CN2022/135481
(87) International publication number: WO 2023/098730

(57) **Abstract**

The present disclosure relates to a compound containing cycloalkyl or haloalkyl, and specifically discloses a compound represented by formula I", a stereoisomer or a pharmaceutically acceptable salt thereof, a preparation method therefor, a pharmaceutical composition containing the compound, and a use thereof in treating tumors.

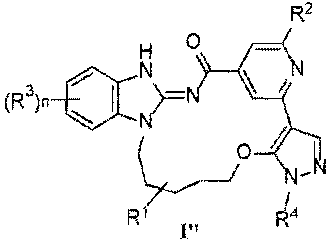

## Description

### CROSS-REFERENCE TO RELATED APPLICATIONS

The present application claims priority and benefits to the following Chinese patent applications filed with the China National Intellectual Property Administration, the disclosures of which are incorporated herein by reference in their entireties:
Chinese Patent Application No. 202111441498.1, filed on Nov. 30, 2021;
Chinese Patent Application No. 202111638379.5, filed on Dec. 29, 2021;
Chinese Patent Application No. 202210564004.7, filed on May 23, 2022;
Chinese Patent Application No. 202210889925.0, filed on Jul. 27, 2022; and
Chinese Patent Application No. 202211492694.6, filed on Nov. 25, 2022.

### TECHNICAL FIELD

The present application relates to a compound containing cycloalkyl or haloalkyl, and particularly discloses a compound represented by a formula I", a stereoisomer thereof or a pharmaceutically acceptable salt thereof, a preparation method therefor, a pharmaceutical composition containing the compound, and use thereof in treating a tumor.

### BACKGROUND

The epidermal growth factor receptor tyrosine kinase inhibitor (EGFR-TKI), as a small molecule inhibitor, inhibits activation of tyrosine kinase by competing with an endogenous ligand for binding to EGFR, to block the EGFR signaling pathway, finally producing a series of biological effects such as the inhibition of proliferation and metastasis of tumor cells, the promotion of apoptosis of tumor cells and the like. Therefore, the EGFR-TKI is one of the main targets for treating lung cancer.

Osimertinib (AZD9291) is a third-generation EGFR-TKI-targeted drug, and although it has a relatively high response rate when addressing drug resistance caused by a T790M mutation, patients may still develop drug resistance. According to the analysis of existing research on the drug resistance of AZD9291, the third mutation, namely EGFR C797S mutation, is obtained, which is one of the main mechanisms causing drug resistance to Osimertinib, and accounts for about 40%. It is of great research significance to provide patients with a fourth-generation EGFR C797S/T790M inhibitor which is safer and more effective.

### SUMMARY

In one aspect, the present application relates to a compound of formula I", a stereoisomer thereof or a pharmaceutically acceptable salt thereof, 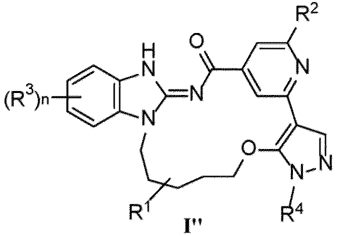 wherein,
R¹ is selected from the group consisting of C₃₋₅ cycloalkyl, C₃₋₅ cycloalkyl-C₁₋₃ alkyl-, C₁₋₆ haloalkyl, or C₁₋₆ alkyl;
R² is selected from the group consisting of hydrogen, halogen, OH, NH₂, C₁₋₈ alkyl, or C₁₋₈ alkoxy, wherein the C₁₋₈ alkyl or C₁₋₈ alkoxy is optionally substituted with one or more R';
R' is selected from the group consisting of halogen, OH, cyano, NH₂, or nitro;
R³ is selected from the group consisting of halogen, cyano, nitro, NH₂, OH, C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, 5- to 10-membered heteroaryl, -NHR^{b}, -NR^{a}R^{b}, -OR^{a}, - SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, -N(C₁₋₄ alkyl)C(O)OR^{a}, or - NHC(O)NHR^{a}, wherein the C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, or 5- to 10-membered heteroaryl is optionally substituted with one or more R^{c};
R^{c} is selected from the group consisting of oxo, OH, NH₂, halogen, cyano, nitro, C₁₋₈ alkyl, C₁₋₈ alkoxy, -N(C₁₋₈ alkyl)₂, -NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, - NHS(O)₂R^{a}, -C₁₋₆ alkyl -S(O)₂-C₁₋₆ alkyl, or 3- to 8-membered heterocycloalkyl optionally substituted with one or more groups independently selected from the group consisting of OH, halogen, or C₁₋₆ alkyl;
R^{a} and R^{b} are each independently selected from the group consisting of the following groups optionally substituted with one or more R": C₁₋₈ alkyl, C₂₋₈ alkenyl, C₂₋₈ alkynyl, C₃₋₁₀ cycloalkyl, 3- to 10-membered heterocycloalkyl, C₆₋₁₀ aryl, or 5- to 10-membered heteroaryl;
R" are each independently selected from the group consisting of halogen, oxo, deuterium, NH₂, OH, cyano, nitro, C₁₋₈ alkyl, C₁₋₈ haloalkyl, C₁₋₈ alkoxy, -N(C₁₋₈ alkyl)₂, -NH(C₁₋₈ alkyl), C₃₋₆ cycloalkyl, or 3- to 10-membered heterocycloalkyl optionally substituted with C₁₋₈ alkyl;
n is selected from the group consisting of 0, 1, 2, 3, or 4;
R⁴ is selected from the group consisting of C₁₋₆ alkyl or C₃₋₅ cycloalkyl, provided that when R¹ is selected from C₁₋₆ alkyl, R⁴ is selected from C₃₋₅ cycloalkyl.

In another aspect, the present application relates to a compound of formula I', a stereoisomer thereof or a pharmaceutically acceptable salt thereof, 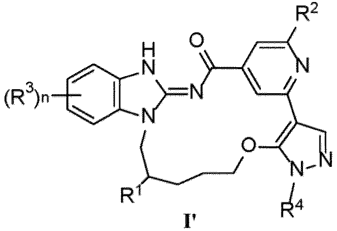 wherein,
R¹ is selected from the group consisting of C₃₋₅ cycloalkyl, C₃₋₅ cycloalkyl-C₁₋₃ alkyl-, C₁₋₆ haloalkyl, or C₁₋₆ alkyl;
R² is selected from the group consisting of hydrogen, C₁₋₈ alkyl, or C₁₋₈ alkoxy, wherein the C₁₋₈ alkyl or C₁₋₈ alkoxy is optionally substituted with one or more R';
R' is selected from the group consisting of halogen, OH, cyano, NH₂, or nitro;
R³ is selected from the group consisting of halogen, cyano, nitro, NH₂, OH, C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, 5- to 10-membered heteroaryl, -NHR^{b}, -NR^{a}R^{b}, -OR^{a}, - SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, or -N(C₁₋₄ alkyl)C(O)OR^{a}, wherein the C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, or 5- to 10-membered heteroaryl is optionally substituted with one or more R^{c};
R^{c} is selected from the group consisting of oxo, halogen, cyano, nitro, C₁₋₈ alkoxy, -N(C₁₋₈ alkyl)₂, -NH(C₁₋₈ alkyl), - C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, or 3- to 8-membered heterocycloalkyl optionally substituted with one or more groups independently selected from the group consisting of OH, halogen, or C₁₋₆ alkyl;
R^{a} and R^{b} are each independently selected from the group consisting of the following groups optionally substituted with one or more R": C₁₋₈ alkyl, C₂₋₈ alkenyl, C₂₋₈ alkynyl, C₃₋₁₀ cycloalkyl, 3- to 10-membered heterocycloalkyl, C₆₋₁₀ aryl, or 5- to 10-membered heteroaryl;
R" are each independently selected from the group consisting of halogen, NH₂, OH, cyano, nitro, C₁₋₈ alkyl, C₁₋₈ alkoxy, -N(C₁₋₈ alkyl)₂, -NH(C₁₋₈ alkyl), C₃₋₆ cycloalkyl, or 3- to 10-membered heterocycloalkyl;
n is selected from the group consisting of 0, 1, 2, 3, or 4;
R⁴ is selected from the group consisting of C₁₋₆ alkyl or C₃₋₅ cycloalkyl,
provided that when R¹ is selected from C₁₋₆ alkyl, R⁴ is selected from C₃₋₅ cycloalkyl.

In some embodiments, R¹ is selected from the group consisting of C₃₋₅ cycloalkyl, C₃₋₅ cycloalkyl-C₁₋₃ alkyl-, or C₁₋₆ haloalkyl.

In some embodiments, R¹ is selected from the group consisting of C₃₋₄ cycloalkyl, C₃₋₄ cycloalkyl-C₁₋₂ alkyl-, or C₁₋₃ haloalkyl.

In some embodiments, R¹ is selected from the group consisting of cyclopropyl, cyclopropyl-CH₂-, or halomethyl. In some embodiments, R¹ is selected from the group consisting of cyclopropyl, cyclopropyl-CH₂-, or methyl substituted with one or more fluoro.

In some embodiments, R¹ is selected from cyclopropyl.

In some embodiments, R¹ is selected from cyclopropyl-CH₂.

In some embodiments, R¹ is selected from the group consisting of -CH₂F, -CHF₂, or -CF₃.

In some embodiments, R¹ is selected from methyl.

In some embodiments, R¹ is selected from the group consisting of C₃₋₄ cycloalkyl, C₃₋₄ cycloalkyl-C₁₋₂ alkyl-, C₁₋₃ haloalkyl, or C₁₋₃ alkyl.

In some embodiments, R¹ is selected from the group consisting of cyclopropyl, cyclopropyl-CH₂-, halomethyl, or methyl.

In some embodiments, R¹ is selected from the group consisting of cyclopropyl, cyclopropyl-CH₂-, methyl substituted with one or more fluoro, or methyl.

In some embodiments, R² is selected from the group consisting of hydrogen, halogen, OH, NH₂, C₁₋₆ alkyl, or C₁₋₆ alkoxy, wherein the C₁₋₆ alkyl or C₁₋₆ alkoxy is optionally substituted with one or more R'.

In some embodiments, R² is selected from the group consisting of hydrogen, halogen, OH, NH₂, C₁₋₃ alkyl, or C₁₋₃ alkoxy, wherein the C₁₋₃ alkyl or C₁₋₃ alkoxy is optionally substituted with one or more R'.

In some embodiments, R² is selected from the group consisting of hydrogen, fluoro, chloro, bromo, C₁₋₃ alkyl, or C₁₋₃ alkoxy.

In some embodiments, R² is selected from the group consisting of fluoro, chloro, bromo, or C₁₋₃ alkyl.

In some embodiments, R² is selected from the group consisting of fluoro or methyl.

In some other embodiments, R² is selected from the group consisting of hydrogen, C₁₋₆ alkyl, or C₁₋₆ alkoxy, wherein the C₁₋₆ alkyl or C₁₋₆ alkoxy is optionally substituted with one or more R'. In some other embodiments, R² is selected from the group consisting of hydrogen, C₁₋₃ alkyl, or C₁₋₃ alkoxy, wherein the C₁₋₃ alkyl or C₁₋₃ alkoxy is optionally substituted with one or more R'.

In some other embodiments, R² is selected from the group consisting of hydrogen, C₁₋₃ alkyl, or C₁₋₃ alkoxy.

In some other embodiments, R² is selected from C₁₋₃ alkyl.

In some other embodiments, R² is selected from methyl.

In some embodiments, R' is selected from halogen.

In some embodiments, R³ is selected from the group consisting of halogen, cyano, nitro, NH₂, OH, C₁₋₆ alkyl, C₂₋₆ alkenyl, C₂₋₆ alkynyl, C₃₋₈ cycloalkyl, 3- to 8-membered heterocyclyl, C₆₋₁₀ aryl, 5- to 6-membered heteroaryl, -NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, -N(C₁₋₄ alkyl)C(O)OR^{a}, or -NHC(O)NHR^{a}, wherein the C₁₋₆ alkyl, C₂₋₆ alkenyl, C₂₋₆ alkynyl, C₃₋₈ cycloalkyl, 3- to 8-membered heterocyclyl, C₆₋₁₀ aryl, or 5- to 6-membered heteroaryl is optionally substituted with one or more R^{c}.

In some embodiments, R³ is selected from the group consisting of halogen, cyano, nitro, NH₂, OH, C₁₋₄ alkyl, C₂₋₄ alkenyl, C₂₋₄ alkynyl, C₃₋₆ cycloalkyl, 3- to 6-membered heterocycloalkyl, phenyl, 5- to 6-membered heteroaryl, - NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, -N(C₁₋₄ alkyl)C(O)OR^{a}, or -NHC(O)NHR^{a}, wherein the C₁₋₄ alkyl, C₂₋₄ alkenyl, C₂₋₄ alkynyl, C₃₋₆ cycloalkyl, 3- to 6-membered heterocycloalkyl, phenyl, or 5- to 6-membered heteroaryl is optionally substituted with one or more R^{c}. In some embodiments, R³ is selected from the group consisting of halogen, cyano, NH₂, OH, C₁₋₆ alkyl, C₂₋₆ alkenyl, C₂₋₆ alkynyl, C₃₋₈ cycloalkyl, 3- to 8-membered heterocyclyl, -NHR^{b}, -NR^{a}R^{b}, -OR^{a}, -SR^{a}, -OC(O)R^{a}, -NHC(O)R^{a}, - N(C₁₋₄ alkyl)C(O)R^{a}, -NHS(O)₂R^{a}, -NHC(O)OR^{a}, -N(C₁₋₄ alkyl)C(O)OR^{a}, or -NHC(O)NHR^{a}, wherein the C₁₋₆ alkyl, C₂₋₆ alkenyl, C₂₋₆ alkynyl, C₃₋₈ cycloalkyl, or 3- to 8-membered heterocyclyl is optionally substituted with one or more R^{c}.

In some embodiments, R³ is selected from the group consisting of halogen, C₁₋₄ alkyl, C₂₋₄ alkenyl, 4- to 6-membered heterocycloalkyl, -NHR^{b}, -NR^{a}R^{b}, -OR^{a}, -SR^{a}, -OC(O)R^{a}, -NHC(O)R^{a}, -NHC(O)OR^{a}, -N(C₁₋₄ alkyl)C(O)OR^{a}, or - NHC(O)NHR^{a}; wherein the C₁₋₄ alkyl, C₂₋₄ alkenyl, or 4- to 6-membered heterocycloalkyl is optionally substituted with one or more R^{c}.

In some embodiments, R³ is selected from the group consisting of fluoro, chloro, bromo, C₁₋₃ alkyl, C₂₋₄ alkenyl, 4-to 5-membered azacycloalkyl, -NHR^{b}, -NR^{a}R^{b}, -OR^{a}, -SR^{a}, -OC(O)R^{a}, -NHC(O)R^{a}, -NHC(O)OR^{a}, or -N(C₁₋₄ alkyl)C(O)OR^{a}, or -NHC(O)NHR^{a}; the C₁₋₃ alkyl, C₂₋₄ alkenyl, or 4- to 5-membered azacycloalkyl is optionally substituted with one or more R^{c}.

In some embodiments, R³ is selected from the group consisting of bromo, methyl, butenyl, azetidinyl, pyrrolidinyl, - NHR^{b}, -NR^{a}R^{b}, -OR^{a}, -NHC(O)R^{a}, or -NHC(O)NHR^{a}; the methyl, butenyl, azetidinyl, or pyrrolidinyl is optionally substituted with one or more R^{c}.

In some embodiments, R³ is selected from the group consisting of bromo, 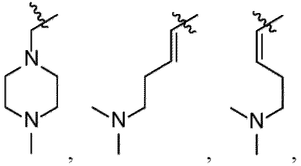 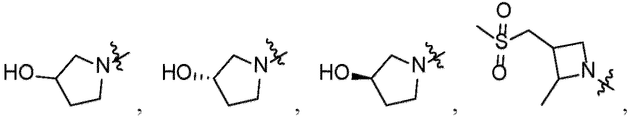 -NHR^{b}, -NR^{a}R^{b}, -OR^{a}, -NHC(O)R^{a}, or - NHC(O)NHR^{a}.

In some specific embodiments, R³ is selected from the group consisting of -NHR^{b}, -NR^{a}R^{b}, -OR^{a}, -NHC(O)R^{a}, or - NHC(O)NHR^{a}.

In some specific embodiments, R³ is selected from the group consisting of -NHR^{b} or -NR^{a}R^{b}. In some specific embodiments, R³ is selected from the group consisting of -OR^{a}. In some specific embodiments, R³ is selected from the group consisting of -NHC(O)R^{a} or -NHC(O)NHR^{a}.

In some embodiments, R³ is selected from the group consisting of bromo, 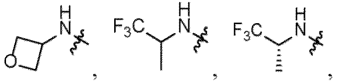 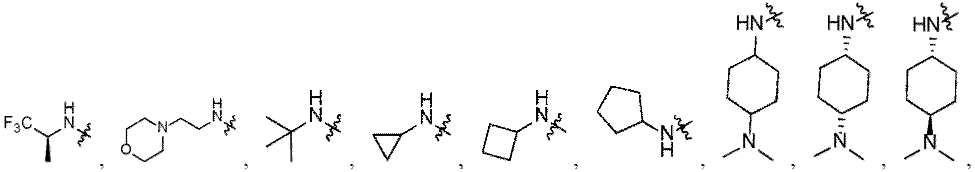 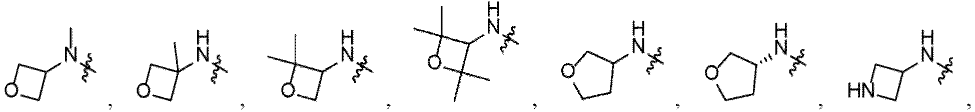 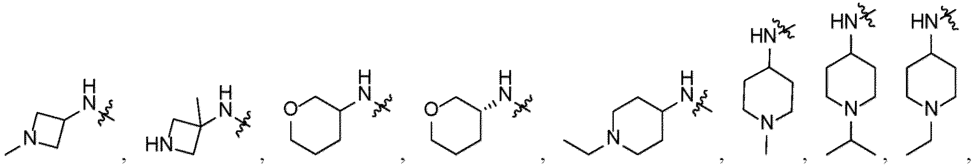 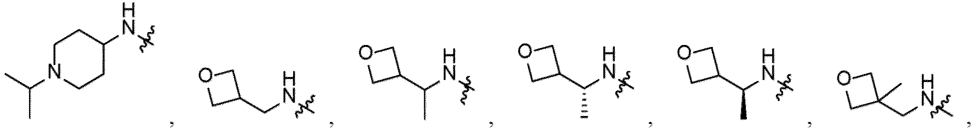 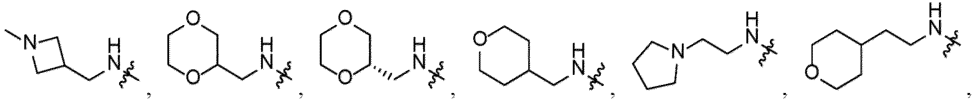 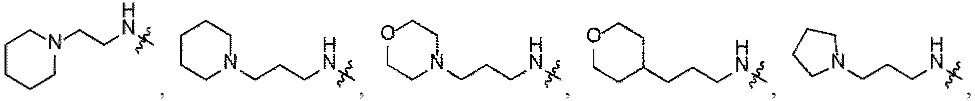 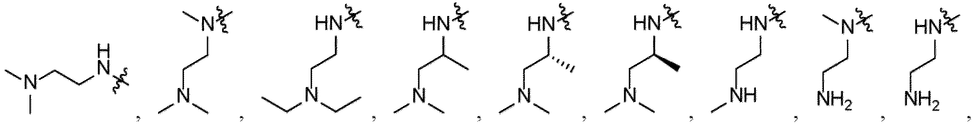 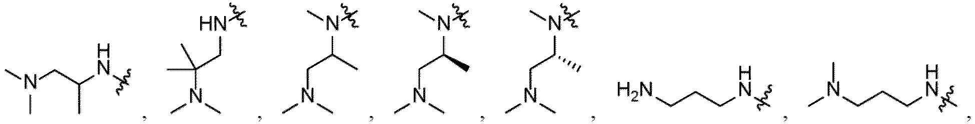 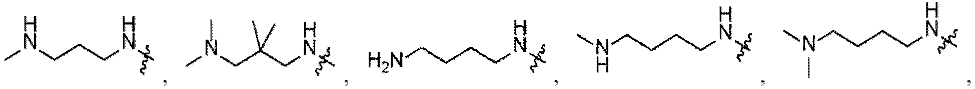 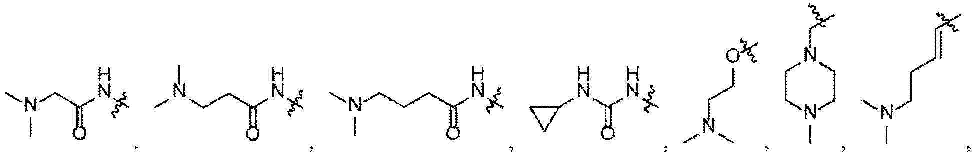 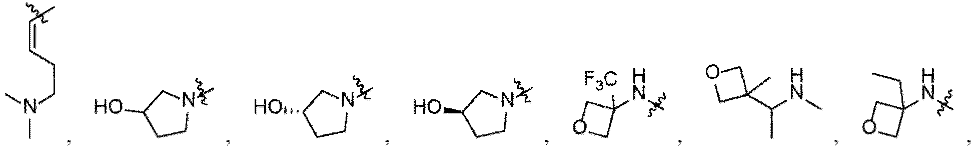 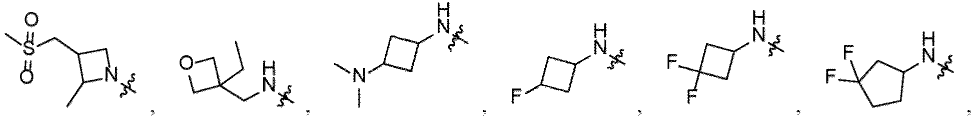 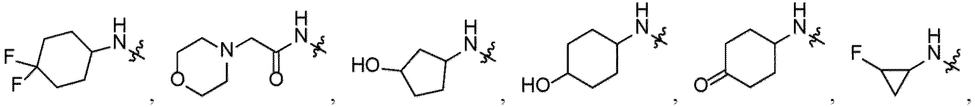 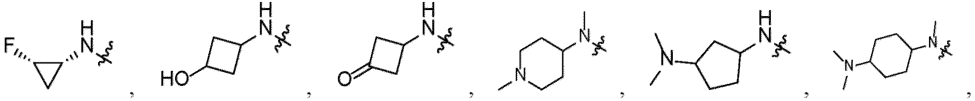 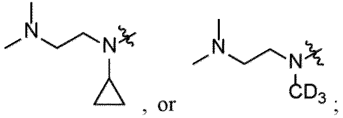 or R³ is selected from the group consisting of 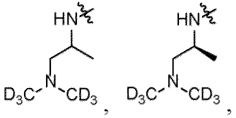 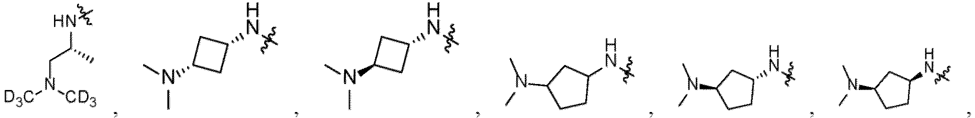 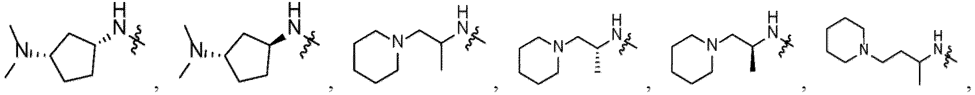 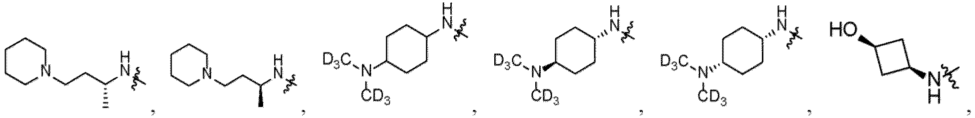 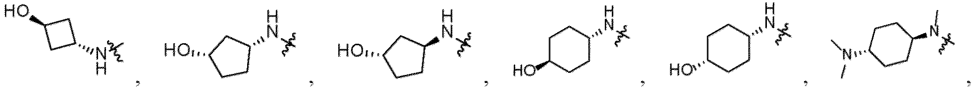 or

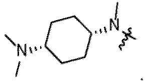

In some specific embodiments, R³ is selected from the group consisting of

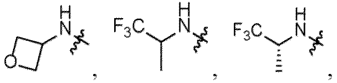

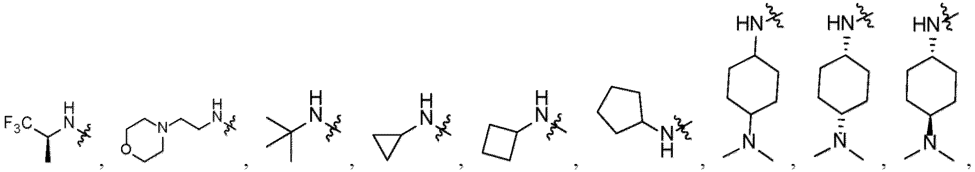

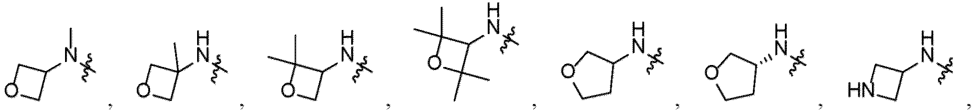

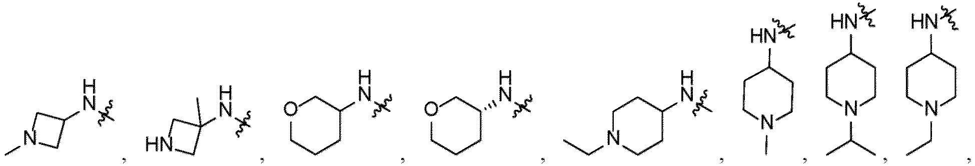

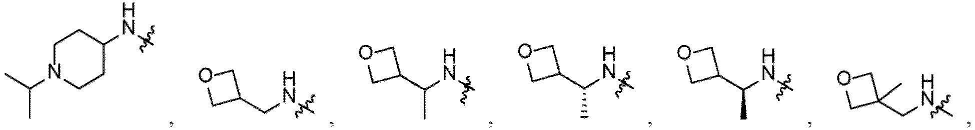

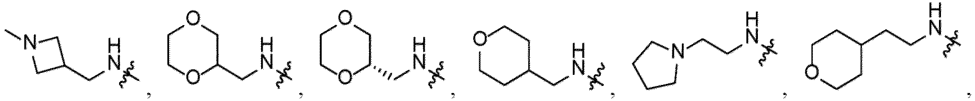

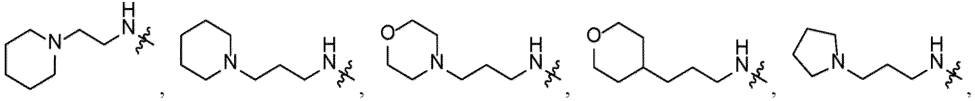

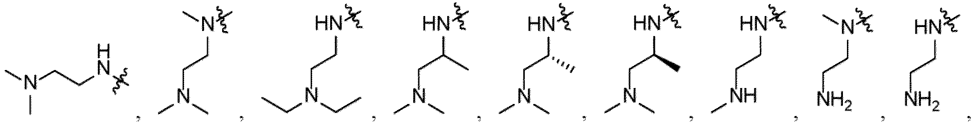

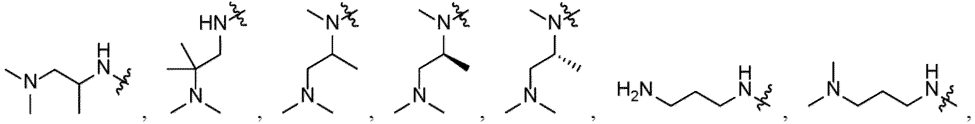

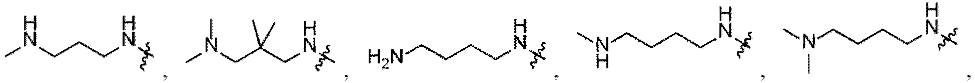

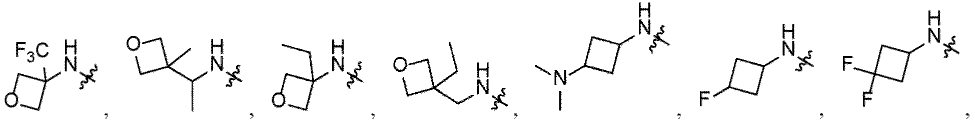

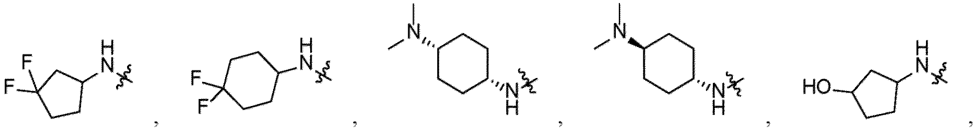

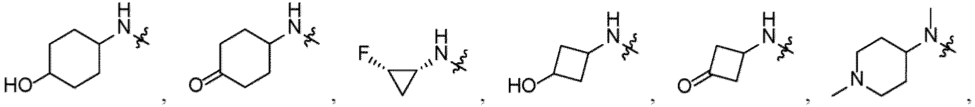

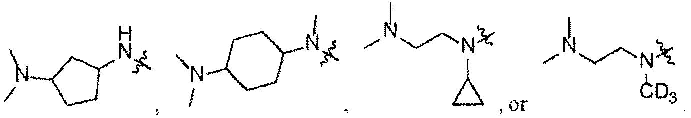

In some other embodiments, R³ is selected from the group consisting of fluoro, chloro, bromo, C₁₋₄ alkyl, C₂₋₄ alkenyl, 5- to 6-membered heterocyclyl, -NHR^{b}, -NR^{a}R^{b}, -OR^{a}, -SR^{a}, -OC(O)R^{a}, -NHC(O)R^{a}, -NHC(O)OR^{a}, -N(C₁₋₄ alkyl)C(O)OR^{a}, or -NHC(O)NHR^{a}; wherein the C₁₋₄ alkyl, C₂₋₄ alkenyl, or 5- to 6-membered heterocyclyl is optionally substituted with one or more R^{c}.

In some other embodiments, R³ is selected from the group consisting of bromo, methyl, butenyl, pyrrolidinyl, -NHR^{b}, -NR^{a}R^{b}, -OR^{a}, -NHC(O)R^{a}, or -NHC(O)NHR^{a}; wherein the methyl, butenyl, or pyrrolidinyl is optionally substituted with one or more R^{c}.

In some other embodiments, R³ is selected from the group consisting of bromo, 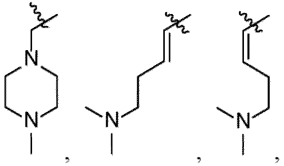 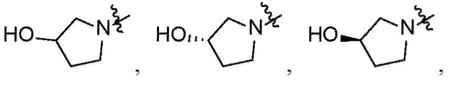 -NHR^{b}, -NR^{a}R^{b}, -OR^{a}, -NHC(O)R^{a}, or -NHC(O)NHR^{a}.

In some other embodiments, R³ is selected from the group consisting of bromo,

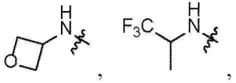

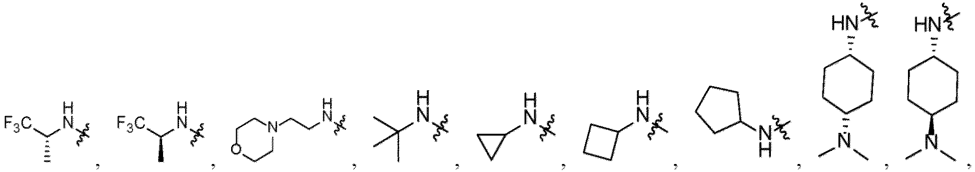

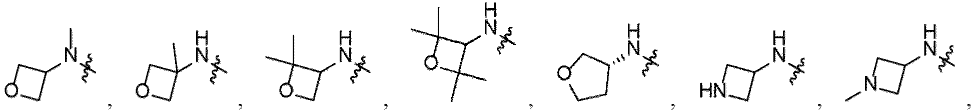

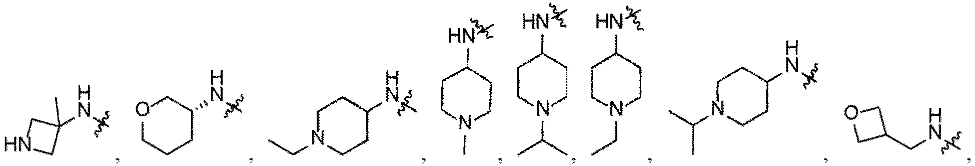

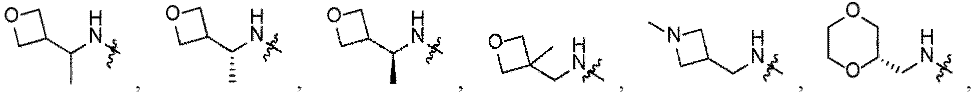

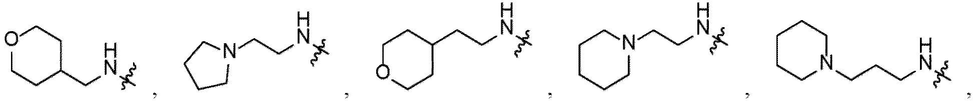

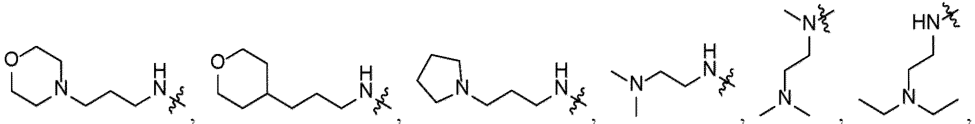

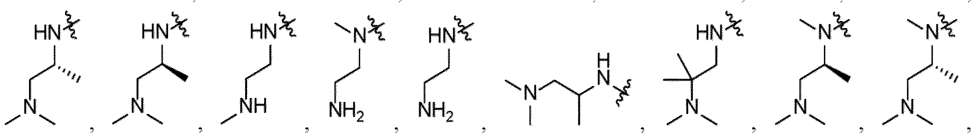

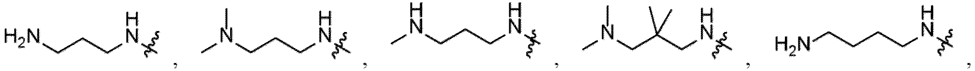

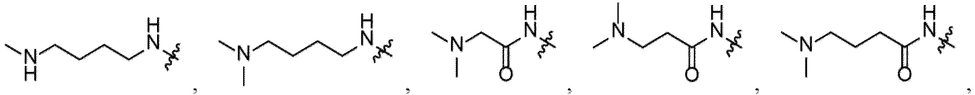

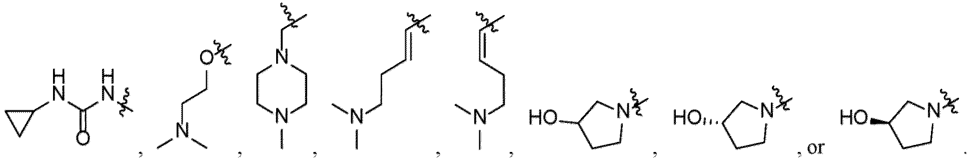

In some other embodiments, R³ is selected from the group consisting of halogen, cyano, nitro, NH₂, OH, C₁₋₆ alkyl, C₂₋₆ alkenyl, C₂₋₆ alkynyl, C₃₋₈ cycloalkyl, 3- to 8-membered heterocyclyl, C₆₋₁₀ aryl, 5- to 6-membered heteroaryl, - NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, or -N(C₁₋₄ alkyl)C(O)OR^{a}, wherein the C₁₋₆ alkyl, C₂₋₆ alkenyl, C₂₋₆ alkynyl, C₃₋₈ cycloalkyl, 3- to 8-membered heterocyclyl, C₆₋₁₀ aryl, or 5- to 6-membered heteroaryl is optionally substituted with one or more R^{c}.

In some other embodiments, R³ is selected from the group consisting of halogen, cyano, nitro, NH₂, OH, C₁₋₄ alkyl, C₂₋₄ alkenyl, C₂₋₄ alkynyl, C₃₋₆ cycloalkyl, 3- to 6-membered heterocycloalkyl, phenyl, 5- to 6-membered heteroaryl, -NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, or -N(C₁₋₄ alkyl)C(O)OR^{a}, wherein the C₁₋₄ alkyl, C₂₋₄ alkenyl, C₂₋₄ alkynyl, C₃₋₆ cycloalkyl, 3- to 6-membered heterocycloalkyl, phenyl, or 5- to 6-membered heteroaryl is optionally substituted with one or more R^{c}.

In some other embodiments, R³ is selected from the group consisting of halogen, cyano, nitro, NH₂, OH, C₁₋₃ alkyl, C₂₋₃ alkenyl, C₂₋₃ alkynyl, C₃₋₆ cycloalkyl, 3- to 6-membered heterocycloalkyl, phenyl, 5- to 6-membered heteroaryl, -NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₃ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, or -N(C₁₋₃ alkyl)C(O)OR^{a}, wherein the C₁₋₃ alkyl, C₂₋₃ alkenyl, C₂₋₃ alkynyl, C₃₋₆ cycloalkyl, 3- to 6-membered heterocycloalkyl, phenyl, or 5- to 6-membered heteroaryl is optionally substituted with one or more R^{c}.

In some other embodiments, R³ is selected from the group consisting of halogen, cyano, NH₂, OH, or -NHR^{b}.

In some other embodiments, R³ is selected from the group consisting of fluoro, chloro, bromo, or -NHR^{b}.

In some other embodiments, R³ is selected from the group consisting of bromo,

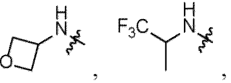

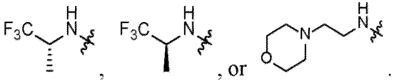

In some embodiments, R^{c} is selected from the group consisting of OH, NH₂, oxo, halogen, cyano, nitro, C₁₋₆ alkyl, C₁₋₆ alkoxy, -N(C₁₋₆ alkyl)₂, -NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, - S(O)₂NR^{a}R^{b}, -NHS(O)₂R^{a}, -C₁₋₆ alkyl-S(O)₂-C₁₋₆ alkyl, or 3- to 6-membered heterocycloalkyl optionally substituted with one or more groups independently selected from the group consisting of OH, halogen, or C₁₋₄ alkyl.

In some embodiments, R^{c} is selected from the group consisting of OH, NH₂, oxo, halogen, cyano, nitro, C₁₋₄ alkyl, C₁₋₄ alkoxy, -N(C₁₋₄ alkyl)₂, -NH(C₁₋₄ alkyl), -C(O)R^{a}, -C(O)OR^{a}, -C(O)NR^{a}R^{b}, -OC(O)R^{a}, -NHC(O)R^{a}, -S(O)₂R^{a}, - S(O)₂NR^{a}R^{b}, -NHS(O)₂R^{a}, -C₁₋₄ alkyl-S(O)₂-C₁₋₄ alkyl, or 4- to 6-membered heterocycloalkyl optionally substituted with one or more groups independently selected from the group consisting of OH, halogen, or C₁₋₃ alkyl.

In some embodiments, R^{c} is selected from the group consisting of OH, NH₂, halogen, cyano, C₁₋₄ alkyl, -C₁₋₄ alkoxy, -N(C₁₋₄ alkyl)₂, -NH(C₁₋₄ alkyl), -C₁₋₄ alkyl-S(O)₂-C₁₋₄ alkyl, or 4- to 6-membered heterocycloalkyl optionally substituted with one or more groups independently selected from the group consisting of OH, halogen, or C₁₋₃ alkyl. In some embodiments, R^{c} is selected from the group consisting of OH, C₁₋₃ alkyl, -N(C₁₋₃ alkyl)₂, -C₁₋₃ alkyl-S(O)₂-C₁₋₃ alkyl, or 6-membered heterocycloalkyl optionally substituted with one or more groups independently selected from the group consisting of OH, halogen, or C₁₋₃ alkyl.

In some embodiments, R^{c} is selected from the group consisting of OH, C₁₋₃ alkyl, -N(C₁₋₃ alkyl)₂, -C₁₋₃ alkyl-S(O)₂-C₁₋₃ alkyl, or piperazinyl optionally substituted with one or more C₁₋₃ alkyl.

In some embodiments, R^{c} is selected from the group consisting of OH, methyl,

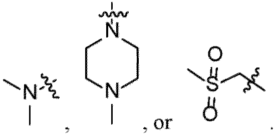

In some other embodiments, R^{c} is selected from the group consisting of oxo, OH, NH₂, halogen, cyano, nitro, C₁₋₈ alkoxy, -N(C₁₋₈ alkyl)₂, -NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, - S(O)₂NR^{a}R^{b}, -NHS(O)₂R^{a}, or 3- to 8-membered heterocycloalkyl optionally substituted with one or more groups independently selected from the group consisting of OH, halogen, or C₁₋₆ alkyl.

In some other embodiments, R^{c} is selected from the group consisting of OH, NH₂, oxo, halogen, cyano, nitro, C₁₋₆ alkoxy, -N(C₁₋₆ alkyl)₂, -NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, - S(O)₂NR^{a}R^{b}, -NHS(O)₂R^{a}, or 3- to 6-membered heterocycloalkyl optionally substituted with one or more groups independently selected from the group consisting of OH, halogen, or C₁₋₄ alkyl.

In some other embodiments, R^{c} is selected from the group consisting of OH, NH₂, oxo, halogen, cyano, nitro, C₁₋₄ alkoxy, -N(C₁₋₄ alkyl)₂, -NH(C₁₋₄ alkyl), -C(O)R^{a}, -C(O)OR^{a}, -C(O)NR^{a}R^{b}, -OC(O)R^{a}, -NHC(O)R^{a}, -S(O)₂R^{a}, - S(O)₂NR^{a}R^{b}, -NHS(O)₂R^{a}, or 4- to 6-membered heterocycloalkyl optionally substituted with one or more groups independently selected from the group consisting of OH, halogen, or C₁₋₃ alkyl.

In some other embodiments, R^{c} is selected from the group consisting of OH, NH₂, halogen, cyano, C₁₋₄ alkoxy, - N(C₁₋₄ alkyl)₂, -NH(C₁₋₄ alkyl), or 4- to 6-membered heterocycloalkyl optionally substituted with one or more groups independently selected from the group consisting of OH, halogen, or C₁₋₃ alkyl.

In some other embodiments, R^{c} is selected from the group consisting of OH, -N(C₁₋₃ alkyl)₂, or 6-membered heterocycloalkyl optionally substituted with one or more groups independently selected from the group consisting of OH, halogen, or C₁₋₃ alkyl.

In some other embodiments, R^{c} is selected from the group consisting of OH, -N(C₁₋₃ alkyl)₂, or piperazinyl optionally substituted with one or more C₁₋₃ alkyl.

In some other embodiments, R^{c} is selected from the group consisting of OH,

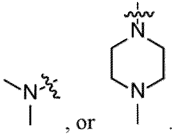

In some other embodiments, R^{c} is selected from the group consisting of oxo, halogen, cyano, nitro, C₁₋₆ alkoxy, - N(C₁₋₆ alkyl)₂, -NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, or 3- to 6-membered heterocycloalkyl optionally substituted with one or more groups independently selected from the group consisting of OH, halogen, or C₁₋₄ alkyl.

In some other embodiments, R^{c} is selected from the group consisting of oxo, halogen, cyano, nitro, C₁₋₄ alkoxy, - N(C₁₋₄ alkyl)₂, -NH(C₁₋₄ alkyl), -C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, or 4- to 6-membered heterocycloalkyl optionally substituted with one or more groups independently selected from the group consisting of OH, halogen, or C₁₋₃ alkyl.

In some other embodiments, R^{c} is selected from the group consisting of oxo, halogen, cyano, C₁₋₄ alkoxy, -N(C₁₋₄ alkyl)₂, -NH(C₁₋₄ alkyl), or 4- to 6-membered heterocycloalkyl optionally substituted with one or more groups independently selected from the group consisting of OH, halogen, or C₁₋₃ alkyl.

In some embodiments, R^{a} and R^{b} are each independently selected from the group consisting of the following groups optionally substituted with one or more R": C₁₋₆ alkyl, C₂₋₆ alkenyl, C₂₋₆ alkynyl, C₃₋₆ cycloalkyl, 3- to 8-membered heterocycloalkyl, C₆₋₁₀ aryl, or 5- to 6-membered heteroaryl.

In some embodiments, R^{a} and R^{b} are each independently selected from the group consisting of the following groups optionally substituted with one or more R": C₁₋₈ alkyl, C₂₋₈ alkenyl, C₂₋₈ alkynyl, C₃₋₈ cycloalkyl, or 3- to 8-membered heterocycloalkyl.

In some embodiments, R^{a} and R^{b} are each independently selected from the group consisting of the following groups optionally substituted with one or more R": C₁₋₈ alkyl, C₃₋₈ cycloalkyl, or 3- to 8-membered heterocycloalkyl. In some specific embodiments, R^{a} and R^{b} are each independently selected from the group consisting of the following groups optionally substituted with one or more R": C₁₋₆ alkyl, C₃₋₆ cycloalkyl, or 3- to 6-membered heterocycloalkyl.

In some embodiments, R^{a} and R^{b} are each independently selected from the group consisting of the following groups optionally substituted with one or more R": C₁₋₅ alkyl, C₃₋₆ cycloalkyl, or 4- to 6-membered heterocycloalkyl.

In some embodiments, R^{a} and R^{b} are each independently selected from the group consisting of the following groups optionally substituted with one or more R": methyl, ethyl, propyl, butyl, pentyl, cyclopropyl, cyclobutyl, cyclopentyl, cyclohexyl, oxetanyl, azetidinyl, tetrahydrofuranyl, tetrahydropyranyl, or piperidinyl.

In some embodiments, R^{a} and R^{b} are each independently selected from the group consisting of C₃₋₆ cycloalkyl or 4-to 6-membered heterocycloalkyl optionally substituted with one or more R". In some embodiments, R^{a} and R^{b} are each independently selected from the group consisting of C₁₋₅ alkyl optionally substituted with one or more R". In some embodiments, R^{a} and R^{b} are each independently selected from the group consisting of C₃₋₆ cycloalkyl optionally substituted with one or more R". In some embodiments, R^{a} and R^{b} are each independently selected from the group consisting of 4- to 6-membered heterocycloalkyl optionally substituted with one or more R". In some specific embodiments, R^{a} and R^{b} are each independently selected from the group consisting of the following groups optionally substituted with one or more R": methyl, ethyl, propyl, butyl, pentyl, cyclopropyl, cyclobutyl, cyclopentyl, or cyclohexyl. In some specific embodiments, R^{a} and R^{b} are each independently selected from the group consisting of the following groups optionally substituted with one or more R": propyl or cyclohexyl. In some specific embodiments, R^{a} and R^{b} are each independently selected from propyl optionally substituted with one or more R". In some specific embodiments, R^{a} and R^{b} are each independently selected from cyclohexyl optionally substituted with one or more R".

In some other embodiments, R^{a} and R^{b} are each independently selected from the group consisting of the following groups optionally substituted with one or more R": C₁₋₄ alkyl, C₂₋₄ alkenyl, C₂₋₄ alkynyl, C₃₋₆ cycloalkyl, 3- to 6-membered heterocycloalkyl, phenyl, or 5- to 6-membered heteroaryl.

In some other embodiments, R^{a} and R^{b} are each independently selected from the group consisting of the following groups optionally substituted with one or more R": C₁₋₄ alkyl or 3- to 6-membered heterocycloalkyl.

In some other embodiments, R^{a} and R^{b} are each independently selected from the group consisting of the following groups optionally substituted with one or more R": propyl, ethyl, or oxetanyl.

In some embodiments, -N(C₁₋₈ alkyl)₂ appearing in the definition of R" is selected from the group consisting of - N(C₁₋₈ alkyl)₂ and -N(C₁₋₈ deuteroalkyl)₂.

In some embodiments, R" are each independently selected from the group consisting of halogen, oxo, deuterium, NH₂, OH, cyano, nitro, C₁₋₈ alkyl, C₁₋₈ haloalkyl, C₁₋₈ alkoxy, -N(C₁₋₈ alkyl)₂, -N(C₁₋₈ deuteroalkyl)₂, -NH(C₁₋₈ alkyl), C₃₋₆ cycloalkyl, or 3- to 10-membered heterocycloalkyl optionally substituted with C₁₋₈ alkyl.

In some embodiments, R" are each independently selected from the group consisting of halogen, oxo, deuterium, NH₂, OH, cyano, nitro, C₁₋₈ alkyl, C₁₋₈ haloalkyl, C₁₋₈ alkoxy, -N(C₁₋₈ alkyl)₂, -N(C₁₋₆ deuteroalkyl)₂, -NH(C₁₋₈ alkyl), C₃₋₆ cycloalkyl, or 3- to 10-membered heterocycloalkyl optionally substituted with C₁₋₈ alkyl.

In some embodiments, R" are each independently selected from the group consisting of halogen, oxo, deuterium, NH₂, OH, cyano, nitro, C₁₋₆ alkyl, C₁₋₆ haloalkyl, C₁₋₆ alkoxy, -N(C₁₋₆ alkyl)₂, -N(C₁₋₆ deutetoalkyl)₂, -NH(C₁₋₆ alkyl), C₃₋₆ cycloalkyl, or 3- to 6-membered heterocycloalkyl optionally substituted with C₁₋₆ alkyl. In some specific embodiments, R" are each independently selected from the group consisting of C₁₋₆ alkyl, -N(C₁₋₆ alkyl)₂, -N(C₁₋₆ deuteroalkyl)₂, -NH(C₁₋₆ alkyl), C₃₋₆ cycloalkyl, or 3- to 6-membered heterocycloalkyl optionally substituted with C₁₋₆ alkyl. In some specific embodiments, R" are each independently selected from the group consisting of C₁₋₆ alkyl, -N(C₁₋₆ alkyl)₂, -N(C₁₋₆ deuteroalkyl)₂, or 3- to 6-membered heterocycloalkyl optionally substituted with C₁₋₃ alkyl. In some specific embodiments, R" are each independently selected from the group consisting of C₁₋₄ alkyl, -N(C₁₋₄ alkyl)₂, -N(C₁₋₄ deuteroalkyl)₂, or 4- to 6-membered heterocycloalkyl optionally substituted with C₁₋₃ alkyl.

In some embodiments, R" are each independently selected from the group consisting of halogen, oxo, deuterium, NH₂, OH, cyano, nitro, C₁₋₃ alkyl, C₁₋₃ haloalkyl, C₁₋₃ alkoxy, -N(C₁₋₃ alkyl)₂, -N(C₁₋₃ deuteroalkyl)₂, -NH(C₁₋₃ alkyl), C₄₋₆ cycloalkyl, or 3- to 6-membered heterocycloalkyl optionally substituted with C₁₋₃ alkyl.

In some embodiments, R" are each independently selected from the group consisting of fluoro, chloro, bromo, oxo, deuterium, NH₂, OH, cyano, C₁₋₄ alkyl, C₁₋₄ fluoroalkyl, C₁₋₄ alkoxy, -N(C₁₋₄ alkyl)₂, -N(C₁₋₄ deuteroalkyl)₂, -NH(C₁₋₄ alkyl), C₅₋₆ cycloalkyl, or 4- to 6-membered heterocycloalkyl optionally substituted with C₁₋₄ alkyl.

In some embodiments, R" are each independently selected from the group consisting of fluoro, OH, oxo, deuterium, -NH₂, methyl, ethyl, propyl, trifluoromethyl, -N(CH₃)₂, -N(CD₃)₂, -N(CH₂CH₃)₂, -NHCH₃, morpholinyl, pyrrolidinyl, piperidinyl, tetrahydropyranyl, 1,4-dioxanyl, or oxetanyl optionally substituted with methyl or ethyl. In some embodiments, R" are each independently selected from the group consisting of halogen, NH₂, OH, oxo, deuterium, cyano, nitro, C₁₋₈ alkyl, C₁₋₈ haloalkyl, C₁₋₈ alkoxy, -N(C₁₋₈ alkyl), -NH(C₁₋₈ alkyl), C₃₋₆ cycloalkyl, or 3-to 10-membered heterocycloalkyl optionally substituted with C₁₋₈ alkyl;

In some embodiments, R" are each independently selected from the group consisting of halogen, oxo, deuterium, NH₂, OH, cyano, nitro, C₁₋₆ alkyl, C₁₋₆ haloalkyl, C₁₋₆ alkoxy, -N(C₁₋₆ alkyl)₂, -NH(C₁₋₆ alkyl), C₃₋₆ cycloalkyl, or 3-to 6-membered heterocycloalkyl optionally substituted with C₁₋₆ alkyl. In some specific embodiments, R" are each independently selected from the group consisting of C₁₋₆ alkyl, -N(C₁₋₆ alkyl)₂, -NH(C₁₋₆ alkyl), C₃₋₆ cycloalkyl, or 3- to 6-membered heterocycloalkyl optionally substituted with C₁₋₆ alkyl. In some specific embodiments, R" are each independently selected from the group consisting of C₁₋₆ alkyl, -N(C₁₋₆ alkyl)₂, or 3- to 6-membered heterocycloalkyl optionally substituted with C₁₋₃ alkyl. In some specific embodiments, R" are each independently selected from the group consisting of C₁₋₄ alkyl, -N(C₁₋₄ alkyl)₂, or 4- to 6-membered heterocycloalkyl optionally substituted with C₁₋₃ alkyl.

In some embodiments, R" are each independently selected from the group consisting of halogen, oxo, deuterium, NH₂, OH, cyano, nitro, C₁₋₃ alkyl, C₁₋₃ haloalkyl, C₁₋₃ alkoxy, -N(C₁₋₃ alkyl)₂, -NH(C₁₋₃ alkyl), C₄₋₆ cycloalkyl, or 3-to 6-membered heterocycloalkyl optionally substituted with C₁₋₃ alkyl.

In some embodiments, R" are each independently selected from the group consisting of fluoro, chloro, bromo, oxo, deuterium, NH₂, OH, cyano, C₁₋₄ alkyl, fluoro C₁₋₄ alkyl, C₁₋₄ alkoxy, -N(C₁₋₄ alkyl)₂, -NH(C₁₋₄ alkyl), C₅₋₆ cycloalkyl, or 4- to 6-membered heterocycloalkyl optionally substituted with C₁₋₄ alkyl.

In some embodiments, R" are each independently selected from the group consisting of fluoro, OH, oxo, deuterium, -NH₂, methyl, ethyl, propyl, trifluoromethyl, -N(CH₃)₂, -N(CH₂CH₃)₂, -NHCH₃, morpholinyl, pyrrolidinyl, piperidinyl, tetrahydropyranyl, 1,4-dioxanyl, or oxetanyl optionally substituted with methyl or ethyl.

In some specific embodiments, R" are each independently selected from the group consisting of halogen, OH, oxo, C₁₋₆ alkyl, C₁₋₆ haloalkyl, or -N(C₁₋₄ alkyl)₂. In some specific embodiments, R" are each independently selected from the group consisting of halogen, OH, oxo, C₁₋₄ alkyl, C₁₋₄ haloalkyl, or -N(C₁₋₂ alkyl)₂. In some specific embodiments, R" are each independently selected from the group consisting of fluoro, OH, oxo, methyl, ethyl, propyl, trifluoromethyl, or -N(CH₃)₂.

In some other embodiments, R" are each independently selected from the group consisting of halogen, NH₂, OH, cyano, nitro, C₁₋₈ alkyl, C₁₋₈ alkoxy, -N(C₁₋₈ alkyl)₂, -NH(C₁₋₈ alkyl), C₃₋₆ cycloalkyl, or 3- to 10-membered heterocycloalkyl.

In some other embodiments, R" are each independently selected from the group consisting of halogen, NH₂, OH, cyano, nitro, C₁₋₆ alkyl, C₁₋₆ alkoxy, -N(C₁₋₆ alkyl)₂, -NH(C₁₋₆ alkyl), C₃₋₆ cycloalkyl, or 3- to 6-membered heterocycloalkyl.

In some other embodiments, R" are each independently selected from the group consisting of halogen, NH₂, OH, cyano, nitro, C₁₋₃ alkyl, C₁₋₃ alkoxy, -N(C₁₋₃ alkyl)₂, -NH(C₁₋₃ alkyl), C₄₋₆ cycloalkyl, or 3- to 6-membered heterocycloalkyl.

In some other embodiments, R" are each independently selected from the group consisting of fluoro, chloro, bromo, NH₂, OH, cyano, C₁₋₄ alkyl, C₁₋₄ alkoxy, -N(C₁₋₄ alkyl)₂, -NH(C₁₋₄ alkyl), C₅₋₆ cycloalkyl, or 4- to 6-membered heterocycloalkyl. In some other embodiments, R" are each independently selected from the group consisting of fluoro, -NH₂, methyl, ethyl, propyl, -N(CH₃)₂, -N(CH₂CH₃)₂, -NHCH₃, morpholinyl, pyrrolidinyl, piperidinyl, tetrahydropyranyl, 1,4-dioxanyl, or oxetanyl. In some other embodiments, R" are each independently selected from the group consisting of halogen or 3- to 6-membered heterocycloalkyl.

In some other embodiments, R" are each independently selected from the group consisting of fluoro, chloro, bromo, or 6-membered heterocycloalkyl.

In some other embodiments, R" are each independently selected from the group consisting of fluoro or morpholinyl.

In some embodiments, R^{a} and R^{b} are each independently selected from the group consisting of methyl, CD₃, 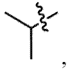 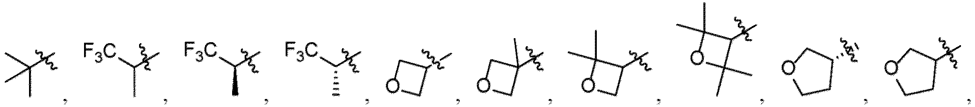 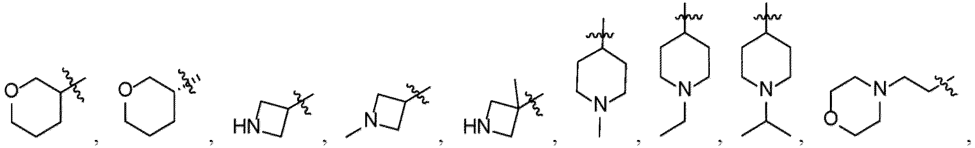 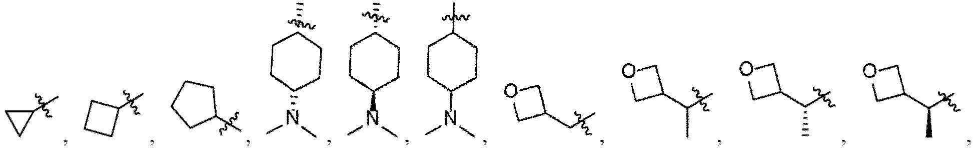 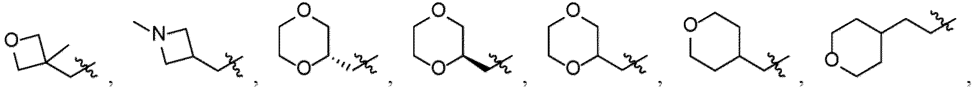 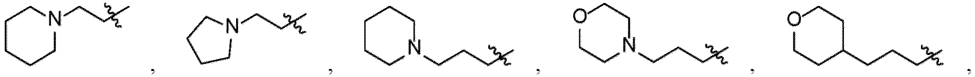 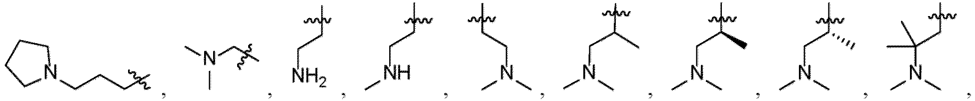 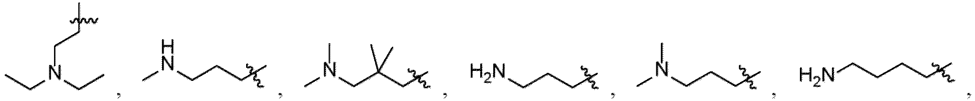 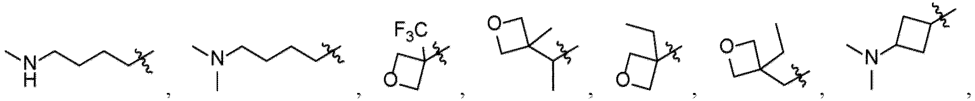 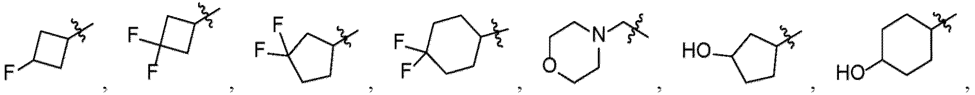 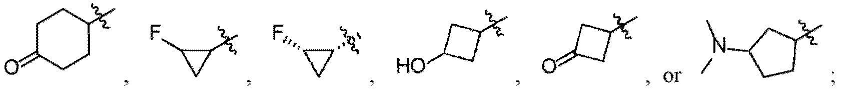 or R^{a} and R^{b} are independently selected from the group consisting of

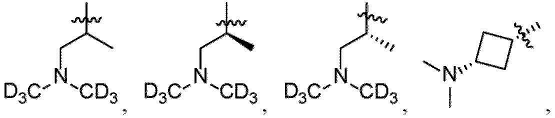

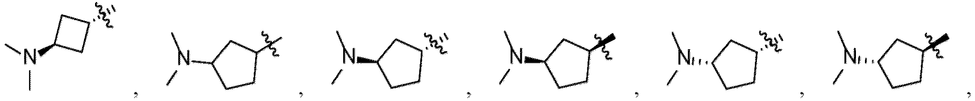

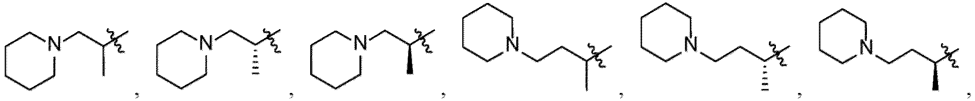

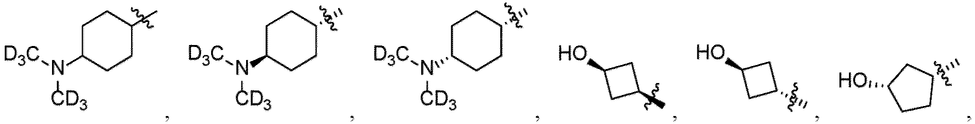

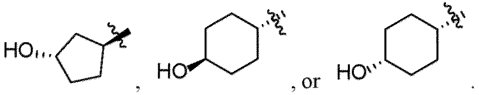

In some other embodiments, R^{a} and R^{b} are each independently selected from the group consisting of methyl,

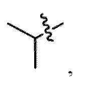

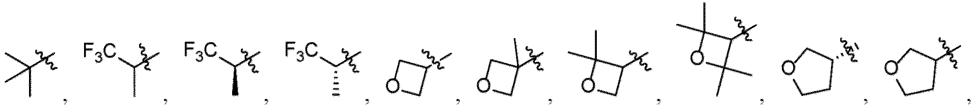

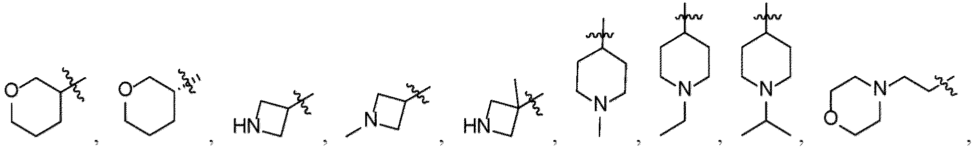

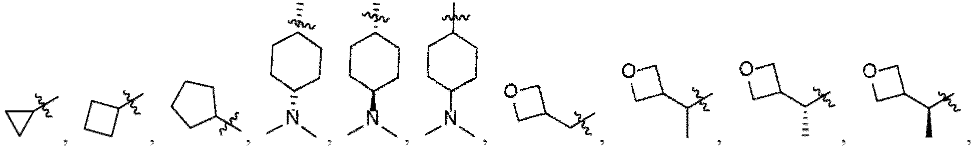

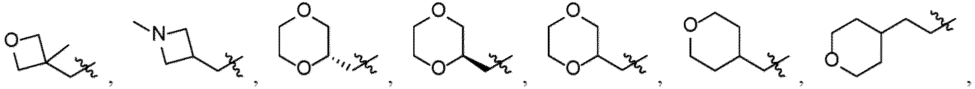

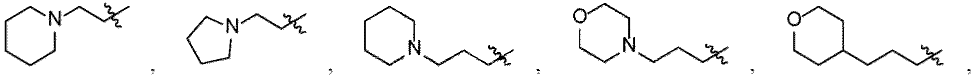

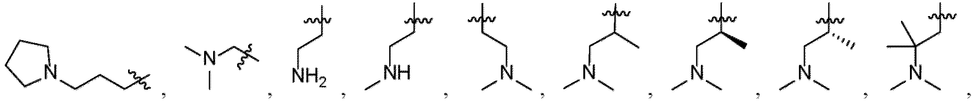

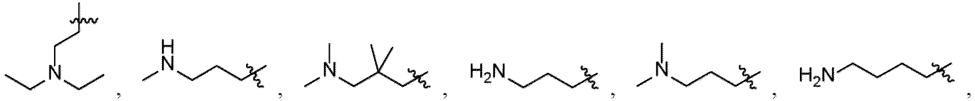

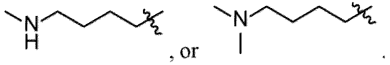

In some other embodiments, R^{a} and R^{b} are each independently selected from the group consisting of

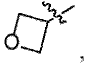

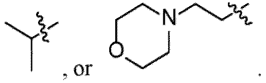

In some embodiments, n is selected from the group consisting of 0, 1, 2, or 3. In some embodiments, n is selected from the group consisting of 0, 1, and 2. In some specific embodiments, n is selected from the group consisting of 1 or 2. In some specific embodiments, n is selected from 1.

In some embodiments, R⁴ is selected from the group consisting of C₁₋₃ alkyl or C₃₋₄ cycloalkyl.

In some embodiments, R⁴ is selected from the group consisting of methyl, ethyl, cyclopropyl, or cyclobutyl.

In some embodiments, R⁴ is selected from the group consisting of methyl or cyclopropyl. In some specific embodiments, R⁴ is selected from cyclopropyl.

In some specific embodiments, R⁴ is selected from methyl.

In some embodiments, R¹ is selected from the group consisting of C₁₋₆ haloalkyl or C₃₋₄ cycloalkyl, or R¹ is selected from the group consisting of halomethyl or cyclopropyl;
R² is selected from C₁₋₃ alkyl, or R² is selected from methyl;
R³ is selected from the group consisting of -NHR^{b} or -NR^{a}R^{b},
R^{a} and R^{b} are each independently selected from the group consisting of the following groups optionally substituted with one or more R": C₁₋₆ alkyl, C₃₋₆ cycloalkyl, or 3- to 6-membered heterocycloalkyl, or R^{a} and R^{b} are each independently selected from the following optionally substituted with one or more R": C₁₋₅ alkyl, C₃₋₆ cycloalkyl, or 4- to 6-membered heterocycloalkyl; or R^{a} and R^{b} are each independently selected from the following groups optionally substituted with one or more R": methyl, ethyl, propyl, butyl, pentyl, cyclopropyl, cyclobutyl, cyclopentyl, cyclohexyl, oxetanyl, azetidinyl, tetrahydrofuranyl, tetrahydropyranyl, or piperidinyl; or R^{a} and R^{b} are each independently selected from the group consisting of the following groups optionally substituted with one or more R": methyl, ethyl, propyl, butyl, pentyl, cyclopropyl, cyclobutyl, cyclopentyl, or cyclohexyl; or R^{a} and R^{b} are each independently selected from the group consisting of the following groups optionally substituted with one or more R": propyl or cyclohexyl;
R" are each independently selected from the group consisting of fluoro, chloro, bromo, oxo, deuterium, NH₂, OH, cyano, C₁₋₄ alkyl, C₁₋₄ fluoroalkyl, C₁₋₄ alkoxy, -N(C₁₋₄ alkyl)₂, -N(C₁₋₄ deuteroalkyl)₂, -NH(C₁₋₄ alkyl), C₅₋₆ cycloalkyl, or 4- to 6-membered heterocycloalkyl optionally substituted with C₁₋₄ alkyl; or R" are each independently selected from the group consisting of fluoro, OH, oxo, deuterium, -NH₂, methyl, ethyl, propyl, trifluoromethyl, - N(CH₃)₂, -N(CD₃)₂, -N(CH₂CH₃)₂, -NHCH₃, morpholinyl, pyrrolidinyl, piperidinyl, tetrahydropyranyl, 1,4-dioxanyl, or oxetanyl optionally substituted with methyl or ethyl;
R⁴ is selected from C₁₋₃ alkyl, or R⁴ is selected from methyl.

In some embodiments, the compound of formula I", the stereoisomer thereof or the pharmaceutically acceptable salt thereof of the present application is selected from the group consisting of a compound of formula I"A or a compound formula I"B, a stereoisomer thereof or a pharmaceutically acceptable salt thereof, 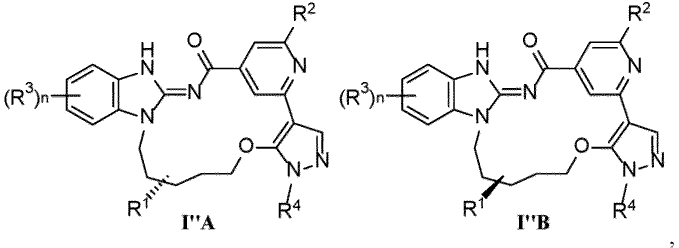 wherein n, R¹, R², R³, or R⁴ is as defined above.

In some embodiments, the compound of formula I', the stereoisomer thereof or the pharmaceutically acceptable salt thereof of the present application is selected from the group consisting of a compound of formula I'A or a compound of formula I'B, a stereoisomer thereof or a pharmaceutically acceptable salt thereof, 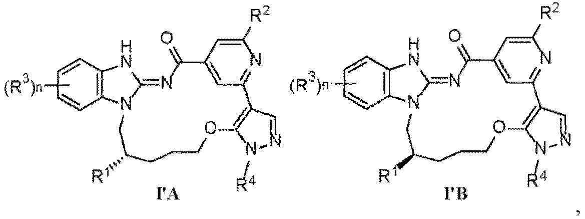 wherein n, R¹, R², R³, or R⁴ is as defined above.

In some embodiments, the compound of formula I', the stereoisomer thereof or the pharmaceutically acceptable salt thereof of the present application is selected from the group consisting of a compound of formula I, a stereoisomer thereof or a pharmaceutically acceptable salt thereof, 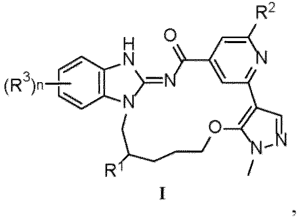 wherein n, R¹, R², or R³ is as defined above.

In some embodiments, in the compound of formula I:
R¹ is selected from the group consisting of C₃₋₅ cycloalkyl, C₃₋₅ cycloalkyl-C₁₋₃ alkyl-, or C₁₋₆ haloalkyl;
R² is selected from the group consisting of hydrogen, C₁₋₈ alkyl, or C₁₋₈ alkoxy, wherein the C₁₋₈ alkyl or C₁₋₈ alkoxy is optionally substituted with one or more R';
R' is selected from the group consisting of halogen, hydroxy, cyano, amino, or nitro;
R³ is selected from the group consisting of halogen, cyano, nitro, NH₂, OH, C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, 5- to 10-membered heteroaryl, -NHR^{b}, -NR^{a}R^{b}, -OR^{a}, - SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, or -N(C₁₋₄ alkyl)C(O)OR^{a}, wherein the C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, or 5- to 10-membered heteroaryl is optionally substituted with one or more R^{c};
R^{c} is selected from the group consisting of oxo, halogen, cyano, nitro, C₁₋₈ alkoxy, -N(C₁₋₈ alkyl)₂, -NH(C₁₋₈ alkyl),-C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, or 3- to 8-membered heterocycloalkyl optionally substituted with one or more hydroxy, halogen, or C₁₋₆ alkyl;
R^{a} and R^{b} are each independently selected from the group consisting of the following groups optionally substituted with one or more R": C₁₋₈ alkyl, C₂₋₈ alkenyl, C₂₋₈ alkynyl, C₃₋₁₀ cycloalkyl, 3- to 10-membered heterocycloalkyl, C₆₋₁₀ aryl, or 5- to 10-membered heteroaryl;
R" are each independently selected from the group consisting of halogen, NH₂, OH, cyano, nitro, C₁₋₈ alkyl, C₁₋₈ alkoxy, -N(C₁₋₈ alkyl)₂, -NH(C₁₋₈ alkyl), C₃₋₆ cycloalkyl, or 3- to 10-membered heterocycloalkyl;
n is selected from the group consisting of 0, 1, 2, 3, or 4.

In some embodiments, heteroatoms in the heterocycloalkyl, heterocyclyl, or heteroaryl described herein are selected from the group consisting of N, O, or S, and the remaining ring atoms are selected from carbon; in some embodiments, heteroatoms in the heterocycloalkyl, heterocyclyl, or heteroaryl described herein are selected from N or O, and the remaining ring atoms are selected from carbon; in some embodiments, the number of heteroatoms in the heterocycloalkyl, heterocyclyl, or heteroaryl described herein is selected from 1, 2, 3, 4, or 5; in some embodiments, the number of heteroatoms in the heterocycloalkyl, heterocyclyl, or heteroaryl described herein is selected from 1, 2, or 3.

In some embodiments, the compound of formula I', the compound of formula I'A, the compound of formula I'B, the compound of formula I, the stereoisomer thereof or the pharmaceutically acceptable salt thereof of the present application is selected from the group consisting of a compound of formula IA or a compound of formula IB, a stereoisomer thereof or a pharmaceutically acceptable salt thereof, 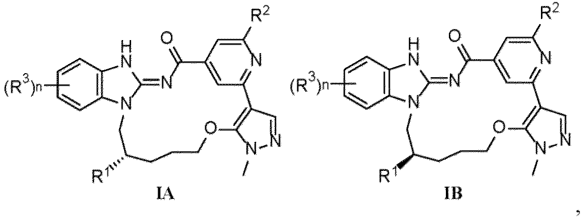 wherein n, R¹, R², or R³ is as defined above.

In some embodiments, the compound of formula I', the compound of formula I'A, the compound of formula I'B, the compound of formula I, the compound of formula IA, the compound of formula IB, the stereoisomer thereof or the pharmaceutically acceptable salt thereof of the present application is selected from the group consisting of a compound of formula I-1, a compound of formula 1-1A, a compound of formula I-1B, a compound of formula I-2, a compound of formula I-2A, a compound of formula I-2B, a compound of formula II, a compound of formula IIA, a compound of formula IIB, a compound of formula II-1, a compound of formula II-1A, a compound of formula II-1B, a compound of formula II-2, a compound of formula II-2A, or a compound of formula II-2B, a stereoisomer thereof or a pharmaceutically acceptable salt thereof, 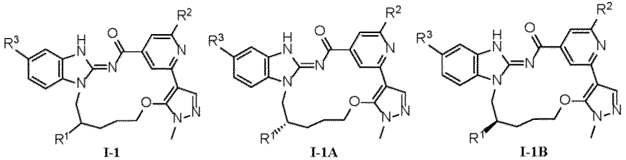 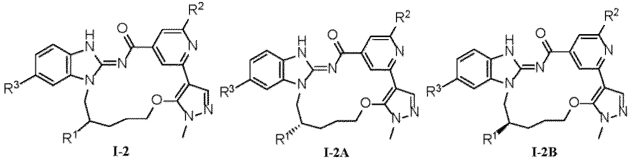 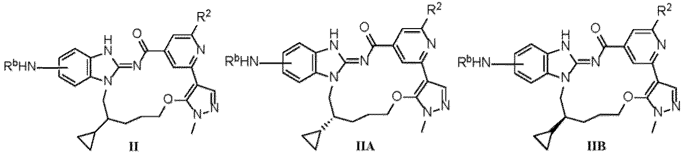 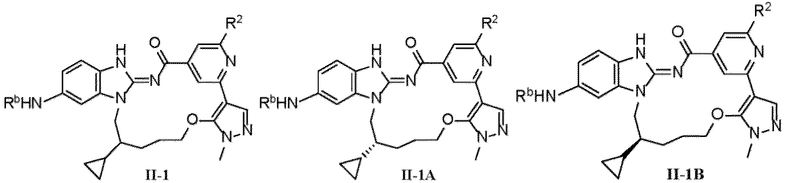 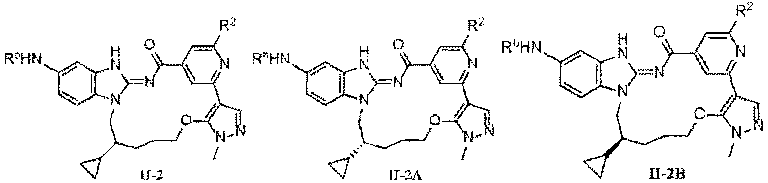 wherein R^{b}, R¹, R², and R³ are as defined above.

In some embodiments, the compound of formula I', the compound of formula I'A, the compound of formula I'B, the stereoisomer thereof or the pharmaceutically acceptable salt thereof of the present application is selected from the group consisting of a compound of formula III-1, a compound of formula III-1A, a compound of formula III-1B, a compound of formula III-2, a compound of formula III-2A, a compound of formula III-2B, a compound of formula IV, a compound of formula IVA, a compound of formula IVB, a compound of formula IV-1, a compound of formula IV-1A, a compound of formula IV-1B, a compound of formula IV-2, a compound of formula IV-2A, a compound of formula IV-2B, a stereoisomer thereof or a pharmaceutically acceptable salt thereof, 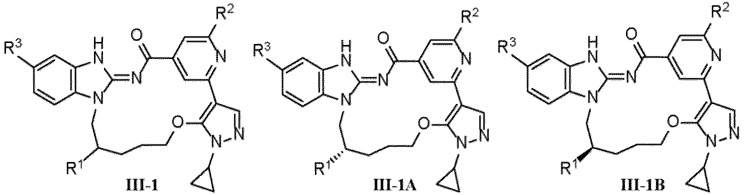 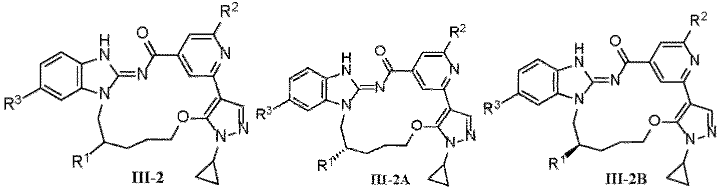 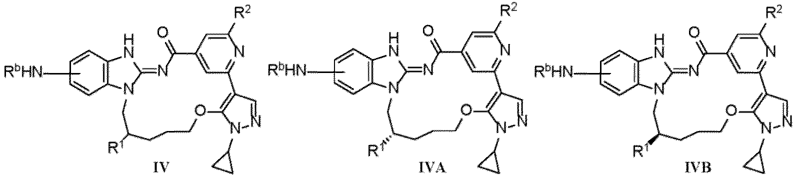 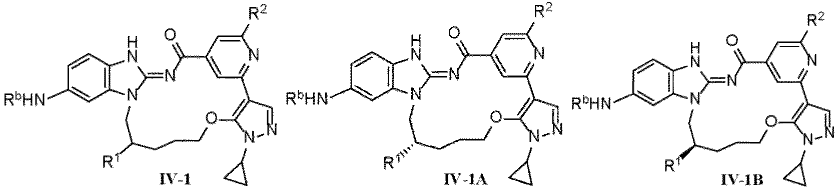 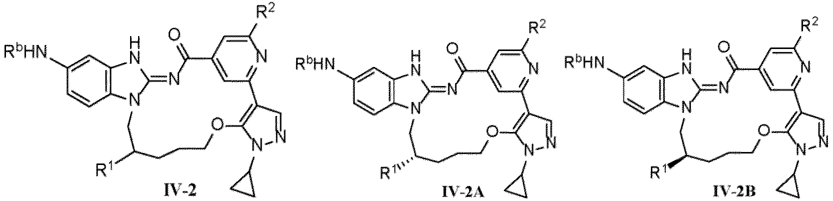 wherein R^{b}, R¹, R², and R³ are as defined above.

In some embodiments, the present application encompasses the variables defined above and embodiments thereof, as well as any combination thereof. It should be understood that any embodiment of the compounds of the present disclosure as described above, and any specific substituents set forth herein with respect to particular n, R¹, R², R³, and R⁴ substituents in the compounds of the present disclosure as described above, may be independently combined with other embodiments of the present invention and/or substituents of the compounds to form embodiments of the present invention not specifically set forth above. Further, where a list of substituents is disclosed in the detailed description and/or claims with respect to any particular n, R¹, R², R³, and R⁴ substituents, it should be understood that one or more substituents may be deleted from the list and the remaining list of substituents will be considered an embodiment of the present invention.

In some embodiments, the present application provides a compound of the following formula, a stereoisomer thereof or a pharmaceutically acceptable salt thereof: 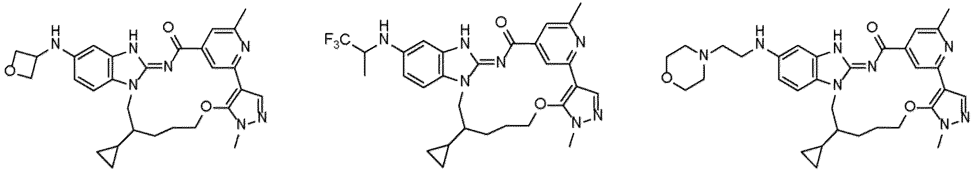 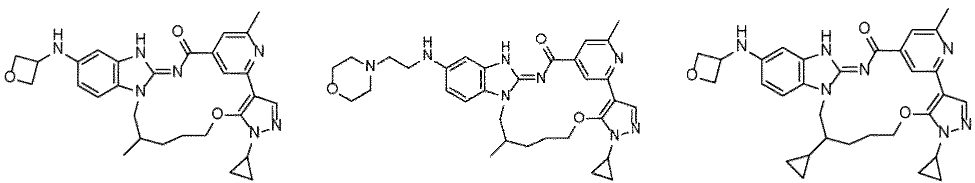 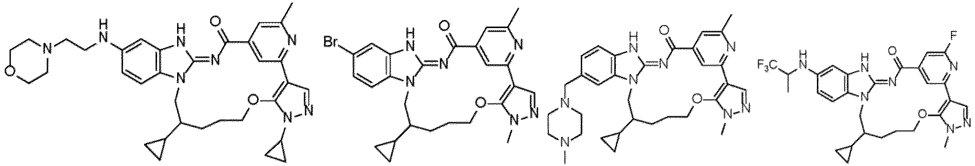 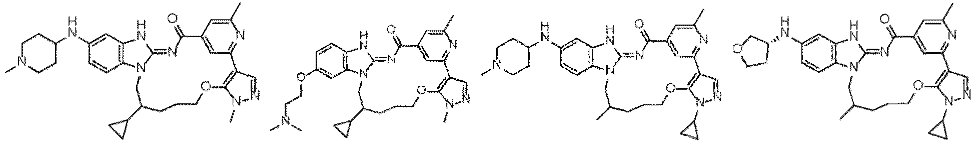 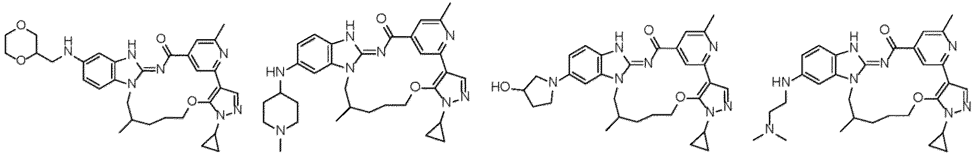 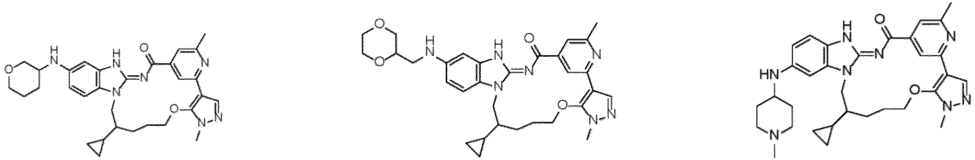 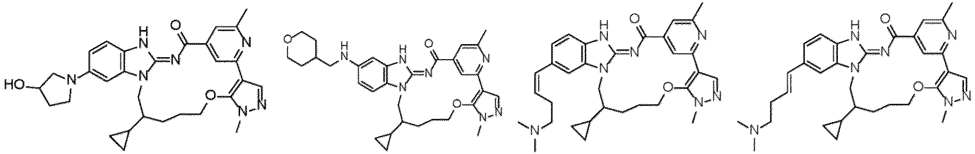 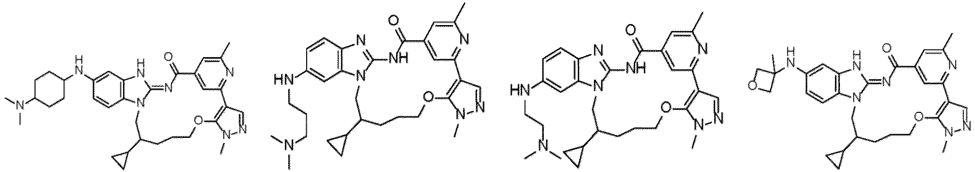 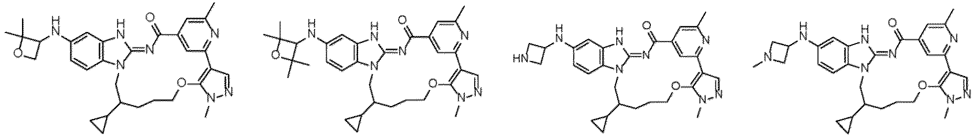 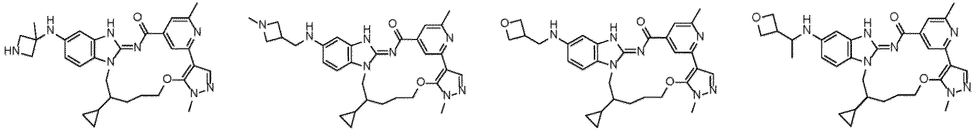 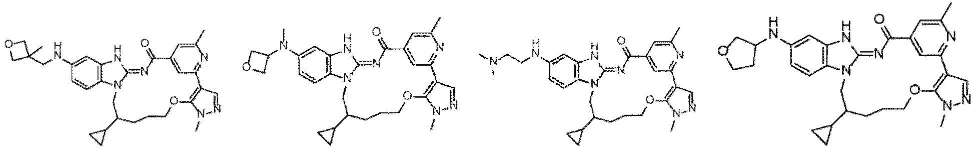 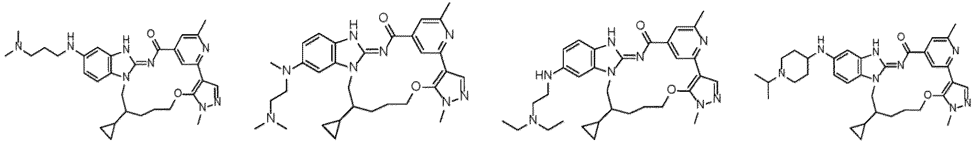 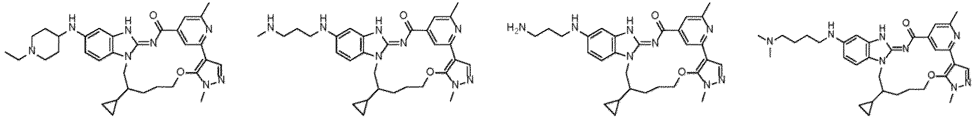 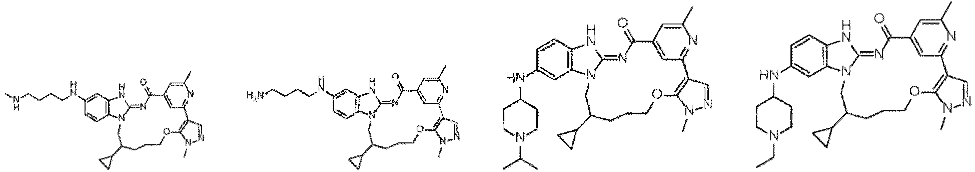 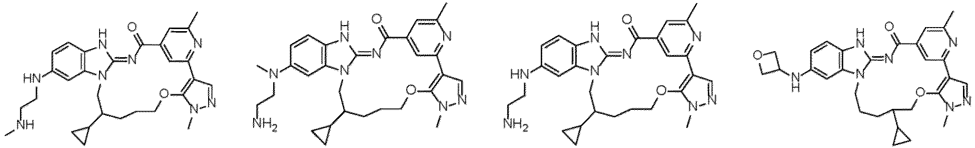 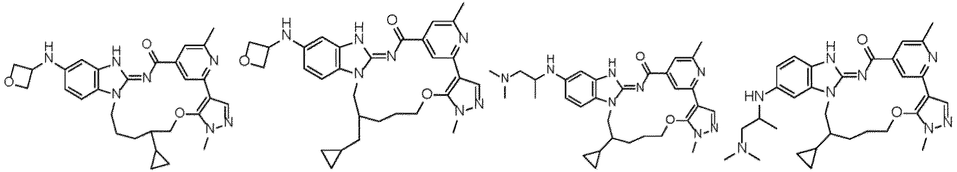 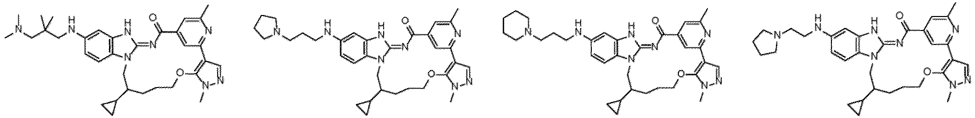 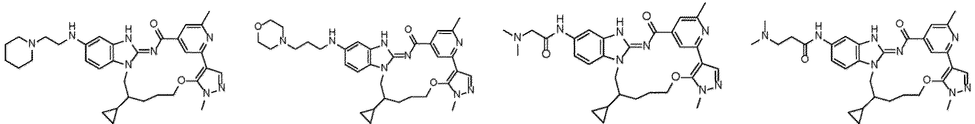 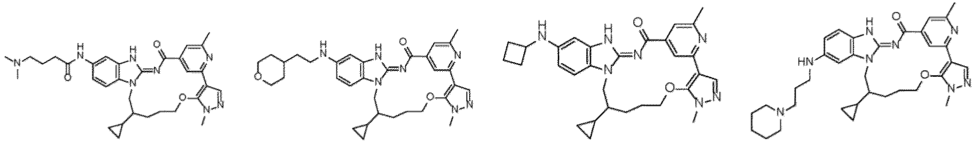 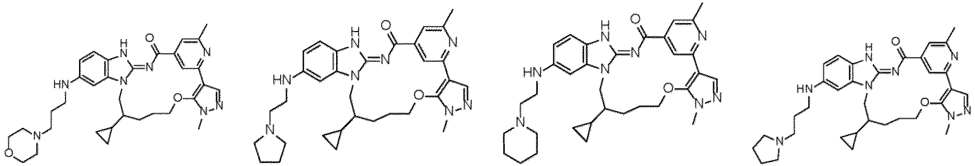 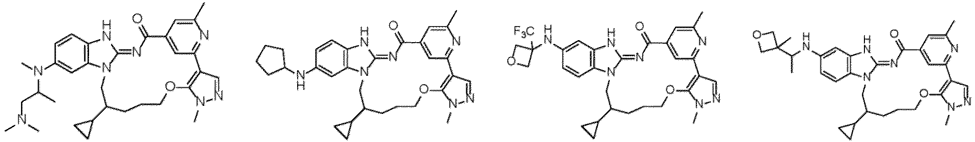 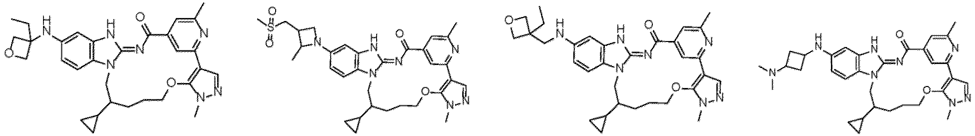 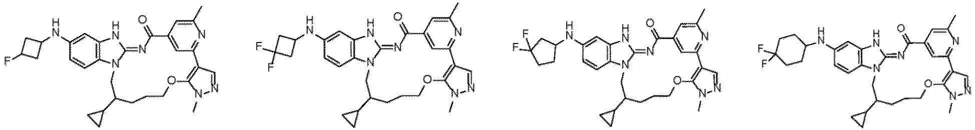 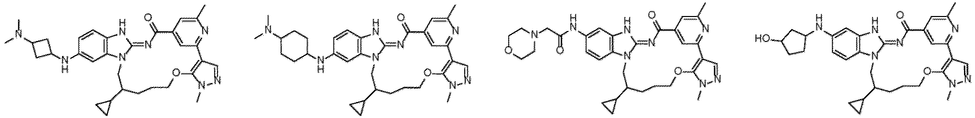 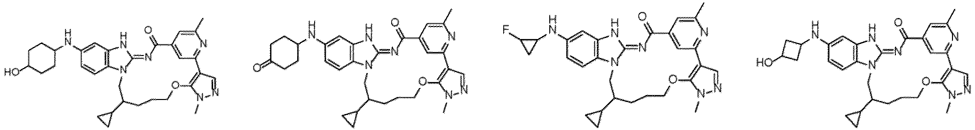 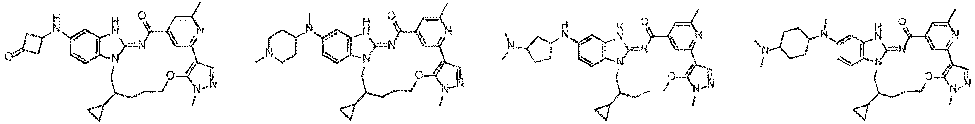 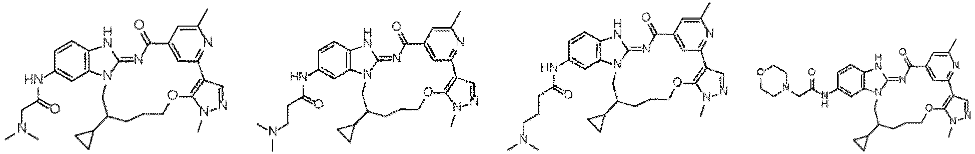 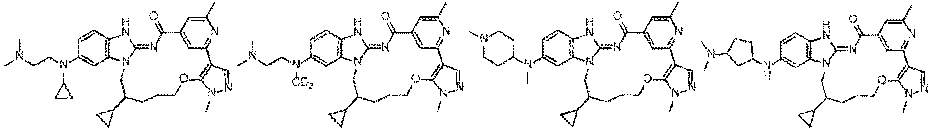 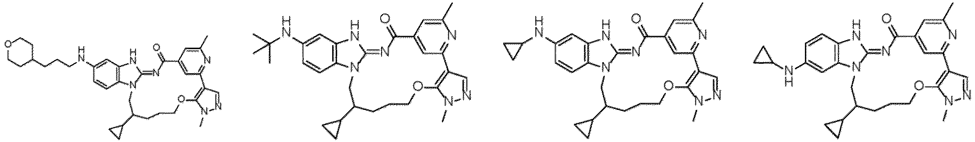 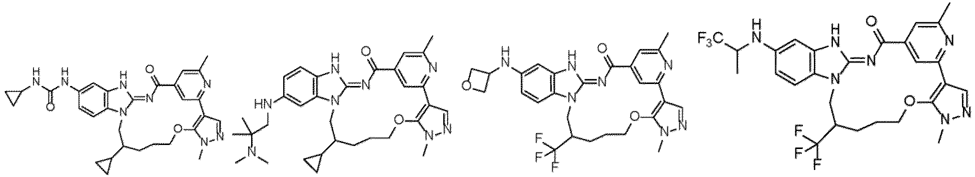 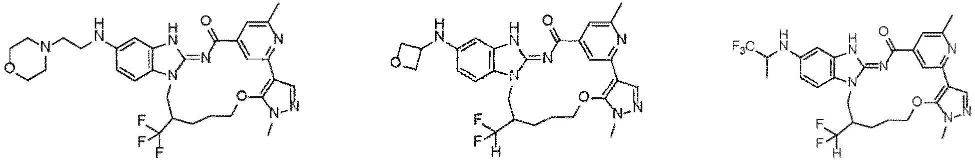 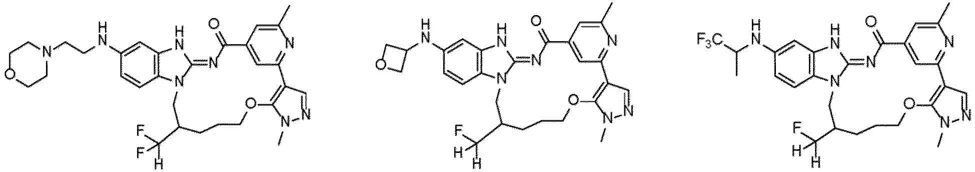 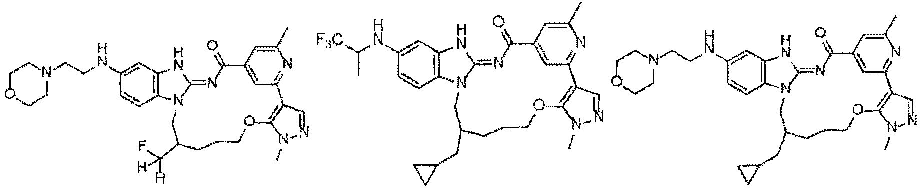 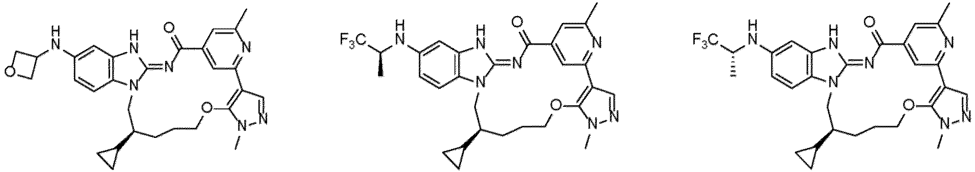 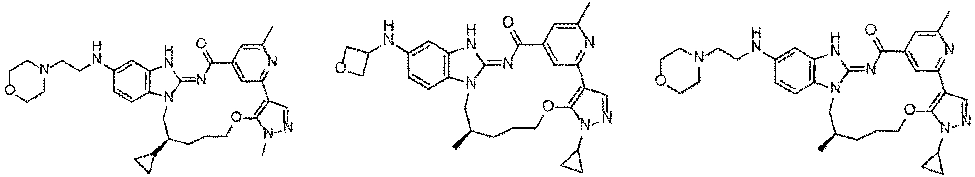 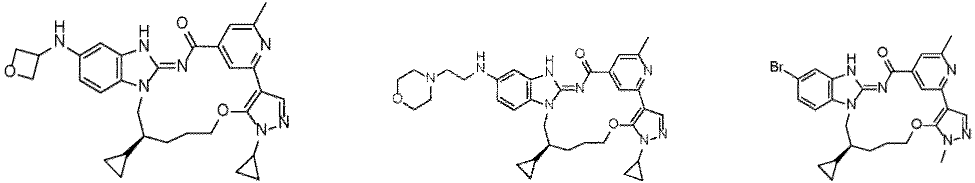 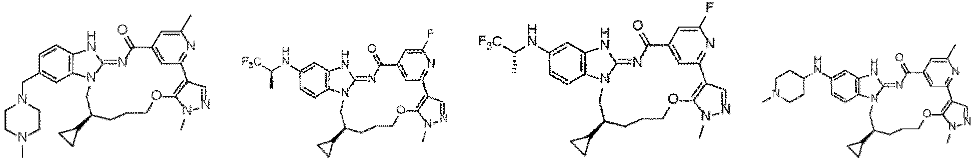 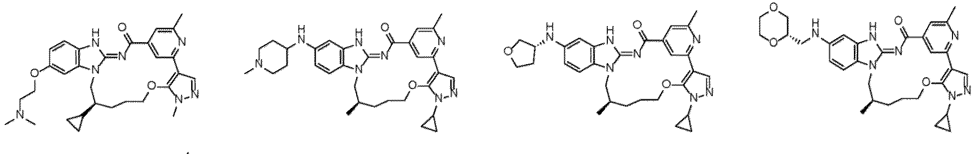 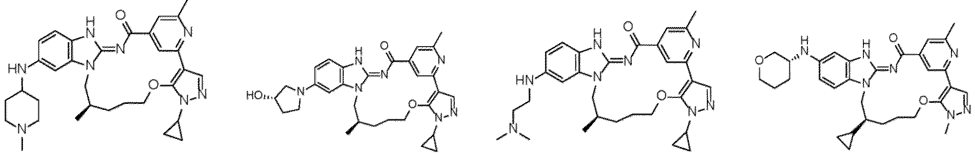 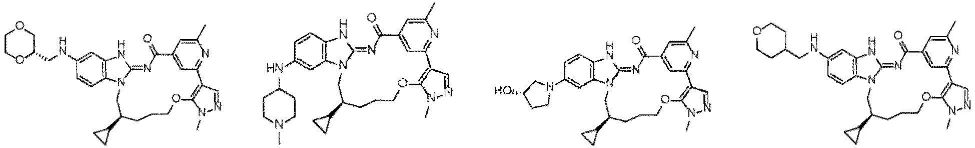 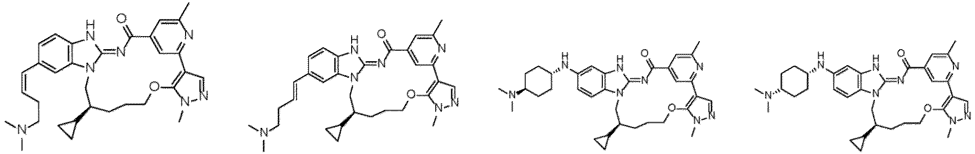 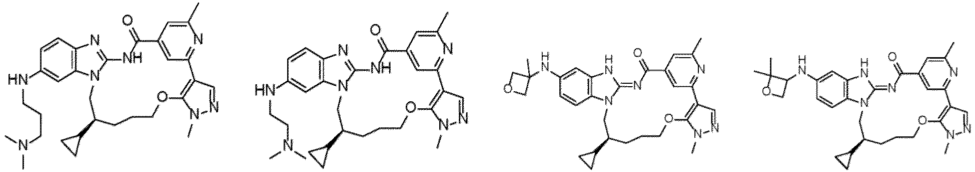 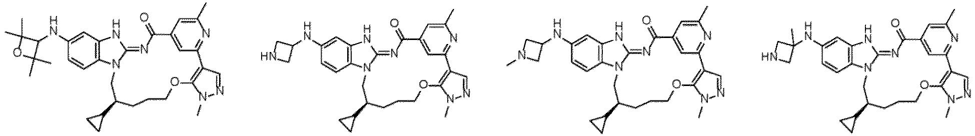 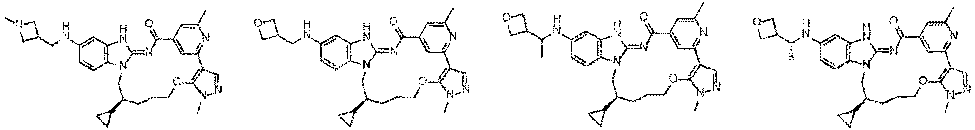 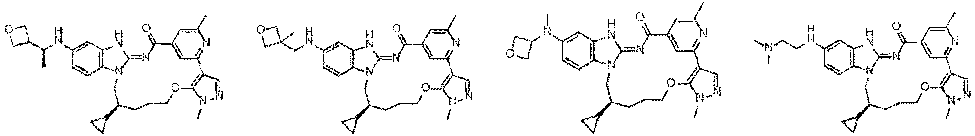 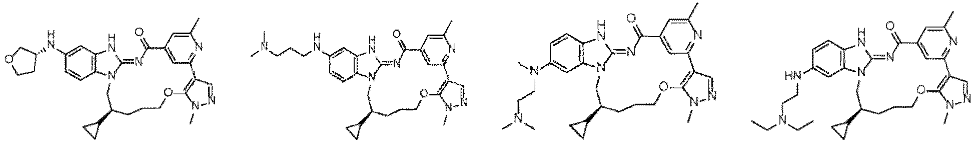 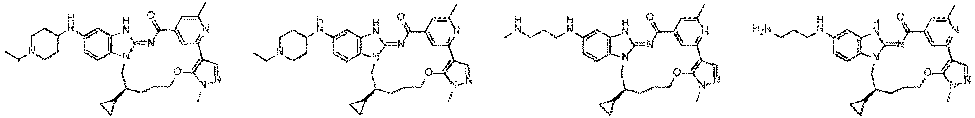 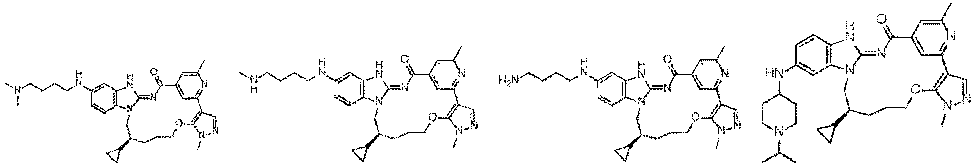 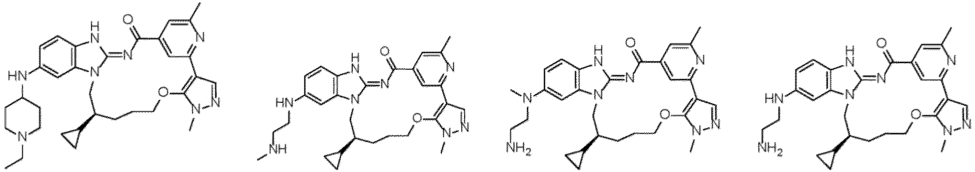 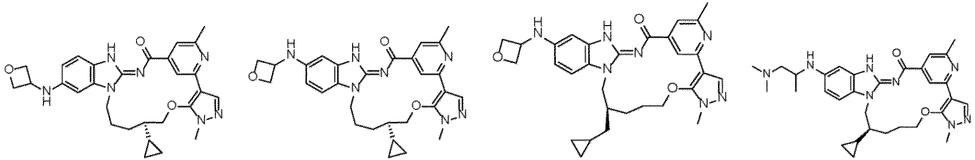 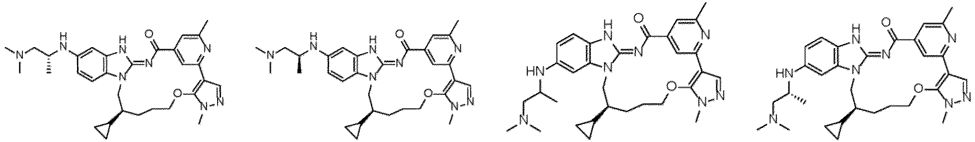 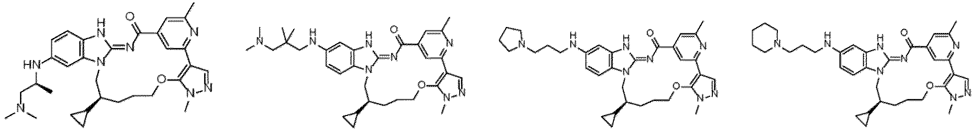 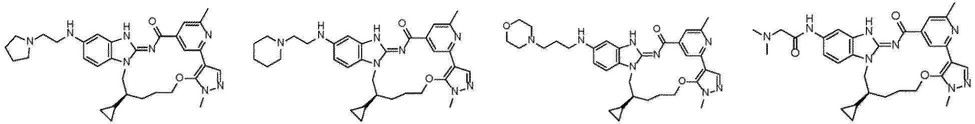 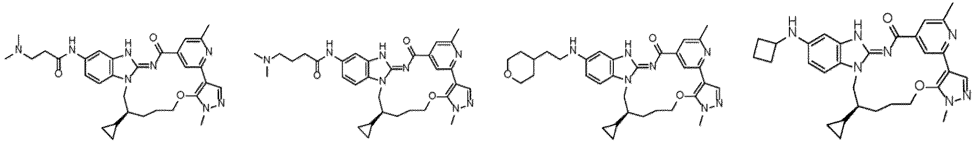 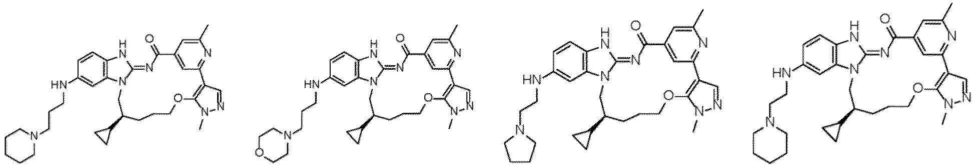 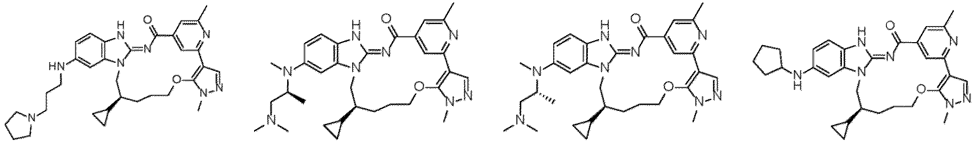 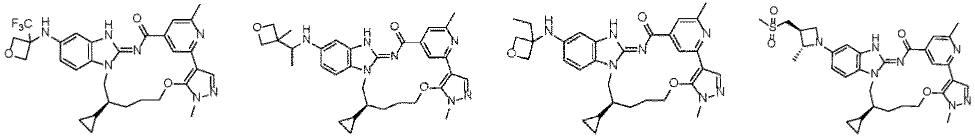 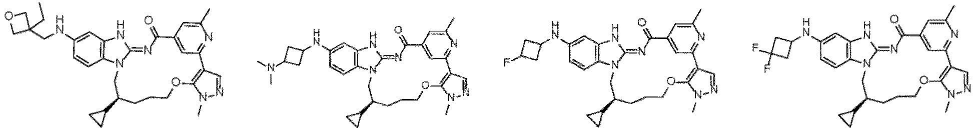 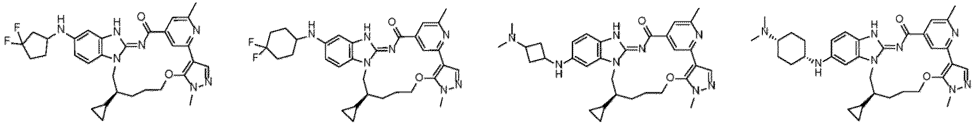 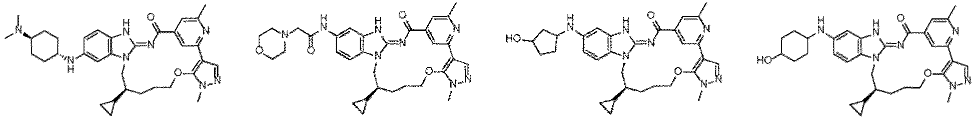 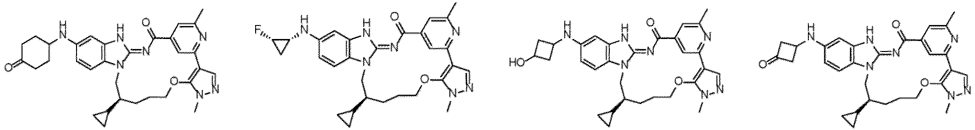 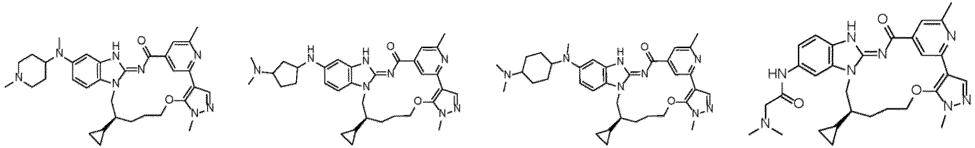 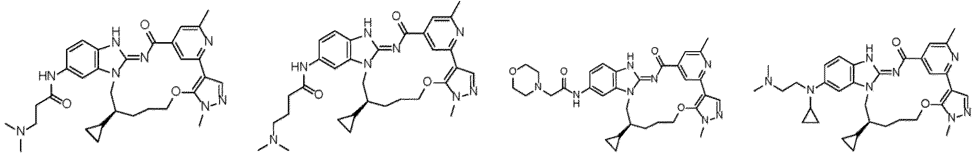 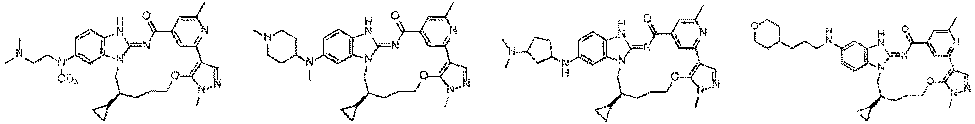 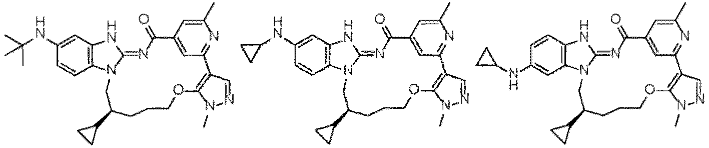 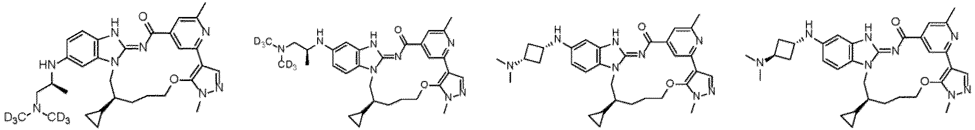 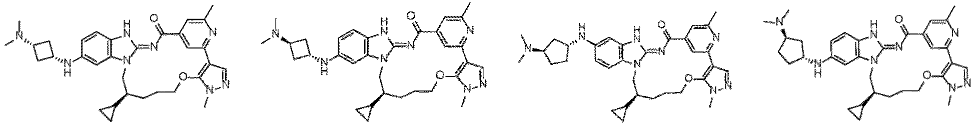 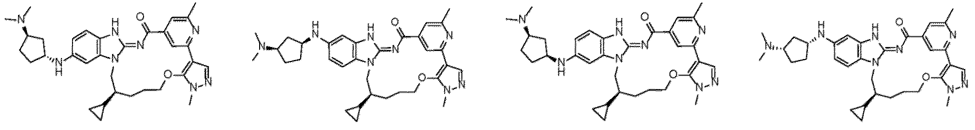 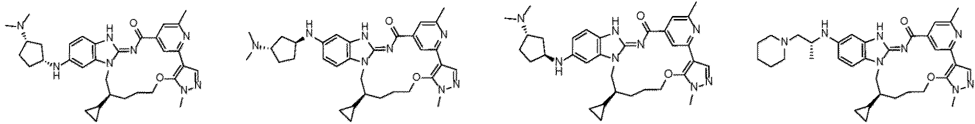 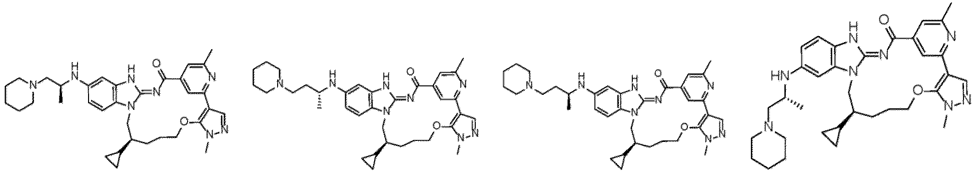 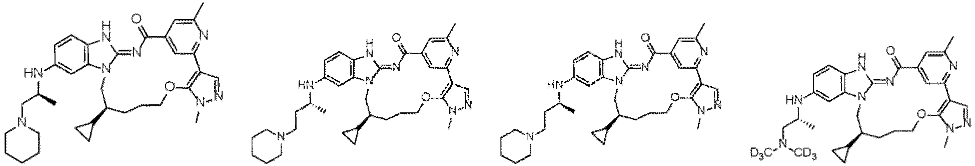 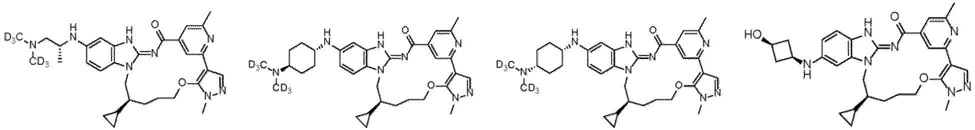 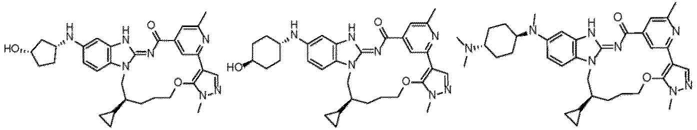 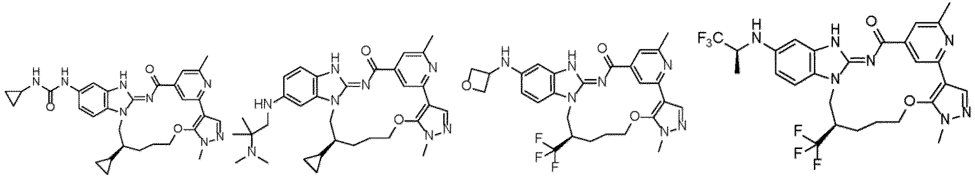 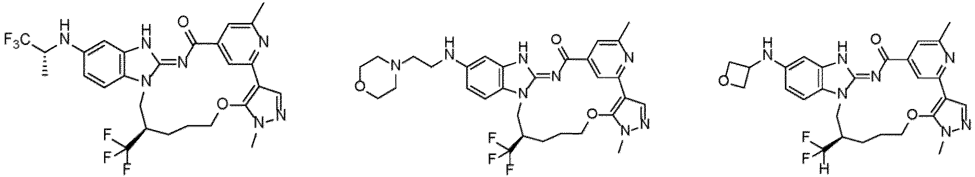 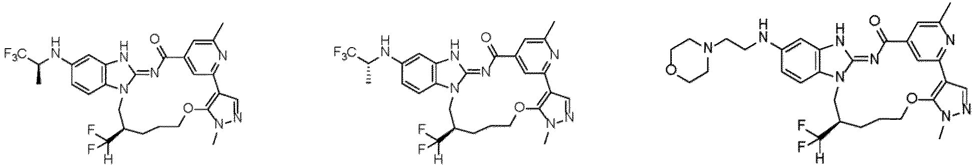 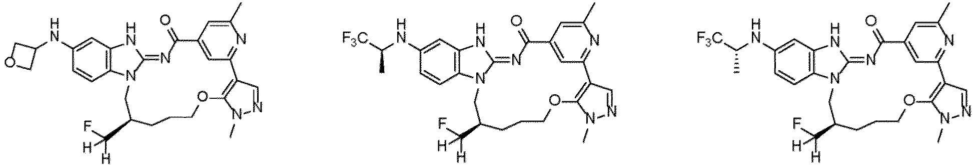 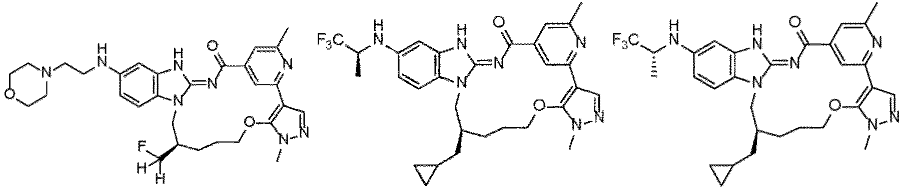 or

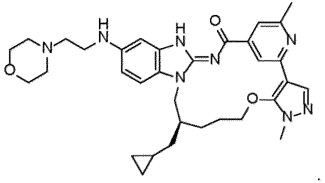

In another aspect, the present application relates to a pharmaceutical composition comprising the compound (the compound of formula I", the compound of formula I', or the compound of formula I), the stereoisomer thereof or the pharmaceutically acceptable salt thereof of the present application. In some embodiments, the pharmaceutical composition of the present application further comprises a pharmaceutically acceptable excipient.

In another aspect, the present application relates to a method for treating an EGFR-mediated disease in a subject (e.g., a mammal), comprising administering to the subject, preferably a human, in need of such treatment a therapeutically effective amount of the compound (e.g., the compound of formula I", the compound of formula I', or the compound of formula I), the stereoisomer thereof or the pharmaceutically acceptable salt thereof, or the pharmaceutical composition thereof of the present application.

In another aspect, the present application relates to use of the compound (such as the compound of formula I", the compound of formula I', or the compound of formula I), the stereoisomer thereof or the pharmaceutically acceptable salt thereof, or the pharmaceutical composition thereof of the present application for preparing a medicament for preventing or treating an EGFR-mediated disease.

In another aspect, the present application relates to use of the compound (such as the compound of formula I", the compound of formula I', or the compound of formula I), the stereoisomer thereof or the pharmaceutically acceptable salt thereof, or the pharmaceutical composition thereof of the present application for preventing or treating an EGFR-mediated disease.

In another aspect, the present application relates to the compound (such as the compound of formula I", the compound of formula I', or the compound of formula I), the stereoisomer thereof or the pharmaceutically acceptable salt thereof, or the pharmaceutical composition thereof of the present application, for use in preventing or treating an EGFR-mediated disease.

In some embodiments, the EGFR-mediated disease described herein is selected from the group consisting of diseases mediated by EGFR mutants. The mutant is selected from the group consisting of one, two, three, or four of L858R, T790M, d19, and C797S. In some embodiments, the mutant is selected from the group consisting of two of L858R and T790M. In some embodiments, the mutant is selected from the group consisting of a double mutant of d19 and T790M. Further, in some embodiments, the mutant comprises a C797S mutant; further, the mutant is selected from a triple mutant of L858R, T790M, and C797S; or the mutant is selected from a triple mutant of d19, T790M, and C797S.

In some embodiments, the EGFR-mediated disease described herein is selected from cancer.

In some embodiments, the EGFR-mediated disease described herein is selected from lung cancer.

In some embodiments, the EGFR-mediated disease described herein is selected from non-small cell lung cancer. The compounds of the present application have one or more advantages of good kinase and cellular activity (including wild type and mutant types, e.g., d19, T790M, C797S, and L858R), stable *in vivo* and *in vitro* metabolism, and excellent *in vivo* drug effect.

### Definitions

Unless otherwise stated, the following terms used in the present application shall have the following meanings. A certain term, unless otherwise specifically defined, should not be considered ambiguous or unclear, but construed according to its common meaning in the art. When referring to a trade name, it is intended to refer to its corresponding commercial product or its active ingredient.

The term "substituted" means that any one or more hydrogen atoms on a specific atom are substituted with substituents, as long as the valence of the specific atom is normal and the resulting compound is stable. When the substituent is oxo (i.e., =O), it means that two hydrogen atoms are substituted and oxo is not possible on an aromatic group.

The term "optional" or "optionally" means that the subsequently described event or circumstance may, but does not necessarily, occur. The description includes instances where the event or circumstance occurs and instances where it does not. For example, ethyl "optionally" substituted with halogen means that the ethyl may be unsubstituted (CH₂CH₃), monosubstituted (for example, CH₂CH₂F), polysubstituted (for example, CHFCH₂F, CH₂CHF₂ and the like), or fully substituted (CF₂CF₃). It can be understood by those skilled in the art that for any groups comprising one or more substituents, no substitutions or substitution modes that are impossible to spatially exist and/or synthesize will be introduced.

In some embodiments, the "one or more" described herein is selected from the group consisting of one, two, three, four, five, or six. In some embodiments, the "one or more" is selected from the group consisting of one, two, or three. In some embodiments, the "one or more" is selected from the group consisting of one or two.

Cₘ₋ₙ used herein means that the moiety has an integer number of carbon atoms in the given range, wherein any carbon present in a substituent for the moiety is not included in the m to n carbons of Cₘ₋ₙ. For example, "C₁₋₆" means that the group may have 1 carbon atom, 2 carbon atoms, 3 carbon atoms, 4 carbon atoms, 5 carbon atoms, or 6 carbon atoms.

When any variable (e.g., R) occurs once or more in the constitution or structure of a compound, the definition of the variable in each case is independent. Therefore, for example, if a group is substituted with 2 R, the definition of each R is independent.

When the number of a connecting group is 0, for example, -(CH₂)₀-, it means that the connecting group is a covalent single bond.

When one of the variables is selected from a single bond, it means that two groups linked by the single bond are directly connected.

When a bond of a substituent is crosslinked to two atoms on a ring, the substituent can be bonded to any atoms on the ring. For example, a structural unit 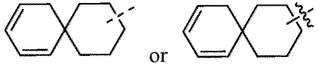 represents that substitution may occur in any one position of cyclohexyl or cyclohexadienyl. " 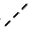 " represents that the substituent is connected to other structures. For example, when the heterocycloalkyl of R¹ is selected from 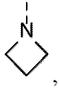 it means that 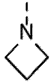 is connected to L:

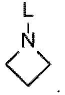

The term "halo-" or "halogen" refers to fluorine, chlorine, bromine, and iodine. When the group of the present application has a group for halogenation, the number of halogenations is optionally selected from one or more. In some embodiments, the "one or more" described herein is selected from the group consisting of one, two, three, four, five, or six. In some embodiments, the "one or more" is selected from the group consisting of one, two, or three. In some embodiments, the "one or more" is selected from the group consisting of one or two. For example, the C₁₋₆ haloalkyl described herein include C₁₋₆ alkyl substituted with one, two, three, four, five, or six halogen atoms.

The term "hydroxy" refers to -OH group.

The term "cyano" refers to -CN group.

The term "amino" refers to -NH₂ group.

The term "nitro" refers to -NO₂ group.

The term "alkyl" refers to hydrocarbyl with a general formula of CₙH₂ₙ₊₁. The alkyl may be linear or branched. For example, the term "C₁-₆ alkyl" refers to alkyl containing 1 to 6 carbon atoms (for example, methyl, ethyl, *n*-propyl, isopropyl, *n*-butyl, isobutyl, *sec*-butyl, *tert*-butyl, *n*-pentyl, 1-methylbutyl, 2-methylbutyl, 3-methylbutyl, neopentyl, hexyl, 2-methylpentyl, and the like). The alkyl moieties (i.e., alkyl) of alkoxy, alkylamino, dialkylamino, alkylsulfonyl, and alkylthio are similarly defined as above.

The term "alkoxy" refers to -O-alkyl.

The term "alkylamino" refers to -NH-alkyl.

The term "dialkylamino" refers to -N(alkyl)₂.

The term "alkylsulfonyl" refers to -SO₂-alkyl.

The term "alkenyl" refers to linear or branched unsaturated aliphatic hydrocarbyl consisting of carbon atoms and hydrogen atoms with at least one double bond. The alkenyl may be C₂₋₈ alkenyl having 2, 3, 4, 5, 6, 7, or 8 carbon atoms. Non-limiting examples of the alkenyl include, but are not limited to, ethenyl, 1-propenyl, 2-propenyl, 1-butenyl, isobutenyl, 1,3-butadienyl, and the like.

The term "alkynyl" refers to linear or branched unsaturated aliphatic hydrocarbyl consisting of carbon atoms and hydrogen atoms with at least one triple bond. The alkynyl may be C₂₋₈ alkynyl having 2, 3, 4, 5, 6, 7, or 8 carbon atoms. Non-limiting examples of the alkynyl include, but are not limited to, ethynyl (-C≡CH), 1-propinyl (-C≡C-CH₃), 2-propinyl (-CH₂-C≡CH), 1,3-butadiynyl (-C≡C-C≡CH), and the like.

The term "cycloalkyl" refers to a carbon ring that is fully saturated and may exist as a monocyclic ring, a bridged ring, or a spiro ring. Unless otherwise indicated, the carbon ring is generally a 3- to 10-membered ring (e.g., a 3-, 4-, 5-, 6-, 7-, or 8-membered ring). Non-limiting examples of the cycloalkyl include, but are not limited to, cyclopropyl, cyclobutyl, cyclopentyl, cyclohexyl, norbornyl (bicyclo[2.2.1]heptyl), bicyclo[2.2.2]octyl, adamantyl, and the like. The term "heterocycloalkyl" refers to a fully saturated cyclic group that may exist as a monocyclic ring, a bridged ring, or a spiro ring. Unless otherwise indicated, the heterocycloalkyl is generally a 3- to 10-membered ring (e.g., a 3-, 4-, 5-, 6-, 7-, or 8-membered ring) containing 1 to 3 heteroatoms (preferably 1 or 2 heteroatoms) independently selected from the group consisting of sulfur, oxygen, and/or nitrogen. Examples of 3-membered heterocycloalkyl include, but are not limited to, oxiranyl, thiiranyl, and aziranyl; non-limiting examples of 4-membered heterocycloalkyl include, but are not limited to, azetidinyl, oxetanyl, and thietanyl; examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl; examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl; examples of 7-membered heterocycloalkyl include, but are not limited to, azepanyl, oxepanyl, and thiepanyl. Monocyclic heterocycloalkyl having 5 or 6 ring atoms is preferred.

The term "heterocyclyl" refers to a fully saturated or partially unsaturated (but not fully unsaturated heteroaromatic group) nonaromatic ring (e.g., heterocycloalkyl) that may exist as a monocyclic ring, a bridged ring, or a spiro ring. Unless otherwise indicated, the heterocyclyl is generally a 3- to 10-membered ring (e.g., a 3-, 4-, 5-, 5-, 7-, or 8-membered ring) containing 1 to 3 heteroatoms (preferably 1 or 2 heteroatoms) independently selected from the group consisting of sulfur, oxygen, and/or nitrogen. Non-limiting examples of the heterocyclyl include, but are not limited to, oxiranyl, oxetanyl, tetrahydrofuranyl, 1,4-dioxanyl, dihydrofuranyl, pyrrolidinyl, *N*-methylpyrrolidinyl, dihydropyrrolyl, piperidinyl, piperazinyl, pyrazolidinyl, 4*H*-pyranyl, morpholinyl, sulfomorpholinyl, tetrahydrothienyl, and the like.

The term "aryl" refers to an all-carbon aromatic monocyclic or fused polycyclic group having a conjugated π-electron system. For example, the aryl may have 6-20 carbon atoms, 6-14 carbon atoms, or 6-12 (e.g., 6, 7, 8, 9, 10, 11, or 12) carbon atoms. Non-limiting examples of the aryl include, but are not limited to, phenyl, naphthyl, anthryl, 1,2,3,4-tetrahydronaphthalene, and the like.

The term "heteroaryl" refers to a monocyclic or fused polycyclic system that comprises at least one ring atom selected from the group consisting of N, O and S, with the remaining ring atoms being C, and that has at least one aromatic ring. Preferably, the heteroaryl has a single 4- to 8-membered ring, in particular a 5- to 8-membered ring, or has a plurality of fused rings comprising 6-14 ring atoms, in particular 6-10 ring atoms. Non-limiting examples of the heteroaryl include, but are not limited to, pyrrolyl, furanyl, thienyl, imidazolyl, oxazolyl, pyrazolyl, pyridinyl, pyrimidinyl, pyrazinyl, quinolyl, isoquinolyl, tetrazolyl, triazolyl, triazinyl, benzofuranyl, benzothienyl, indolyl, isoindolyl, and the like.

The term "treat" or "treatment" means administering the compound or formulation described herein to ameliorate or eliminate a disease or one or more symptoms associated with the disease, including:
(i) inhibiting a disease or disease state, i.e., arresting its progression; and
(ii) alleviating a disease or disease state, i.e., causing its regression.

The term "prevent" or "prevention" means administering the compound or formulation described herein to prevent a disease or one or more symptoms associated with the disease, including preventing the occurrence of a disease or disease state in a subject (e.g., a mammal), particularly when such a mammal is predisposed to the disease state but has not yet been diagnosed with it.

The term "effective amount" refers to an amount of the compound of the present application for (i) treating or preventing a specific disease, condition or disorder; (ii) alleviating, improving or eliminating one or more symptoms of a specific disease, condition or disorder, or (iii) preventing or delaying onset of one or more symptoms of a specific disease, condition or disorder. The amount of the compound of the present application considered as the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organism.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, water, and the like.

The term "subject" includes a mammal, such as a human, dog, cow, horse, pig, sheep, goat, cat, mouse, rabbit, rat or transgenic non-human animal. In some embodiments, the subject is a human.

The word "comprise" and variations thereof such as "comprises" or "comprising" will be understood in an open, non-exclusive sense, i.e., "including but not limited to".

The compounds and intermediates of the present application may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerization. A specific example of a proton tautomer is an imidazole moiety where a proton can transfer between two ring nitrogen atoms. A valence tautomer includes the interconversion via recombination of some bonding electrons.

The present application also comprises isotopically labeled compounds of the present application which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present application (e.g., those labeled with ³H and ¹⁴C) can be used to analyze compounds and/or substrate tissue distribution. Tritiated (i.e., ³H) and carbon-14 (i.e., ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (i.e., ²H) may provide certain therapeutic advantages (e.g., increased *in vivo* half-life or reduced dosage) resulting from greater metabolic stability and hence may be preferred in some circumstances in which deuterium substitution may be partial or complete, wherein partial deuterium substitution refers to substitution of at least one hydrogen with at least one deuterium.

The compound of the present application can be asymmetrical, for example, having one or more stereoisomers. Unless otherwise stated, all stereoisomers are included, for example, enantiomers and diastereoisomers. The compound with asymmetrical carbon atoms of the present application can be separated in an optically pure form or in a racemic form. The optically pure form can be separated from a racemic mixture or can be synthesized using a chiral raw material or a chiral reagent.

The pharmaceutical composition of the present application can be prepared by combining the compound of the present application with a suitable pharmaceutically acceptable excipient, and can be formulated, for example, into a solid, semisolid, liquid or gaseous formulation such as tablet, pill, capsule, powder, granule, ointment, emulsion, suspension, suppository, injection, inhalant, gel, microsphere, aerosol, and the like.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous and intravenous administration.

The pharmaceutical composition of the present application can be manufactured using methods well known in the art, such as conventional mixing, dissolving, granulating, dragee-making, levigating, emulsifying, and lyophilizing. In all of the administration methods of the compound described herein, the daily dose administered is from 0.01 to 200 mg/kg body weight, given in individual or separated doses.

The compounds of the present application can be prepared using a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application.

The chemical reactions of the specific embodiments of the present application are conducted in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protecting groups for reactive functional groups. For example, reference may be made to Greene's Protective Groups in Organic Synthesis (4th Ed.) Hoboken, New Jersey: John Wiley & Sons, Inc. All references cited in the present application are incorporated herein by reference in their entireties.

In some embodiments, the compounds of the general formula of the present application can be prepared by those skilled in the art of organic synthesis according to the route below: 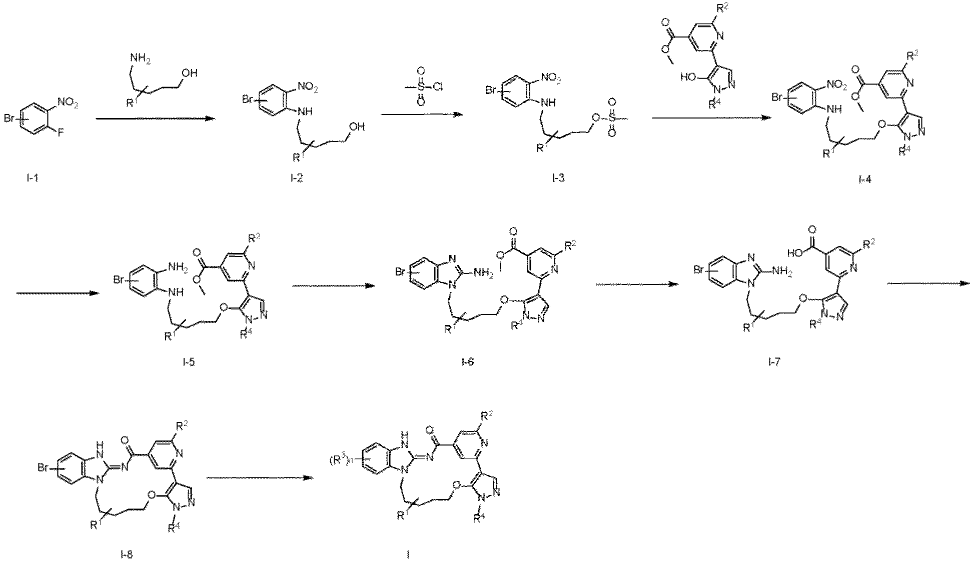 wherein R¹, R², R³, R⁴, and n are as defined above.

Preparation of intermediate I-2: subjecting compound I-1 to a reaction with 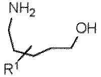 (wherein R¹ is as defined herein) in the presence of a suitable solvent (e.g., *N*,*N*-dimethylformamide and the like) and an inorganic base (e.g., potassium carbonate, etc.). The reaction is performed at about 25 °C to about 50 °C.

Preparation of intermediate I-3: subjecting compound I-2 to a reaction in the presence of a suitable solvent (e.g., dichloromethane, etc.) and an organic base (e.g., triethylamine, etc.). The reaction is performed at about 0 °C to about 20 °C.

Preparation of intermediate I-4: subjecting compound I-3 to a reaction with 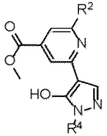 (wherein R² and R⁴ are as defined herein) in the presence of a suitable solvent (e.g., *N*,*N*-dimethylformamide, etc.) and an inorganic base (e.g., potassium carbonate, etc.). The reaction is performed at about 50 °C to about 80 °C.

Preparation of intermediate I-5: subjecting compound I-4 to a reaction in the presence of a suitable solvent (e.g., methanol, etc.) and a catalyst (e.g., Raney nickel, etc.). The reaction is performed at about 0 °C to about 30 °C. Preparation of intermediate I-6: subjecting compound I-5 to a reaction with cyanogen bromide in the presence of a suitable solvent (e.g., dichloromethane/*tert*-butanol, etc.). The reaction is performed at about 0 °C to about 30 °C. Preparation of intermediate I-7: subjecting compound I-6 to a reaction in the presence of a suitable solvent (e.g., tetrahydrofuran, etc.) and an inorganic base (e.g., sodium hydroxide, etc.). The reaction is performed at about 0 °C to about 30 °C.

Preparation of intermediate I-8: subjecting compound I-7 to a reaction in the presence of a suitable solvent (e.g., dichloromethane, etc.) and a condensing agent (e.g., TBTU, etc.). The reaction is performed at about 0 °C to about 30 °C.

Preparation of an intermediate I: subjecting compound I-8 to a reaction in the presence of a suitable solvent (e.g., tert-amyl alcohol, etc.), a base (e.g., potassium *tert*-butoxide, etc.), a ligand (e.g., 2-(di-*tert*-butylphosphine)biphenyl, etc.), and a catalyst (e.g., Pd₂(dba)₃, etc.). The reaction is performed at about 70 °C to about 110 °C.

The following abbreviations are used in this application:
Pd(dppf)Cl₂CH₂Cl₂ represents 1,1'-bis(diphenylphosphino)ferrocenepalladium dichloride dichloromethane complex; HATU represents 2-(7-azabenzotriazole)-*N*,*N*,*N*',*N*'-tetramethyluronium hexafluorophosphate; TBTU represents O-benzotriazole-*N*,*N*,*N*',*N*'-tetramethyluronium tetrafluoroborate; Pd₂(dba)₃ represents tris(dibenzylideneacetone)dipalladium; Pd(dppf)Cl₂ represents 1,1 '-bis(diphenylphosphino)ferrocene]palladium dichloride; X-Phos represents 2-dicyclohexylphosphine-2',4',6'-triisopropylbiphenyl; SEM represents 2-(trimethylsilyl)ethoxymethyl; Boc represents *tert*-butyloxycarbonyl; LiAlH₄ represents lithium aluminium hydride; Cbz represents benzyloxycarbonyl.

### DETAILED DESCRIPTION

For clarity, the present application is further described with the following examples, which are, however, not intended to limit the scope of the present application. All reagents used in the present application are commercially available and can be used without further purification.

### Example 1:

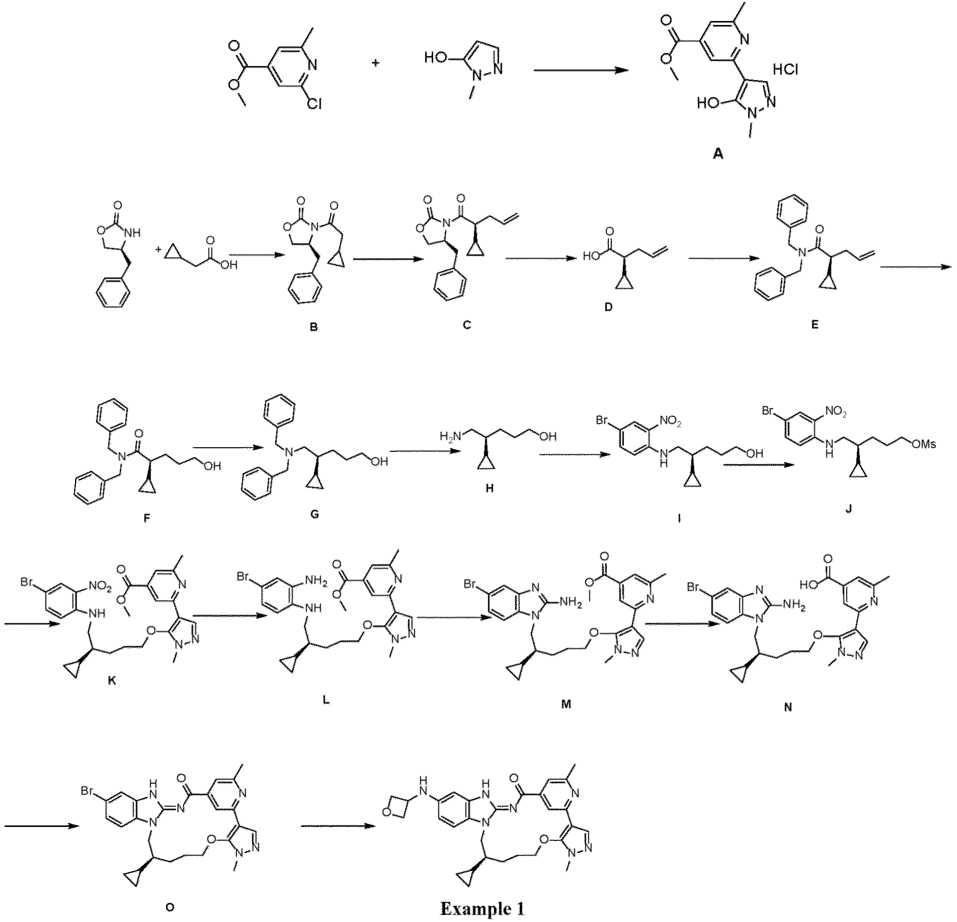

### (1) Preparation method for compound A:

Methyl 2-chloro-6-methylisonicotinate (20.0 g), 1-methyl-5-hydroxypyrazole (21.4 g), Pd(dppf)Cl₂CH₂Cl₂ (2.64 g), and sodium carbonate (25.125 g) were dissolved in anisole (400 mL). The reaction solution was purged with argon 3 times, and stirred at 130 °C for about 10 h. After the reaction was completed, the reaction solution was cooled to room temperature and filtered under vacuum. The filter cake was rinsed with 80 mL of toluene. 60 mL of methanol was added to the filtrate, and 40 mL of 4 M hydrogen chloride/dioxane solution was slowly added dropwise. After the dropwise addition, the mixture was stirred at room temperature for 2 h. After the stirring was completed, the mixture was filtered under vacuum. The filter cake was rinsed with 40 mL of toluene to give the target compound, which was dried under vacuum at 50 °C for 8 h to give compound **A** (18.3 g), ESI-MS: m/z = 248.26[M+H] ⁺.

### (2) Preparation method for compound B:

(*S*)-4-benzyl-2-oxazolidinone (10.0 g), cyclopropylacetic acid (7.3 g), and 4-dimethylaminopyridine (7.6 g) were dispersed in dichloromethane (150 mL), and 1-(3-dimethylaminopropyl)-3-ethylcarbodiimide hydrochloride (21.6 g) was added at 20-25 °C. After the addition, the mixture was stirred at 20-25 °C for 12 h. After the reaction was completed, 50 mL of dichloromethane was added to the reaction solution. The dichloromethane phase was washed with water (100 mL), 1 M hydrochloric acid (100 mL × 2), and 1 M sodium bicarbonate solution (100 mL × 2), and the dichloromethane phase was retained, dried over anhydrous sodium sulfate, concentrated until no liquid flowed out, and subjected to column chromatography to give compound **B** (7.65 g), ESI-MS: m/z = 260.16[M+H]⁺.

### (3) Preparation method for compound C:

Compound **B** (4.0 g) was dispersed in tetrahydrofuran (50 mL), and the dispersion was purged with nitrogen three times and cooled (set temperature: -78 °C). When the internal temperature reached -70 °C, sodium bis(trimethylsilyl)amide (2 M, 11.6 mL) was added dropwise over 15 min, and the mixture was stirred for 1 h with the temperature kept within -70 °C. Then 3-bromopropylene (3.7 g) was added dropwise, and the mixture was stirred for 1 h with the temperature kept within -70 °C, slowly warmed to room temperature, and stirred for 12 h. After the reaction was completed, the reaction solution was poured into an ammonium chloride solution (1 M, 400 mL), and the resulting mixture was extracted with ethyl acetate (30 mL × 2). The ethyl acetate phases were combined, dried over anhydrous sodium sulfate, concentrated until no liquid flowed out, and subjected to column chromatography to give compound **C** (2.8 g), ESI-MS: m/z = 300.18[M+H]⁺.

### (4) Preparation method for compound D:

Compound **C** (1.0 g) was dispersed in tetrahydrofuran (10 mL), and the dispersion was cooled to 0-5 °C in an ice bath. A solution of lithium hydroxide monohydrate (240 mg) in water (2 mL) was added, and the mixture was stirred for 5 min. Then hydrogen peroxide (30%, 1.34 mL) was added, and the mixture was stirred for 1 h with the temperature kept at 0-5 °C. After the reaction was completed, a sodium sulfite solution (1 M, 15 mL) and a sodium bicarbonate solution (1 M, 15 mL) were added to the reaction solution, and the resulting mixture was extracted with dichloromethane (15 mL × 2). The aqueous phase was retained, adjusted to pH 1-2 with 4 M hydrochloric acid, and extracted with dichloromethane (15 mL × 2). The dichloromethane phases were combined, dried over anhydrous sodium sulfate, and concentrated until no liquid flowed out to give compound **D** (3.34 mmol), which was directly used in the next step without further purification.

### (5) Preparation method for compound E:

Compound **D** (3.34 mmol) was dissolved in dichloromethane (10 mL), and dibenzylamine (659 mg), *N,N-*diisopropylethylamine (1.3 g), and HATU (1.5 g) were sequentially added. After the addition, the mixture was stirred at 20-25 °C for 12 h. After the reaction was completed, the reaction solution was washed with water (10 mL × 2), and the dichloromethane phase was retained, dried over anhydrous sodium sulfate, concentrated until no liquid flowed out, and subjected to column chromatography to give compound **E** (800 mg). ESI-MS: m/z = 320.30[M+H] ⁺.

### (6) Preparation method for compound F:

Compound **E** (800 mg) was dispersed in tetrahydrofuran (10 mL), and 9-borabicyclo[3.3.1]nonane (0.5 M, 12.5 mL) was added. The mixture was stirred at 20-25 °C for 1 h, and then cooled to 0-5 °C in an ice bath. A solution of sodium hydroxide (200 mg) in water (2 mL) and hydrogen peroxide (30%, 1.42 mL) were sequentially added. The mixture was stirred at 20-25 °C for 2 h. After the reaction was completed, the reaction solution was poured into water (30 mL), and the resulting mixture was extracted with ethyl acetate (10 mL × 2). The ethyl acetate phases were combined, dried over anhydrous sodium sulfate, and concentrated until no liquid flowed out to give compound **F** (700 mg), which was directly used in the next step without further purification. ESI-MS: m/z = 338.30[M+H] ⁺.

### (7) Preparation method for compound G:

Compound **F** (700 mg) was dispersed in tetrahydrofuran (10 mL), and the dispersion was cooled to 0-5 °C. Lithium aluminum hydride (1 M, 4.1 mL) was added dropwise, and the mixture was stirred at 20-25 °C for 2 h. After the reaction was completed, a saturated sodium sulfate solution was added dropwise to the reaction solution until no gas was discharged. Then the mixture was dried over anhydrous sodium sulfate, filtered, and concentrated until no liquid flowed out to give compound **G** (630 mg), which was directly used in the next step without further purification. ESI-MS: m/z = 324.35 [M+H] ⁺.

### (8) Preparation method for compound H:

Compound **G** (630 mg) was dispersed in methanol (10 mL), and Pd/C (10%, 50 mg) was added. The mixture was purged with hydrogen three times, and stirred in a water bath at 40-45 °C for 2 h. After the reaction was completed, the reaction solution was filtered and concentrated until no liquid flowed out to give compound **H** (1.948 mmol), which was directly used in the next step without further purification.

### (9) Preparation method for compound I:

Compound **H** (1.948 mmol) was dissolved in *N*,*N*-dimethylformamide (10 mL), and 4-bromo-1-fluoro-2-nitrobenzene (428 mg) and potassium carbonate (592 mg) were sequentially added. The mixture was stirred in a water bath at 40-45 °C for 6 h. After the reaction was completed, the reaction solution was poured into 50 mL of water, and the resulting mixture was extracted with ethyl acetate (10 mL × 2). The ethyl acetate phases were combined, dried over anhydrous sodium sulfate, concentrated until no liquid flowed out, and subjected to column chromatography to give compound **I** (470 mg). ESI-MS: m/z = 343.14[M+H] ⁺.

### (10) Preparation method for compound J:

Compound **I** (470 mg) was dissolved in dichloromethane (8 mL), and triethylamine (208 mg) was added. The mixture was cooled to 0-5 °C. A solution of methylsulfonyl chloride (188 mg) in dichloromethane (2 mL) was added dropwise. After the dropwise addition, the mixture was stirred at 20-25 °C for 1 h. After the reaction was completed, the reaction solution was washed with a sodium bicarbonate solution (1 M, 10 mL × 2). The dichloromethane phase was retained, dried over anhydrous sodium sulfate, and concentrated until no liquid flowed out to give compound **J** (1.369 mmol), which was directly used in the next step without further purification.

### (11) Preparation method for compound K:

Compound **J** (1.369 mmol), compound **A** (339 mg), and potassium carbonate (416 mg) were dispersed in *N,N-*dimethylformamide (10 mL), and the mixture was reacted at 60 °C for 6 h. After the reaction was completed, the reaction solution was poured into 50 mL of water, and extracted with ethyl acetate (10 mL × 2). The ethyl acetate phases were combined. The organic phase was washed once with water, dried over anhydrous sodium sulfate, concentrated until no liquid flowed out, and subjected to column chromatography to give compound **K** (650 mg). ESI-MS: m/z = 572.16[M+H] ⁺.

### (12) Preparation method for compound L:

Compound **K** (650 mg) was dissolved in methanol (5 mL), and Raney nickel (20 mg) was added. The mixture was purged with hydrogen 3 times, and reacted at 25 °C for 5 h. After the reaction was completed, the reaction solution was filtered, and the filtrate was dried over anhydrous sodium sulfate and concentrated until no liquid flowed out to give compound **L** (1.135 mmol), which was directly used in the next step without further purification.

### (13) Preparation method for compound M:

Compound **L** (1.135 mmol) was dissolved in *tert*-butanol (1 mL) and dichloromethane (4 mL), and a solution of cyanogen bromide (144 mg) in dichloromethane (1 mL) was added dropwise. The mixture was reacted at 25 °C for 12 h. After the reaction was completed, a sodium bicarbonate solution (1 M, 10 mL) was added to the reaction solution, and the resulting mixture was subjected to liquid separation. The dichloromethane phase was retained. The organic phase was washed with a sodium bicarbonate solution (1 M, 10 mL × 2), dried over anhydrous sodium sulfate, and concentrated until no liquid flowed out to give compound **M** (460 mg), which was directly used in the next step without further purification. ESI-MS: m/z = 567.17[M+H] ⁺.

### (14) Preparation method for compound N:

Compound **M** (460 mg) was dissolved in tetrahydrofuran (3 mL), and a solution of sodium hydroxide (130 mg) in water (3 mL) was added. The mixture was stirred at 20-25 °C for 45 min. After the reaction was completed, the mixture was adjusted to pH 5-6 with 6 M hydrochloric acid, and concentrated by rotary evaporation under reduced pressure to remove tetrahydrofuran and water. Dichloromethane (10 mL) and triethylamine (246 mg) were added, and the mixture was stirred for complete dissolution, dried over anhydrous sodium sulfate, filtered, and concentrated until no liquid flowed out to give compound **N** (0.811 mmol), which was directly used in the next step without further purification. ESI-MS: m/z = 553.16[M+H] ⁺.

### (15) Preparation method for compound O:

Compound **N** (0.811 mmol) was redissolved in dichloromethane (5 mL), and TBTU (312 mg) was added. The mixture was stirred at 20-25 °C for 16 h. After the reaction was completed, the reaction solution was washed with water (10 mL × 3). The organic phase was dried over anhydrous sodium sulfate, concentrated until no liquid flowed out, and subjected to column chromatography to give compound **O** (310 mg). ESI-MS: m/z = 535.22[M+H] ⁺.

### (16) Preparation method for Example 1:

Compound **O** (100 mg), 3-oxacyclobutylamine (16 mg), Pd₂(dba)₃ (43 mg), 2-(di-*tert*-butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 2). The organic phases were combined, dried over anhydrous sodium sulfate, concentrated until no liquid flowed out, and purified by preparative liquid phase chromatography to give **Example 1** (20 mg). ESI-MS: m/z = 528.48[M+H] ⁺.

¹H NMR (500 MHz, DMSO-*d*₆) δ 12.40 (s, 1H), 8.33 (s, 1H), 7.90 (s, 1H), 7.54 (d, *J* = 1.2 Hz, 1H), 7.36 (d, *J* = 8.7 Hz, 1H), 6.64 (d, *J=* 2.1 Hz, 1H), 6.48 (dd, *J=* 8.7, 2.2 Hz, 1H), 6.38 (d, *J=* 6.0 Hz, 1H), 4.85 (t, *J=* 6.2 Hz, 2H), 4.49 (q, *J=* 6.2 Hz, 1H), 4.44 (td, *J=* 5.9, 4.1 Hz, 2H), 4.31 - 4.25 (m, 1H), 4.21 (dd, *J=* 14.0, 3.5 Hz, 1H), 4.07 - 3.98 (m, 2H), 3.72 (s, 3H), 2.54 (s, 3H), 2.19 (q, *J=* 6.7 Hz, 1H), 2.13 (dt, *J=* 10.4, 5.1 Hz, 1H), 1.97 (dd, *J=* 11.7, 6.5 Hz, 1H), 1.80 (t, *J* = 8.5 Hz, 1H), 1.69 (q, *J* = 6.9, 5.5 Hz, 1H), 0.62 - 0.53 (m, 1H), 0.29 (dt, *J* = 9.4, 4.8 Hz, 1H), -0.07 (dq, *J* = 9.5, 4.9 Hz, 1H), -0.18 (tt, *J* = 9.5, 4.5 Hz, 1H), -0.62 (dq, *J* = 9.6, 4.8 Hz, 1H).

### Example 2:

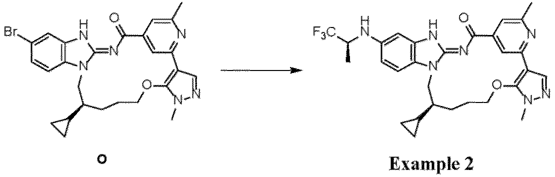

Compound **O** (100 mg), (*S*)-trifluoroisopropylamine hydrochloride (32 mg), Pd₂(dba)₃ (43 mg), *2-(di-tert-*butylphosphine)biphenyl (28 mg), and potassium *tert*-butoxide (126 mg) were dispersed in *tert*-amyl alcohol (10 mL). The mixture was purged with nitrogen 3 times and reacted at 100 °C for 2 h. After the reaction was completed, 50 mL of water was added to the reaction solution, and the resulting mixture was extracted with ethyl acetate (50 mL × 3). The organic phases were combined, dried over anhydrous sodium sulfate, concentrated until no liquid flowed out, and purified by preparative liquid phase chromatography to give **Example 2** (10 mg). ESI-MS: m/z = 568.48[M+H] ⁺.

¹H NMR (500 MHz, DMSO-*d*₆) δ 12.44 (s, 1H), 8.34 (s, 1H), 7.91 (s, 1H), 7.55 (s, 1H), 7.37 (d, *J =* 8.7 Hz, 1H), 6.93 (d, *J* = 2.2 Hz, 1H), 6.73 (dd, *J* = 8.8, 2.3 Hz, 1H), 5.99 (d, *J* = 8.6 Hz, 1H), 4.29 (d, *J* = 7.5 Hz, 1H), 4.22 (dq, *J* = 8.1, 5.1, 4.3 Hz, 2H), 4.03 (td, *J* = 9.5, 4.3 Hz, 2H), 3.73 (s, 3H), 2.54 (s, 3H), 2.20 (q, *J* = 6.7, 6.3 Hz, 1H), 2.13 (dq, *J* = 9.0, 4.4, 3.8 Hz, 1H), 1.98 (td, *J* = 9.9, 9.2, 4.5 Hz, 1H), 1.80 (d, *J* = 9.3 Hz, 1H), 1.70 (tt, *J =* 8.6, 3.7 Hz, 1H), 1.33 (d, *J* = 6.7 Hz, 3H), 0.59 (tt, *J* = 8.6, 3.6 Hz, 1H), 0.30 (dt, *J =* 8.9, 4.4 Hz, 1H), -0.03 - -0.09 (m, 1H), - 0.18 (dt, *J* = 9.1, 4.4 Hz, 1H), -0.61 (dd, *J* = 9.4, 4.8 Hz, 1H).

### Example 3:

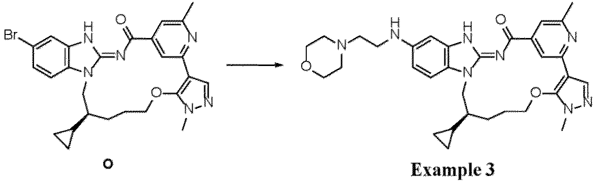

Compound **O** (110 mg), 4-(2-aminoethyl)morpholine (40 mg), Pd₂(dba)₃ (47 mg), 2-(di-*tert-*butylphosphine)biphenyl (31 mg), and potassium *tert*-butoxide (126 mg) were dispersed in *tert*-amyl alcohol (10 mL). The mixture was purged with nitrogen 3 times and reacted at 100 °C for 2 h. After the reaction was completed, 50 mL of water was added to the reaction solution, and the resulting mixture was extracted with ethyl acetate (50 mL × 3). The organic phases were combined, dried over anhydrous sodium sulfate, concentrated until no liquid flowed out, and purified by preparative liquid phase chromatography to give **Example 3** (30 mg). ESI-MS: m/z = 585.49[M+H] ⁺.

¹H NMR (500 MHz, DMSO-*d*₆) δ 12.39 (s, 1H), 8.34 (s, 1H), 8.16 (s, 1H), 7.91 (s, 1H), 7.55 (d, *J =* 1.3 Hz, 1H), 7.34 (d, *J* = 8.7 Hz, 1H), 6.76 (d, *J* = 2.2 Hz, 1H), 6.61 (dd, *J* = 8.7, 2.2 Hz, 1H), 4.33 - 4.26 (m, 1H), 4.22 (dd, *J* = 13.9, 3.4 Hz, 1H), 4.02 (dt, *J* = 14.3, 8.2 Hz, 2H), 3.73 (s, 3H), 3.61 (t, *J* = 4.6 Hz, 4H), 3.13 (t, *J* = 6.7 Hz, 2H), 2.54 (s, 6H), 2.44 (s, 3H), 2.21 (t, *J* = 6.8 Hz, 1H), 2.14 (dt, *J* = 10.4, 5.4 Hz, 1H), 2.03 - 1.94 (m, 1H), 1.80 (d, *J=* 10.0 Hz, 1H), 1.70 (dd, *J* = 11.1, 5.9 Hz, 1H), 0.58 (dt, *J* = 9.8, 5.2 Hz, 1H), 0.30 (dt, *J* = 9.3, 4.7 Hz, 1H), -0.06 (dq, *J* = 9.5, 4.8 Hz, 1H), -0.17 (dt, *J* = 9.1, 4.7 Hz, 1H), -0.61 (dt, *J* = 9.6, 4.8 Hz, 1H).

### Example 4:

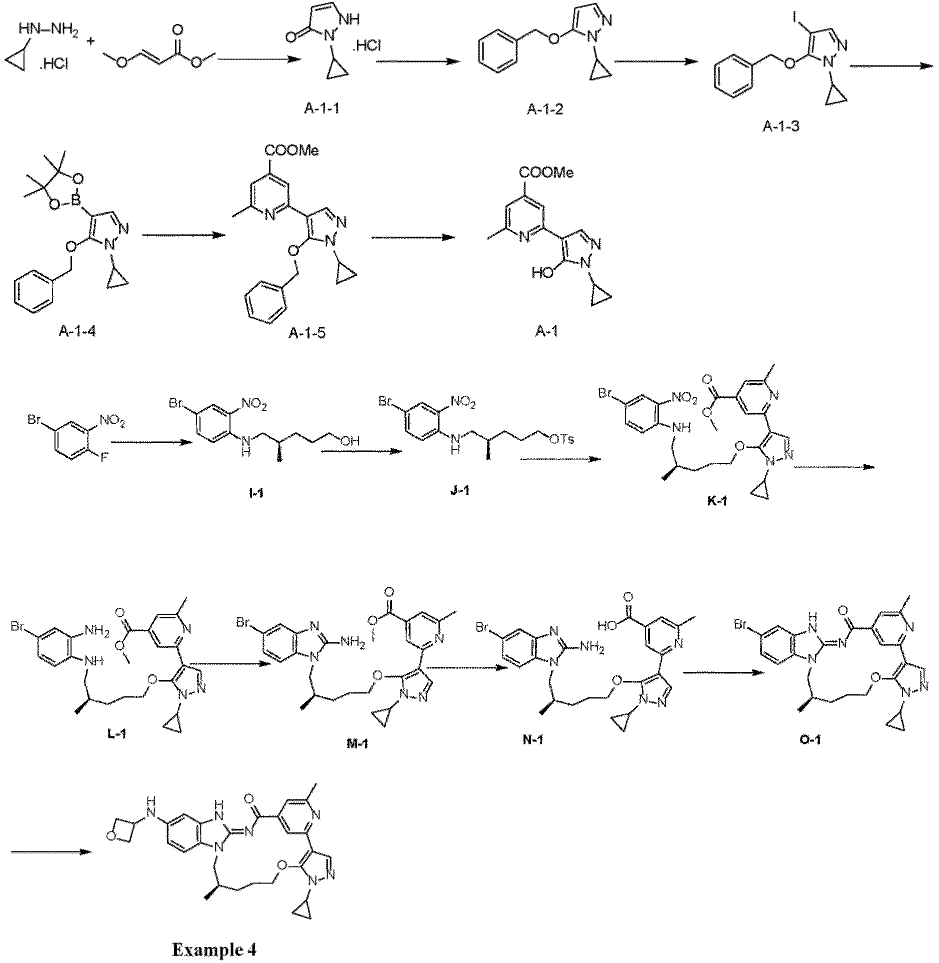

### (1) Preparation method for compound A-1-1:

Cyclopropyl hydrazine hydrochloride (5.0 g) was dispersed in methanol (50 mL), and methyl *trans*-3-methoxyacrylate (5.34 g) was added dropwise at 20-25 °C. Then the mixture was heated to 50-60 °C and reacted for 3.5. After the reaction was completed, the reaction solution was concentrated. Tetrahydrofuran (60 mL) was added to the residue, and the mixture was stirred at room temperature for 2 h. After the stirring was completed, the mixture was filtered under vacuum. The filter cake was rinsed with 20 mL of tetrahydrofuran and dried under vacuum at 50 °C for 8 h to give compound **A-1-1** (6.4 g). ESI-MS: m/z = 125.08[M+H] ⁺.

### (2) Preparation method for compound A-1-2:

Compound A-1-1 (6.0 g) and sodium methoxide (2.02 g) were dispersed in methanol (60 mL), and the mixture was stirred at 20-25 °C for 2 h. The reaction solution was concentrated, and dried by rotary evaporation after tetrahydrofuran (50 mL × 2) was added. Then tetrahydrofuran (100 mL) was added, and the mixture was stirred at 20-25 °C for 1 h, filtered under vacuum, and concentrated to dryness. Then tetrahydrofuran (60 mL), triphenylphosphine (12.7 g), and benzyl alcohol (4.85 g) were sequentially added, and the mixture was cooled to 0-10 °C. Diisopropyl azodicarboxylate (10.6 g) was added dropwise, and the mixture was reacted for 2 h with the temperature kept at this temperature. After the reaction was completed, water (100 mL) and ethyl acetate (100 mL) were added to the reaction solution, and the resulting mixture was subjected to liquid separation. The organic phase was dried over anhydrous sodium sulfate, concentrated until no liquid flowed out, and subjected to column chromatography to give compound **A-1-2** (7.0 g). ESI-MS: m/z = 215.10[M+H] ⁺.

### (3) Preparation method for compound A-1-3:

Compound A-1-2 (7.0 g) was dispersed in dichloromethane (70 mL), and *N*-iodosuccinimide (7.35 g) was added. The mixture was stirred at 20-25 °C for 2 h. After the reaction was completed, water (100 mL) was added to the reaction solution, and the resulting mixture was extracted with dichloromethane (50 mL × 2). The organic phases were combined, dried over anhydrous sodium sulfate, concentrated until no liquid flowed out, and subjected to column chromatography to give compound **A-1-3** (5.9 g). ESI-MS: m/z = 341.04[M+H] ⁺.

### (4) Preparation method for compound A-1-4:

Compound A-1-3 (5.9 g) was dispersed in tetrahydrofuran (60 mL), and the mixture was cooled to -20 °C to -10 °C under nitrogen atmosphere. Isopropyl magnesium chloride (2 mol/L, 17.34 mL) was added dropwise, and the mixture was reacted for 2 h with the temperature kept at this temperature. Isopropanol pinacol borate (8.07 g) was added dropwise. After the dropwise addition, the mixture was reacted for 2 h with the temperature kept at this temperature. After the reaction was completed, the reaction solution was poured into an ammonium chloride solution (10%, 100 mL), and the resulting mixture was extracted with ethyl acetate (100 mL × 2). The organic phases were combined, dried over anhydrous sodium sulfate, and concentrated until no liquid flowed out to give compound **A-1-4** (7.2 g), which was directly used in the next step without further purification. ESI-MS: m/z = 341.02[M+H] ⁺.

### (5) Preparation method for compound A-1-5:

Compound A-1-4 (3.6 g), methyl 2-bromo-6-methylisonicotinate (2.43 g), potassium carbonate (2.9 g), and Pd(dppf)Cl₂ (0.39 g) were dispersed in *N*,*N*-dimethylformamide (30 mL). The mixture was purged with nitrogen 3 times and reacted at 90-100 °C for 8 h. After the reaction was completed, water (300 mL) was added to the reaction solution, and the resulting mixture was extracted with ethyl acetate (150 mL × 2). The organic phases were combined, dried over anhydrous sodium sulfate, concentrated until no liquid flowed out, and subjected to column chromatography to give compound **A-1-5** (1.06 g). ESI-MS: m/z = 364.25[M+H] ⁺.

### (6) Preparation method for compound A-1:

Compound A-1-5 (1.06 g) was dispersed in tetrahydrofuran (20 mL), and Pd/C (10%, 200 mg) was added. The mixture was purged with hydrogen three times, and stirred at 20-25 °C for 0.5 h. After the reaction was completed, the reaction solution was filtered and concentrated until no liquid flowed out to give compound **A-1** (0.8 g), which was directly used in the next step without further purification. ESI-MS: m/z = 274.11[M+H] ⁺.

### (7) Preparation method for compound 1-1:

(*R*)-5-amino-4-methyl-1-pentanol (4.65 g), 2-fluoro-5-bromonitrobenzene (8.32 g), and potassium carbonate (11.50 g) were added to *N*,*N*-dimethylformamide (85 mL), and the mixture was reacted at 50 °C for 4 h. After the reaction was completed, the reaction solution was poured into 500 mL of water, and the resulting mixture was extracted with ethyl acetate (400 mL × 2). The ethyl acetate phases were combined, dried over anhydrous sodium sulfate, and concentrated until no liquid flowed out to give compound **1-1** (4.5 g), which was directly used in the next step without further purification. ESI-MS: m/z = 317.10 [M+H]⁺.

### (8) Preparation method for compound J-1:

Compound I-1 (4.31 g) was dissolved in dichloromethane (80 mL), and triethylamine (2.75 g) was slowly added. The mixture was cooled to 0-5 °C, andp-toluenesulfonyl chloride (2.86 g) was slowly added dropwise. After the dropwise addition, the mixture was reacted at 20-25 °C for 3 h. After the reaction was completed, the reaction solution was washed with a sodium bicarbonate solution (1 M, 100 mL × 2) and subjected to liquid separation. The organic phase was dried over anhydrous sodium sulfate and concentrated until no liquid flowed out to give compound **J-1** (5.1 g), which was directly used in the next step without further purification. ESI-MS: m/z = 471.10 [M+H]⁺.

### (9) Preparation method for compound K-1:

Compound J-1 (1.38 g), compound A-1 (0.8 g), and potassium carbonate (1.0 g) were added to *N,N-*dimethylformamide (10 mL), and the mixture was reacted at 70 °C for 2.5 h. After the reaction was completed, the reaction solution was poured into 150 mL of water, and the resulting mixture was extracted with ethyl acetate (150 mL × 2). The ethyl acetate phases were combined, dried over anhydrous sodium sulfate, concentrated until no liquid flowed out, and subjected to column chromatography to give compound **K-1** (0.8 g). ESI-MS: m/z = 572.19 [M+H]⁺.

### (10) Preparation method for compound L-1:

Compound K-1 (0.8 g) was dissolved in methanol (30 mL), and Raney Ni (0.2 g) was slowly added. The reaction solution was purged with nitrogen 2 times and then with hydrogen 3 times, and reacted at 25 °C for 2 h. After the reaction was completed, the reaction solution was filtered, and the filtrate was concentrated until no liquid flowed out to give compound **L-1** (1.40 mmol), which was directly used in the next step without further purification. ESI-MS: m/z = 542.21 [M+H]⁺.

### (11) Preparation method for compound M-1:

Compound L-1 (1.40 mmol) was dissolved in methanol (30 mL), and a solution of cyanogen bromide (0.3 g) in acetonitrile (3 mL) was added dropwise. The mixture was reacted at 40-50 °C for 2 h. After the reaction was completed, a sodium bicarbonate solution (1 M, 20 mL) was added to the reaction solution, and the resulting mixture was extracted with dichloromethane (15 mL × 2). The dichloromethane phases were combined, dried over anhydrous sodium sulfate, concentrated until no liquid flowed out, and concentrated to give compound **M-1** (0.7 g), which was directly used in the next step without further purification. ESI-MS: m/z = 567.21 [M+H]⁺.

### (12) Preparation method for compound N-1:

Compound M-1 (0.7 g) was dissolved in tetrahydrofuran (30 mL), and a solution of sodium hydroxide (0.2 g) in water (6 mL) was added dropwise. The mixture was reacted at 20-25 °C for 3 h. After the reaction was completed, the reaction solution was adjusted to pH 5-6 with 6 M hydrochloric acid, and concentrated by rotary evaporation under reduced pressure to remove tetrahydrofuran and water. Dichloromethane (50 mL) and triethylamine (2.25 g) were added, and the mixture was stirred for complete dissolution, dried, and filtered to give compound **N-1** (1.23 mmol) in dichloromethane, which was directly used in the next step without further purification. ESI-MS: m/z = 553.18 [M+H]⁺.

### (13) Preparation method for compound O-1:

TBTU (0.47 g) was added to a solution of compound N-1 (1.23 mmol) in dichloromethane, and the mixture was reacted at 20-25 °C for 16 h. After the reaction was completed, the reaction solution was washed with water (20 mL × 3). The organic phase was dried over anhydrous sodium sulfate, concentrated until no liquid flowed out, and subjected to column chromatography to give compound **O-1** (0.48 g). ESI-MS: m/z = 535.23 [M+H]⁺.

### (14) Preparation method for Example 4:

Compound **O-1** (100 mg), 3-oxacyclobutylamine (21 mg), Pd₂(dba)₃ (37 mg), 2-(di-*tert*-butylphosphine)biphenyl (23 mg), and potassium *tert*-butoxide (126 mg) were dispersed in *tert*-amyl alcohol (5 mL). The mixture was purged with nitrogen 3 times and reacted at 70-75 °C for 2 h. After the reaction was completed, water (50 mL) was added to the reaction solution, and the resulting mixture was extracted with dichloromethane (50 mL × 2). The organic phases were combined, dried over anhydrous sodium sulfate, concentrated until no liquid flowed out, and purified by preparative liquid phase chromatography to give **Example 4** (36 mg). ESI-MS: m/z = 528.43[M+H] ⁺.

¹H NMR (500 MHz, chloroform-*d*) δ 12.09 (s, 1H), 8.48 (d, *J* = 1.3 Hz, 1H), 8.09 (s, 1H), 7.63 (d, *J* = 1.4 Hz, 1H), 7.05 (d, *J* = 8.5 Hz, 1H), 6.56 (dd, *J* = 8.7, 2.2 Hz, 1H), 6.45 (d, *J* = 6.0 Hz, 1H), 5.30 (s, 1H), 4.99 (t, *J* = 6.2 Hz, 2H), 4.62 - 4.38 (m, 4H), 4.29 (d, *J* = 13.7 Hz, 1H), 4.02 (s, 1H), 3.58 (t, *J* = 11.8 Hz, 1H), 3.49-3.40 (m, 1H), 2.79 (s, 1H), 2.60 (s, 3H), 2.25 *(d, J=* 7.1 Hz, 1H), 1.93 (dtd, *J* = 19.1, 10.2, 9.5, 5.6 Hz, 2H), 1.48 (dq, *J* = 13.4, 7.1, 5.8 Hz, 1H), 1.40-1.32 (d, *J* = 10.5 Hz, 1H), 1.16-1.13 (m, 1H), 1.09 - 0.97 (m, 2H), 0.89 (d, *J* = 6.6 Hz, 3H).

### Example 5:

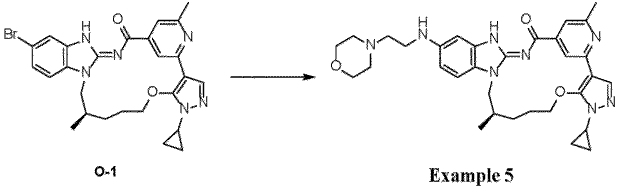

Compound **O-1** (100 mg), 4-(2-aminoethyl)morpholine (46 mg), Pd₂(dba)₃ (37 mg), 2*-*(di*-tert-*butylphosphine)biphenyl (23 mg), and potassium *tert*-butoxide (126 mg) were dispersed in *tert*-amyl alcohol (5 mL). The mixture was purged with nitrogen 3 times and reacted at 70-75 °C for 2 h. After the reaction was completed, water (50 mL) was added to the reaction solution, and the resulting mixture was extracted with dichloromethane (50 mL × 2). The organic phases were combined, dried over anhydrous sodium sulfate, concentrated until no liquid flowed out, and purified by preparative liquid phase chromatography to give **Example 5** (15 mg). ESI-MS: m/z = 585.36[M+H] ⁺.

¹H NMR (500 MHz, DMSO-*d*₆) δ 12.41 (s, 1H), 8.44 (s, 1H), 8.16 (s, 1H), 7.87 (s, 1H), 7.55 (s, 1H), 7.44 - 7.41 (m, 1H), 6.77 (d, *J* = 2.3 Hz, 1H), 6.61 (dd, *J* = 8.6, 2.2 Hz, 1H), 4.39 (td, *J* = 9.2, 4.5 Hz, 1H), 4.12 (ddd, *J* = 25.9, 11.6, 4.0 Hz, 2H), 3.78 (dd, *J* = 13.6, 10.1 Hz, 1H), 3.60 (t, *J* = 4.5 Hz, 5H), 3.13 (t, *J* = 6.7 Hz, 2H), 2.78 (s, 1H), 2.54 (s, 4H), 2.52 (s, 1H), 2.43 (t, *J* = 4.7 Hz, 3H), 2.23 (t, *J =* 6.8 Hz, 1H), 2.03 - 1.95 (m, 1H), 1.93 (d, *J* = 10.9 Hz, 1H), 1.49 - 1.40 (m, 1H), 1.23 (d, *J* = 5.0 Hz, 1H), 1.17 - 1.14 (m, 1H), 1.03 (t, *J=* 5.5 Hz, 3H), 0.81 (d, *J* = 6.5 Hz, 3H).

### Example 6:

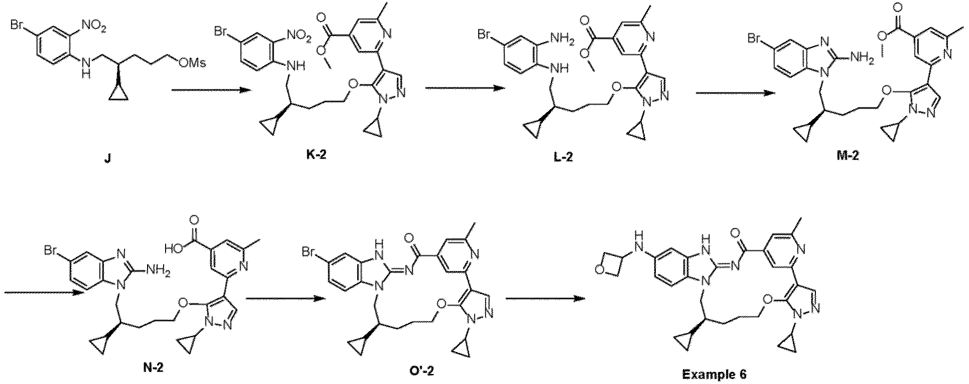

### (1) Preparation method for compound K-2:

Compound J (600 mg), compound A-1 (400 mg), and potassium carbonate (433 mg) were added to *N,N-*dimethylformamide (10 mL), and the mixture was reacted at 60 °C for 5 h. After the reaction was completed, the reaction solution was poured into 50 mL of water, and the resulting mixture was extracted with ethyl acetate (30 mL × 2). The ethyl acetate phases were combined, dried over anhydrous sodium sulfate, concentrated until no liquid flowed out, and subjected to column chromatography to give compound **K-2** (610 mg). ESI-MS: m/z = 598.21[M+H]⁺.

### (2) Preparation method for compound L-2:

Compound K-2 (600 mg) was dissolved in methanol (10 mL), and Raney Ni (0.1 g) was slowly added. The reaction solution was purged with nitrogen 2 times and then with hydrogen 3 times, and reacted at 25 °C for 4 h. After the reaction was completed, the reaction solution was filtered, and the filtrate was concentrated until no liquid flowed out to give compound **L-2** (1.003 mmol), which was directly used in the next step without further purification. ESI-MS: m/z = 568.22[M+H] ⁺.

### (3) Preparation method for compound M-2:

Compound L-2 (1.003 mmol) was dissolved in tert-butanol (2 mL) and dichloromethane (8 mL), and a solution of cyanogen bromide (128 mg) in dichloromethane (1 mL) was added dropwise. The mixture was reacted at 20-25 °C for 12 h. After the reaction was completed, a sodium bicarbonate solution (1 M, 10 mL) was added to the reaction solution, and the resulting mixture was subjected to liquid separation. The dichloromethane phase was retained. The organic phase was washed with a sodium bicarbonate solution (1 M, 10 mL × 2), dried over anhydrous sodium sulfate, and concentrated until no liquid flowed out to give compound **M-2** (500 mg), which was directly used in the next step without further purification. ESI-MS: m/z = 593.24[M+H] ⁺.

### (4) Preparation method for compound N-2:

Compound M-2 (500 mg) was dissolved in tetrahydrofuran (10 mL), and a solution of sodium hydroxide (135 mg) in water (6 mL) was added dropwise. The mixture was reacted at 20-25 °C for 2 h. After the reaction was completed, the reaction solution was adjusted to pH 5-6 with 6 M hydrochloric acid, and concentrated by rotary evaporation under reduced pressure to remove tetrahydrofuran and water. Dichloromethane (20 mL) and triethylamine (250 mg) were added, and the mixture was stirred for complete dissolution, dried, and filtered to give compound **N-2** (0.842 mmol), which was directly used in the next step without further purification. ESI-MS: m/z = 579.20[M+H] ⁺.

### (5) Preparation method for compound O'-2:

TBTU (324 mg) was added to a solution of compound N-2 (0.842 mmol) in dichloromethane, and the mixture was reacted at 20-25 °C for 16 h. After the reaction was completed, the reaction solution was washed with water (20 mL × 3). The organic phase was dried over anhydrous sodium sulfate, concentrated until no liquid flowed out, and subjected to column chromatography to give compound **O'-2** (350 mg). ESI-MS: m/z = 561.19[M+H] ⁺.

### (6) Preparation method for Example 6:

Compound **O'-2** (100 mg), 3-oxacyclobutylamine (16 mg), Pd₂(dba)₃ (41 mg), 2-(di-*tert*-butylphosphine)biphenyl (27 mg), and potassium *tert*-butoxide (120 mg) were dispersed in *tert*-amyl alcohol (5 mL). The mixture was purged with nitrogen 3 times and reacted at 70-75 °C for 2 h. After the reaction was completed, 50 mL of water was added to the reaction solution, and the resulting mixture was extracted with dichloromethane (50 mL × 2). The organic phases were combined, dried over anhydrous sodium sulfate, concentrated until no liquid flowed out, and purified by preparative liquid phase chromatography to give **Example 6** (26 mg). ESI-MS: m/z = 554.31[M+H] ⁺.

### Example 7:

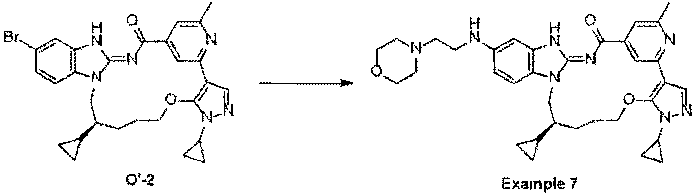

Compound **O'-2** (100 mg), 4-(2-aminoethyl)morpholine (28 mg), Pd₂(dba)₃ (41 mg), 2-(di-*tert-*butylphosphine)biphenyl (27 mg), and potassium *tert*-butoxide (120 mg) were dispersed in *tert*-amyl alcohol (5 mL). The mixture was purged with nitrogen 3 times and reacted at 70-75 °C for 2 h. After the reaction was completed, 50 mL of water was added to the reaction solution, and the resulting mixture was extracted with dichloromethane (50 mL × 2). The organic phases were combined, dried over anhydrous sodium sulfate, concentrated until no liquid flowed out, and purified by preparative liquid phase chromatography to give **Example 7** (15 mg). ESI-MS: m/z = 611.36[M+H] ⁺.

### Example 8:

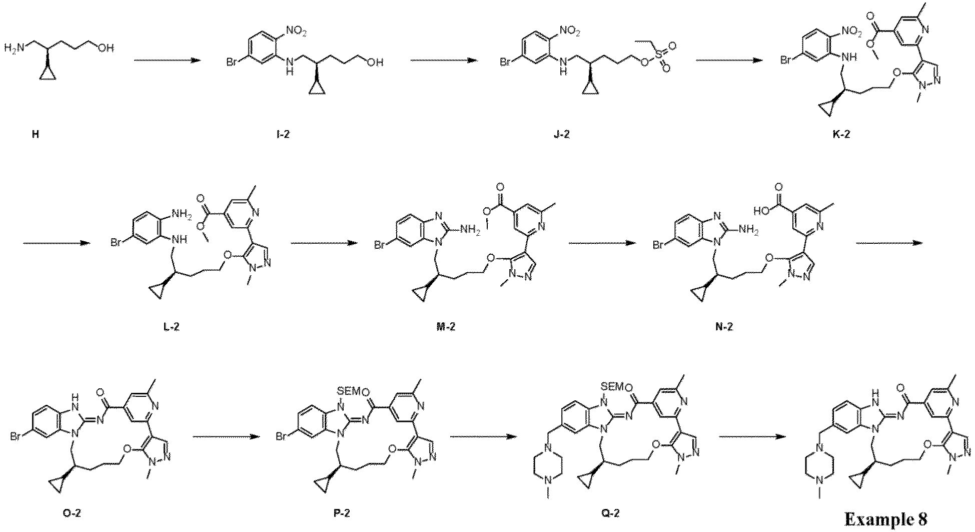

### (1) Preparation method for compound 1-2:

Compound **H** (1.0 g), 2-fluoro-4-bromonitrobenzene (1.54 g), and potassium carbonate (2.12 g) were added to *N,N-*dimethylformamide (30 mL), and the mixture was stirred in a water bath at 40-45 °C for 6 h. After the reaction was completed as monitored by LC-MS, the reaction solution was poured into 200 mL of water, and the resulting mixture was extracted with ethyl acetate (30 mL × 2). The ethyl acetate phases were combined, dried over anhydrous sodium sulfate, concentrated until no liquid flowed out, and subjected to column chromatography to give compound **1-2** (1.54 g). ESI-MS: m/z = 343.15[M+H] ⁺.

### (2) Preparation method for compound J-2:

Compound **1-2** (1.54 g) was dissolved in dichloromethane (20 mL), and triethylamine (681 mg) was slowly added. The mixture was cooled to 0-5 °C, and ethylsulfonyl chloride (692 mg) was slowly added dropwise. After the dropwise addition, the mixture was reacted at 20-25 °C for 3 h. After the reaction was completed, the reaction solution was washed with a sodium bicarbonate solution (1 M, 20 mL × 2) and subjected to liquid separation. The organic phase was dried over anhydrous sodium sulfate and concentrated until no liquid flowed out to give compound **J-2,** which was directly used in the next step without further purification.

### (3) Preparation method for compound K-2:

Compound **J-2** obtained in the previous step, compound **A** (1.10 g), and potassium carbonate (1.36 g) were added to *N*,*N*-dimethylformamide (20 mL), and the mixture was reacted at 70 °C for 2.5 h. After the reaction was completed as monitored by LC-MS, the reaction solution was poured into 100 mL of water, and the resulting mixture was extracted with ethyl acetate (20 mL × 2). The ethyl acetate phases were combined, dried over anhydrous sodium sulfate, concentrated until no liquid flowed out, and subjected to column chromatography to give compound **K-2** (2.01 g). ESI-MS: m/z = 572.20[M+H]⁺.

### (4) Preparation method for compound L-2:

Compound **K-2** (2.0 g) was dissolved in methanol (15 mL) and tetrahydrofuran (15 mL), and Raney nickel (200 mg) was added. The mixture was purged with hydrogen 3 times, and reacted at 25 °C for 5 h. After the reaction was completed as detected by TLC, the reaction solution was filtered, and the filtrate was dried over anhydrous sodium sulfate and concentrated until no liquid flowed out to give compound **L-2,** which was directly used in the next step without further purification. ESI-MS: m/z = 542.21 [M+H]⁺.

### (5) Preparation method for compound M-2:

Compound **L-2** obtained in the previous step was dissolved in *tert*-butanol (5 mL) and dichloromethane (20 mL), and a solution of cyanogen bromide (444 mg) in dichloromethane (2 mL) was added dropwise. The mixture was reacted at 25 °C for 12 h. After the reaction was completed as detected by LC-MS, a sodium bicarbonate solution (1 M, 30 mL) was added to the reaction solution, and the resulting mixture was subjected to liquid separation. The dichloromethane phase was retained. The organic phase was washed with a sodium bicarbonate solution (1 M, 30 mL × 2), dried over anhydrous sodium sulfate, and concentrated until no liquid flowed out to give compound **M-2** (1.80 g), which was directly used in the next step without further purification. ESI-MS: m/z = 567.19[M+H] ⁺.

### (6) Preparation method for compound N-2:

Compound **M-2** (1.80 g) was dissolved in tetrahydrofuran (10 mL), and a solution of sodium hydroxide (507 mg) in water (10 mL) was added. The mixture was stirred at 20-25 °C for 45 min. After the reaction was completed as monitored by LC-MS, the mixture was adjusted to pH 5-6 with 6 M hydrochloric acid, and concentrated by rotary evaporation under reduced pressure to remove tetrahydrofuran and water. Dichloromethane (30 mL) and triethylamine (963 mg) were added, and the mixture was stirred for complete dissolution, dried over anhydrous sodium sulfate, filtered, and concentrated until no liquid flowed out to give compound **N-2,** which was directly used in the next step without further purification. ESI-MS: m/z = 553.16[M+H] ⁺.

### (7) Preparation method for compound O-2:

Compound **N-2** obtained in the previous step was dissolved in dichloromethane (30 mL), and TBTU (1.20 g) was added. The mixture was reacted at 20-25 °C for 16 h. After the reaction was completed as detected by LC-MS, the reaction solution was washed with water (30 mL × 3). The organic phase was dried over anhydrous sodium sulfate, concentrated until no liquid flowed out, and subjected to column chromatography to give compound **O-2** (900 mg). ESI-MS: m/z = 535.21[M+H] ⁺.

### (8) Preparation method for compound P-2:

Compound **O-2** (100 mg) was added to *N*,*N-*dimethylformamide (2 mL), and 2-(trimethylsilyl)ethoxymethyl chloride (47 mg) and *N*,*N*-diisopropylethylamine (48 mg) were sequentially added dropwise at 20-25 °C. The mixture was purged with nitrogen 3 times, and reacted at 60 °C for 3 h. After the reaction was completed as monitored by LC-MS, the reaction solution was poured into 15 mL of water, and the resulting mixture was extracted with ethyl acetate (10 mL × 2). The ethyl acetate phases were combined, dried over anhydrous sodium sulfate, concentrated until no liquid flowed out, and subjected to column chromatography to give compound **P-2** (90 mg). ESI-MS: m/z = 665.20[M+H] ⁺.

### (9) Preparation method for compound Q-2:

Compound **P-2** (90 mg), potassium trifluoro((4-methylpiperazin-1-yl)methyl)borate (52 mg), X-Phos (15 mg), palladium acetate (3.6 mg), and cesium carbonate (130 mg) were added to 1,4-dioxane (3 mL) and water (0.3 mL). The mixture was purged with nitrogen 3 times and reacted at 100 °C for 2 h. After the reaction was completed as detected by LC-MS, the reaction solution was poured into 15 mL of water, and the resulting mixture was extracted with ethyl acetate (10 mL × 2). The ethyl acetate phases were combined, dried over anhydrous sodium sulfate, concentrated until no liquid flowed out, and subjected to column chromatography to give compound **Q-2** (70 mg). ESI-MS: m/z = 699.38[M+H] ⁺.

### (10) Preparation method for Example 8:

Compound **Q-2** (20 mg) was added to dichloromethane (2 mL), and trifluoroacetic acid (0.3 mL) was added dropwise at 20-25 °C. The mixture was reacted at 35 °C for 6 h. After the reaction was completed as detected by LC-MS, the reaction solution was concentrated until no liquid flowed out, and purified by preparative liquid phase chromatography to give **Example 8** (13 mg). ESI-MS: m/z = 569.35 [M+H] ⁺.

¹H NMR (500 MHz, chloroform-*d*) δ 12.05 (s, 1H), 8.41 (s, 1H), 8.15 (s, 1H), 7.66 (s, 1H), 7.40 (s, 1H), 7.27 (d, *J* = 9.1 Hz, 1H), 7.21 (d, *J =* 8.1 Hz, 1H), 4.41 (dd, *J =* 14.2, 3.0 Hz, 1H), 4.38 - 4.32 (m, 1H), 4.03 (dd, *J =* 14.0, 7.2 Hz, 1H), 3.96 (dd, *J =* 9.6, 4.7 Hz, 1H), 3.79 (s, 3H), 3.62 (s, 2H), 2.64 (s, 3H), 2.57 - 2.40 (m, 6H), 2.30 (s, 3H), 2.28 (s, 1H), 2.21 (dd, *J* = 15.5, 7.9 Hz, 3H), 2.01 (d, *J* = 6.3 Hz, 1H), 1.64 (t, *J* = 7.5 Hz, 1H), 0.59 - 0.51 (m, 1H), 0.44 (dq, *J=* 8.9, 4.4 Hz, 1H), 0.04 (d, *J=* 7.2 Hz, 1H), -0.04 (q, *J=* 5.3, 4.8 Hz, 1H), -0.37 (dq, *J=* 9.7, 5.0 Hz, 1H).

### Example 9:

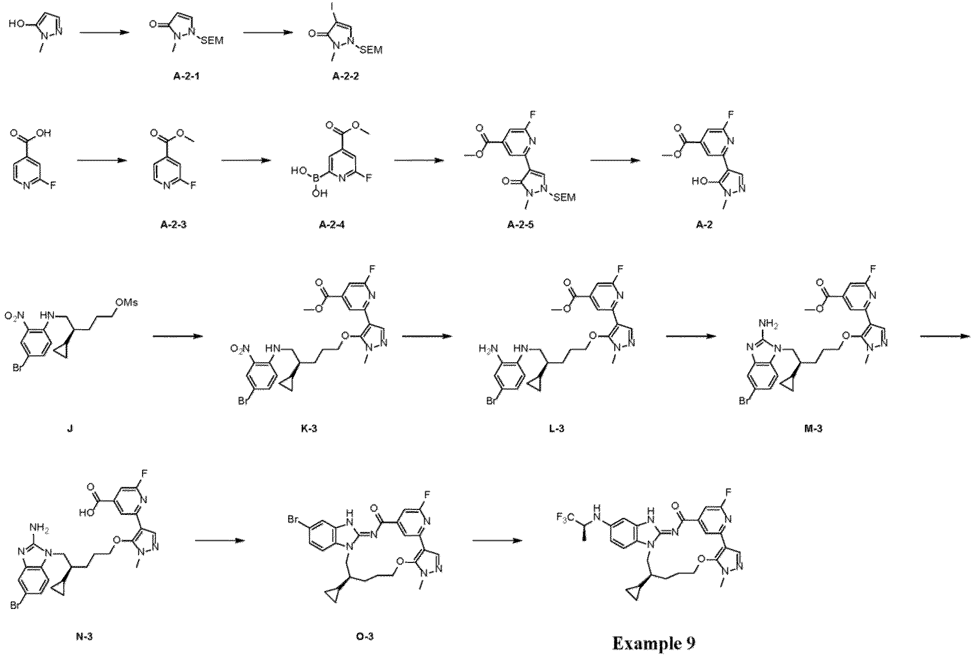

### (1) Preparation method for compound A-2-1:

1-Methyl-5-hydroxypyrazole (30 g) and potassium carbonate (90 g) were added to acetonitrile (900 mL), and 2-(trimethylsilyl)ethoxymethyl chloride (90 mL) was added dropwise at 25 °C. After the dropwise addition, the mixture was reacted at 20-25 °C for 16 h. After the reaction was completed, the reaction solution was filtered, and the filtrate was concentrated by rotary evaporation to dryness. Methyl *tert*-butyl ether (300 mL) was added to the residue, and the resulting mixture was heated to 65 °C for complete dissolution. Then *n*-heptane (120 mL) was added, and the mixture was naturally cooled for crystallization and filtered under vacuum. The filter cake was dried to give compound **A-2-1.** ESI-MS: m/z = 229.30[M+H]⁺.

### (2) Preparation method for compound A-2-2:

Compound **A-2-1** (26 g), *N*-iodosuccinimide (30.7 g), and benzoic acid (2.8 g) were added to dichloromethane (260 mL), and the mixture was reacted at 20-25 °C for 3 h. After the reaction was completed, the reaction solution was washed with water (500 mL × 2) and subjected to liquid separation. The organic phase was dried over anhydrous sodium sulfate, concentrated until no liquid flowed out, and subjected to column chromatography to give compound **A-2-2.** ESI-MS: m/z = 355.20[M+H]⁺.

### (3) Preparation method for compound A-2-3:

2-Fluoroisonicotinic acid (20 g), iodomethane (40.2 g), and potassium carbonate (39 g) were added to *N,N-*dimethylformamide (200 mL), and the mixture was reacted at 40 °C for 16 h. After the reaction was completed, the reaction solution was poured into 1500 mL of water, and the resulting mixture was extracted with ethyl acetate (500 mL × 2). The organic phases were combined, dried over anhydrous sodium sulfate, concentrated until no liquid flowed out, and subjected to column chromatography to give compound **A-2-3.** ESI-MS: m/z = 156.08[M+H]⁺.

### (4) Preparation method for compound A-2-4:

Bis(pinacolato)diboron (14.8 g), (1,5-cyclooctadiene)(methoxy)iridium(I) dimer (570 mg), and 4,4-di-*tert-*butylbipyridine (468 mg) were added to cyclohexane (150 mL). The mixture was purged with nitrogen 3 times and stirred at 25 °C for 1 h. Then a solution of compound **A-2-3** (9 g) in cyclohexane (30 mL) was added dropwise. After the dropwise addition, the mixture was reacted at 75 °C for 4 h. After the reaction was completed, the reaction solution was concentrated by rotary evaporation to dryness to give compound **A-2-4,** which was directly used in the next step without further purification. ESI-MS: m/z = 199.99[M+H]⁺.

### (5) Preparation method for compound A-2-5:

Compound **A-2-2** (5.9 g), compound **A-2-4** (5.0 g), Pd(dppf)Cl₂ (1.3 g), and cesium carbonate (8.2 g) were added to *N*,*N*-dimethylformamide (50 mL). The mixture was purged with nitrogen 3 times and stirred at 100 °C for 4 h. After the reaction was completed, the reaction solution was poured into 300 mL of water, and the resulting mixture was extracted with dichloromethane (100 mL × 2). The organic phases were combined, dried over anhydrous sodium sulfate, concentrated until no liquid flowed out, and subjected to column chromatography to give compound **A-2-5.**

ESI-MS: m/z = 382.34[M+H]⁺.

### (6) Preparation method for compound A-2:

Compound **A-2-5** (5.9 g) was added to a solution of hydrogen chloride in 1,4-dioxane (4 M, 20 mL). The mixture was stirred at 25 °C for 3 h. After the reaction was completed, the reaction solution was concentrated by rotary evaporation to dryness to give compound **A-2,** which was directly used in the next step without further purification.

ESI-MS: m/z = 252.17[M+H]⁺.

### (7) Preparation method for compound K-3:

Compound **J** (1.0 g), compound **A-2** (596 mg), and potassium carbonate (721 mg) were added to *N,N-*dimethylformamide (20 mL), and the mixture was reacted at 60 °C for 6 h. After the reaction was completed as monitored by LC-MS, the reaction solution was poured into 100 mL of water, and extracted with ethyl acetate (30 mL × 2). The ethyl acetate phases were combined. The organic phase was washed once with water, dried over anhydrous sodium sulfate, concentrated until no liquid flowed out, and subjected to column chromatography to give compound **K-3** (1.1 g). ESI-MS: m/z = 576.15[M+H] ⁺.

### (8) Preparation method for compound L-3:

Compound **K-3** (1.1 g) was dissolved in methanol (15 mL), and Raney nickel (100 mg) was added. The mixture was purged with hydrogen 3 times, and reacted at 25 °C for 5 h. After the reaction was completed as detected by TLC, the reaction solution was filtered, and the filtrate was dried over anhydrous sodium sulfate and concentrated until no liquid flowed out to give compound **L-3,** which was directly used in the next step without further purification.

### (9) Preparation method for compound M-3:

Compound **L-3** obtained in the previous step was dissolved in *tert*-butanol (4 mL) and dichloromethane (16 mL), and a solution of cyanogen bromide (243 mg) in dichloromethane (1 mL) was added dropwise. The mixture was reacted at 25 °C for 12 h. After the reaction was completed as detected by LC-MS, a sodium bicarbonate solution (1 M, 20 mL) was added to the reaction solution, and the resulting mixture was subjected to liquid separation. The dichloromethane phase was retained. The organic phase was washed with a sodium bicarbonate solution (1 M, 20 mL × 2), dried over anhydrous sodium sulfate, and concentrated until no liquid flowed out to give compound **M-3** (850 mg), which was directly used in the next step without further purification. ESI-MS: m/z = 571.13[M+H] ⁺.

### (10) Preparation method for compound N-3:

Compound **M-3** (850 mg) was dissolved in tetrahydrofuran (10 mL), and a solution of sodium hydroxide (238 mg) in water (3 mL) was added. The mixture was stirred at 20-25 °C for 45 min. After the reaction was completed as monitored by LC-MS, the mixture was adjusted to pH 5-6 with 6 M hydrochloric acid, and concentrated by rotary evaporation under reduced pressure to remove tetrahydrofuran and water. Dichloromethane (10 mL) and triethylamine (456 mg) were added, and the mixture was stirred for complete dissolution, dried over anhydrous sodium sulfate, filtered, and concentrated until no liquid flowed out to give compound **N-3,** which was directly used in the next step without further purification. ESI-MS: m/z = 557.15[M+H] ⁺.

### (11) Preparation method for compound O-3:

Compound **N-3** obtained in the previous step was dissolved in dichloromethane (10 mL), and TBTU (573 mg) was added. The mixture was reacted at 20-25 °C for 16 h. After the reaction was completed as detected by LC-MS, the reaction solution was washed with water (10 mL × 3). The organic phase was dried over anhydrous sodium sulfate, concentrated until no liquid flowed out, and subjected to column chromatography to give compound **O-3** (620 mg) as a grey-brown solid. ESI-MS: m/z = 539.16[M+H] ⁺.

### (12) Preparation method for Example 9:

Compound **O-3** (100 mg), (*S*)-trifluoroisopropylamine hydrochloride (42 mg), Pd₂(dba)₃ (43 mg), *2-(di-tert-*butylphosphine)biphenyl (28 mg), and potassium *tert*-butoxide (126 mg) were dispersed in *tert*-amyl alcohol (10 mL). The mixture was purged with nitrogen 3 times and reacted at 100 °C for 2 h. After the reaction was completed as detected by LC-MS, 10 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 9** (12 mg). ESI-MS: m/z = 572.34[M+H] ⁺.

¹H NMR (500 MHz, DMSO-*d*₆) δ 12.52 (s, 1H), 8.43 (d, *J=* 2.2 Hz, 1H), 7.87 (s, 1H), 7.40 (d, *J=* 8.8 Hz, 1H), 7.29 (d, *J=* 1.9 Hz, 1H), 6.93 (d, *J=* 2.2 Hz, 1H), 6.75 (dd, *J=* 8.7, 2.3 Hz, 1H), 6.03 (d, *J=* 8.6 Hz, 1H), 4.36 - 4.28 (m, 1H), 4.23 (dt, *J* = 14.1, 3.7 Hz, 2H), 4.06 (td, *J* = 9.3, 4.1 Hz, 2H), 3.74 (s, 3H), 2.21 (q, *J* = 6.8, 6.0 Hz, 1H), 2.15 (dt, *J=* 10.1, 5.0 Hz, 1H), 2.04 - 1.96 (m, 1H), 1.79 (s, 1H), 1.76 - 1.66 (m, 1H), 1.33 (d, *J=* 6.7 Hz, 3H), 0.63 - 0.56 (m, 1H), 0.30 (tt, *J=* 9.0, 4.7 Hz, 1H), -0.04 (dq, *J=* 9.7, 4.7 Hz, 1H), -0.18 (tt, *J=* 9.6, 4.8 Hz, 1H), -0.62 (dt, *J* = 9.5, 5.0 Hz, 1H).

### Example 10:

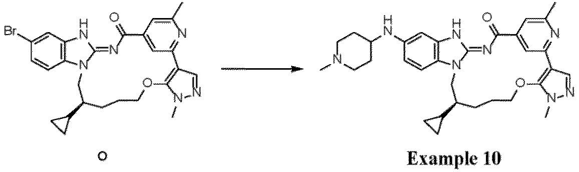

Compound **O** (100 mg), 4-amino-1-methylpiperidine (32 mg), Pd₂(dba)₃ (43 mg), 2-(di-*tert*-butylphosphine)biphenyl (28 mg), and potassium *tert*-butoxide (126 mg) were added to *tert-amyl* alcohol (10 mL). The mixture was purged with nitrogen 3 times and reacted at 100 °C for 2 h. After the reaction was completed, 50 mL of water was added to the reaction solution, and the resulting mixture was extracted with ethyl acetate (50 mL × 3). The organic phases were combined, dried over anhydrous sodium sulfate, concentrated until no liquid flowed out, and purified by preparative liquid phase chromatography to give **Example 10** (30 mg). ESI-MS: m/z = 569.56[M+H] ⁺.

¹H NMR (500 MHz, DMSO-*d*₆) δ 12.35 (s, 1H), 8.33 (s, 1H), 8.21 (s, 1H), 7.90 (s, 1H), 7.54 (s, 1H), 7.32 (d, *J=* 8.6 Hz, 1H), 6.78 (d, *J* = 2.2 Hz, 1H), 6.59 (dd, *J=* 8.7, 2.2 Hz, 1H), 4.28 (q, *J* = 7.8 Hz, 1H), 4.21 (dd, *J=* 13.9, 3.5 Hz, 1H), 4.02 (td, *J* = 14.0, 13.4, 7.5 Hz, 2H), 3.73 (s, 3H), 3.24 - 3.14 (m, 1H), 2.89 (d, *J=* 11.3 Hz, 2H), 2.54 (s, 3H), 2.30 (s, 3H), 2.27 - 2.19 (m, 3H), 2.16 - 2.08 (m, 1H), 1.97 (dd, *J* = 13.7, 8.8 Hz, 3H), 1.81 (d, *J* = 9.2 Hz, 1H), 1.70 (dq, *J* = 13.7, 6.4, 5.5 Hz, 1H), 1.48 (q, *J* = 11.2 Hz, 2H), 0.58 (tt, *J=* 8.6, 3.6 Hz, 1H), 0.30 (tt, *J=* 9.3, 4.8 Hz, 1H), -0.06 (dq, *J* = 9.7, 4.9 Hz, 1H), -0.16 (dp, *J* = 9.4, 4.7 Hz, 1H), -0.59 (dt, *J=* 9.7, 4.9 Hz, 1H).

### Example 11:

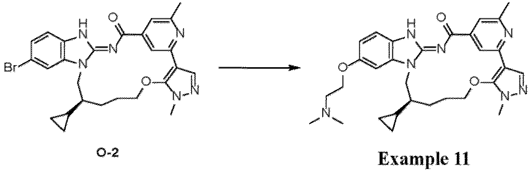

Compound **O-2** (60 mg), copper hydroxide (22 mg), and potassium carbonate (30 mg) were added to *N,N-*dimethylethanolamine (5 mL). The mixture was purged with nitrogen 3 times and reacted at 130 °C for 18 h. After the reaction was completed, 30 mL of water was added to the reaction solution, and the resulting mixture was extracted with ethyl acetate (30 mL × 3). The organic phases were combined, dried over anhydrous sodium sulfate, concentrated until no liquid flowed out, and purified by preparative liquid phase chromatography to give **Example 11** (15 mg). ESI-MS: m/z = 544.41[M+H] ⁺.

¹H NMR (500 MHz, DMSO-*d*₆) δ 12.43 (s, 1H), 8.38 (s, 1H), 7.95 (s, 1H), 7.59 (s, 1H), 7.30 (d, *J =* 8.5 Hz, 1H), 6.81 (d, *J* = 2.1 Hz, 1H), 6.61 (dd, *J* = 8.5, 2.0 Hz, 1H), 4.33 (q, *J* = 8.1 Hz, 1H), 4.27 (dd, *J* = 14.1, 3.7 Hz, 1H), 4.09 (td, *J =* 9.5, 4.5 Hz, 2H), 3.99 (t, *J =* 5.3 Hz, 2H), 3.77 (s, 3H), 2.59 (s, 3H), 2.53 (t, *J =* 6.9 Hz, 2H), 2.28 (s, 1H), 2.25 (s, 6H), 2.18 (td, *J* = 9.6, 4.9 Hz, 1H), 2.04 (tt, *J* = 13.9, 10.5, 5.0 Hz, 1H), 1.89 (d, *J* = 9.5 Hz, 1H), 1.78 (tt, *J* = 13.2, 5.6 Hz, 1H), 0.66 (qd, *J =* 8.4, 4.2 Hz, 1H), 0.38 (dp, *J =* 9.2, 4.7 Hz, 1H), 0.00 (dq, *J* = 9.6, 5.0 Hz, 1H), - 0.06 (dp, *J* = 9.4, 4.7 Hz, 1H), -0.55 (dt, *J* = 9.6, 4.9 Hz, 1H).

### Example 12:

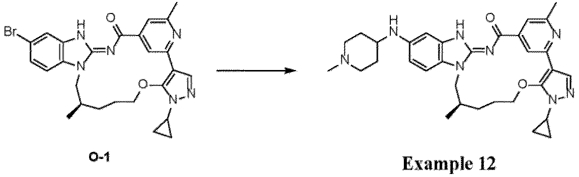

Compound **O-1** (100 mg), 4-amino-1-methylpiperidine (32 mg), Pd₂(dba)₃ (37 mg), *2-(di-tert-*butylphosphine)biphenyl (23 mg), and potassium *tert*-butoxide (126 mg) were added to *tert*-amyl alcohol (4 mL). The mixture was purged with nitrogen 3 times and reacted at 70-75 °C for 4 h. After the reaction was completed, water (50 mL) was added to the reaction solution, and the resulting mixture was extracted with dichloromethane (100 mL × 2). The organic phases were combined, dried over anhydrous sodium sulfate, concentrated until no liquid flowed out, and purified by preparative liquid phase chromatography to give **Example 12** (47 mg). ESI-MS: m/z = 569.45 [M+H] ⁺.

¹H NMR (500 MHz, chloroform-*d*) δ 12.20 (s, 1H), 8.46 (d, *J =* 1.4 Hz, 1H), 8.15 (s, 1H), 7.90 (d, *J =* 1.3 Hz, 1H), 7.34 (d, *J* = 8.5 Hz, 1H), 6.58 (d, *J=* 9.5 Hz, 2H), 4.48 (td, *J* = 9.1, 5.0 Hz, 1H), 4.32 (dd, *J=* 13.7, 3.3 Hz, 1H), 4.06 - 4.01 (m, 1H), 3.85 (ddt, *J* = 15.4, 5.2, 3.3 Hz, 2H), 3.79 - 3.75 (m, 2H), 3.73 (s, 3H), 3.69 - 3.61 (m, 2H), 3.52 - 3.46 (m, 2H), 3.14 (dd, *J* = 12.4, 3.8 Hz, 1H), 3.05 (dd, *J* = 12.4, 7.5 Hz, 1H), 2.83 (s, 1H), 2.65 (s, 3H), 2.28 *(d, J=* 7.1 Hz, 1H), 2.12 (ddt, *J* = 14.1, 9.3, 4.5 Hz, 1H), 1.92 (dtd, *J* = 19.1, 10.2, 9.5, 5.6 Hz, 2H), 1.50 (dq,J= 13.4, 7.1, 5.8 Hz, 1H), 1.40 - 1.35 (m, 1H), 1.18 - 1.13 (m, 1H), 1.07 - 1.01 (m, 2H), 0.89 (d, *J* = 6.6 Hz, 3H).

### Example 13:

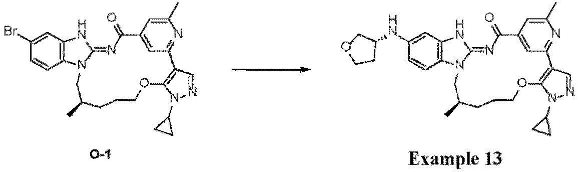

Compound **O-1** (100 mg), (*R*)-3-aminotetrahydrofuran hydrochloride (35 mg), Pd₂(dba)₃ (37 mg), *2-(di-tert-*butylphosphine)biphenyl (23 mg), and potassium *tert*-butoxide (126 mg) were added to *tert*-amyl alcohol (4 mL). The mixture was purged with nitrogen 3 times and reacted at 70-75 °C for 4 h. After the reaction was completed, water (50 mL) was added to the reaction solution, and the resulting mixture was extracted with dichloromethane (100 mL × 2). The organic phases were combined, dried over anhydrous sodium sulfate, concentrated until no liquid flowed out, and purified by preparative liquid phase chromatography to give **Example 13** (18 mg). ESI-MS: m/z = 542.39[M+H] ⁺.

¹H NMR (500 MHz, chloroform-*d*) δ 11.93 (s, 1H), 8.51 (d, *J* = 1.4 Hz, 1H), 8.10 (s, 1H), 7.66 (d, *J* = 1.3 Hz, 1H), 7.08 (d, *J* = 8.5 Hz, 1H), 6.57 (d, *J=* 8.2 Hz, 2H), 4.47 (dt, *J* = 9.1, 4.5 Hz, 1H), 4.32 (dd, *J=* 13.7, 3.4 Hz, 1H), 4.10 - 4.02 (m, 2H), 4.00 - 3.91 (m, 2H), 3.85 (td, *J* = 8.6, 5.5 Hz, 1H), 3.74 (dd, *J* = 9.3, 2.8 Hz, 1H), 3.63 (dd, *J* = 13.7, 9.6 Hz, 1H), 3.48 (tt, *J* = 7.4, 3.9 Hz, 1H), 2.83 (s, 1H), 2.63 (s, 3H), 2.29 (dtd, *J* = 10.5, 5.7, 5.0, 2.5 Hz, 2H), 2.14 (td, *J* = 9.7, 4.9 Hz, 1H), 1.96 (dq, *J* = 9.6, 4.8 Hz, 1H), 1.89 (dddd, *J=* 13.0, 8.1, 5.6, 3.1 Hz, 1H), 1.56 - 1.47 (m, 1H), 1.37 (dtd, *J=* 7.5, 3.9, 2.0 Hz, 1H), 1.19 - 1.14 (m, 1H), 1.10 - 1.01 (m, 2H), 0.92 (d, *J* = 6.5 Hz, 3H).

### Example 14:

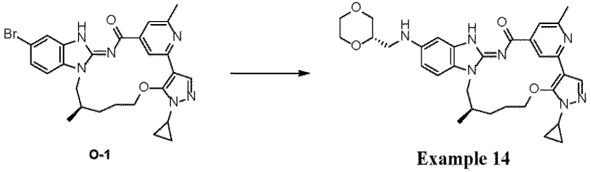

Compound **O-1** (100 mg), (S)-1,4-dioxacycloalkane-2-methanamine hydrochloride (43 mg), Pd₂(dba)₃ (37 mg), 2-(di-*tert*-butylphosphine)biphenyl (23 mg), and potassium *tert*-butoxide (126 mg) were added to *tert*-amyl alcohol (4 mL). The mixture was purged with nitrogen 3 times and reacted at 70-75 °C for 4 h. After the reaction was completed, water (50 mL) was added to the reaction solution, and the resulting mixture was extracted with dichloromethane (100 mL × 2). The organic phases were combined, dried over anhydrous sodium sulfate, concentrated until no liquid flowed out, and purified by preparative liquid phase chromatography to give **Example 14** (11 mg). ESI-MS: m/z = 572.41[M+H] ⁺.

¹H NMR (500 MHz, chloroform-*d*) δ 11.91 (s, 1H), 8.51 (d, *J=* 1.3 Hz, 1H), 8.09 (s, 1H), 7.66 (d, *J* = 1.4 Hz, 1H), 7.07 (d, *J* = 8.6 Hz, 1H), 6.61 (dd, *J=* 8.6, 2.1 Hz, 1H), 6.57 (d, *J* = 2.1 Hz, 1H), 4.48 (td, *J=* 9.1, 5.0 Hz, 1H), 4.32 (dd, *J* = 13.7, 3.3 Hz, 1H), 4.06 - 4.01 (m, 1H), 3.85 (ddt, *J* = 15.4, 5.2, 3.3 Hz, 2H), 3.79 - 3.72 (m, 2H), 3.68 - 3.60 (m, 2H), 3.51 - 3.45 (m, 2H), 3.16 (dd, *J=* 12.4, 3.8 Hz, 1H), 3.07 (dd, *J=* 12.4, 7.5 Hz, 1H), 2.83 (s, 1H), 2.63 (s, 3H), 2.27 (d, *J=* 7.1 Hz, 1H), 2.14 (ddt, *J* = 14.1, 9.3, 4.5 Hz, 1H), 1.96 (dtd, *J* = 19.1, 10.2, 9.5, 5.6 Hz, 2H), 1.51 (dq, *J* = 13.4, 7.1, 5.8 Hz, 1H), 1.40 - 1.35 (m, 1H), 1.18 - 1.13 (m, 1H), 1.07 - 1.01 (m, 2H), 0.91 (d, *J* = 6.6 Hz, 3H).

### Example 15:

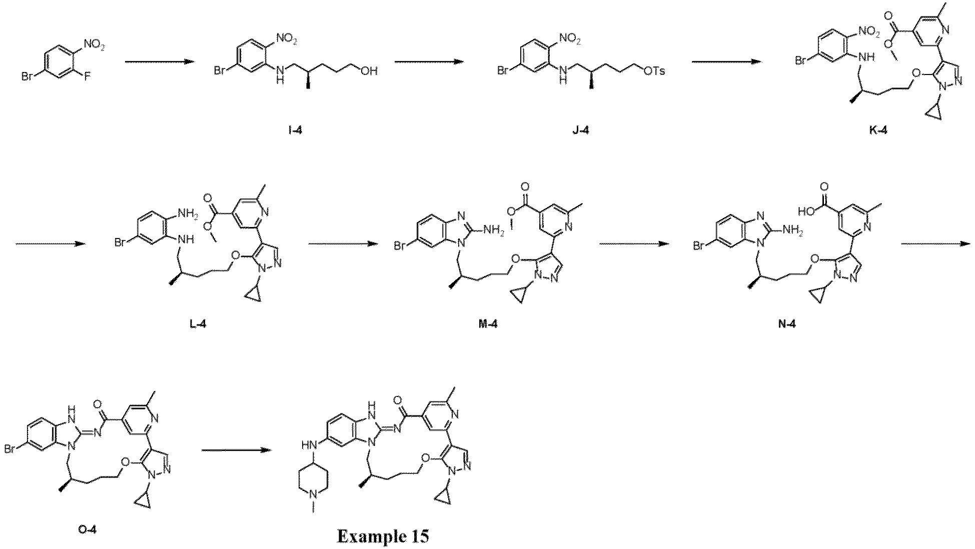

### (1) Preparation method for compound 1-4:

(*R*)-5-amino-4-methyl-1-pentanol (5.58 g), 2-fluoro-4-bromonitrobenzene (9.98 g), and potassium carbonate (13.82 g) were added to *N*,*N*-dimethylformamide (85 mL), and the mixture was reacted at 50 °C for 4 h. After the reaction was completed, the reaction solution was poured into 500 mL of water, and the resulting mixture was extracted with ethyl acetate (400 mL × 2). The ethyl acetate phases were combined, dried over anhydrous sodium sulfate, and concentrated until no liquid flowed out to give compound **1-4** (5.52 g), which was directly used in the next step without further purification. ESI-MS: m/z = 317.10 [M+H]⁺.

### (2) Preparation method for compound J-4:

Compound **1-4** (5.52 g) was dissolved in dichloromethane (80 mL), and triethylamine (3.52 g) was slowly added. The mixture was cooled to 0-5 °C, and p-toluenesulfonyl chloride (3.66 g) was slowly added dropwise. After the dropwise addition, the mixture was reacted at 20-25 °C for 3 h. After the reaction was completed, the reaction solution was washed with a sodium bicarbonate solution (1 M, 100 mL × 2) and subjected to liquid separation. The organic phase was dried over anhydrous sodium sulfate and concentrated until no liquid flowed out to give compound **J-4** (6.1 g), which was directly used in the next step without further purification. ESI-MS: m/z = 471.10 [M+H]⁺.

### (3) Preparation method for compound K-4:

Compound **J-4** (1.38 g), compound **A-1** (0.8 g), and potassium carbonate (1.0 g) were added to *N,N-*dimethylformamide (10 mL), and the mixture was reacted at 70 °C for 2.5 h. After the reaction was completed, the reaction solution was poured into 150 mL of water, and the resulting mixture was extracted with ethyl acetate (150 mL × 2). The ethyl acetate phases were combined, dried over anhydrous sodium sulfate, concentrated until no liquid flowed out, and subjected to column chromatography to give compound **K-4** (0.9 g). ESI-MS: m/z = 572.20[M+H]⁺.

### (4) Preparation method for compound L-4:

Compound **K-4** (0.8 g) was dissolved in methanol (30 mL), and Raney nickel (0.2 g) was slowly added. The reaction solution was purged with nitrogen 2 times and then with hydrogen 3 times, and reacted at 25 °C for 2 h. After the reaction was completed, the reaction solution was filtered, and the filtrate was concentrated until no liquid flowed out to give compound **L-4,** which was directly used in the next step without further purification. ESI-MS: m/z = 542.24[M+H]⁺.

### (5) Preparation method for compound M-4:

Compound **L-4** obtained in the previous step was dissolved in methanol (30 mL), and a solution of cyanogen bromide (0.3 g) in acetonitrile (3 mL) was added dropwise. The mixture was reacted at 40-50 °C for 2 h. After the reaction was completed, a sodium bicarbonate solution (1 M, 20 mL) was added to the reaction solution, and the resulting mixture was extracted with dichloromethane (15 mL × 2). The dichloromethane phases were combined, dried over anhydrous sodium sulfate, concentrated until no liquid flowed out, and concentrated to give compound **M-4** (0.7 g).

ESI-MS: m/z = 567.25 [M+H]⁺.

### (6) Preparation method for compound N-4:

Compound **M-4** (0.7 g) was dissolved in tetrahydrofuran (30 mL), and a solution of sodium hydroxide (0.2 g) in water (6 mL) was added dropwise. The mixture was reacted at 20-25 °C for 3 h. After the reaction was completed, the reaction solution was adjusted to pH 5-6 with 6 M hydrochloric acid, and concentrated by rotary evaporation under reduced pressure to remove tetrahydrofuran and water. Dichloromethane (50 mL) and triethylamine (2.25 g) were added, and the mixture was stirred for complete dissolution, dried, and filtered to give compound **N-4,** which was directly used in the next step without further purification. ESI-MS: m/z = 553.19[M+H]⁺.

### (7) Preparation method for compound O-4:

TBTU (0.47 g) was added to a solution of compound **N-4** obtained in the previous step in dichloromethane, and the mixture was reacted at 20-25 °C for 16 h. After the reaction was completed, the reaction solution was washed with water (20 mL × 3). The organic phase was dried over anhydrous sodium sulfate, concentrated until no liquid flowed out, and subjected to column chromatography to give compound **O-4** (0.51 g). ESI-MS: m/z = 535.27[M+H]⁺.

### (8) Preparation method for Example 15:

Compound **O-4** (50 mg), 4-amino-1-methylpiperidine (13 mg), Pd₂(dba)₃ (21 mg), 2-(di-*tert-*butylphosphine)biphenyl (14 mg), and potassium *tert*-butoxide (61 mg) were added to *tert*-amyl alcohol (3 mL). The mixture was purged with nitrogen 3 times and reacted at 70-75 °C for 4 h. After the reaction was completed, water (10 mL) was added to the reaction solution, and the resulting mixture was extracted with ethyl acetate (10 mL × 2). The organic phases were combined, dried over anhydrous sodium sulfate, concentrated until no liquid flowed out, and purified by preparative liquid phase chromatography to give **Example 15** (5 mg). ESI-MS: m/z = 569.49[M+H] ⁺.

¹H NMR (500 MHz, DMSO-*d*₆) δ 12.38 (s, 1H), 8.44 (s, 1H), 8.15 (s, 1H), 7.87 (s, 1H), 7.54 (s, 1H), 7.23 (d, *J=* 8.6 Hz, 1H), 6.68 (d, *J* = 2.0 Hz, 1H), 6.54 (dd, *J=* 8.6, 2.0 Hz, 1H), 4.40 (td, *J* = 9.3, 4.5 Hz, 1H), 4.13 (ddd, *J* = 18.7, 11.6, 4.0 Hz, 2H), 3.85 - 3.81 (m, 1H), 3.64 - 3.62 (m, 1H), 3.28 (s, 1H), 2.81 - 2.73 (m, 3H), 2.54 (s, 3H), 2.24 (s, 1H), 2.20 (s, 3H), 2.09 (t, *J* = 11.2 Hz, 2H), 2.03 - 1.98 (m, 1H), 1.96 - 1.89 (m, 3H), 1.46 (s, 1H), 1.44 - 1.35 (m, 2H), 1.20 - 1.14 (m, 1H), 1.03 (ddd, *J* = 11.6, 7.7, 3.7 Hz, 3H), 0.82 (d, *J=* 6.5 Hz, 3H).

### Example 16:

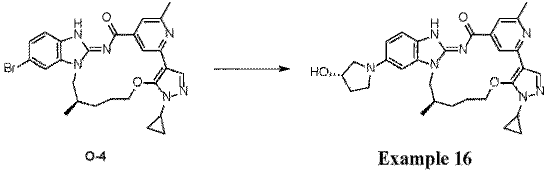

Compound **O-4** (50 mg), (S)-3-pyrrolidinol (10 mg), Pd₂(dba)₃ (21 mg), 2-(di-*tert*-butylphosphine)biphenyl (14 mg), and potassium *tert*-butoxide (61 mg) were added to *tert*-amyl alcohol (3 mL). The mixture was purged with nitrogen 3 times and reacted at 70-75 °C for 4 h. After the reaction was completed, water (10 mL) was added to the reaction solution, and the resulting mixture was extracted with ethyl acetate (10 mL × 2). The organic phases were combined, dried over anhydrous sodium sulfate, concentrated until no liquid flowed out, and purified by preparative liquid phase chromatography to give **Example 16** (10 mg). ESI-MS: m/z = 542.26[M+H] ⁺.

¹H NMR (500 MHz, DMSO-*d*₆) δ 12.41 (s, 1H), 8.35 (s, 1H), 7.91 (s, 1H), 7.55 (s, 1H), 7.34 (d, *J* = 8.6 Hz, 1H), 6.69 (d, *J=* 2.2 Hz, 1H), 6.47 (dd, *J* = 8.7, 2.2 Hz, 1H), 4.98 (s, 1H), 4.43 (dq, *J=* 7.0, 3.4 Hz, 1H), 4.29 (q, *J* = 8.2, 7.6 Hz, 1H), 4.22 (dd, *J=* 14.0, 3.7 Hz, 1H), 4.14 (dd, *J=* 13.8, 9.1 Hz, 1H), 4.04 (dt, *J=* 7.0, 4.6 Hz, 1H), 3.46 (dd, *J* = 10.0, 5.0 Hz, 1H), 3.12 (dd, *J* = 10.0, 2.5 Hz, 1H), 2.79 (s, 1H), 2.60 (s, 3H), 2.25 (d, *J* = 7.1 Hz, 1H), 2.09 (t, *J* = 11.2 Hz, 2H), 2.03 - 1.98 (m, 1H), 1.96 - 1.89 (m, 3H), 1.48 (dq, *J* = 13.4, 7.1, 5.8 Hz, 1H), 1.40-1.32 (d, *J* = 10.5 Hz, 2H), 1.16-1.13 (m, 1H), 1.09 - 0.97 (m, 2H), 0.89 (d, *J=* 6.6 Hz, 3H).

### Example 17:

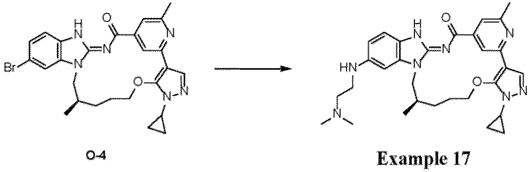

Compound **O-4** (70 mg), *N*,*N*-dimethylethylenediamine (23 mg), Pd₂(dba)₃ (24 mg), *2-(di-tert-*butylphosphine)biphenyl (16 mg), and potassium *tert*-butoxide (88 mg) were added to *tert*-amyl alcohol (3 mL). The mixture was purged with nitrogen 3 times and reacted at 80 °C for 2 h. After the reaction was completed, water (10 mL) was added to the reaction solution, and the resulting mixture was extracted with ethyl acetate (10 mL × 2). The organic phases were combined, dried over anhydrous sodium sulfate, concentrated until no liquid flowed out, and purified by preparative liquid phase chromatography to give **Example 17** (20 mg). ESI-MS: m/z = 543.43 [M+H] ⁺. ¹H NMR (500 MHz, DMSO-*d*₆) δ 12.41 (s, 1H), 8.45 (s, 1H), 8.19 *(d, J=* 2.6 Hz, 1H), 7.88 *(d, J=* 3.4 Hz, 1H), 7.55 (d, *J =* 1.2 Hz, 1H), 7.26 (d, *J =* 8.5 Hz, 1H), 6.73 (d, *J* = 2.1 Hz, 1H), 6.57 (dd, *J* = 8.6, 2.1 Hz, 1H), 4.40 (dt, *J =* 9.5, 4.8 Hz, 1H), 4.18 - 4.10 (m, 2H), 3.84 (dd, *J* = 13.6, 10.2 Hz, 1H), 3.65 - 3.61 (m, 1H), 3.21 (t, *J* = 6.4 Hz, 2H), 2.81 (s, 1H), 2.63 (t, *J =* 6.5 Hz, 2H), 2.55 (d, *J =* 3.6 Hz, 3H), 2.33 (d, *J =* 4.7 Hz, 6H), 2.25 (s, 1H), 2.01 (d, *J =* 13.8 Hz, 1H), 1.97 - 1.89 (m, 1H), 1.47 (s, 1H), 1.16 (ddd, *J=* 9.1, 4.6, 2.7 Hz, 1H), 1.09 - 1.01 (m, 3H), 0.84 (t, *J* = 5.2 Hz, 3H).

### Example 18:

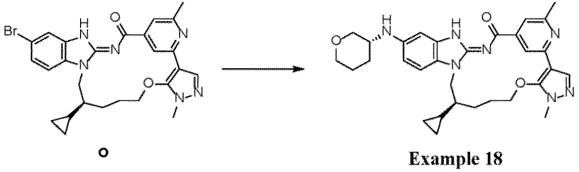

Compound **O** (100 mg), (*R*)-3-aminotetrahydropyran hydrochloride (31 mg), Pd₂(dba)₃ (43 mg), 2-(di-*tert-*butylphosphine)biphenyl (28 mg), and potassium *tert*-butoxide (126 mg) were added to *tert*-amyl alcohol (3 mL). The mixture was purged with nitrogen 3 times and reacted at 100 °C for 2 h. After the reaction was completed, 50 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 18** (69 mg). ESI-MS: m/z = 556.46[M+H] ⁺.

¹H NMR (500 MHz, DMSO-*d*₆) δ 12.36 (s, 1H), 8.34 (s, 1H), 7.91 (s, 1H), 7.54 (d, *J* = 1.2 Hz, 1H), 7.24 (d, *J* = 8.6 Hz, 1H), 6.82 (d, *J =* 2.1 Hz, 1H), 6.58 (dd, *J =* 8.6, 2.1 Hz, 1H), 5.45 (s, 1H), 4.28 (td, *J =* 8.5, 5.2 Hz, 1H), 4.21 (dd, *J=* 13.9, 3.7 Hz, 1H), 4.12 - 4.01 (m, 2H), 3.92 (ddd, *J* = 10.9, 4.0, 1.6 Hz, 1H), 3.73 (s, 3H), 3.45 (s, 1H), 3.38 - 3.33 (m, 1H), 3.12 (dd, *J=* 11.0, 8.4 Hz, 1H), 2.54 (s, 3H), 2.21 (dd, *J=* 13.3, 6.6 Hz, 1H), 2.13 (tt, *J=* 8.8, 4.9 Hz, 1H), 2.00 (td, *J =* 13.4, 5.0 Hz, 2H), 1.84 (d, *J =* 9.9 Hz, 1H), 1.71 (dq, *J =* 12.2, 3.7 Hz, 2H), 1.59 (dtd, *J =* 17.7, 10.0, 8.3, 4.4 Hz, 1H), 1.50 - 1.42 (m, 1H), 0.86 - 0.83 (m, 1H), 0.67 - 0.58 (m, 1H), 0.32 (dp, *J=* 9.2, 4.7 Hz, 1H), -0.05 (dq, *J* = 9.6, 4.8 Hz, 1H), -0.13 (dq, *J* = 9.3, 4.9 Hz, 1H), -0.57 (dt, *J=* 9.6, 4.8 Hz, 1H).

### Example 19:

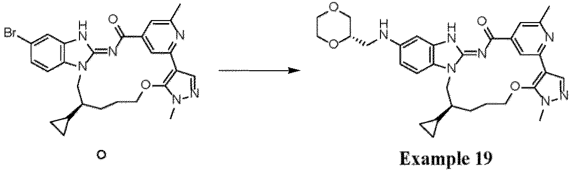

Compound **O** (100 mg), (*S*)-1,4-dioxacycloalkane-2-methanamine hydrochloride (43 mg), Pd₂(dba)₃ (43 mg), 2-(di-*tert*-butylphosphine)biphenyl (28 mg), and potassium *tert*-butoxide (126 mg) were added to *tert-*amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 19** (13 mg). ESI-MS: m/z = 572.34[M+H] ⁺.

¹H NMR (500 MHz, chloroform-*d*) δ 11.93 (s, 1H), 8.39 (s, 1H), 8.15 (s, 1H), 7.65 (s, 1H), 7.15 (d, *J* = 9.1 Hz, 1H), 6.57 (d, *J* = 6.5 Hz, 2H), 4.35 (tt, *J* = 8.8, 4.5 Hz, 2H), 3.98 - 3.90 (m, 2H), 3.84 (ddd, *J* = 17.8, 9.9, 4.1 Hz, 3H), 3.78 (s, 3H), 3.76 (d, *J* = 10.1 Hz, 1H), 3.66 (td, *J* = 11.4, 3.0 Hz, 1H), 3.51 (dd, *J* = 11.4, 9.8 Hz, 1H), 3.22 (dd, *J* = 12.4, 3.8 Hz, 1H), 3.13 (dd, *J* = 12.3, 7.5 Hz, 1H), 2.64 (s, 3H), 2.27 (q, *J* = 7.8, 7.4 Hz, 1H), 2.18 (p, *J* = 9.2, 7.5 Hz, 2H), 1.81 (q, *J=* 7.8, 7.1 Hz, 2H), 0.87 (dtt, *J* = 10.5, 6.6, 3.0 Hz, 1H), 0.52 (dq, *J* = 8.6, 4.8, 3.6 Hz, 1H), 0.44 (tt, *J* = 8.9, 4.9 Hz, 1H), 0.12 (tt, *J* = 9.0, 5.1 Hz, 1H), -0.03 (d, *J* = 4.8 Hz, 1H), -0.32 (dq, *J* = 9.9, 5.1 Hz, 1H).

### Example 20:

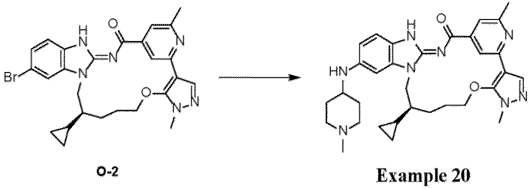

Compound **O-2** (100 mg), 4-atnino-1-methylpiperidine (26 mg), Pd₂(dba)₃ (43 mg), *2-(di-tert-*butylphosphine)biphenyl (28 mg), and potassium *tert*-butoxide (126 mg) were added to *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 20** (35 mg). ESI-MS: m/z = 569.45[M+H] ⁺.

¹H NMR (500 MHz, DMSO-*d*₆) δ 12.35 (s, 1H), 8.33 (s, 1H), 7.90 (s, 1H), 7.54 (s, 1H), 7.23 (d, *J* = 8.5 Hz, 1H), 6.71 (d, *J=* 2.1 Hz, 1H), 6.53 (dd, *J=* 8.8, 1.9 Hz, 1H), 5.35 (s, 1H), 4.32 - 4.26 (m, 1H), 4.22 (dd, *J=* 13.9, 3.8 Hz, 1H), 4.03 (td, *J=* 13.9, 13.1, 7.1 Hz, 2H), 3.73 (s, 3H), 3.24 (d, *J=* 9.9 Hz, 1H), 2.75 (d, *J=* 11.4 Hz, 2H), 2.54 (s, 3H), 2.26 - 2.21 (m, 1H), 2.17 (s, 3H), 2.12 (dq, *J* = 9.2, 4.3 Hz, 1H), 2.07 - 1.98 (m, 3H), 1.90 (t, *J* = 13.1 Hz, 2H), 1.81 (s, 1H), 1.72 (dd, *J=* 13.6, 6.4 Hz, 1H), 1.39 (dtd, *J* = 24.7, 11.5, 11.1, 5.6 Hz, 2H), 0.59 (dq, *J=* 8.5, 5.1, 3.5 Hz, 1H), 0.33 (td, *J* = 9.1, 5.0 Hz, 1H), -0.04 (dt, *J* = 9.4, 4.6 Hz, 1H), -0.13 (dp, *J* = 9.5, 4.6 Hz, 1H), -0.54 (dq, *J* = 9.6, 4.8 Hz, 1H).

### Example 21:

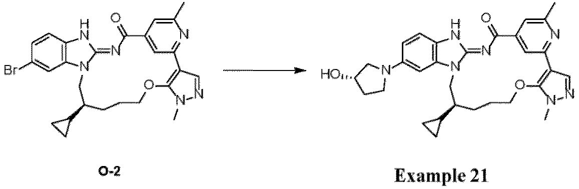

Compound **O-2** (60 mg), (*S*)-3-pyrrolidinol hydrochloride (17 mg), Pd₂(dba)₃ (25 mg), 2-(di*-tert-*butylphosphine)biphenyl (16 mg), and potassium *tert*-butoxide (75 mg) were added to *tert*-amyl alcohol (10 mL), and the mixture was purged with nitrogen 3 times, and reacted at 85-95 °C for 3 h. After the reaction was completed, the mixture was cooled to room temperature and filtered under vacuum to remove the palladium catalyst. 30 mL of water was added to the filtrate, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 21** (25 mg). ESI-MS: m/z = 542.41[M+H] ⁺.

¹H NMR (500 MHz, DMSO-*d*₆) δ 12.41 (s, 1H), 8.35 (s, 1H), 7.91 (s, 1H), 7.55 (s, 1H), 7.34 (d, *J =* 8.6 Hz, 1H), 6.69 (d, *J* = 2.2 Hz, 1H), 6.47 (dd, *J* = 8.7, 2.2 Hz, 1H), 4.98 (s, 1H), 4.43 (dq, *J=* 7.0, 3.4 Hz, 1H), 4.29 (q, *J* = 8.2, 7.6 Hz, 1H), 4.22 (dd, *J* = 14.0, 3.7 Hz, 1H), 4.14 (dd, *J* = 13.8, 9.1 Hz, 1H), 4.04 (dt, *J* = 7.0, 4.6 Hz, 1H), 3.73 (s, 3H), 3.46 (dd, *J* = 10.0, 5.0 Hz, 1H), 3.12 (dd, *J* = 10.0, 2.5 Hz, 1H), 2.54 (s, 3H), 2.25 - 2.18 (m, 1H), 2.16 - 2.06 (m, 2H), 2.03 - 1.98 (m, 2H), 1.94 - 1.84 (m, 2H), 1.72 (dt, *J* = 13.6, 6.1 Hz, 1H), 1.50 - 1.42 (m, 1H), 0.63 (dd, *J* = 9.8, 5.3 Hz, 1H), 0.31 (tt, *J* = 9.3, 4.6 Hz, 1H), -0.05 (dt, *J =* 9.5, 4.7 Hz, 1H), -0.14 (tt, *J =* 9.1, 4.6 Hz, 1H), -0.56 (dd, *J* = 9.5, 5.0 Hz, 1H).

### Example 22:

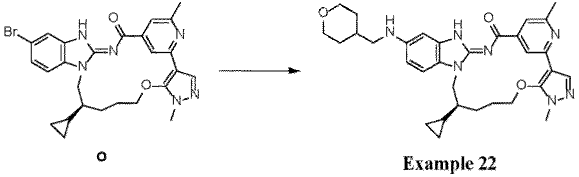

Compound **O** (70 mg), 4-aminomethyl tetrahydropyran (23 mg), Pd₂(dba)₃ (30 mg), 2-(di-*tert-*butylphosphine)biphenyl (20 mg), and potassium *tert*-butoxide (88 mg) were added to *tert-*amyl alcohol (5 mL). The mixture was purged with nitrogen 3 times and reacted at 90 °C for 2 h. After the reaction was completed, 50 mL of water was added to the reaction solution, and the resulting mixture was extracted with ethyl acetate (50 mL × 3). The organic phases were combined, dried over anhydrous sodium sulfate, concentrated until no liquid flowed out, and purified by preparative liquid phase chromatography to give **Example 22** (35 mg). ESI-MS: m/z = 570.41[M+H] ⁺. ¹H NMR (500 MHz, DMSO-*d*₆) δ 12.37 (s, 1H), 8.34 (s, 1H), 7.90 (s, 1H), 7.54 (s, 1H), 7.23 (d, *J =* 8.6 Hz, 1H), 6.71 (d, *J* = 2.3 Hz, 1H), 6.55 (dd,J= 8.6, 2.0 Hz, 1H), 4.28 (q, *J* = 7.7 Hz, 1H), 4.22 (dd, *J* = 14.0, 3.6 Hz, 1H), 4.10 - 4.01 (m, 2H), 3.89 - 3.83 (m, 2H), 3.73 (s, 3H), 3.25 (td, *J* = 9.3, 4.6 Hz, 2H), 2.96 (qd, *J* = 12.7, 6.7 Hz, 2H), 2.54 (s, 3H), 2.21 (q, *J* = 7.7, 6.4 Hz, 1H), 2.13 (dq, *J* = 14.2, 7.7, 6.2 Hz, 1H), 2.05 - 1.97 (m, 1H), 1.87 - 1.77 (m, 2H), 1.75 - 1.66 (m, 3H), 1.29 - 1.20 (m, 2H), 0.61 (tq, *J* = 8.4, 4.4, 3.4 Hz, 1H), 0.32 (dp, *J* = 9.4, 4.7 Hz, 1H), -0.04 (dt, *J* = 9.7, 4.8 Hz, 1H), -0.13 (tt, *J* = 9.3, 4.7 Hz, 1H), -0.57 (dq, *J* = 9.6, 4.8 Hz, 1H).

### Example 23/24:

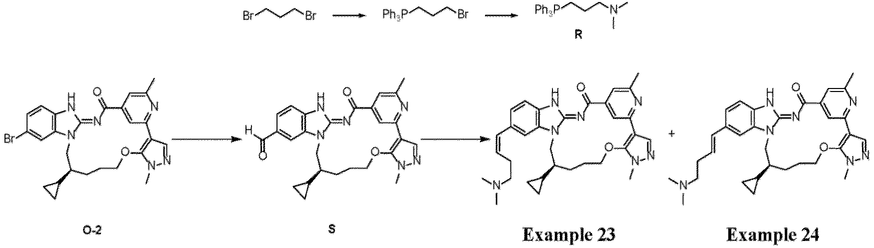

### (1) Preparation method for compound (3-bromopropyl)triphenylphosphine:

1,3-Dibromopropane (90 mmol), triphenylphosphine (25 g), and toluene (260 mL) were sequentially added to a reaction flask, and the mixture was reacted at 110 °C for 4 h. The reaction solution was filtered under vacuum. The filter cake was rinsed with 40 mL of cyclohexane to give compound **(3-bromopropyl)triphenylphosphine,** which was directly used in the next step without further purification. ESI-MS: m/z = 383.01[M]⁺.

### (2) Preparation method for compound R:

Compound (3-bromopropyl)triphenylphosphine (5 g), methanol (26 mL), and dimethylamine (40 mmol) were sequentially added to a reaction flask, and the mixture was stirred at room temperature for 1 h, and then heated to 65 °C and reacted for 4 h. The reaction solution was concentrated until no liquid flowed out to give compound **R** (4.0 g), which was directly used in the next step without further purification. ESI-MS: m/z = 348.40[M]⁺.

### (3) Preparation method for compound S:

Compound **O-2** (165 mg), *N*-formyl saccharin (131 mg), palladium acetate (7mg), 1,4-bis(diphenylphosphino)butane (20 mg), triethylsilane (58 mg), and sodium carbonate (66 mg) were added to *N*,*N*-dimethylformamide (5 mL). The mixture was purged with nitrogen 3 times and reacted at 80 °C for 4 h. The reaction solution was poured into 20 mL of water, and the resulting mixture was extracted with ethyl acetate (10 mL × 3). The organic phases were combined, dried over anhydrous sodium sulfate, and concentrated until no liquid flowed out to give compound **S,** which was directly used in the next step without further purification. ESI-MS: m/z = 485.33 [M+H]⁺.

### (4) Preparation method for Example 23/24:

Compound **R** (323 mg) was added to tetrahydrofuran (5 mL), and the mixture was purged with nitrogen 3 times and cooled to -78 °C. *n*-Butyllithium (0.5 mL) was added, and the mixture was stirred at -78 °C for 3 h. A solution of compound S obtained in the previous step in tetrahydrofuran (5 mL) was added to the reaction solution, and the mixture was gradually heated to 60 °C and reacted for 3 h. After the reaction was completed, the mixture was poured into 20 mL of ice water, and the resulting mixture was concentrated by rotary evaporation under reduced pressure to remove tetrahydrofuran, and extracted with dichloromethane (50 mL × 3). The organic phases were combined, dried over anhydrous sodium sulfate, concentrated until no liquid flowed out, and purified by preparative liquid phase chromatography to give **Example 23** (15 mg), ESI-MS: m/z = 554.46[M+H] ⁺ and **Example 24** (20 mg), ESI-MS: m/z = 554.46[M+H] ⁺. Example 23: ¹H NMR (500 MHz, DMSO-*d*₆) 8.41 (s, 1H), 8.25 (s, 1H), 7.96 (s, 1H), 7.60 (dd, *J* = 9.1, 1.3 Hz, 2H), 7.56 (d, *J =* 8.2 Hz, 1H), 7.21 (dd, *J =* 8.3, 1.4 Hz, 1H), 6.61 (dd, *J =* 11.7, 2.1 Hz, 1H), 5.72 (dt, *J* = 11.6, 6.6 Hz, 1H), 4.38 - 4.32 (m, 2H), 4.26 - 4.22 (m, 1H), 4.10 (d, *J* = 5.6 Hz, 1H), 3.78 (s, 3H), 2.60 (s, 3H), 2.52 - 2.45 (m, 2H), 2.22 (s, 6H), 2.05 (ddt, *J =* 14.1, 10.8, 5.0 Hz, 2H), 1.90 (s, 1H), 1.81 - 1.72 (m, 1H), 1.34 - 1.25 (m, 3H), 0.68 (dd, *J* = 11.7, 7.1 Hz, 1H), 0.34 (dq, *J* = 8.9, 4.7 Hz, 1H), -0.00 (dq, *J* = 9.7, 5.0 Hz, 1H), - 0.19 (tt, *J* = 9.1, 4.7 Hz, 1H), -0.63 (dq, *J* = 9.7, 4.8 Hz, 1H). Example 24: ¹H NMR (500 MHz, DMSO-*d*₆) 8.43 (s, 1H), 8.34 (s, 1H), 7.99 (s, 1H), 7.77 (d, *J* = 1.5 Hz, 1H), 7.63 (d, *J* = 1.2 Hz, 1H), 7.53 (d, *J* = 8.2 Hz, 1H), 7.32 (dd, *J* = 8.4, 1.5 Hz, 1H), 6.60 (d, *J* = 15.8 Hz, 1H), 6.39 (dt, *J* = 15.8, 6.8 Hz, 1H), 4.39 - 4.33 (m, 2H), 4.29 - 4.26 (m, 1H), 4.14 - 4.10 (m, 1H), 3.80 (s, 3H), 3.45 (t, *J=* 6.5 Hz, 2H), 2.62 (s, 3H), 2.55 (d, *J* = 7.3 Hz, 1H), 2.46 (t, *J* = 7.5 Hz, 2H), 2.32 (s, 6H), 2.08 (dt, *J* = 13.0, 4.6 Hz, 1H), 1.96 (d, *J* = 9.5 Hz, 1H), 1.83 - 1.75 (m, 1H), 1.64 - 1.54 (m, 1H), 0.71 (dq, *J* = 9.8, 5.3, 4.2 Hz, 1H), 0.37 (dq, *J* = 9.4, 5.0 Hz, 1H), 0.01 (dt, *J* = 9.7, 5.0 Hz, 1H), -0.18 (dq, *J* = 9.0, 4.7 Hz, 1H), -0.59 (dq, *J=* 9.5, 4.8 Hz, 1H).

### Example 25/26:

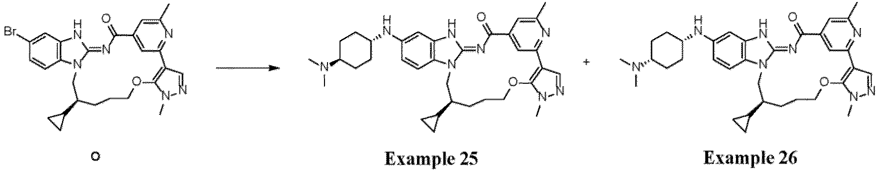

Compound **O** (100 mg), *N*,*N*-dimethyl-1,4-cyclohexanediamine (30 mg), Pd₂(dba)₃ (43 mg), 2-(di-*tert-*butylphosphine)biphenyl (28 mg), and potassium *tert*-butoxide (126 mg) were added to *tert-*amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and subjected to preparative chromatography (liquid phase chromatography conditions: column: YMC-TA-C18 250 × 50 mm, 10 µm; mobile phase: A: 0.1% formic acid/water, C: acetonitrile, gradient: 20% C-50% C (0-60 min); flow rate: 60 mL/min; wavelength: 254 nm) to give **Example 25** (13 mg, retention time: 14.2 min), ESI-MS: m/z = 597.44[M+H] ⁺, and **Example 26** (19 mg, retention time: 19.6 min), ESI-MS: m/z = 597.44[M+H] ⁺. Example 25: ¹H NMR (500 MHz, chloroform-*d*) δ 12.15 (s, 1H), 8.51 (s, 1H), 8.41 (s, 1H), 8.15 (s, 1H), 7.70 (s, 1H), 7.15 (d, *J* = 8.5 Hz, 1H), 6.54 (d, *J* = 7.8 Hz, 2H), 4.40 - 4.32 (m, 2H), 3.95 (p, *J* = 5.9 Hz, 2H), 3.79 (s, 3H), 3.16 (d, *J* = 12.0 Hz, 1H), 2.92 (d, *J* = 12.4 Hz, 1H), 2.62 (d, *J* = 5.1 Hz, 3H), 2.60 (s, 6H), 2.27 (d, *J* = 12.3 Hz, 3H), 2.17 - 2.09 (m, 3H), 1.83 - 1.74 (m, 2H), 1.48 (t, *J* = 10.5 Hz, 2H), 1.26 (t, *J* = 6.0 Hz, 2H), 0.87 (dt, *J=* 14.0, 7.0 Hz, 1H), 0.57 - 0.48 (m, 1H), 0.45 (dq, *J* = 10.0, 5.1 Hz, 1H), 0.12 (dd, *J=* 10.2, 5.3 Hz, 1H), -0.02 (s, 1H), -0.31 (dq, *J* = 10.2, 5.0 Hz, 1H). Example 26: ¹H NMR (500 MHz, chloroform-*d*) δ 11.98 (s, 1H), 8.53 (s, 1H), 8.39 (s, 1H), 8.15 (s, 1H), 7.67 (s, 1H), 7.15 (d, *J* = 8.6 Hz, 1H), 6.72 (dd, *J* = 8.7, 2.1 Hz, 1H), 6.60 (d, *J* = 2.1 Hz, 1H), 4.39 - 4.32 (m, 2H), 3.94 (dq, *J* = 13.9, 7.4, 6.2 Hz, 2H), 3.78 (s, 3H), 3.69 - 3.64 (m, 1H), 2.83 (d, *J* = 11.1 Hz, 1H), 2.69 (s, 6H), 2.64 (s, 3H), 2.31 - 2.25 (m, 1H), 2.22 - 2.15 (m, 2H), 2.12 - 2.06 (m, 2H), 1.97 (d, *J* = 21.7 Hz, 4H), 1.82 - 1.73 (m, 2H), 1.68 - 1.60 (m, 2H), 0.52 (d, *J* = 12.0 Hz, 1H), 0.44 (dq, *J=* 8.9, 4.5, 4.1 Hz, 1H), 0.17 - 0.09 (m, 1H), -0.04 (q, *J* = 4.8 Hz, 1H), -0.31 (dd, *J* = 9.8, 5.3 Hz, 1H).

### Example 27:

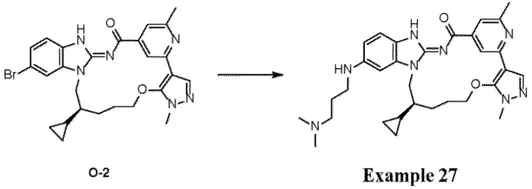

Compound **O-2** (60 mg), 3-dimethylaminopropylamine (14 mg), Pd₂(dba)₃ (25 mg), 2-(di-*tert-*butylphosphine)biphenyl (17 mg), and potassium *tert*-butoxide (75 mg) were added to *tert*-amyl alcohol (3 mL). The mixture was purged with nitrogen 3 times and reacted at 100 °C for 2 h. After the reaction was completed, a 1 M aqueous sodium bicarbonate solution (10 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 27** (20 mg). ESI-MS: m/z = 279.36[1/2M+H] ⁺.

¹H NMR (500 MHz, DMSO-*d*₆) δ 12.37 (s, 1H), 8.34 (s, 1H), 7.91 (s, 1H), 7.54 (d, *J* = 1.2 Hz, 1H), 7.25 (d, *J* = 8.6 Hz, 1H), 6.70 (d, *J* = 2.0 Hz, 1H), 6.53 (dd, *J* = 8.6, 2.0 Hz, 1H), 4.29 (td, *J* = 8.4, 5.8 Hz, 1H), 4.22 (dd, *J* = 14.0, 3.6 Hz, 1H), 4.08 - 4.00 (m, 2H), 3.73 (s, 3H), 3.07 (t, *J* = 6.9 Hz, 2H), 2.54 (d, *J* = 1.2 Hz, 4H), 2.33 (t, *J* = 7.1 Hz, 2H), 2.22 (dt, *J* = 12.4, 6.9 Hz, 1H), 2.15 (s, 6H), 2.00 (td, *J* = 7.8, 4.6 Hz, 1H), 1.84 (d, *J* = 9.3 Hz, 1H), 1.71 (h, *J* = 6.7 Hz, 3H), 0.60 (td, *J* = 8.2, 3.5 Hz, 1H), 0.32 (tt, *J* = 9.3, 4.6 Hz, 1H), -0.05 (dt, *J* = 9.4, 4.6 Hz, 1H), -0.12 (tt, *J* = 7.6, 4.4 Hz, 1H), -0.57 (dq, *J* = 9.5, 4.7 Hz, 1H).

### Example 28:

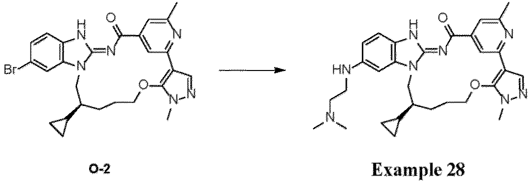

Compound **O-2** (60 mg), *N,N-*dimethylethylenediatnine (12 mg), Pd₂(dba)₃ (25 mg), 2-(di-*tert-*butylphosphine)biphenyl (17 mg), and potassium *tert*-butoxide (75 mg) were added to *tert*-amyl alcohol (3 mL). The mixture was purged with nitrogen 3 times and reacted at 100 °C for 2 h. After the reaction was completed, a 1 M aqueous sodium bicarbonate solution (10 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 28** (25 mg). ESI-MS: m/z = 272.30[1/2M+H] ⁺.

¹H NMR (500 MHz, DMSO-*d*₆) δ 12.43 (s, 1H), 8.38 (s, 1H), 7.95 (s, 1H), 7.59 (s, 1H), 7.30 (d, *J* = 8.5 Hz, 1H), 6.81 (d, *J* = 2.1 Hz, 1H), 6.61 (dd, *J* = 8.5, 2.0 Hz, 1H), 5.41 (s, 1H), 4.33 (q, *J* = 8.1 Hz, 1H), 4.27 (dd, *J* = 14.1, 3.7 Hz, 1H), 4.09 (td, *J* = 9.5, 4.5 Hz, 2H), 3.77 (s, 3H), 3.20 (t, *J* = 5.3 Hz, 2H), 2.59 (s, 3H), 2.53 (t, *J* = 6.9 Hz, 2H), 2.28 (s, 1H), 2.25 (s, 6H), 2.18 (td, *J* = 9.6, 4.9 Hz, 1H), 2.05 (tt, *J=* 13.9, 10.5, 5.0 Hz, 1H), 1.89 (d, *J* = 9.5 Hz, 1H), 1.76 (tt, *J* = 13.2, 5.6 Hz, 1H), 0.65 (qd, *J* = 8.4, 4.2 Hz, 1H), 0.37 (dp, *J* = 9.2, 4.7 Hz, 1H), 0.00 (dq, *J* = 9.6, 5.0 Hz, 1H), -0.07 (dp, *J* = 9.4, 4.7 Hz, 1H), -0.51 (dt, *J* = 9.6, 4.9 Hz, 1H).

### Example 29:

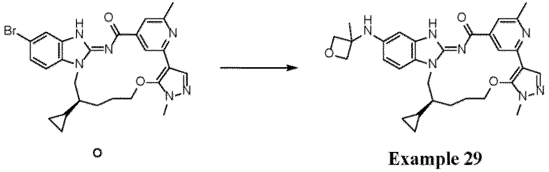

Compound **O** (100 mg), 3-methyloxetane-3-amine (20 mg), Pd₂(dba)₃ (43 mg), 2-(di-*tert*-butylphosphine)biphenyl (28 mg), and potassium *tert*-butoxide (126 mg) were added to *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 29** (20 mg). ESI-MS: m/z = 542.37[M+H] ⁺.

¹H NMR (500 MHz, DMSO-*d*₆) δ 12.38 (s, 1H), 8.33 (s, 1H), 7.90 (s, 1H), 7.54 (s, 1H), 7.29 (d, *J* = 8.5 Hz, 1H), 6.40 (dd, *J=* 8.6, 2.1 Hz, 1H), 6.33 (d, *J=* 2.1 Hz, 1H), 6.11 (s, 1H), 4.63 (dd, *J* = 15.9, 5.8 Hz, 2H), 4.52 (d, *J* = 5.8 Hz, 2H), 4.33 - 4.25 (m, 1H), 4.22 (dd, *J* = 14.0, 3.4 Hz, 1H), 4.07 - 3.97 (m, 2H), 3.72 (s, 3H), 2.54 (s, 3H), 2.22 (dd, *J* = 13.6, 6.9 Hz, 1H), 2.12 (td, *J* = 11.3, 10.2, 5.5 Hz, 1H), 2.05 - 1.95 (m, 1H), 1.81 - 1.69 (m, 2H), 1.58 (s, 3H), 0.57 (tt, *J* = 8.7, 3.6 Hz, 1H), 0.33 (dp, *J* = 9.3, 4.7 Hz, 1H), -0.05 (dt, *J* = 9.7, 4.8 Hz, 1H), -0.13 (dp, *J* = 9.5, 4.7 Hz, 1H), -0.60 (dq, *J* = 9.7, 4.8 Hz, 1H).

### Example 30:

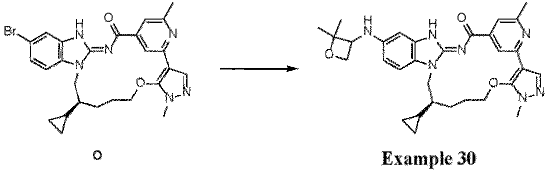

Compound O (50 mg), 2,2-dimethyloxetan-3-amine (19 mg), Pd₂(dba)₃ (21 mg), 2-(di-*tert*-butylphosphine)biphenyl (14 mg), and potassium *tert*-butoxide (63 mg) were added to *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 30** (10 mg). ESI-MS: m/z = 556.37[M+H] ⁺.

¹H NMR (500 MHz, DMSO-*d*₆) δ 12.40 (s, 1H), 8.33 (s, 1H), 7.90 (s, 1H), 7.54 (d, *J* = 1.2 Hz, 1H), 7.28 (d, *J* = 8.5 Hz, 1H), 6.79 - 6.72 (m, 2H), 5.70 (d, *J=* 9.2 Hz, 1H), 4.44 (td, *J=* 5.9, 4.1 Hz, 2H), 4.29 (td, *J=* 8.7, 6.1 Hz, 1H), 4.23 (dd, *J* = 14.0, 3.5 Hz, 1H), 4.04 (t, *J* = 8.3 Hz, 2H), 3.97 (dd, *J* = 13.9, 9.6 Hz, 1H), 3.73 (s, 3H), 2.54 (s, 3H), 2.29 - 2.19 (m, 1H), 2.17 - 2.09 (m, 1H), 2.00 - 1.95 (m, 1H), 1.84 (t, *J* = 8.5 Hz, 1H), 1.77 - 1.67 (m, 1H), 1.46 (s, 3H), 1.44 (s, 3H), 0.55 (tt, *J=* 8.3, 3.1 Hz, 1H), 0.33 (dq, *J* = 9.5, 4.7 Hz, 1H), -0.04 (dt, *J* = 9.9, 4.9 Hz, 1H), -0.19 (dp, *J* = 9.7, 4.8 Hz, 1H), -0.62 (dq, *J* = 9.7, 4.9 Hz, 1H).

### Example 31:

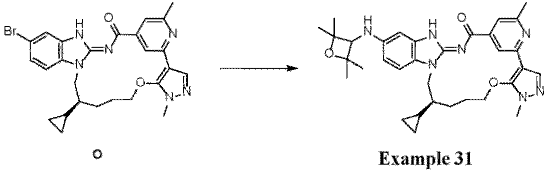

Compound **O** (70 mg), 2,2,4,4-tetramethyloxabutan-3-amine hydrochloride (43 mg), Pd₂(dba)₃ (24 mg), *2-(di-tert-*butylphosphine)biphenyl (16 mg), and potassium *tert*-butoxide (88 mg) were added to *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 31** (10 mg). ESI-MS: m/z = 584.34[M+H] ⁺. ¹H NMR (500 MHz, DMSO-*d*₆) δ 12.42 (s, 1H), 8.33 (s, 1H), 7.90 (s, 1H), 7.54 (d, *J* = 1.2 Hz, 1H), 7.28 (d, *J* = 8.5 Hz, 1H), 6.78 - 6.70 (m, 2H), 5.76 (d, *J=* 9.2 Hz, 1H), 4.29 (td, *J=* 8.7, 6.1 Hz, 1H), 4.23 (dd, *J=* 14.0, 3.5 Hz, 1H), 4.04 (t, *J* = 8.3 Hz, 2H), 3.97 (dd, *J* = 13.9, 9.6 Hz, 1H), 3.73 (s, 3H), 2.54 (s, 3H), 2.29 - 2.19 (m, 1H), 2.17 - 2.09 (m, 1H), 2.00 - 1.95 (m, 1H), 1.84 (t, *J=* 8.5 Hz, 1H), 1.77 - 1.67 (m, 1H), 1.46 (s, 3H), 1.44 (s, 3H), 1.30 (s, 3H), 1.19 (s, 3H), 0.55 (tt, *J=* 8.3, 3.1 Hz, 1H), 0.33 (dq, *J* = 9.5, 4.7 Hz, 1H), -0.04 (dt, *J* = 9.9, 4.9 Hz, 1H), -0.19 (dp, *J* = 9.7, 4.8 Hz, 1H), -0.62 (dq, *J* = 9.7, 4.9 Hz, 1H).

### Example 32:

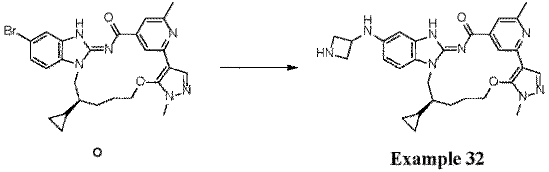

Compound **O** (70 mg), 1-*tert*-butoxycarbonyl-3-aminocyclobutylamine (45 mg), Pd₂(dba)₃ (24 mg), 2-(di-*tert-*butylphosphine)biphenyl (16 mg), and potassium *tert*-butoxide (88 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, and concentrated until no liquid flowed out. The crude product obtained by concentrating the filtrate was directly dissolved in hydrogen chloride-methanol solution (4 M, 5 mL), and the mixture was stirred for 30 min. After the reaction was completed as monitored by LC-MS, the reaction solution was concentrated and purified by preparative liquid phase chromatography to give **Example 32** (16 mg). ESI-MS: m/z = 527.37 [M+H] ⁺.

¹H NMR (500 MHz, DMSO-*d*₆) δ 8.38 (s, 1H), 8.33 (s, 1H), 7.90 (s, 1H), 7.54 (s, 1H), 7.30 (d, *J=* 8.5 Hz, 1H), 6.68 - 6.62 (m, 1H), 6.51 (dd, *J* = 8.5, 2.0 Hz, 1H), 6.43 (s, 1H), 4.35 (s, 1H), 4.29 (t, *J=* 4.3 Hz, 1H), 4.19 (dd, *J* = 14.6, 5.6 Hz, 2H), 4.10 - 4.01 (m, 2H), 3.73 (s, 3H), 3.67 (td, *J=* 9.8, 4.8 Hz, 2H), 2.54 (s, 3H), 2.26 - 2.18 (m, 1H), 2.13 (dq, *J* = 13.1, 7.2, 6.1 Hz, 1H), 1.99 (tt, *J=* 12.4, 5.5 Hz, 2H), 1.82 (d, *J* = 9.6 Hz, 1H), 1.71 (q, *J* = 9.0, 6.1 Hz, 1H), 0.60 (dt, *J* = 9.6, 4.6 Hz, 1H), 0.31 (dt, *J* = 9.2, 4.5 Hz, 1H), -0.05 (dq, *J* = 9.9, 5.0 Hz, 1H), -0.18 (tt, *J* = 9.4, 4.7 Hz, 1H), -0.62 (dq, *J* = 9.5, 4.8 Hz, 1H).

### Example 33:

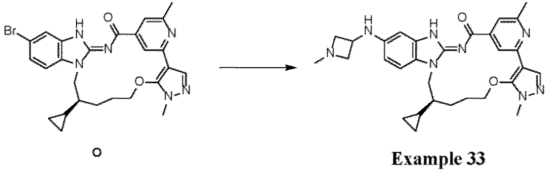

Compound **O** (50 mg), 1-methylazetidin-3-amine (30 mg), Pd₂(dba)₃ (21 mg), 2-(di-*tert*-butylphosphine)biphenyl (14 mg), and potassium *tert*-butoxide (63 mg) were added to *tert-*amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 33** (10 mg). ESI-MS: m/z = 541.41[M+H] ⁺.

¹H NMR (500 MHz, chloroform-*d*) δ 12.30 (s, 1H), 8.49 (s, 1H), 8.45 (s, 1H), 8.17 (s, 1H), 7.80 (s, 1H), 7.15 (d, *J* = 8.6 Hz, 1H), 6.65 (dd, *J* = 8.7, 2.0 Hz, 1H), 6.30 (d, *J* = 2.1 Hz, 1H), 4.40 - 4.32 (m, 2H), 4.06 - 4.00 (m, 1H), 3.95 - 3.90 (m, 2H), 3.81 (s, 1H), 3.79 (s, 3H), 3.67 (t, *J =* 8.7 Hz, 1H), 2.66 (s, 3H), 2.64 (s, 3H), 2.28 - 2.10 (m, 3H), 1.78 (d, *J* = 14.6 Hz, 2H), 1.28 (dd, *J=* 18.1, 8.7 Hz, 2H), 0.49 (td, *J* = 9.0, 8.5, 4.1 Hz, 1H), 0.41 (dq, *J* = 8.7, 4.5, 4.0 Hz, 1H), 0.10 - 0.03 (m, 1H), -0.06 (dt, *J=* 9.7, 5.2 Hz, 1H), -0.40 (dq, *J=* 10.0, 5.1 Hz, 1H).

### Example 34:

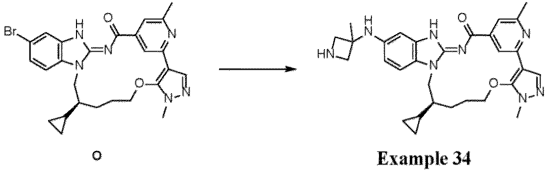

Compound **O** (70 mg), 1-Boc-3-amino-3-methylacridine (31 mg), Pd₂(dba)₃ (24 mg), *2-(di-tert-*butylphosphine)biphenyl (16 mg), and potassium *tert*-butoxide (88 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, and concentrated until no liquid flowed out. The crude product obtained by concentrating the filtrate was directly dissolved in hydrogen chloride-methanol solution (4 M, 5 mL), and the mixture was stirred for 30 min. After the reaction was completed as monitored by LC-MS, the reaction solution was concentrated and purified by preparative liquid phase chromatography to give **Example 34** (6 mg). ESI-MS: m/z = 541.21 [M+H] ⁺.

¹H NMR (500 MHz, DMSO-*d*₆) δ 8.34 (s, 1H), 8.29 (s, 2H), 7.91 (s, 1H), 7.54 (d, *J* = 1.2 Hz, 1H), 7.31 (d, *J* = 8.4 Hz, 1H), 6.50 - 6.43 (m, 2H), 6.19 (s, 1H), 4.32 - 4.27 (m, 1H), 4.22 (dd, *J=* 14.0, 3.6 Hz, 1H), 4.16 - 4.11 (m, 1H), 4.06 (s, 1H), 3.92 (s, 2H), 3.73 (s, 3H), 2.64 (s, 3H), 2.28 - 2.19 (m, 1H), 2.17 - 2.10 (m, 1H), 2.02 - 1.97 (m, 1H), 1.81 (s, 1H), 1.73 (t, *J* = 10.7 Hz, 1H), 1.57 (s, 3H), 1.24 (t, *J* = 4.6 Hz, 2H), 0.65 - 0.57 (m, 1H), 0.32 (dt, *J* = 10.4, 5.3 Hz, 1H), -0.02 - -0.08 (m, 1H), -0.19 (d, *J* = 9.4 Hz, 1H), -0.65 (dd, *J=* 9.6, 5.0 Hz, 1H).

### Example 35:

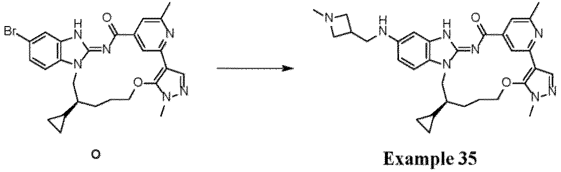

Compound **O** (60 mg), (1-methylpyrrolidin-3-yl)methylatnine (14 mg), Pd₂(dba)₃ (26 mg), 2-(di-*tert-*butylphosphine)biphenyl (17 mg), and potassium *tert*-butoxide (75 mg) were added to *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 35** (20 mg). ESI-MS: m/z = 278.66[1/2M+H] ⁺.

¹H NMR (500 MHz, DMSO-*d*₆) δ 12.40 (s, 1H), 8.34 (s, 1H), 8.24 (d, *J* = 2.3 Hz, 1H), 7.91 (d, *J* = 2.3 Hz, 1H), 7.54 (s, 1H), 7.25 (d, *J* = 8.5 Hz, 1H), 6.74 (s, 1H), 6.54 (d, *J* = 8.5 Hz, 1H), 4.30 (t, *J* = 7.9 Hz, 1H), 4.21 (dd, *J* = 14.2, 3.7 Hz, 1H), 4.05 (dt, *J* = 9.5, 6.0 Hz, 2H), 3.73 (s, 3H), 3.53 (t, *J* = 7.8 Hz, 2H), 3.28 - 3.21 (m, 4H), 2.78 - 2.70 (m, 1H), 2.54 (s, 3H), 2.39 (s, 3H), 2.22 (s, 1H), 2.14 (s, 1H), 2.00 (d, *J=* 9.9 Hz, 1H), 1.86 (s, 1H), 1.70 (d, *J* = 14.3 Hz, 1H), 0.61 (q, *J* = 7.2, 5.4 Hz, 1H), 0.32 (dq, *J* = 9.3, 4.8 Hz, 1H), -0.05 (dd, *J* = 9.7, 4.9 Hz, 1H), -0.14 (dq, *J* = 9.2, 4.8 Hz, 1H), -0.59 (dd, *J* = 9.8, 5.2 Hz, 1H).

### Example 36:

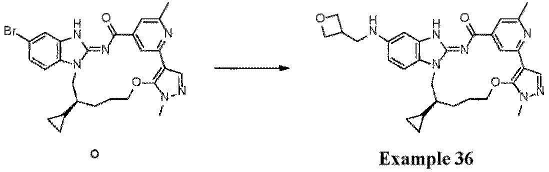

Compound **O** (70 mg), 3-aminomethyloxetane (14 mg), Pd₂(dba)₃ (24 mg), 2-(di-*tert*-butylphosphine)biphenyl (16 mg), and potassium *tert*-butoxide (88 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 36** (32 mg). ESI-MS: m/z = 542.35 [M+H] ⁺.

¹H NMR (500 MHz, DMSO-*d*₆) δ 12.39 (s, 1H), 8.34 (s, 1H), 7.91 (s, 1H), 7.54 (d, *J* = 1.2 Hz, 1H), 7.25 (d, *J* = 8.5 Hz, 1H), 6.76 (d, *J* = 2.1 Hz, 1H), 6.53 (dd, *J* = 8.6, 2.0 Hz, 1H), 4.68 (ddd, *J* = 7.5, 5.9, 1.6 Hz, 2H), 4.34 (td, *J* = 5.9, 2.2 Hz, 2H), 4.29 (dt, *J* = 8.4, 4.2 Hz, 1H), 4.22 (dd, *J* = 14.0, 3.6 Hz, 1H), 4.10 - 4.01 (m, 2H), 3.73 (s, 3H), 3.36 (d, *J* = 7.4 Hz, 2H), 3.24 - 3.19 (m, 1H), 2.54 (s, 3H), 2.21 (dd, *J* = 13.0, 6.9 Hz, 1H), 2.14 (dq, *J* = 9.7, 4.9, 4.3 Hz, 1H), 2.04 - 1.97 (m, 1H), 1.85 (d, *J=* 10.8 Hz, 1H), 1.76 - 1.67 (m, 1H), 0.67 - 0.58 (m, 1H), 0.32 (dp, *J* = 9.2, 4.5 Hz, 1H), -0.05 (dq, *J* = 9.5, 4.9 Hz, 1H), -0.14 (tt, *J* = 9.3, 4.7 Hz, 1H), -0.59 (dq, *J* = 9.7, 5.0 Hz, 1H).

### Example 37:

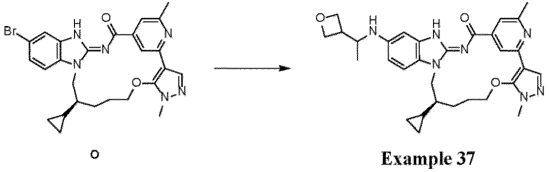

Compound **O** (70 mg), 1-(oxetanacyclo-3-yl)ethan-1-amine (16 mg), Pd₂(dba)₃ (24 mg), *2-(di-tert-*butylphosphine)biphenyl (16 mg), and potassium *tert*-butoxide (88 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 37** (10 mg). ESI-MS: m/z = 556.39[M+H] ⁺.

¹H NMR (500 MHz, DMSO-*d*₆) δ 12.40 (s, 1H), 8.36 (s, 1H), 7.93 (s, 1H), 7.57 (s, 1H), 7.26 (d, *J* = 8.5 Hz, 1H), 6.86 (d, *J* = 12.8 Hz, 1H), 6.58 (d, *J* = 8.3 Hz, 1H), 5.36 (dd, *J* = 23.0, 8.6 Hz, 1H), 4.65 (ddt, *J* = 18.1, 14.3, 7.2 Hz, 2H), 4.40 (p, *J* = 6.0 Hz, 2H), 4.36 - 4.28 (m, 1H), 4.24 (d, *J* = 13.7 Hz, 1H), 4.15 - 4.03 (m, 2H), 3.89 (q, *J* = 7.4, 6.6 Hz, 1H), 3.75 (s, 3H), 3.07 (p, *J* = 7.8 Hz, 1H), 2.56 (s, 3H), 2.24 (s, 1H), 2.16 (s, 1H), 2.05 - 1.99 (m, 1H), 1.86 (s, 1H), 1.79 - 1.69 (m, 1H), 1.05 (dd, *J* = 22.8, 6.2 Hz, 3H), 0.67 - 0.58 (m, 1H), 0.35 (dp, *J* = 9.2, 4.5 Hz, 1H), - 0.05 (dq,J= 9.5, 4.9 Hz, 1H), -0.14 (tt, *J* = 9.3, 4.7 Hz, 1H), -0.59 (dq, *J* = 9.7, 5.0 Hz, 1H).

### Example 38/39:

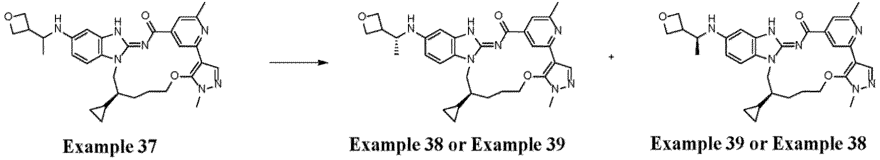

Example 37 (80 mg) was subjected to resolution (resolution conditions: column: YMC-SB 250 mm × 30 mm, 10 µm; mobile phase: A: n-hexane; B: ethanol, gradient: 20% C-70% B (0-80 min); flow rate: 20 mL/min; wavelength: 254 nm) by preparative chromatography to give **Example 38** (27 mg, retention time: 31.0 min), ESI-MS: m/z = 556.39[M+H] ⁺, and **Example 39** (30 mg, retention time: 43.5 min), ESI-MS: m/z = 556.39 [M+H] ⁺. **Example 38:** ¹H NMR (500 MHz, DMSO-*d*₆) δ 12.38 (s, 1H), 8.34 (s, 1H), 7.91 (s, 1H), 7.55 (d, *J* = 1.2 Hz, 1H), 7.33 (d, *J* = 8.6 Hz, 1H), 6.80 (d, *J* = 2.1 Hz, 1H), 6.62 (dd, *J* = 8.8, 2.2 Hz, 1H), 5.38 (d, *J* = 8.8 Hz, 1H), 4.62 (ddd, *J* = 10.9, 7.9, 6.1 Hz, 2H), 4.38 (td, *J* = 6.2, 4.5 Hz, 2H), 4.29 (q, *J* = 7.7 Hz, 1H), 4.22 (dd, *J* = 13.9, 3.5 Hz, 1H), 4.07 - 3.98 (m, 2H), 3.73 (s, 3H), 3.70 (dt, *J* = 8.9, 6.2 Hz, 1H), 3.05 (dtd, *J* = 14.4, 8.1, 6.3 Hz, 1H), 2.54 (s, 3H), 2.21 (p, *J* = 6.7, 6.3 Hz, 1H), 2.13 (dq, *J* = 13.8, 8.3, 6.7 Hz, 1H), 2.02 - 1.95 (m, 1H), 1.85 - 1.77 (m, 1H), 1.69 (h, *J* = 7.2 Hz, 1H), 1.04 (d, *J* = 6.2 Hz, 3H), 0.63 - 0.55 (m, 1H), 0.31 (dq, *J* = 9.3, 5.1 Hz, 1H), -0.06 (dq, *J* = 9.5, 4.8 Hz, 1H), -0.15 (dp, *J* = 9.4, 4.6 Hz, 1H), -0.58 (dq, *J* = 9.7, 4.9 Hz, 1H). **Example 39:** ¹H NMR (500 MHz, DMSO-*d*₆) δ 12.38 (s, 1H), 8.34 (s, 1H), 7.91 (s, 1H), 7.55 (s, 1H), 7.33 (d, *J* = 8.6 Hz, 1H), 6.89 - 6.73 (m, 1H), 6.62 (d, *J* = 8.7 Hz, 1H), 5.37 (d, *J* = 8.8 Hz, 1H), 4.63 (dt, *J* = 10.9, 6.9 Hz, 2H), 4.38 (td, *J=* 6.2, 3.7 Hz, 2H), 4.29 (q, *J=* 7.8 Hz, 1H), 4.22 (dd, *J* = 14.1, 3.5 Hz, 1H), 4.02 (td, *J* = 13.7, 12.1, 7.2 Hz, 2H), 3.73 (s, 3H), 3.69 (d, *J* = 7.5 Hz, 1H), 3.05 (q, *J* = 7.5 Hz, 1H), 2.54 (s, 3H), 2.26 - 2.17 (m, 1H), 2.13 (s, 1H), 2.03 - 1.94 (m, 1H), 1.81 (s, 1H), 1.69 (d, *J* = 14.8 Hz, 1H), 1.04 (d, *J* = 6.2 Hz, 3H), 0.59 (d, *J* = 9.7 Hz, 1H), 0.30 (tt, *J* = 9.3, 4.7 Hz, 1H), -0.06 (dq, *J* = 9.6, 4.9 Hz, 1H), -0.14 (dq, *J* = 13.3, 7.9, 6.2 Hz, 1H), -0.59 (dd, *J* = 9.8, 5.1 Hz, 1H).

### Example 40:

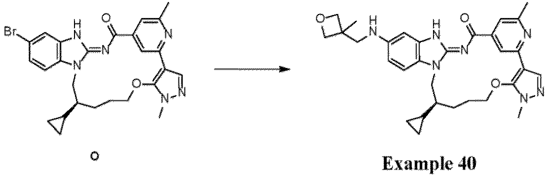

Compound **O** (70 mg), 3-methyl-3-atninomethyl-1-oxetane (16 mg), Pd₂(dba)₃ (24 mg), *2-(di-tert-*butylphosphine)biphenyl (16 mg), and potassium *tert*-butoxide (88 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 40** (41 mg). ESI-MS: m/z = 556.40[M+H] ⁺.

¹H NMR (500 MHz, DMSO-*d*₆) δ 12.40 (s, 1H), 8.35 (s, 1H), 7.92 (s, 1H), 7.55 (s, 1H), 7.25 (d, *J* = 8.5 Hz, 1H), 6.80 (d, *J* = 2.1 Hz, 1H), 6.62 (dd, *J* = 8.5, 2.0 Hz, 1H), 5.67 (s, 1H), 4.44 (d, *J* = 5.5 Hz, 2H), 4.31 - 4.19 (m, 4H), 4.06 (dd, *J* = 14.2, 9.1 Hz, 2H), 3.73 (s, 3H), 3.27 (d, *J* = 3.3 Hz, 2H), 2.55 (s, 3H), 2.22 (dt, *J* = 12.9, 6.8 Hz, 1H), 2.13 (tt, *J* = 8.8, 4.7 Hz, 1H), 1.99 (ddt, *J* = 14.2, 9.5, 5.0 Hz, 1H), 1.85 (p, *J* = 9.3 Hz, 1H), 1.72 (dd, *J* = 11.8, 6.6 Hz, 1H), 1.35 (s, 3H), 0.60 (tt, *J* = 8.5, 3.6 Hz, 1H), 0.31 (dp, *J* = 9.3, 4.6 Hz, 1H), -0.06 (dq, *J* = 9.7, 4.9 Hz, 1H), - 0.15 (dq, *J* = 9.1, 4.6 Hz, 1H), -0.60 (dq, *J* = 9.7, 4.8 Hz, 1H).

### Example 41:

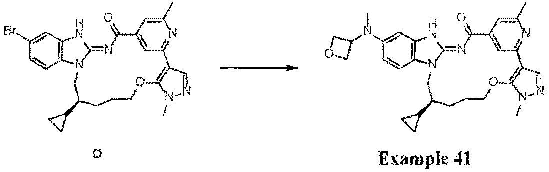

Compound **O** (70 mg), 3-methylaminooxacyclobutane (14 mg), Pd₂(dba)₃ (30 mg), 2-(di-*tert-*butylphosphine)biphenyl (20 mg), and potassium *tert*-butoxide (88 mg) were added to *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 41** (30 mg). ESI-MS: m/z = 542.42[M+H] ⁺. ¹H NMR (500 MHz, DMSO-*d*₆) δ 12.49 (s, 1H), 8.34 (s, 1H), 7.90 (s, 1H), 7.54 (s, 1H), 7.37 (d, *J* = 8.7 Hz, 1H), 6.89 (s, 1H), 6.67 (d, *J* = 8.7 Hz, 1H), 4.79 (q, *J* = 6.2, 5.6 Hz, 2H), 4.57 (h, *J* = 5.7 Hz, 2H), 4.48 (t, *J* = 5.8 Hz, 1H), 4.30 (q, *J* = 7.9 Hz, 1H), 4.19 (qd, *J* = 14.1, 6.4 Hz, 2H), 4.02 (q, *J* = 7.3, 6.8 Hz, 1H), 3.73 (s, 3H), 2.83 (s, 3H), 2.54 (s, 3H), 2.18 (d, *J* = 33.7 Hz, 2H), 2.04 - 1.96 (m, 1H), 1.83 (s, 1H), 1.70 (d, *J* = 13.3 Hz, 1H), 0.70 - 0.59 (m, 1H), 0.29 (tt, *J* = 9.2, 4.6 Hz, 1H), -0.08 (dq, *J* = 9.7, 4.8 Hz, 1H), -0.25 (tt, *J* = 9.7, 4.7 Hz, 1H), -0.70 (dd, *J* = 9.4, 5.0 Hz, 1H).

### Example 42:

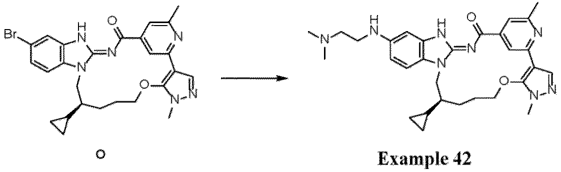

Compound **O** (70 mg), *N*,*N*-dimethylethylenediamine (23 mg), Pd₂(dba)₃ (30 mg), 2-(di-*tert-*butylphosphine)biphenyl (20 mg), and potassium *tert*-butoxide (88 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 42** (15 mg). ESI-MS: m/z = 543.42[M+H] ⁺.

¹H NMR (500 MHz, chloroform-*d*) δ 8.41 (s, 1H), 8.15 (s, 1H), 7.72 (s, 1H), 7.13 (d, *J* = 8.7 Hz, 1H), 6.61 (dd, *J* = 8.6, 2.1 Hz, 1H), 6.49 (d, *J* = 2.1 Hz, 1H), 4.35 (dt, *J* = 10.1, 3.1 Hz, 2H), 3.97 - 3.88 (m, 2H), 3.79 (s, 3H), 3.11 (q, *J* = 6.4 Hz, 2H), 2.78 (t, *J* = 5.7 Hz, 2H), 2.63 (s, 3H), 2.42 (s, 6H), 2.30 - 2.23 (m, 1H), 2.20 - 2.11 (m, 2H), 1.83 - 1.74 (m, 2H), 0.50 (tq, *J* = 8.4, 5.0, 4.2 Hz, 1H), 0.42 (dq, *J* = 8.8, 4.5 Hz, 1H), 0.09 (tt, *J* = 9.1, 5.2 Hz, 1H), -0.05 (dt, *J* = 9.8, 4.8 Hz, 1H), -0.34 (dq, *J* = 10.0, 5.1 Hz, 1H).

### Example 43:

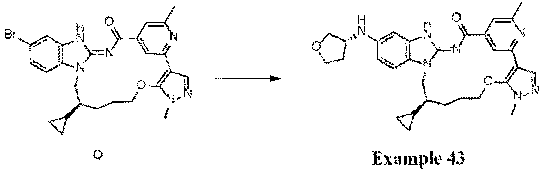

Compound **O** (100 mg), (*R*)-tetrahydrofuran-3-amine hydrochloride (28 mg), Pd₂(dba)₃ (43 mg), 2-(di-*tert-*butylphosphine)biphenyl (28 mg), and potassium *tert*-butoxide (126 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 43** (33 mg). ESI-MS: m/z = 542.39[M+H] ⁺.

¹H NMR (500 MHz, DMSO-*d*₆) δ 12.41 (s, 1H), 8.39 (s, 1H), 7.96 (s, 1H), 7.59 (s, 1H), 7.31 (d, *J* = 8.5 Hz, 1H), 6.78 (s, 1H), 6.60 (d, *J* = 8.6 Hz, 1H), 5.89 (d, *J* = 6.7 Hz, 1H), 4.30 (dd, *J* = 31.8, 11.0 Hz, 2H), 4.10 (d, *J* = 9.4 Hz, 2H), 4.01 (t, *J* = 7.4 Hz, 1H), 3.87 (q, *J* = 7.7 Hz, 1H), 3.78 (s, 3H), 3.59 (dd, *J* = 8.9, 3.9 Hz, 1H), 3.47 - 3.28 (m, 2H), 2.59 (s, 3H), 2.33 - 2.11 (m, 3H), 2.10 - 2.00 (m, 1H), 1.92 - 1.71 (m, 3H), 0.72 - 0.62 (m, 1H), 0.36 (dd, *J* = 11.3, 6.1 Hz, 1H), -0.00 (dt, *J* = 9.7, 5.0 Hz, 1H), -0.05 - -0.16 (m, 1H), -0.49 - -0.60 (m, 1H).

### Example 44:

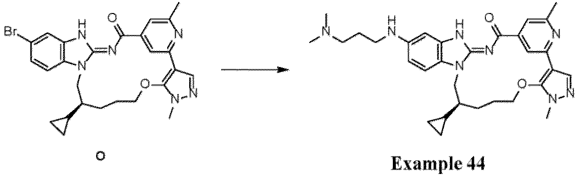

Compound **O** (100 mg), *N*,*N*-dimethyl-1,3-diaminopropane (23 mg), Pd₂(dba)₃ (43 mg), *2-(di-tert-*butylphosphine)biphenyl (28 mg), and potassium *tert*-butoxide (126 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 44** (20 mg). ESI-MS: m/z = 557.44[M+H] ⁺.

¹H NMR (500 MHz, chloroform-*d*) δ 8.47 (s, 1H), 8.41 (s, 1H), 8.15 (s, 1H), 7.72 (s, 1H), 7.13 (d, *J* = 8.6 Hz, 1H), 6.62 (dd, *J* = 8.7, 2.1 Hz, 1H), 6.48 (d, *J* = 2.1 Hz, 1H), 4.38 - 4.33 (m, 2H), 3.97 - 3.94 (m, 1H), 3.93 - 3.89 (m, 1H), 3.79 (s, 3H), 3.10 (dq, *J* = 13.4, 6.4 Hz, 2H), 3.02 - 2.95 (m, 2H), 2.65 (s, 6H), 2.64 (s, 3H), 2.31 - 2.25 (m, 1H), 2.17 (ddd, *J* = 23.0, 11.6, 5.1 Hz, 2H), 2.00 (q, *J* = 7.0 Hz, 2H), 1.78 (t, *J* = 8.9 Hz, 2H), 0.51 (td, *J* = 9.1, 4.6 Hz, 1H), 0.42 (tt, *J* = 9.1, 5.0 Hz, 1H), 0.08 (tt, *J* = 8.6, 5.0 Hz, 1H), -0.04 (dt, *J* = 9.6, 5.1 Hz, 1H), -0.35 (dq, *J* = 9.9, 5.1 Hz, 1H).

### Example 45:

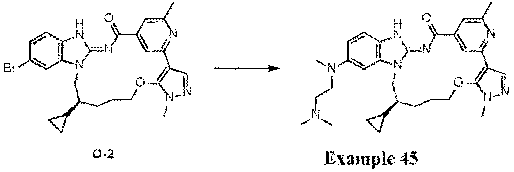

Compound **O-2** (100 mg), *N*,*N*,*N*'-trimethylethylenediamine (28 mg), Pd₂(dba)₃ (43 mg), 2-(di-*tert-*butylphosphine)biphenyl (28 mg), and potassium *tert*-butoxide (126 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 45** (23 mg). ESI-MS: m/z = 279.37[1/2M+H]⁺.

¹H NMR (500 MHz, DMSO-*d*₆) δ 12.42 (s, 1H), 8.35 (s, 1H), 7.91 (s, 1H), 7.55 (s, 1H), 7.34 (d, *J* = 8.7 Hz, 1H), 6.89 (d, *J* = 2.3 Hz, 1H), 6.66 (dd, *J* = 8.8, 2.3 Hz, 1H), 4.30 (q, *J* = 7.9 Hz, 1H), 4.23 (dd, *J* = 13.9, 3.6 Hz, 1H), 4.14 (dd, *J* = 13.9, 9.2 Hz, 1H), 4.03 (dt, *J* = 9.0, 5.8 Hz, 1H), 3.73 (s, 3H), 3.57 - 3.49 (m, 2H), 3.42 (ddd, *J* = 14.6, 8.5, 6.0 Hz, 2H), 2.94 (s, 3H), 2.54 (s, 3H), 2.43 (qd, *J* = 9.1, 7.7, 4.1 Hz, 2H), 2.20 (s, 6H), 2.01 (td, *J* = 9.5, 4.5 Hz, 1H), 1.84 (d, *J* = 9.5 Hz, 1H), 1.72 (tt, *J* = 12.5, 5.2 Hz, 1H), 0.63 (qt, *J* = 8.6, 5.0 Hz, 1H), 0.31 (tt, *J* = 9.3, 4.8 Hz, 1H), -0.05 (dq, *J* = 9.7, 4.9 Hz, 1H), -0.17 (dp, *J* = 9.5, 4.7 Hz, 1H), -0.60 (dq, *J* = 9.7, 4.8 Hz, 1H).

### Example 46:

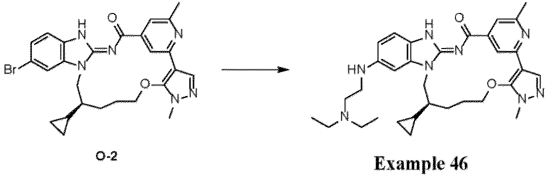

Compound **O-2** (70 mg), *N*,*N*-diethylethylenediamine (18 mg), Pd₂(dba)₃ (30 mg), 2-(di-*tert-*butylphosphine)biphenyl (20 mg), and potassium *tert*-butoxide (88 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 46** (20 mg). ESI-MS: m/z = 571.41[M+H] ⁺.

¹H NMR (500 MHz, DMSO-*d*₆) δ 12.41 (s, 1H), 8.34 (s, 1H), 7.91 (s, 1H), 7.54 (s, 1H), 7.27 (d, *J* = 8.5 Hz, 1H), 6.79 (d, *J* = 2.1 Hz, 1H), 6.56 (dd, *J* = 8.6, 2.1 Hz, 1H), 4.33 - 4.26 (m, 1H), 4.22 (dd, *J* = 14.1, 3.6 Hz, 1H), 4.10 - 4.01 (m, 2H), 3.73 (s, 3H), 3.21 (t, *J* = 6.7 Hz, 2H), 2.77 (t, *J* = 6.7 Hz, 2H), 2.69 (q, *J* = 7.2 Hz, 4H), 2.54 (s, 3H), 2.27 - 2.18 (m, 1H), 2.13 (tt, *J* = 9.3, 4.8 Hz, 1H), 2.00 (tq, *J* = 8.9, 4.4, 3.8 Hz, 1H), 1.86 (d, *J* = 10.2 Hz, 1H), 1.71 (tt, *J* = 8.8, 3.9 Hz, 1H), 1.03 (t, *J* = 7.1 Hz, 6H), 0.61 (dt, *J* = 9.4, 4.9 Hz, 1H), 0.31 (dt, *J* = 9.0, 4.4 Hz, 1H), -0.05 (dt, *J* = 9.8, 4.8 Hz, 1H), -0.15 (dp, *J* = 9.6, 4.7 Hz, 1H), -0.59 (dq, *J* = 9.7, 4.8 Hz, 1H).

### Example 47:

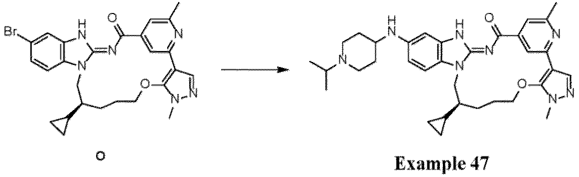

Compound **O** (75 mg), 1-isopropylpiperidine-4-amine (40 mg), Pd₂(dba)₃ (32 mg), 2-(di-*tert-*butylphosphine)biphenyl (21 mg), and potassium *tert*-butoxide (94 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 47** (20 mg). ESI-MS: m/z = 299.39[1/2M+H] ⁺.

¹H NMR (500 MHz, chloroform-*d*) δ 8.49 (s, 1H), 8.42 (s, 1H), 8.15 (s, 1H), 7.73 (s, 1H), 7.14 (d, *J* = 8.5 Hz, 1H), 6.59 - 6.53 (m, 2H), 4.36 (dq, *J=* 8.6, 5.8, 4.6 Hz, 2H), 3.97 - 3.89 (m, 2H), 3.79 (s, 3H), 3.39 - 3.29 (m, 4H), 2.64 (s, 3H), 2.57 (s, 1H), 2.28 (dd, *J* = 13.4, 6.8 Hz, 1H), 2.24 - 2.13 (m, 4H), 1.98 (dd, *J=* 25.5, 12.2 Hz, 2H), 1.80 (d, *J=* 11.9 Hz, 2H), 1.28 (s, 3H), 1.26 (s, 3H), 0.87 (dt, *J=* 13.2, 6.6 Hz, 1H), 0.51 (dd, *J=* 10.5, 5.8 Hz, 1H), 0.44 (tt, *J=* 9.0, 4.9 Hz, 1H), 0.10 (tt, *J* = 8.8, 4.8 Hz, 1H), -0.01 - -0.08 (m, 1H), -0.34 (dq, *J=* 10.1, 5.1 Hz, 1H).

### Example 48:

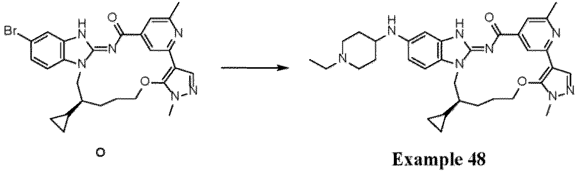

Compound O (75 mg), 1-ethylpiperidine-4-atnine dihydrochloride (56 mg), Pd₂(dba)₃ (32 mg), *2-(di-tert-*butylphosphine)biphenyl (21 mg), and potassium *tert*-butoxide (94 mg) were added to tert-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 48** (10 mg). ESI-MS: m/z = 292.38[1/2M+H] ⁺.

¹H NMR (500 MHz, chloroform-*d*) δ 8.48 (s, 1H), 8.44 (s, 1H), 8.15 (s, 1H), 7.79 (s, 1H), 7.14 (d, *J* = 8.5 Hz, 1H), 6.58 - 6.50 (m, 2H), 4.41 - 4.33 (m, 2H), 3.96 (dt, *J* = 8.9, 5.9 Hz, 1H), 3.89 (dd, *J* = 13.9, 7.7 Hz, 1H), 3.79 (s, 3H), 3.30 (d, *J* = 13.3 Hz, 2H), 3.18 (s, 1H), 2.73 (q, *J* = 7.2 Hz, 2H), 2.64 (s, 3H), 2.34 (d, *J* = 10.9 Hz, 1H), 2.28 (q, *J* = 6.5 Hz, 1H), 2.25 - 2.19 (m, 2H), 2.11 (t, *J* = 8.8 Hz, 3H), 1.84 - 1.77 (m, 3H), 1.21 (t, *J* = 7.3 Hz, 3H), 0.89 - 0.86 (m, 1H), 0.51 (t, *J* = 8.5 Hz, 1H), 0.43 (tt, *J* = 9.1, 5.0 Hz, 1H), 0.09 (tt, *J* = 9.1, 5.3 Hz, 1H), -0.04 (p, *J* = 4.9 Hz, 1H), -0.34 (dt, *J* = 9.6, 5.0 Hz, 1H).

### Example 49:

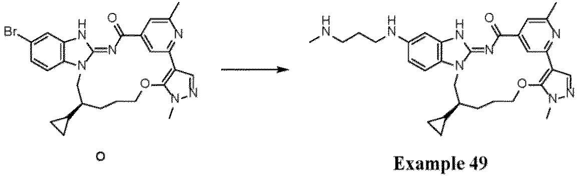

Compound **O** (75 mg), 3-methylaminopropylamine (15 mg), Pd₂(dba)₃ (32 mg), 2-(di-*tert*-butylphosphine)biphenyl (21 mg), and potassium *tert*-butoxide (94 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 49** (25 mg). ESI-MS: m/z = 272.30[1/2M+H] ⁺.

¹H NMR (500 MHz, DMSO-*d*₆) δ 8.36 (s, 1H), 8.33 (s, 1H), 7.90 (s, 1H), 7.54 (s, 1H), 7.34 (d, *J* = 8.7 Hz, 1H), 6.74 (d, *J* = 2.2 Hz, 1H), 6.60 - 6.57 (m, 1H), 4.32 - 4.26 (m, 1H), 4.22 (dd, *J* = 13.9, 3.5 Hz, 1H), 4.02 (dd, *J* = 14.5, 9.3 Hz, 2H), 3.73 (s, 3H), 3.06 (t, *J* = 6.9 Hz, 2H), 2.73 (t, *J* = 7.0 Hz, 1H), 2.67 - 2.62 (m, 1H), 2.54 (s, 3H), 2.52 (d, *J* = 2.1 Hz, 3H), 2.23 - 2.18 (m, 1H), 2.14 (d, *J* = 9.3 Hz, 1H), 2.03 - 1.96 (m, 2H), 1.83 - 1.76 (m, 2H), 1.71 *(d, J=* 12.8 Hz, 1H), 0.58 (dd, *J* = 10.5, 5.8 Hz, 1H), 0.30 (tt, *J* = 9.0, 4.9 Hz, 1H), -0.06 (tt, *J* = 8.8, 4.8 Hz, 1H), -0.10 - - 0.15 (m, 1H), -0.61 (dq, *J* = 10.1, 5.1 Hz, 1H).

### Example 50:

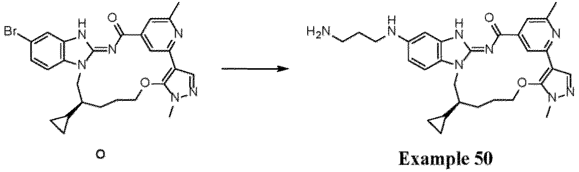

Compound **O** (85 mg), *tert*-butyl (3-aminopropyl)carbamate (33 mg), Pd₂(dba)₃ (34 mg), 2-(di-*tert-*butylphosphine)biphenyl (22 mg), and potassium *tert*-butoxide (100 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, and concentrated until no liquid flowed out. The crude product obtained by concentrating the filtrate was directly dissolved in hydrogen chloride-methanol solution (4 M, 5 mL), and the mixture was stirred for 30 min. After the reaction was completed as monitored by LC-MS, the reaction solution was concentrated and purified by preparative liquid phase chromatography to give **Example 50** (9 mg). ESI-MS: m/z = 529.31[M+H] ⁺.

¹H NMR (500 MHz, DMSO-*d*₆) δ 8.38 (s, 1H), 8.34 (s, 1H), 7.88 (s, 1H), 7.52 (s, 1H), 7.31 (d, *J* = 8.7 Hz, 1H), 6.72 (d, *J* = 2.2 Hz, 1H), 6.60 - 6.56 (m, 1H), 4.33 - 4.27 (m, 1H), 4.20 (dd, *J* = 13.9, 3.5 Hz, 1H), 4.01 (dd, *J* = 14.5, 9.3 Hz, 2H), 3.73 (s, 3H), 3.04 (t, *J* = 6.9 Hz, 2H), 2.76 (t, *J* = 7.0 Hz, 1H), 2.66 - 2.61 (m, 1H), 2.56 (s, 3H), 2.23 - 2.18 (m, 1H), 2.13 (d, *J* = 9.3 Hz, 1H), 2.02 - 1.97 (m, 2H), 1.82 - 1.76 (m, 2H), 1.67 (d, *J* = 12.8 Hz, 1H), 0.56 (dd, *J* = 10.5, 5.8 Hz, 1H), 0.31 (tt, *J* = 9.0, 4.9 Hz, 1H), -0.05 (tt, *J* = 8.8, 4.8 Hz, 1H), -0.10 - -0.15 (m, 1H), -0.60 (dq, *J* = 10.1, 5.1 Hz, 1H).

### Example 51:

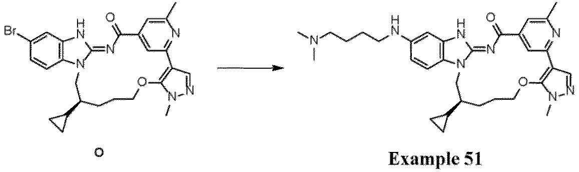

Compound **O** (100 mg), 4-dimethylaminobutylamine (21 mg), Pd₂(dba)₃ (43 mg), 2-(di-*tert*-butylphosphine)biphenyl (28 mg), and potassium *tert*-butoxide (126 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 51** (10 mg). ESI-MS: m/z = 571.44[M+H] ⁺.

¹H NMR (500 MHz, chloroform-*d*) δ 8.46 (s, 1H), 8.40 (s, 1H), 8.15 (s, 1H), 7.70 (s, 1H), 7.13 (d, *J* = 8.7 Hz, 1H), 6.60 (dd, *J* = 8.6, 2.1 Hz, 1H), 6.52 (d, *J* = 2.2 Hz, 1H), 4.40 - 4.31 (m, 2H), 3.97 - 3.94 (m, 1H), 3.90 (d, *J* = 6.4 Hz, 1H), 3.78 (s, 3H), 3.08 (td, *J* = 6.4, 3.3 Hz, 2H), 2.95 - 2.89 (m, 2H), 2.69 (s, 6H), 2.63 (s, 3H), 2.27 (dt, *J* = 13.5, 6.9 Hz, 1H), 2.16 (ddt, *J* = 21.9, 13.5, 7.2 Hz, 2H), 1.87 - 1.73 (m, 4H), 1.68 (p, *J* = 6.8 Hz, 2H), 0.50 (td, *J* = 9.8, 8.3, 3.4 Hz, 1H), 0.42 (tt, *J* = 8.9, 4.9 Hz, 1H), 0.09 (tt, *J* = 9.1, 4.9 Hz, 1H), -0.04 (dq, *J* = 9.8, 4.9 Hz, 1H), -0.34 (dq, *J* = 9.9, 5.1 Hz, 1H).

### Example 52:

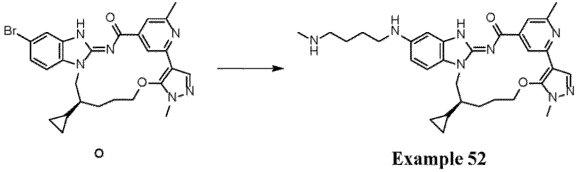

Compound **O** (100 mg), *tert*-butyl (4-aminobutyl)(methyl)carbamate (47 mg), Pd₂(dba)₃ (43 mg), 2-(di-*tert-*butylphosphine)biphenyl (28 mg), and potassium *tert*-butoxide (126 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, and concentrated until no liquid flowed out. The crude product obtained by concentrating the filtrate was directly dissolved in hydrogen chloride-methanol solution (4 M, 5 mL), and the mixture was stirred for 30 min. After the reaction was completed as monitored by LC-MS, the reaction solution was concentrated and purified by preparative liquid phase chromatography to give **Example 52** (22 mg). ESI-MS: m/z = 557.31[M+H] ⁺.

¹H NMR (500 MHz, chloroform-*d*) δ 8.35 (s, 2H), 8.32 (s, 1H), 8.14 (s, 1H), 7.59 (s, 1H), 7.05 (d, *J* = 8.6 Hz, 1H), 6.62 - 6.54 (m, 2H), 4.29 - 4.21 (m, 2H), 3.90 - 3.86 (m, 1H), 3.76 (d, *J* = 8.8 Hz, 1H), 3.73 (s, 3H), 3.02 (dt, *J* = 23.3, 7.0 Hz, 4H), 2.69 (s, 3H), 2.60 (s, 3H), 2.15 (q, *J* = 6.6 Hz, 1H), 2.08 (d, *J* = 6.1 Hz, 1H), 2.02 - 1.95 (m, 1H), 1.90 (t, *J* = 7.7 Hz, 2H), 1.74 - 1.65 (m, 4H), 0.41 (s, 1H), 0.35 (dq, *J* = 8.6, 4.6, 4.1 Hz, 1H), -0.00 - -0.06 (m, 1H), -0.12 (dt, *J* = 9.7, 5.0 Hz, 1H), -0.45 (dd, *J* = 9.7, 5.1 Hz, 1H).

### Example 53:

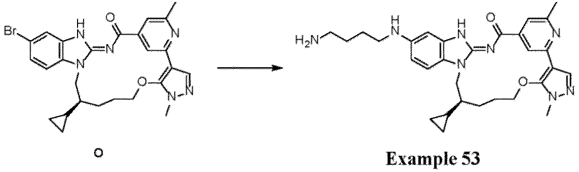

Compound **O** (100 mg), 1,4-butanediamine hydrochloride (61 mg), Pd₂(dba)₃ (43 mg), *2-(di-tert-*butylphosphine)biphenyl (28 mg), and potassium *tert*-butoxide (126 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 53** (25 mg). ESI-MS: m/z = 543.42[M+H] ⁺.

¹H NMR (500 MHz, Methanol-*d*₄) δ 8.34 (s, 1H), 8.02 (s, 1H), 7.58 (s, 1H), 7.24 - 7.17 (m, 1H), 6.66 (d, *J* = 8.4 Hz, 2H), 4.34 - 4.28 (m, 1H), 4.27 - 4.20 (m, 1H), 3.98 (p, *J* = 5.1 Hz, 1H), 3.84 (d, *J* = 20.1 Hz, 1H), 3.73 (s, 3H), 3.17 - 3.09 (m, 2H), 2.99 (t, *J* = 7.4 Hz, 2H), 2.53 (s, 3H), 2.19 (d, *J* = 13.0 Hz, 2H), 2.00 (d, *J* = 17.1 Hz, 1H), 1.76 (ddd, *J* = 36.2, 13.9, 6.7 Hz, 6H), 0.52 - 0.43 (m, 1H), 0.33 (t, *J* = 8.9 Hz, 1H), -0.08 - -0.17 (m, 2H), -0.60 (d, *J* = 8.9 Hz, 1H).

### Example 54:

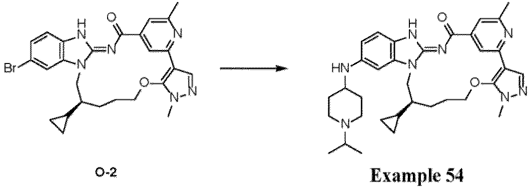

Compound **O-2** (70 mg), 1-isopropylpiperidine-4-amine (22 mg), Pd₂(dba)₃ (30 mg), 2-(di-*tert-*butylphosphine)biphenyl (20 mg), and potassium *tert*-butoxide (88 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 54** (43 mg). ESI-MS: m/z = 299.39[1/2M+H] ⁺.

¹H NMR (500 MHz, DMSO-*d*₆) δ 8.38 (s, 1H), 8.29 (s, 1H), 7.95 (s, 1H), 7.58 (s, 1H), 7.28 (d, *J* = 8.5 Hz, 1H), 6.78 (s, 1H), 6.60 (d, *J* = 8.6 Hz, 1H), 4.35 - 4.31 (m, 1H), 4.26 (dd, *J* = 13.8, 3.5 Hz, 1H), 4.10 - 4.06 (m, 2H), 3.77 (s, 3H), 3.42 (t, *J* = 10.0 Hz, 1H), 3.03 (p, *J* = 8.0, 6.7 Hz, 2H), 2.62 (d, *J* = 10.6 Hz, 2H), 2.58 (s, 3H), 2.26 (dd, *J* = 13.0, 6.6 Hz, 1H), 2.21 - 2.14 (m, 1H), 2.10 - 2.01 (m, 3H), 1.91 - 1.84 (m, 1H), 1.81 - 1.71 (m, 1H), 1.53 (q, *J* = 13.9, 13.0 Hz, 2H), 1.13 (d, *J* = 6.6 Hz, 6H), 0.63 (qd, *J* = 8.5, 5.2, 3.6 Hz, 1H), 0.36 (tt, *J* = 9.4, 4.6 Hz, 1H), 0.03 - -0.03 (m, 1H), -0.11 (dt, *J* = 9.3, 4.3 Hz, 1H), -0.54 (dt, *J* = 9.7, 4.9 Hz, 1H).

### Example 55:

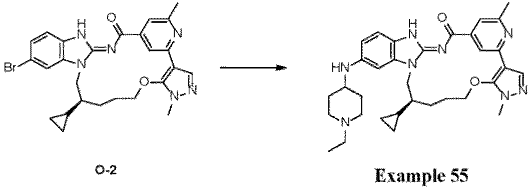

Compound **O-2** (70 mg), 1-ethylpiperidine-4-atnine dihydrochloride (32 mg), Pd₂(dba)₃ (30 mg), 2-(di-*tert-*butylphosphine)biphenyl (20 mg), and potassium *tert*-butoxide (88 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 55** (35 mg). ESI-MS: m/z = 292.40[1/2M+H] ⁺.

¹H NMR (500 MHz, DMSO-*d*₆) δ 12.42 (s, 1H), 8.38 (s, 1H), 8.27 (s, 1H), 7.95 (s, 1H), 7.58 (s, 1H), 7.29 (d, *J* = 8.6 Hz, 1H), 6.78 (s, 1H), 6.60 (d, *J* = 8.6 Hz, 1H), 4.33 (d, *J* = 8.2 Hz, 1H), 4.29 - 4.24 (m, 1H), 4.08 (dt, *J* = 13.2, 6.7 Hz, 2H), 3.77 (s, 3H), 3.42 (d, *J* = 10.0 Hz, 1H), 3.07 (t, *J* = 10.8 Hz, 2H), 2.63 (d, *J* = 7.1 Hz, 2H), 2.58 (s, 3H), 2.43 (d, *J* = 12.6 Hz, 2H), 2.31 - 2.23 (m, 1H), 2.20 - 2.13 (m, 1H), 2.05 (d, *J* = 14.8 Hz, 3H), 1.86 (s, 1H), 1.76 (dt, *J* = 13.6, 6.6 Hz, 1H), 1.51 (t, *J* = 12.9 Hz, 2H), 1.12 (t, *J* = 7.1 Hz, 3H), 0.64 (q, *J* = 8.1 Hz, 1H), 0.37 (td, *J* = 9.5, 9.0, 4.6 Hz, 1H), 0.00 (dd, *J* = 9.6, 4.9 Hz, 1H), -0.09 (dt, *J* = 9.6, 4.6 Hz, 1H), -0.52 (dd, *J* = 9.1, 4.9 Hz, 1H).

### Example 56/57:

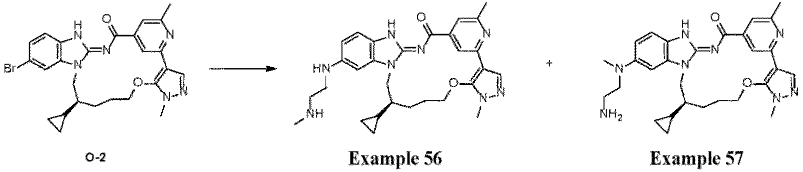

Compound **O-2** (70 mg), *N*-methylethylenediamine (15 mg), Pd₂(dba)₃ (30 mg), 2-(di-*tert*-butylphosphine)biphenyl (20 mg), and potassium *tert*-butoxide (88 mg) were added to *tert-amyl* alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and subjected to preparative chromatography (liquid phase chromatography conditions: column: YMC AQ C18 250 mm × 30 mm, 10 µm; mobile phase: A: 0.1% formic acid, B: acetonitrile, gradient: 15% C-25% B (0-90 min); flow rate: 25mL/min; wavelength: 254 nm) to give **Example 56** (12 mg, retention time: 60.4 min), ESI-MS: m/z = 529.35 [M+H] ⁺, and **Example 57** (25 mg, retention time: 39.5 min), ESI-MS: m/z = 529.35 [M+H] ⁺. **Example 56:** ¹H NMR (500 MHz, DMSO-*d*₆) δ 8.39 (s, 1H), 7.91 (s, 1H), 7.54 (s, 1H), 7.27 (d, *J* = 8.5 Hz, 1H), 6.80 (d, *J* = 2.1 Hz, 1H), 6.56 (dd, *J* = 8.6, 2.0 Hz, 1H), 5.81 (s, 1H), 4.33 - 4.26 (m, 1H), 4.22 (dd, *J* = 14.0, 3.6 Hz, 1H), 4.12 - 4.01 (m, 2H), 3.73 (s, 3H), 3.28 (t, *J* = 6.3 Hz, 2H), 2.90 (t, *J* = 6.3 Hz, 2H), 2.54 (s, 3H), 2.44 (s, 3H), 2.21 (dt, *J* = 12.2, 5.9 Hz, 1H), 2.13 (tt, *J* = 8.6, 4.6 Hz, 1H), 2.01 (qd, *J* = 9.4, 6.1, 5.6 Hz, 1H), 1.85 (s, 1H), 1.76 -1.66 (m, 1H), 0.62 (tq, *J* = 8.2, 4.9, 4.3 Hz, 1H), 0.31 (tt, *J* = 9.4, 4.7 Hz, 1H), -0.05 (dq, *J* = 9.6, 4.9 Hz, 1H), -0.14 (tt, *J* = 9.4, 4.6 Hz, 1H), -0.60 (dq, *J* = 9.7, 4.9 Hz, 1H). **Example 57:** ¹H NMR (500 MHz, DMSO-*d*₆) δ 8.44 (s, 1H), 7.91 (s, 1H), 7.55 (s, 1H), 7.35 (d, *J* = 8.8 Hz, 1H), 7.00 (s, 1H), 6.72 (d, *J* = 8.8 Hz, 1H), 4.29 (t, *J* = 7.8 Hz, 1H), 4.21 (d, *J* = 7.7 Hz, 2H), 4.03 (p, *J* = 6.2 Hz, 1H), 3.73 (s, 3H), 3.50 (tt, *J* = 15.3, 8.1 Hz, 2H), 2.94 (s, 3H), 2.87 (t, *J* = 7.2 Hz, 2H), 2.54 (s, 3H), 2.27 - 2.19 (m, 1H), 2.15 (s, 1H), 2.02 (d, *J* = 15.6 Hz, 1H), 1.87 (s, 1H), 1.71 (s, 1H), 0.66 (s, 1H), 0.29 (s, 1H), -0.03 - -0.13 (m, 1H), -0.21 (s, 1H), -0.65 (s, 1H).

### Example 58:

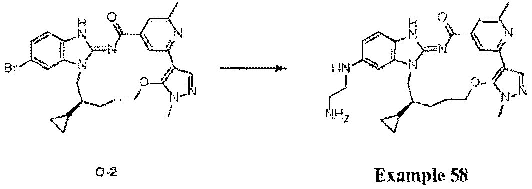

Compound **O-2** (70 mg), ethylenediamine (20 mg), Pd₂(dba)₃ (30 mg), 2-(di-tert-butylphosphine)biphenyl (20 mg), and potassium *tert*-butoxide (88 mg) were added to tert-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 58** (40 mg). ESI-MS: m/z = 258.34[1/2M+H] ⁺.

¹H NMR (500 MHz, DMSO-*d*₆) δ 8.43 (s, 1H), 8.34 (s, 1H), 7.91 (s, 1H), 7.54 (s, 1H), 7.27 (d, *J* = 8.5 Hz, 1H), 6.80 (d, *J* = 2.1 Hz, 1H), 6.56 (dd, *J* = 8.5, 2.0 Hz, 1H), 4.29 (q, *J* = 7.9 Hz, 1H), 4.22 (dd, *J* = 14.1, 3.5 Hz, 1H), 4.13 - 4.00 (m, 2H), 3.73 (s, 3H), 3.27 (t, *J* = 6.4 Hz, 2H), 2.93 (t, *J* = 6.3 Hz, 2H), 2.54 (s, 3H), 2.21 (dt, *J* = 11.9, 5.9 Hz, 1H), 2.14 (dt, *J* = 13.4, 6.4 Hz, 1H), 2.04 - 1.96 (m, 1H), 1.84 (d, *J* = 9.7 Hz, 1H), 1.72 (dd, *J* = 12.1, 6.7 Hz, 1H), 0.63 (td, *J* = 9.5, 8.8, 4.1 Hz, 1H), 0.31 (dp, *J* = 9.4, 4.6 Hz, 1H), -0.05 (dq, *J* = 9.7, 4.9 Hz, 1H), -0.14 (td, *J* = 8.8, 4.5 Hz, 1H), -0.60 (dq, *J* = 9.9, 4.9 Hz, 1H).

### Example 59/60:

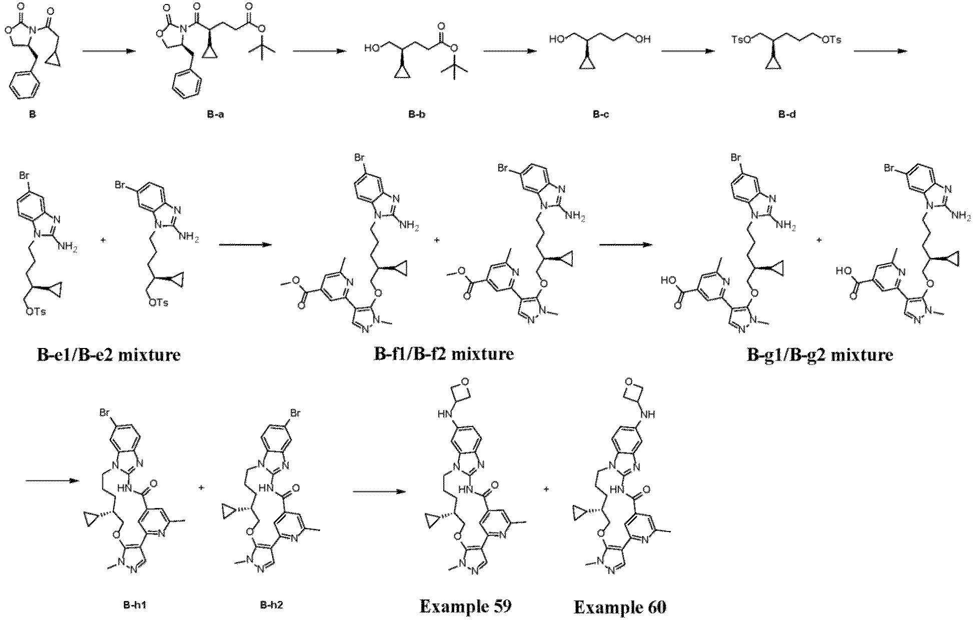

### (1) Preparation method for compound B-a:

Titanium tetrachloride (43.9 g) was added to dichloromethane (300 mL), and the mixture was cooled to 0-2 °C. Tetraisopropyl titanate (19.7 g) was added dropwise with the temperature controlled at 0-2 °C, and the mixture was stirred for 10 min with the temperature kept at this temperature. *N*,*N*-diisopropylethylamine (32.8 g) was added dropwise with the temperature controlled at 0-2 °C, and the mixture was stirred for 10 min with the temperature kept at this temperature. A solution of compound **B** (30.0 g) in dichloromethane (100 mL) was added dropwise with the temperature controlled at 0-2 °C, and the mixture was stirred for 2 h with the temperature kept at this temperature.

Then tert-butyl acrylate (44.5 g) was added dropwise with the temperature controlled at 0-2 °C, and the mixture was reacted for 4 h with the temperature controlled at 0-2 °C. After the reaction was completed as monitored by TLC, the reaction solution was poured into a saturated ammonium chloride solution (600 mL), and the resulting mixture was extracted with dichloromethane (200 mL × 2). The organic phases were combined, dried over anhydrous sodium sulfate, concentrated until no liquid flowed out, and subjected to column chromatography to give compound **B-a** (19.0 g).

### (2) Preparation method for compound B-b:

Sodium borohydride (1.64 g) was added to tetrahydrofuran (70 mL), and the mixture was cooled to 0-2 °C. A solution of compound **B-a** (15.26 g) in tetrahydrofuran (30 mL) was added dropwise with the temperature controlled at 0-2 °C. After the dropwise addition, the ice bath was removed and the mixture was stirred at 20-25 °C for 6 h. After the reaction was completed as monitored by TLC, the reaction solution was poured into a saturated ammonium chloride solution (200 mL), and the resulting mixture was extracted with ethyl acetate (50 mL × 2). The organic phases were combined, dried over anhydrous sodium sulfate, concentrated until no liquid flowed out, and subjected to column chromatography to give compound **B-b** (1.95 g).

### (3) Preparation method for compound B-c:

Compound **B-b** (1.95 g) was added to tetrahydrofuran (50 mL), and the mixture was purged with nitrogen 3 times and cooled to 0-2 °C. A solution of lithium aluminum hydride in tetrahydrofuran (1 M, 10.9 mL) was added dropwise with the temperature controlled at 0-2 °C. After the dropwise addition, the ice bath was removed and the mixture was stirred at 20-25 °C for 6 h. After the reaction was completed as monitored by TLC, water (0.37 mL), 15% sodium hydroxide solution (0.37 mL), and water (1.11 mL) were sequentially added dropwise to the reaction solution with the temperature controlled at 0-5 °C. The reaction solution was filtered. The filter cake was washed with tetrahydrofuran (20 mL), and the filtrate was dried over anhydrous sodium sulfate and concentrated until no liquid flowed out to give compound **B-c** (1.44 g).

### (4) Preparation method for compound B-d:

Compound **B-c** (1.44 g) was dissolved in dichloromethane (50 mL), and triethylamine (5.06 g) and 4-dimethylaminopyridine (10 mg) were slowly added. The mixture was cooled to 0-5 °C, and then a solution of *p-*toluenesulfonyl chloride (5.72 g) in dichloromethane (10 mL) was added dropwise. After the addition was completed, the mixture was reacted at 20-25 °C for 3 h. After the reaction was completed, the reaction solution was washed with a sodium bicarbonate solution (1 M, 20 mL × 2) and subjected to liquid separation. The organic phase was dried over anhydrous sodium sulfate, concentrated until no liquid flowed out, and subjected to column chromatography to give compound **B-d** (1.89 g).

### (5) Preparation method for compound B-e1/B-e2:

Compound **B-d** (1.89 g), 5-bromo-1*H*-benzimidazol-2-amine (888 mg), and 7-methyl-1,5,7-triazabicyclo[4.4.0]dec-5-ene (1.8 mL) were added to acetonitrile (50 mL), and the mixture was purged with nitrogen 3 times and reacted at 15-20 °C for 24 h. After the reaction was completed as monitored by TLC, a 1 M aqueous sodium bicarbonate solution (50 mL) was added to the reaction solution, and the resulting mixture was extracted with ethyl acetate (50 mL × 2). The organic phases were combined, dried over anhydrous sodium sulfate, concentrated until no liquid flowed out, and subjected to column chromatography to give compound **B-e1/B-e2** mixture (1.44 g). ESI-MS: m/z = 492.18[M+H] ⁺.

### (6) Preparation method for compound B-f1/B-f2:

Compound **B-e1/B-e2** (1.44 g), compound **A** (673 mg), and potassium carbonate (601 mg) were added to acetonitrile (70 mL), and the mixture was reacted at 80 °C for 16 h. After the reaction was completed as monitored by TLC, the reaction solution was poured into 100 mL of water, and extracted with ethyl acetate (40 mL × 2). The ethyl acetate phases were combined. The organic phase was washed once with water, dried over anhydrous sodium sulfate, concentrated until no liquid flowed out, and subjected to column chromatography to give compound **B-f1/B-f2 mixture** (853 mg). ESI-MS: m/z = 567.19[M+H] ⁺.

### (7) Preparation method for compound B-g1/B-g2:

Compound **B-f1/B-f2** (853 mg) was dissolved in tetrahydrofuran (10 mL), and a solution of sodium hydroxide (183 mg) in water (3 mL) was added. The mixture was stirred at 20-25 °C for 18 h. After the reaction was completed as monitored by LC-MS, the mixture was adjusted to pH 5-6 with 6 M hydrochloric acid, and concentrated by rotary evaporation under reduced pressure to remove tetrahydrofuran and water. Dichloromethane (10 mL) and triethylamine (456 mg) were added, and the mixture was stirred for complete dissolution, dried over anhydrous sodium sulfate, filtered, and concentrated until no liquid flowed out to give compound **B-g1/B-g2 mixture,** which was directly used in the next step without further purification. ESI-MS: m/z = 553.16[M+H] ⁺.

### (8) Preparation method for compound B-h1/B-h2:

Compound **B-g1/B-g2** obtained in the previous step was dissolved in dichloromethane (10 mL), and TBTU (593 mg) was added. The mixture was reacted at 20-25 °C for 16 h. After the reaction was completed as detected by LC-MS, the reaction solution was washed with water (10 mL × 3). The organic phase was dried over anhydrous sodium sulfate, concentrated until no liquid flowed out, and subjected to column chromatography to give compound **B-h1/B-h2 mixture** (817 mg), which was subjected to preparative SFC (liquid phase chromatography conditions: column: YMC SB 30 mm × 250 mm, 10 µm; mobile phase: carbon dioxide: methanol = 40:60; flow rate: 80 mL/min; wavelength: 254 nm) to give compound **B-h1** (253 mg, retention time: 11.6 min), ESI-MS: m/z = 535.16[M+H] ⁺, and compound **B-h2** (207 mg, retention time: 14.0 min), ESI-MS: m/z = 535.16[M+H] ⁺. Compound **B-h1:** ¹H NMR (500 MHz, chloroform-*d*) δ 12.00 (s, 1H), 8.47 (s, 1H), 8.17 (s, 1H), 7.60 (s, 1H), 7.43 (d, *J* = 1.7 Hz, 1H), 7.35 (dd, *J* = 8.4, 1.8 Hz, 1H), 7.21 (d, *J* = 8.4 Hz, 1H), 4.22 (dd, *J* = 9.1, 4.1 Hz, 1H), 4.18 - 4.10 (m, 2H), 4.01 (ddd, *J* = 14.0, 9.5, 4.8 Hz, 1H), 3.82 (s, 3H), 2.61 (s, 3H), 2.31 - 2.23 (m, 1H), 2.09 (ddt, *J* = 12.6, 9.1, 4.5 Hz, 2H), 1.94 (ddd, *J* = 12.0, 7.4, 4.3 Hz, 1H), 1.08 (dq, *J* = 8.6, 4.4 Hz, 1H), 0.90 (dtd, *J* = 9.9, 5.6, 4.7, 2.3 Hz, 1H), 0.64 (tt, *J* = 9.0, 4.6 Hz, 1H), 0.56 (tt, *J* = 8.9, 4.7 Hz, 1H), 0.28 (dq, *J* = 9.6, 4.8 Hz, 1H), 0.21 (dq, *J* = 9.3, 4.7 Hz, 1H). Compound **B-h2:** ¹H NMR (500 MHz, chloroform-*d*) δ 12.04 (s, 1H), 8.46 (s, 1H), 8.17 (s, 1H), 7.59 (s, 1H), 7.49 (s, 1H), 7.40 (dd, *J* = 8.5, 1.2 Hz, 1H), 7.12 (d, *J* = 8.5 Hz, 1H), 4.22 (dd, *J* = 9.2, 4.1 Hz, 1H), 4.19 - 4.09 (m, 2H), 4.00 (ddd, *J* = 14.1, 9.7, 5.0 Hz, 1H), 3.82 (s, 3H), 2.60 (s, 3H), 2.31 - 2.21 (m, 1H), 2.07 (tt, *J* = 11.3, 8.4, 3.9 Hz, 2H), 1.92 (ddd, *J* = 11.7, 7.5, 4.2 Hz, 1H), 1.07 (qt, *J* = 8.3, 4.3 Hz, 1H), 0.96 - 0.87 (m, 1H), 0.67 - 0.60 (m, 1H), 0.56 (ddt, *J* = 13.3, 8.9, 4.6 Hz, 1H), 0.28 (dq, *J* = 9.6, 4.9 Hz, 1H), 0.20 (dq, *J* = 9.3, 4.8 Hz, 1H).

### (9) Preparation method for Example 59:

Compound **B-h1** (100 mg), 3-oxacyclobutamine (16 mg), Pd₂(dba)₃ (43 mg), 2-(di-*tert*-butylphosphine)biphenyl (28 mg), and potassium *tert*-butoxide (126 mg) were added to tert-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 59** (22 mg). ESI-MS: m/z = 528.35[M+H] ⁺.

¹H NMR (500 MHz, DMSO-*d*₆) δ 12.34 (s, 1H), 8.42 (s, 1H), 7.94 (s, 1H), 7.55 (s, 1H), 7.35 (d, *J* = 8.6 Hz, 1H), 6.64 (s, 1H), 6.47 (d, *J* = 8.5 Hz, 1H), 6.39 (d, *J* = 5.8 Hz, 1H), 4.85 (t, *J* = 6.3 Hz, 2H), 4.53 - 4.46 (m, 1H), 4.42 (t, *J* = 6.0 Hz, 2H), 4.23 (dd, *J* = 9.3, 4.1 Hz, 1H), 4.19 - 4.10 (m, 2H), 3.97 (s, 1H), 3.78 (s, 3H), 2.55 (s, 3H), 2.25 - 2.16 (m, 1H), 1.23 (d, *J* = 3.8 Hz, 3H), 1.14 (d, *J* = 4.1 Hz, 1H), 0.97 (tt, *J* = 8.5, 3.5 Hz, 1H), 0.54 (ddq, *J* = 17.6, 9.0, 4.5, 3.7 Hz, 2H), 0.34 (dq, *J* = 9.5, 4.7 Hz, 1H), 0.21 (dq, *J* = 8.9, 4.8 Hz, 1H).

### (10) Preparation method for Example 60:

Compound **B-h2** (100 mg), 3-oxacyclobutamine (16 mg), Pd₂(dba)₃ (43 mg), 2-(di-*tert*-butylphosphine)biphenyl (28 mg), and potassium *tert*-butoxide (126 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 60** (35 mg). ESI-MS: m/z = 528.35[M+H] ⁺.

¹H NMR (500 MHz, DMSO-*d*₆) δ 12.35 (s, 1H), 8.42 (s, 1H), 7.94 (s, 1H), 7.58 - 7.51 (m, 1H), 7.26 (d, *J* = 8.5 Hz, 1H), 6.60 (d, *J* = 1.9 Hz, 1H), 6.50 (dd, *J* = 8.6, 2.0 Hz, 1H), 6.40 (d, *J=* 7.1 Hz, 1H), 4.90 (t, *J* = 6.5 Hz, 2H), 4.63 (h, *J* = 6.7 Hz, 1H), 4.43 (td, *J* = 6.1, 1.6 Hz, 2H), 4.25 (dd, *J* = 9.2, 4.1 Hz, 1H), 4.21 - 4.11 (m, 2H), 4.01 - 3.94 (m, 1H), 3.78 (s, 3H), 2.55 (s, 3H), 2.29 - 2.19 (m, 1H), 1.98 - 1.89 (m, 2H), 1.15 (ddt, *J* = 15.7, 8.7, 3.0 Hz, 2H), 1.04 - 0.97 (m, 1H), 0.56 (dtt, *J* = 25.5, 8.8, 4.3 Hz, 2H), 0.36 (dt, *J* = 9.3, 4.6 Hz, 1H), 0.27 - 0.20 (m, 1H).

### Example 61:

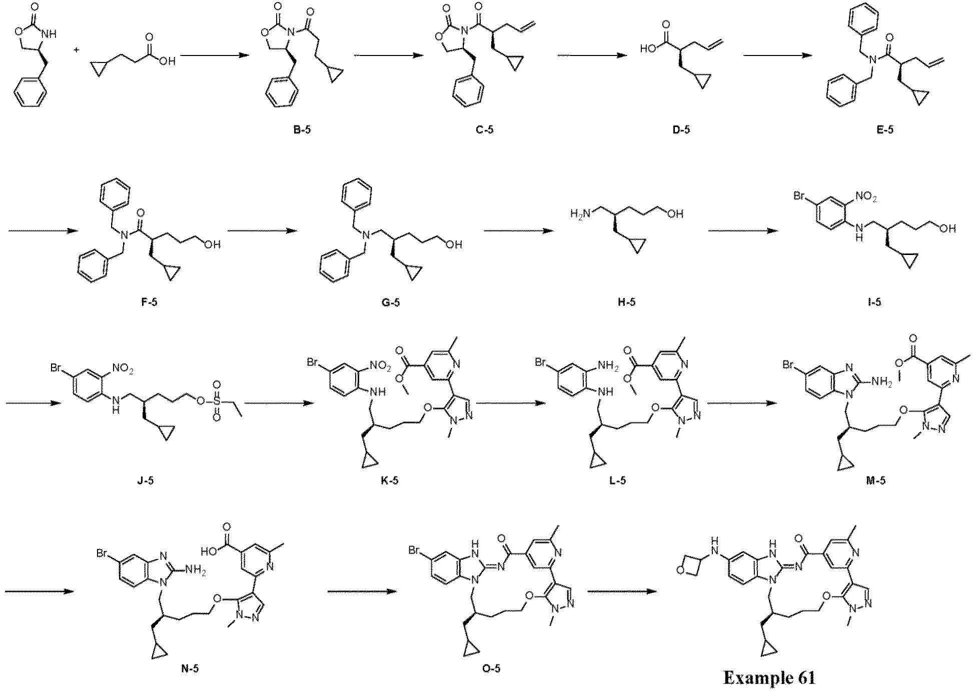

### (1) Preparation method for compound B-5:

(*S*)-4-benzyl-2-oxazolidinone (14.1 g), 3-cyclopropylpropionic acid (10 g), and 4-dimethylaminopyridine (10.7 g) were added to dichloromethane (200 mL), and 1-(3-dimethylaminopropyl)-3-ethylcarbodiimide hydrochloride (30.6 g) was added at 20-25 °C. After the addition, the mixture was stirred at 20-25 °C for 12 h. After the reaction was completed, 75 mL of dichloromethane was added to the reaction solution. The dichloromethane phase was sequentially washed with water (150 mL), 1 M hydrochloric acid (150 mL × 2), and 1 M sodium bicarbonate solution (150 mL × 2), and the dichloromethane phase was retained, dried over anhydrous sodium sulfate, concentrated until no liquid flowed out, and subjected to column chromatography to give compound **B-5** (19.1 g), ESI-MS: m/z = 274.14[M+H] ⁺.

### (2) Preparation method for compound C-5:

Compound **B-5** (6.0 g) was added to tetrahydrofuran (60 mL), and the mixture was purged with nitrogen three times and cooled (set temperature: -78 °C). When the internal temperature reached -70 °C, sodium bis(trimethylsilyl)amide (2 M, 16.5 mL) was added dropwise over 15 min, and the mixture was stirred for 1 h with the temperature kept within -70 °C. Then 3-bromopropylene (5.3 g) was added dropwise, and the mixture was stirred for 1 h with the temperature kept within -70 °C, slowly warmed to room temperature, and stirred for 12 h. After the reaction was completed, the reaction solution was poured into an ammonium chloride solution (1 M, 400 mL), and the resulting mixture was extracted with ethyl acetate (50 mL × 2). The ethyl acetate phases were combined, dried over anhydrous sodium sulfate, concentrated until no liquid flowed out, and subjected to column chromatography to give compound **C-5** (3.5 g), ESI-MS: m/z = 314.19[M+H] ⁺.

### (3) Preparation method for compound D-5:

Compound **C-5** (2.8 g) was added to tetrahydrofuran (30 mL), and the mixture was cooled to 0-5 °C in an ice bath. A solution of lithium hydroxide monohydrate (1.1 g) in water (10 mL) was added, and the mixture was stirred for 5 min. Then hydrogen peroxide (30 wt%, 3.65 mL) was added, and the mixture was stirred for 1 h with the temperature kept at 0-5 °C. After the reaction was completed, a sodium sulfite solution (1 M, 30 mL) and a sodium bicarbonate solution (1 M, 30 mL) were added to the reaction solution, and the resulting mixture was extracted with dichloromethane (30 mL × 2). The aqueous phase was retained, adjusted to pH 1-2 with 4 M hydrochloric acid, and extracted with dichloromethane (30 mL × 2). The dichloromethane phases were combined, dried over anhydrous sodium sulfate, and concentrated until no liquid flowed out to give compound **D-5,** which was directly used in the next step without further purification.

### (4) Preparation method for compound E-5:

Compound **D-5** obtained in the previous step was dissolved in dichloromethane (30 mL), and dibenzylamine (1.8 g), *N*,*N*-diisopropylethylamine (4.43 mL), and HATU (4.1 g) were sequentially added. After the addition, the reaction was stirred at 20-25 °C for 12 h, and completed. The reaction solution was washed with water (30 mL × 2). The dichloromethane phase was retained, dried over anhydrous sodium sulfate, concentrated until no liquid flowed out, and subjected to column chromatography to give compound **E-5** (2.8 g). ESI-MS: m/z = 334.30[M+H] ⁺.

### (5) Preparation method for compound F-5:

Compound **E-5** (2.8 g) was added to tetrahydrofuran (30 mL), and 9-borabicyclo[3.3.1]nonane (0.5 M, 42 mL) was added. The mixture was stirred at 20-25 °C for 1 h, and then cooled to 0-5 °C in an ice bath. A solution of sodium hydroxide (672 mg) in water (5 mL) and hydrogen peroxide (30 wt%, 4.29 mL) were sequentially added. The mixture was stirred at 20-25 °C for 2 h. After the reaction was completed, the reaction solution was poured into water (30 mL), and the resulting mixture was extracted with ethyl acetate (30 mL × 2). The ethyl acetate phases were combined, dried over anhydrous sodium sulfate, concentrated until no liquid flowed out, and subjected to column chromatography to give compound **F-5** (2.08 mg). ESI-MS: m/z = 352.30[M+H] ⁺.

### (6) Preparation method for compound G-5:

Compound **F-5** (2.08 g) was added to tetrahydrofuran (30 mL), and the mixture was cooled to 0-5 °C. Borane dimethyl sulfide (2 M, 10.22 mL) was added dropwise. After the addition, the mixture was stirred at 60 °C for 4 h. After the reaction was completed, the reaction solution was cooled to 0-5 °C in an ice bath, and the reaction was quenched by adding diluted hydrochloric acid (1M) dropwise to the reaction solution. The reaction solution was concentrated by rotary evaporation to dryness. The concentrate was dissolved in ethyl acetate (50 mL). The organic phase was sequentially washed with a saturated sodium bicarbonate solution (50 mL × 1) and saturated brine (50 mL × 1), dried, concentrated, and then separated by column chromatography to give **G-5** (1.45 g). ESI-MS: m/z = 338.40[M+H] ⁺.

### (7) Preparation method for compound H-5:

Compound **G-5** (510 mg) was added to methanol (10 mL), and Pd/C (10%, 51 mg) was added. The mixture was purged with hydrogen three times and stirred in a water bath at 40-45 °C for 2 h. After the reaction was completed, the reaction solution was filtered and concentrated until no liquid flowed out to give compound **H-5,** which was directly used in the next step without further purification.

### (8) Preparation method for compound 1-5:

Compound **H-5** obtained in the previous step was dissolved in *N*,*N*-dimethylformamide (10 mL), and 4-bromo-1-fluoro-2-nitrobenzene (333 mg) and potassium carbonate (460 mg) were sequentially added. The mixture was stirred in a water bath at 40-45 °C for 6 h. After the reaction was completed, the reaction solution was poured into 50 mL of water, and the resulting mixture was extracted with ethyl acetate (10 mL × 2). The ethyl acetate phases were combined, dried over anhydrous sodium sulfate, concentrated until no liquid flowed out, and subjected to column chromatography to give compound **1-5** (387 mg). ESI-MS: m/z = 357.14[M+H] ⁺.

### (9) Preparation method for compound J-5:

Compound **1-5** (387 mg) was dissolved in dichloromethane (8 mL), and triethylamine (164 mg) was added. The mixture was cooled to 0-5 °C. A solution of ethylsulfonyl chloride (168 mg) in dichloromethane (5 mL) was added dropwise. After the dropwise addition, the mixture was reacted at 20-25 °C for 1 h. After the reaction was completed, the reaction solution was washed with a sodium bicarbonate solution (1 M, 30 mL × 2). The dichloromethane phase was retained, dried over anhydrous sodium sulfate, and concentrated until no liquid flowed out to give compound **J-5,** which was directly used in the next step without further purification.

### (10) Preparation method for compound K-5:

Compound **J-5** obtained in the previous step, compound **A** (306 mg), and potassium carbonate (416 mg) were added to *N,N-*dimethylformamide (20 mL), and the mixture was reacted at 60 °C for 20 h. After the reaction was completed, the reaction solution was poured into 50 mL of water, and extracted with ethyl acetate (30 mL × 2). The ethyl acetate phases were combined. The organic phase was washed once with water, dried over anhydrous sodium sulfate, concentrated until no liquid flowed out, and subjected to column chromatography to give compound **K-5** (450 mg). ESI-MS: m/z = 586.22[M+H] ⁺.

### (11) Preparation method for compound L-5:

Compound **K-5** (450 mg) was dissolved in methanol (5 mL), and Raney nickel (20 mg) was added. The mixture was purged with hydrogen 3 times, and reacted at 25 °C for 2 h. After the reaction was completed, the reaction solution was filtered, and the filtrate was dried over anhydrous sodium sulfate and concentrated until no liquid flowed out to give compound **L-5,** which was directly used in the next step without further purification.

### (12) Preparation method for compound M-5:

Compound **L-5** obtained in the previous step was dissolved in *tert*-butanol (10 mL) and dichloromethane (10 mL), and a solution of cyanogen bromide (163 mg) in dichloromethane (5 mL) was added dropwise. The mixture was reacted at 25 °C for 12 h. After the reaction was completed, a sodium bicarbonate solution (1 M, 10 mL) was added to the reaction solution, and the resulting mixture was subjected to liquid separation. The dichloromethane phase was retained. The organic phase was washed with a sodium bicarbonate solution (1 M, 10 mL × 2), dried over anhydrous sodium sulfate, and concentrated until no liquid flowed out to give compound **M-5,** which was directly used in the next step without further purification. ESI-MS: m/z = 581.19[M+H] ⁺.

### (13) Preparation method for compound N-5:

Compound **M-5** obtained in the previous step was dissolved in tetrahydrofuran (5 mL), and a solution of sodium hydroxide (123 mg) in water (5 mL) was added. The mixture was stirred at 20-25 °C for 45 min. After the reaction was completed, the mixture was adjusted to pH 5-6 with 6 M hydrochloric acid, and concentrated by rotary evaporation under reduced pressure to remove tetrahydrofuran and water. Dichloromethane (10 mL) and triethylamine (0.43 mL) were added, and the mixture was stirred for complete dissolution, dried over anhydrous sodium sulfate, filtered, and concentrated until no liquid flowed out to give compound **N-5,** which was directly used in the next step without further purification. ESI-MS: m/z = 567.18[M+H] ⁺.

### (14) Preparation method for compound O-5:

Compound **N-5** obtained in the previous step was dissolved in dichloromethane (5 mL), and TBTU (297 mg) was added. The mixture was reacted at 20-25 °C for 16 h. After the reaction was completed, the reaction solution was washed with water (10 mL × 3). The organic phase was dried over anhydrous sodium sulfate, concentrated until no liquid flowed out, and subjected to column chromatography to give compound **O-5** (240 mg). ESI-MS: m/z = 549.20[M+H] ⁺.

### (15) Preparation method for Example 61:

Compound **O-5** (60 mg), 3-oxacyclobutamine (12 mg), Pd₂(dba)₃ (25 mg), 2-(di-*tert*-butylphosphine)biphenyl (16 mg), and potassium *tert*-butoxide (73 mg) were added to *tert-amyl* alcohol (5 mL). The mixture was purged with nitrogen 3 times and reacted at 90 °C for 2 h. After the reaction was completed, 30 mL of water was added to the reaction solution, and the resulting mixture was extracted with ethyl acetate (30 mL × 3). The organic phases were combined, dried over anhydrous sodium sulfate, concentrated until no liquid flowed out, and purified by preparative liquid phase chromatography to give **Example 61** (8 mg). ESI-MS: m/z = 542.46[M+H] ⁺.

¹H NMR (500 MHz, chloroform-*d*) δ 12.04 (s, 1H), 8.41 (s, 1H), 8.15 (s, 1H), 7.66 (s, 1H), 7.08 (d, *J* = 8.6 Hz, 1H), 6.50 (dd, *J=* 8.5, 2.1 Hz, 1H), 6.43 (d, *J* = 2.2 Hz, 1H), 5.04 (t, *J=* 6.6 Hz, 2H), 4.63 (d, *J* = 7.8 Hz, 1H), 4.55 (t, *J* = 6.1 Hz, 2H), 4.45 - 4.34 (m, 2H), 4.01 (q, *J* = 7.6 Hz, 1H), 3.79 (s, 3H), 2.73 (s, 1H), 2.64 (s, 3H), 2.34 (dt, *J* = 13.8, 6.9 Hz, 1H), 2.13 - 2.06 (m, 1H), 2.01 (d, *J* = 6.4 Hz, 1H), 1.86 (q, *J* = 7.2, 6.0 Hz, 2H), 1.31 (s, 2H), 0.65 - 0.57 (m, 1H), 0.41 (ddt, *J* = 13.3, 8.7, 4.6 Hz, 2H), -0.04 (t, *J* = 4.1 Hz, 1H), -0.09 (dq, *J* = 11.4, 6.6, 5.4 Hz, 1H).

### Example 62:

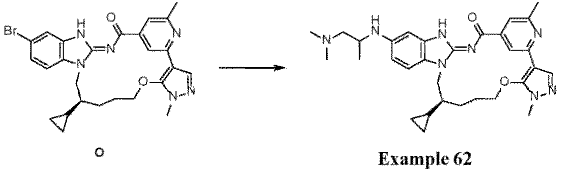

Compound **O** (50 mg), 1-(dimethylamino-2-propylamine) (14 mg), Pd₂(dba)₃ (21 mg), *2-(di-tert-*butylphosphine)biphenyl (14 mg), and potassium *tert*-butoxide (63 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 62** (10 mg). ESI-MS: m/z = 557.45 [M+H] ⁺.

¹H NMR (500 MHz, DMSO-*d*₆) δ 12.40 (s, 1H), 8.39 (s, 1H), 8.00 (s, 1H), 7.56 (s, 1H), 7.30 (d, *J* = 8.8 Hz, 1H), 6.85 (s, 1H), 6.64 (d, *J* = 8.6 Hz, 1H), 4.34 (s, 1H), 4.27 (d, *J* = 13.5 Hz, 1H), 4.04 (d, *J* = 22.0 Hz, 2H), 3.77 (s, 3H), 2.58 (s, 3H), 2.41 (d, *J* = 12.0 Hz, 1H), 2.22 (s, 6H), 2.15 (d, *J* = 10.1 Hz, 2H), 2.05 (d, *J* = 13.1 Hz, 2H), 1.84 (h, *J* = 7.3, 5.2 Hz, 1H), 1.75 (tt, *J* = 12.6, 5.9 Hz, 1H), 1.21 (d, *J* = 6.3 Hz, 3H), 0.91 (s, 1H), 0.65 (s dhept, *J* = 8.5, 5.0 Hz, 1H), 0.37 (tt, *J* = 9.1, 4.8 Hz, 1H), -0.01 (dq, *J* = 9.5, 5.3, 4.7 Hz, 1H), -0.09 (tt, *J* = 9.7, 4.8 Hz, 1H), -0.55 (dq, *J* = 9.7, 4.9 Hz, 1H).

### Example 63:

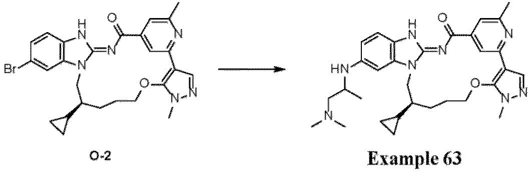

Compound **O-2** (50 mg), 1-(dimethylamino)-2-propylamine (14 mg), Pd₂(dba)₃ (21 mg), 2-(di-*tert-*butylphosphine)biphenyl (14 mg), and potassium *tert*-butoxide (63 mg) were added to *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 63** (10 mg). ESI-MS: m/z = 557.47[M+H] ⁺.

¹H NMR (500 MHz, chloroform-*d*) δ 11.91 (s, 1H), 8.39 (s, 1H), 8.15 (s, 1H), 7.65 (s, 1H), 7.13 (dd, *J* = 8.5, 1.4 Hz, 1H), 6.63 (t, *J* = 2.6 Hz, 1H), 6.59 (dt, *J* = 8.7, 2.4 Hz, 1H), 4.36 (ddt, *J* = 14.1, 9.6, 4.9 Hz, 2H), 3.94 (ddt, *J* = 20.6, 13.4, 4.0 Hz, 2H), 3.78 (s, 3H), 3.58 - 3.48 (m, 1H), 2.64 (s, 4H), 2.33 (d, *J* = 7.3 Hz, 6H), 2.17 (qd, *J* = 7.4, 4.2 Hz, 2H), 2.01 (d, *J* = 6.5 Hz, 1H), 1.85 - 1.76 (m, 2H), 1.33 - 1.30 (m, 1H), 1.27 (d, *J* = 6.2 Hz, 3H), 0.60 - 0.51 (m, 1H), 0.45 (tq, *J* = 9.8, 5.3 Hz, 1H), 0.14 (tt, *J* = 9.2, 5.2 Hz, 1H), -0.02 (d, *J* = 5.7 Hz, 1H), -0.25 - -0.33 (m, 1H).

### Example 64:

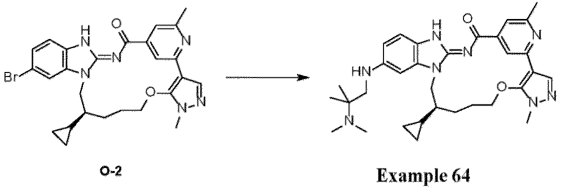

### (1) Preparation method for Example 64:

Compound **O-2** (100 mg), (2-amino-1,1-dimethylethyl)dimethylamine dihydrochloride (42 mg), Pd₂(dba)₃ (43 mg), 2-(di-*tert*-butylphosphine)biphenyl (28 mg), and potassium *tert*-butoxide (126 mg) were dispersed in *tert-amyl* alcohol (3 mL). The mixture was purged with nitrogen 3 times and reacted at 95 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 64** (20 mg). ESI-MS: m/z = 571.42 [M+H] ⁺.

### Example 65:

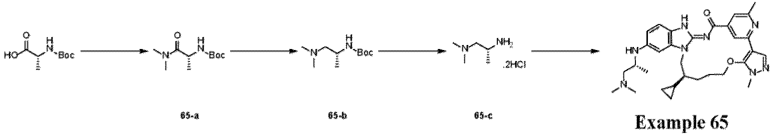

### (1) Preparation method for compound 65-a:

Compound BOC-D-alanine (10.0 g) was dispersed in dichloromethane (150 mL), and dimethylamine hydrochloride (6.5 g), HATU (22.1 g), and *N*,*N*-diisopropylethylamine (20.5 g) were sequentially added. The mixture was reacted at 25 °C for 16 h. After the reaction was completed as detected by TLC, the reaction solution was washed with an aqueous NaHCOs solution (1 M, 100 mL × 2). The organic phase was retained, dried, concentrated by rotary evaporation to dryness, and subjected to column chromatography to give compound **65-a** (9.1 g).

### (2) Preparation method for compound 65-b:

Compound **65-a** (9.1 g) was dispersed in tetrahydrofuran (100 mL), and the mixture was cooled to 0-5 °C. A solution of LiAlH₄ in tetrahydrofuran (1 M, 105 mL) was added dropwise with the temperature kept at this temperature. The mixture was reacted at 0-5 °C for 2 h. After the reaction was completed as detected by TLC, water (7 mL), an aqueous NaOH solution (15%, 7 mL), and water (21 mL) were sequentially added dropwise to the reaction solution, and the resulting mixture was filtered. The filtrate was dried, concentrated by rotary evaporation to dryness, and subjected to column chromatography to give compound **65-b** (4.5 g).

### (3) Preparation method for compound 65-c:

Compound **65-b** (4.5 g) was dispersed in a hydrogen chloride/1,4-dioxane solution (4 M, 56 mL), and the mixture was reacted at 25 °C for 16 h. After the reaction was completed as detected by TLC, the reaction solution was concentrated. Ethyl acetate (40 mL) was added, and the resulting mixture was stirred at 25 °C for 1 h and filtered. The filter cake was dried to give compound **65-c** (2.2 g).

### (4) Preparation method for Example 65:

Compound **O-2** (100 mg), compound **65-c** (23 mg), Pd₂(dba)₃ (43 mg), 2-(di-*tert*-butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 65** (8.6 mg). ESI-MS: m/z = 557.45 [M+H] ⁺.

¹H NMR (500 MHz, DMSO-*d*₆) δ 12.41 (s, 1H), 8.37 (s, 1H), 7.93 (d, *J* = 7.0 Hz, 1H), 7.57 (d, *J* = 6.9 Hz, 1H), 7.28 (d, *J* = 8.2 Hz, 1H), 6.80 (s, 1H), 6.59 (d, *J* = 8.1 Hz, 1H), 5.36 (s, 1H), 4.34 - 4.24 (m, 2H), 4.10 - 4.03 (m, 2H), 3.76 (s, 3H), 3.63 (d, *J* = 6.7 Hz, 1H), 2.58 (s, 3H), 2.45 (t, *J* = 6.1 Hz, 1H), 2.36 - 2.31 (m, 1H), 2.27 (s, 6H), 2.03 (qd, *J* = 11.8, 6.8, 6.1 Hz, 3H), 1.85 (s, 1H), 1.79 - 1.71 (m, 1H), 1.14 (d, *J* = 6.3 Hz, 3H), 0.64 (dhept, *J* = 8.5, 5.0 Hz, 1H), 0.37 (tt, *J* = 9.1, 4.8 Hz, 1H), 0.00 (dq, *J* = 9.5, 5.3, 4.7 Hz, 1H), -0.07 (tt, *J* = 9.7, 4.8 Hz, 1H), -0.52 (dq, *J* = 9.7, 4.9 Hz, 1H).

### Example 66:

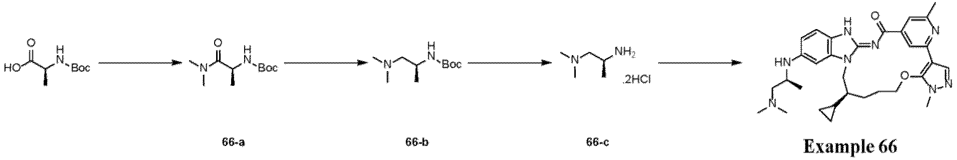

### (1) Preparation method for compound 66-a:

Compound *N*-*tert*-butoxycarbonyl-L-alanine (15.0 g) was dispersed in dichloromethane (200 mL), and dimethylamine hydrochloride (9.7 g), HATU (33.2 g), and *N*,*N*-diisopropylethylamine (30.7 g) were sequentially added. The mixture was reacted at 25 °C for 16 h. After the reaction was completed as detected by TLC, the reaction solution was washed with an aqueous NaHCOs solution (1 M, 100 mL × 2). The organic phase was retained, dried, concentrated by rotary evaporation to dryness, and subjected to column chromatography to give compound **66-a** (13.3 g).

### (2) Preparation method for compound 66-b:

Compound **66-a** (13.3 g) was dispersed in tetrahydrofuran (150 mL), and the mixture was cooled to 0-5 °C. A solution of LiAlH₄ in tetrahydrofuran (1 M, 154 mL) was added dropwise with the temperature kept at this temperature. The mixture was reacted at 0-5 °C for 2 h. After the reaction was completed as detected by TLC, water (11 mL), an aqueous NaOH solution (15%, 11 mL), and water (33 mL) were sequentially added dropwise to the reaction solution, and the resulting mixture was filtered. The filtrate was dried, concentrated by rotary evaporation to dryness, and subjected to column chromatography to give compound **66-b** (5.7 g).

### (3) Preparation method for compound 66-c:

Compound **66-b** (5.7 g) was dispersed in a hydrogen chloride/1,4-dioxane solution (4 M, 70 mL), and the mixture was reacted at 25 °C for 16 h. After the reaction was completed as detected by TLC, the reaction solution was concentrated. Ethyl acetate (50 mL) was added, and the resulting mixture was stirred at 25 °C for 1 h and filtered. The filter cake was dried to give compound **66-c** (3.0 g).

### (4) Preparation method for Example 66:

Compound **O-2** (100 mg) in Example 8, compound **66-c** (23 mg), Pd₂(dba)₃ (43 mg), *2-(di-tert-*butylphosphine)biphenyl (28 mg), and potassium *tert*-butoxide (126 mg) were dispersed in *tert-amyl* alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 66** (13 mg). ESI-MS: m/z = 557.45 [M+H] ⁺.

¹H NMR (500 MHz, DMSO-*d*₆) δ 12.41 (s, 1H), 8.38 (s, 1H), 7.95 (s, 1H), 7.58 (s, 1H), 7.28 (d, *J* = 8.6 Hz, 1H), 6.80 (d, *J* = 2.1 Hz, 1H), 6.58 (dd, *J* = 8.6, 2.0 Hz, 1H), 5.30 (d, *J* = 7.7 Hz, 1H), 4.33 (td, *J* = 8.6, 6.0 Hz, 1H), 4.26 (dd, *J* = 14.0, 3.6 Hz, 1H), 4.07 (td, *J* = 9.5, 4.1 Hz, 2H), 3.77 (s, 3H), 3.60 (p, *J* = 7.0 Hz, 1H), 2.58 (s, 3H), 2.33 (dd, *J* = 12.0, 5.5 Hz, 1H), 2.27 - 2.23 (m, 1H), 2.19 (s, 6H), 2.19 - 2.13 (m, 2H), 2.04 (ddt, *J* = 13.1, 8.8, 4.9 Hz, 1H), 1.86 (h, *J* = 7.3, 5.2 Hz, 1H), 1.75 (tt, *J* = 12.6, 5.9 Hz, 1H), 1.20 (d, *J* = 6.2 Hz, 3H), 0.64 (dhept, *J* = 8.5, 5.0 Hz, 1H), 0.36 (tt, *J=* 9.1, 4.8 Hz, 1H), 0.00 (dq, *J=* 9.5, 5.3, 4.7 Hz, 1H), -0.07 (tt, *J* = 9.7, 4.8 Hz, 1H), -0.53 (dq, *J* = 9.7, 4.9 Hz, 1H).

### Example 67:

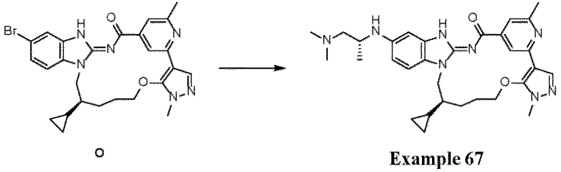

Compound **O** (100 mg), compound **65-c** (23 mg), Pd₂(dba)₃ (43 mg), 2-(di-*tert*-butylphosphine)biphenyl (28 mg), and potassium *tert*-butoxide (126 mg) were dispersed in *tert-amyl* alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 67** (10.1 mg). ESI-MS: m/z = 557.47 [M+H] ⁺.

¹H NMR (500 MHz, DMSO-*d*₆) δ 8.34 (s, 1H), 8.22 (s, 1H), 7.91 (s, 1H), 7.54 (d, *J* = 1.2 Hz, 1H), 7.32 (d, *J* = 8.7 Hz, 1H), 6.79 (d, *J* = 2.2 Hz, 1H), 6.60 (dd, *J* = 8.7, 2.2 Hz, 1H), 4.31 - 4.26 (m, 1H), 4.22 (dd, *J* = 13.9, 3.6 Hz, 1H), 4.03 (ddd, *J* = 13.7, 9.7, 5.3 Hz, 2H), 3.73 (s, 3H), 3.46 (d, *J* = 6.7 Hz, 1H), 2.54 (s, 3H), 2.40 (dd, *J* = 12.1, 6.0 Hz, 1H), 2.23 (s, 6H), 2.22 - 2.19 (m, 1H), 2.14 (dp, *J* = 13.0, 7.4, 6.3 Hz, 1H), 1.98 (dq, *J* = 19.2, 7.5, 6.1 Hz, 1H), 1.80 (s, 1H), 1.74 - 1.65 (m, 1H), 1.25 (dd, *J* = 8.6, 5.0 Hz, 1H), 1.15 (d, *J* = 6.2 Hz, 3H), 0.59 (tt, *J* = 8.6, 3.7 Hz, 1H), 0.31 (tt, *J* = 9.2, 4.7 Hz, 1H), -0.05 (dq, *J* = 9.6, 4.9 Hz, 1H), -0.14 (tt, *J* = 9.2, 4.6 Hz, 1H), -0.58 (dq, *J* = 9.7, 4.9 Hz, 1H).

### Example 68:

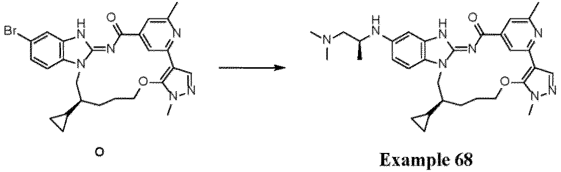

Compound **O** (100 mg), compound **66-c** (23 mg), Pd₂(dba)₃ (43 mg), 2-(di-*tert*-butylphosphine)biphenyl (28 mg), and potassium *tert*-butoxide (126 mg) were dispersed in *tert-amyl* alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 68** (15 mg). ESI-MS: m/z = 557.47 [M+H] ⁺. ¹H NMR (500 MHz, DMSO-*d*₆) δ 12.44 (s, 1H), 8.39 (s, 1H), 7.96 (s, 1H), 7.59 (s, 1H), 7.37 (d, *J* = 8.8 Hz, 1H), 6.83 (s, 1H), 6.65 (d, *J* = 8.6 Hz, 1H), 4.34 (s, 1H), 4.27 (d, *J* = 13.5 Hz, 1H), 4.06 (d, *J* = 22.0 Hz, 2H), 3.78 (s, 3H), 2.59 (s, 3H), 2.40 (d, *J* = 12.0 Hz, 1H), 2.25 (s, 6H), 2.20 (d, *J* = 10.1 Hz, 2H), 2.05 (d, *J* = 13.1 Hz, 2H), 1.85 (h, *J* = 7.3, 5.2 Hz, 1H), 1.75 (tt, *J* = 12.6, 5.9 Hz, 1H), 1.21 (d, *J* = 6.3 Hz, 3H), 0.91 (s, 1H), 0.64 (s dhept, *J* = 8.5, 5.0 Hz, 1H), 0.36 (tt, *J* = 9.1, 4.8 Hz, 1H), -0.00 (dq, *J* = 9.5, 5.3, 4.7 Hz, 1H), -0.09 (tt, *J* = 9.7, 4.8 Hz, 1H), -0.53 (dq, *J* = 9.7, 4.9 Hz, 1H).

### Example 69:

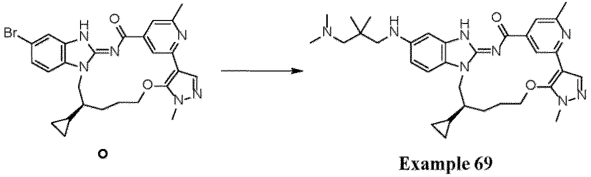

Compound **O** (70 mg), *N*,*N*,2,2-tetratnethyl-1,3-propylenediatnine (20 mg), Pd₂(dba)₃ (30 mg), 2-(di-*tert-*butylphosphine)biphenyl (19 mg), and potassium *tert*-butoxide (88 mg) were dispersed in *tert*-amyl alcohol (5 mL). The mixture was purged with nitrogen 3 times and reacted at 95 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 69** (10 mg). ESI-MS: m/z = 585.41 [M+H] ⁺.

¹H NMR (500 MHz, DMSO-*d*₆) δ 12.36 (s, 1H), 8.33 (s, 1H), 7.90 (s, 1H), 7.54 (d, *J* = 1.3 Hz, 1H), 7.32 (d, *J* = 9.0 Hz, 1H), 6.80 (s, 1H), 6.72 - 6.62 (m, 2H), 4.29 (q, *J* = 7.7 Hz, 1H), 4.22 (dd, *J* = 14.0, 3.5 Hz, 1H), 4.06 - 4.00 (m, 2H), 3.73 (s, 3H), 3.51 (d, *J* = 3.0 Hz, 1H), 2.87 (s, 2H), 2.54 (s, 3H), 2.51 (s, 6H), 2.03 - 1.95 (m, 4H), 1.81 (d, *J* = 11.1 Hz, 1H), 1.73 - 1.68 (m, 1H), 0.96 (s, 6H), 0.61 - 0.56 (m, 1H), 0.30 (dd, *J* = 9.5, 5.5 Hz, 1H), -0.00 (m, 1H), - 0.14 - -0.19 (m, 1H), -0.60 (dd, *J* = 9.5, 4.9 Hz, 1H).

### Example 70:

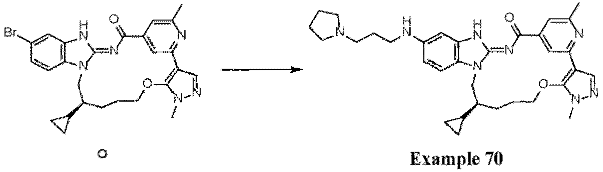

Compound **O** (75 mg), 1-(3-aminopropyl)pyrrolidine (36 mg), Pd₂(dba)₃ (32 mg), 2-(di-*tert*-butylphosphine)biphenyl (21 mg), and potassium *tert*-butoxide (94 mg) were dispersed in *tert-amyl* alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 70** (20 mg). ESI-MS: m/z = 583.40 [M+H] ⁺.

¹H NMR (500 MHz, chloroform-*d*) δ 12.09 (s, 1H), 8.41 (s, 1H), 8.15 (s, 1H), 7.71 (s, 1H), 7.14 (d, *J* = 8.6 Hz, 1H), 6.62 (d, *J* = 8.7 Hz, 1H), 6.49 (s, 1H), 4.41 - 4.31 (m, 2H), 3.93 (dq, *J* = 20.6, 6.0 Hz, 2H), 3.79 (s, 3H), 3.23 (q, *J* = 5.8 Hz, 2H), 2.96 (t, *J* = 5.9 Hz, 2H), 2.86 (s, 4H), 2.64 (s, 3H), 2.27 (d, *J* = 7.3 Hz, 1H), 2.21 - 2.14 (m, 2H), 2.00 (q, *J* = 7.0 Hz, 2H), 1.92 (s, 4H), 1.83 - 1.76 (m, 2H), 0.51 (s, 1H), 0.46 - 0.38 (m, 1H), 0.09 (t, *J* = 10.5 Hz, 1H), - 0.02 - -0.10 (m, 1H), -0.34 (dd, *J* = 9.4, 5.1 Hz, 1H).

### Example 71:

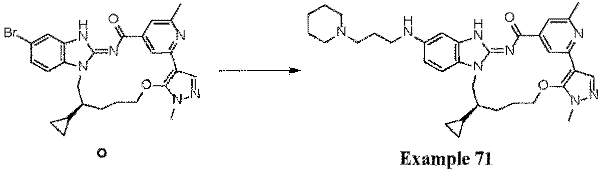

Compound **O** (100 mg), 1-(3-aminopropyl)piperidine (54 mg), Pd₂(dba)₃ (35 mg), 2-(di-*tert-*butylphosphine)biphenyl (23 mg), and potassium *tert*-butoxide (128 mg) were dispersed in *tert*-amyl alcohol (3 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 71** (19 mg). ESI-MS: m/z = 597.42 [M+H] ⁺.

¹H NMR (500 MHz, DMSO-*d*₆) δ 12.36 (s, 1H), 8.34 (s, 1H), 7.90 (s, 1H), 7.55 (d, *J* = 1.2 Hz, 1H), 7.32 (d, *J* = 8.8 Hz, 1H), 6.73 (d, v = 2.3 Hz, 1H), 6.56 (dd, *J* = 8.7, 2.2 Hz, 1H), 4.33 - 4.26 (m, 1H), 4.22 (dd, *J* = 13.9, 3.5 Hz, 1H), 4.07 - 3.97 (m, 2H), 3.73 (s, 3H), 3.02 (t, *J* = 6.9 Hz, 2H), 2.54 (s, 3H), 2.35 (t, *J* = 7.0 Hz, 3H), 2.32 (s, 2H), 2.21 (p, *J* = 6.6, 6.1 Hz, 1H), 2.13 (dq, *J* = 9.5, 4.3 Hz, 1H), 1.98 (dp, *J* = 13.2, 4.8, 4.3 Hz, 1H), 1.81 (d, *J* = 9.3 Hz, 1H), 1.71 (h, *J* = 6.3, 5.7 Hz, 3H), 1.51 (p, *J* = 5.6 Hz, 4H), 1.39 (q, *J* = 6.0 Hz, 2H), 1.25 (td, *J* = 10.8, 9.2, 4.7 Hz, 1H), 0.57 (tdd, *J* = 10.1, 6.5, 4.0 Hz, 1H), 0.30 (dp, *J* = 9.4, 4.6 Hz, 1H), -0.06 (dq, *J* = 9.5, 4.8 Hz, 1H), -0.16 (tt, *J* = 9.5, 4.6 Hz, 1H), -0.61 (dq, *J* = 9.7, 4.9 Hz, 1H).

### Example 72:

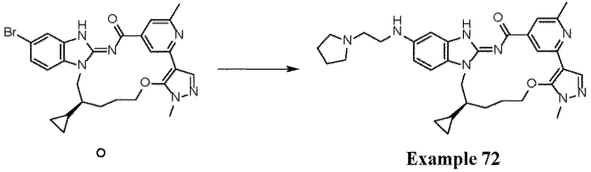

Compound **O** (75 mg), 1-(2-aminoethyl)pyrrolidine (32 mg), Pd₂(dba)₃ (32 mg), 2-(di-*tert*-butylphosphine)biphenyl (21 mg), and potassium *tert*-butoxide (94 mg) were dispersed in *tert-amyl* alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 72** (16 mg). ESI-MS: m/z = 569.46 [M+H] ⁺.

¹H NMR (500 MHz, chloroform-*d*) δ 12.09 (s, 1H), 8.41 (s, 1H), 8.15 (s, 1H), 7.71 (s, 1H), 7.14 (d, *J* = 8.6 Hz, 1H), 6.62 (d, *J* = 8.7 Hz, 1H), 6.49 (s, 1H), 4.41 - 4.31 (m, 2H), 3.93 (dq, *J* = 20.6, 6.0 Hz, 2H), 3.79 (s, 3H), 3.23 (q, *J* = 5.8 Hz, 2H), 2.96 (t, *J* = 5.9 Hz, 2H), 2.86 (s, 4H), 2.64 (s, 3H), 2.27 (d, *J* = 7.3 Hz, 1H), 2.21 - 2.14 (m, 2H), 1.92 (s, 4H), 1.83 - 1.76 (m, 2H), 0.51 (s, 1H), 0.46 - 0.38 (m, 1H), 0.09 (t, *J* = 10.5 Hz, 1H), -0.02 - -0.10 (m, 1H), -0.34 (dd, *J* = 9.4, 5.1 Hz, 1H).

### Example 73:

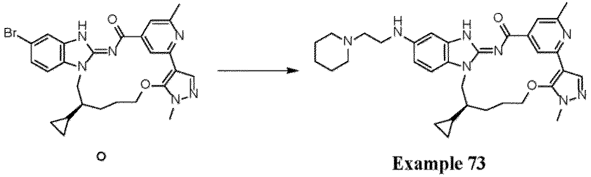

Compound **O** (75 mg), 1-(2-aminoethyl)piperidine (36 mg), Pd₂(dba)₃ (32 mg), 2-(di-*tert*-butylphosphine)biphenyl (21 mg), and potassium *tert*-butoxide (94 mg) were dispersed in *tert-amyl* alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 73** (31 mg). ESI-MS: m/z = 583.40 [M+H] ⁺.

¹H NMR (500 MHz, chloroform-*d*) δ 12.17 (s, 1H), 8.57 (s, 1H), 8.42 (s, 1H), 8.16 (s, 1H), 7.75 (d, *J* = 1.4 Hz, 1H), 7.13 (d, *J* = 8.7 Hz, 1H), 6.65 (dd, *J* = 8.7, 2.1 Hz, 1H), 6.45 (d, *J* = 2.1 Hz, 1H), 4.35 (dd, *J* = 13.6, 3.9 Hz, 2H), 3.93 (ddt, *J* = 21.8, 13.8, 6.5 Hz, 2H), 3.79 (s, 3H), 3.18 (dq, *J=* 12.2, 6.7, 6.2 Hz, 2H), 2.95 (q, *J* = 6.8 Hz, 2H), 2.81 (s, 4H), 2.64 (s, 3H), 2.21 (ddt, *J* = 30.1, 22.6, 7.5 Hz, 3H), 1.80 (p, *J* = 5.9 Hz, 6H), 1.56 (s, 2H), 0.50 (d, *J* = 8.0 Hz, 1H), 0.42 (dq, *J* = 8.9, 4.7 Hz, 1H), 0.14 - 0.05 (m, 1H), -0.05 (dq, *J* = 10.0, 4.9 Hz, 1H), -0.35 (dq, *J* = 9.9, 5.1 Hz, 1H).

### Example 74:

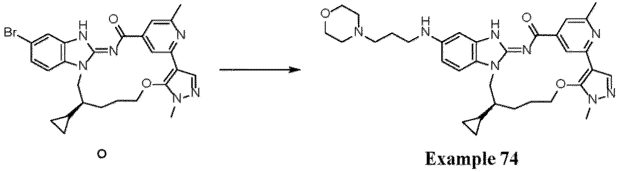

Compound **O** (75 mg), *N*-(3-aminopropyl)morpholine (40 mg), Pd₂(dba)₃ (32 mg), 2-(di-*tert-*butylphosphine)biphenyl (21 mg), and potassium *tert*-butoxide (94 mg) were dispersed in *tert*-amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 74** (31 mg). ESI-MS: m/z = 599.45 [M+H] ⁺.

¹H NMR (500 MHz, chloroform-*d*) δ 12.03 (s, 1H), 8.44 - 8.38 (m, 1H), 8.15 (s, 1H), 7.68 (d, *J* = 1.3 Hz, 1H), 7.15 (d, *J* = 8.6 Hz, 1H), 6.58 (dd, *J* = 8.6, 2.1 Hz, 1H), 6.53 (d, *J* = 2.1 Hz, 1H), 4.40 - 4.32 (m, 2H), 3.94 (ddd, *J* = 16.7, 12.6, 6.5 Hz, 2H), 3.80 (d, *J* = 4.5 Hz, 4H), 3.78 (s, 3H), 3.16 (td, *J* = 6.4, 1.9 Hz, 2H), 2.64 (s, 3H), 2.62 (s, 2H), 2.60 (d, *J* = 7.1 Hz, 4H), 2.27 (q, *J* = 6.5 Hz, 1H), 2.20 - 2.11 (m, 2H), 1.87 (p, *J* = 6.6 Hz, 2H), 1.79 (td, *J* = 13.2, 12.2, 7.8 Hz, 2H), 0.51 (dq, *J* = 12.6, 3.6 Hz, 1H), 0.43 (dq, *J* = 8.8, 4.6 Hz, 1H), 0.10 (tt, *J* = 9.0, 5.2 Hz, 1H), -0.04 (q, *J* = 4.9 Hz, 1H), -0.33 (dq, *J* = 9.9, 5.1 Hz, 1H).

### Example 75:

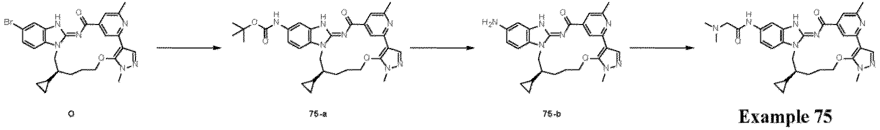

### (1) Preparation method for compound 75-a:

Compound **O** (1.0 g), *tert*-butyl carbamate (437 mg), Pd₂(dba)₃ (430 mg), 2-(di-*tert*-butylphosphine)biphenyl (280 mg), and potassium *tert*-butoxide (1.26 g) were dispersed in *tert*-amyl alcohol (20 mL). The mixture was purged with nitrogen 3 times and reacted at 100 °C for 2 h. After the reaction was completed, an aqueous sodium bicarbonate solution (1 M, 100 mL) was added to the reaction solution, and the resulting mixture was extracted with ethyl acetate. The organic phases were combined, dried over anhydrous sodium sulfate, concentrated until no liquid flowed out, and subjected to column chromatography to give compound **75-a** (480 mg). ESI-MS: m/z = 572.30 [M+H] ⁺.

### (2) Preparation method for compound 75-b:

Compound **75-a** (480 mg) was dispersed in dichloromethane (10 mL), and trifluoroacetic acid (5 mL) was added dropwise at 25 °C. After the dropwise addition, the mixture was reacted at 25 °C for 2 h. After the reaction was completed, the reaction solution was concentrated. An aqueous sodium bicarbonate solution (1 M, 20 mL) and ethyl acetate (30 mL) were added to the concentrate, and the resulting mixture was subjected to liquid separation. The organic phase was retained and concentrated until no liquid flowed out to give compound **75-b** (350 mg). ESI-MS: m/z = 472.31 [M+H] ⁺.

### (3) Preparation method for Example 75:

Compound **75-b** (30 mg) and *N,N*-dimethylglycine (7 mg) were dispersed in dichloromethane (5 mL), and HATU (29 mg) and triethylamine (19 mg) were sequentially added. The mixture was reacted at 25 °C for 2 h. After the reaction was completed, the reaction solution was washed with an aqueous sodium bicarbonate solution (1 M, 5 mL), dried over anhydrous sodium sulfate, concentrated until no liquid flowed out, and purified by preparative liquid phase chromatography to give **Example 75** (15 mg). ESI-MS: m/z = 557.32 [M+H] ⁺.

¹H NMR (500 MHz, DMSO-*d*₆) δ 9.84 (s, 1H), 8.36 (s, 1H), 8.09 (d, *J* = 1.9 Hz, 1H), 7.93 (s, 1H), 7.57 (dd, *J* = 8.7, 2.0 Hz, 1H), 7.50 - 7.37 (m, 2H), 4.32 - 4.25 (m, 2H), 4.9 - 4.01 (m, 2H), 3.75 (s, 3H), 3.13 (t, *J* = 7.1 Hz, 2H), 2.57 (s, 3H), 2.33 (s, 6H), 2.24 (q, *J* = 6.3 Hz, 1H), 2.14 (dt, *J* = 9.3, 4.9 Hz, 1H), 1.99 (td, *J* = 11.6, 8.9, 5.3 Hz, 1H), 1.86 - 1.70 (m, 2H), 0.60 (dq, *J* = 12.8, 7.9, 5.7 Hz, 1H), 0.37 (tt, *J* = 9.4, 4.8 Hz, 1H), -0.01 (dt, *J* = 9.8, 5.1 Hz, 1H), - 0.05 (dp, *J* = 9.4, 4.7 Hz, 1H), -0.51 (dq, *J* = 9.6, 4.9 Hz, 1H).

### Example 76:

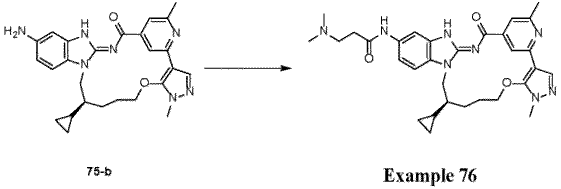

Compound **75-b** (30 mg) and 3-dimethylaminopropionic acid hydrochloride (9 mg) were dispersed in dichloromethane (5 mL), and HATU (29 mg) and triethylamine (19 mg) were sequentially added. The mixture was reacted at 25 °C for 2 h. After the reaction was completed, the reaction solution was washed with an aqueous sodium bicarbonate solution (1 M, 5 mL), dried over anhydrous sodium sulfate, concentrated until no liquid flowed out, and purified by preparative liquid phase chromatography to give **Example 76** (14 mg). ESI-MS: m/z = 571.35 [M+H] ⁺. ¹H NMR (500 MHz, DMSO-*d*₆) δ 12.68 (s, 1H), 10.15 (s, 1H), 8.35 (s, 1H), 7.94 (d, *J* = 1.9 Hz, 1H), 7.91 (s, 1H), 7.55 (d, *J* = 8.1 Hz, 2H), 7.44 (dd, *J* = 8.7, 2.0 Hz, 1H), 4.32 - 4.25 (m, 2H), 4.13 - 4.08 (m, 1H), 4.05 - 4.01 (m, 1H), 3.73 (s, 3H), 2.70 (t, *J* = 7.1 Hz, 2H), 2.55 (s, 3H), 2.52 (d, *J* = 7.0 Hz, 2H), 2.28 (s, 6H), 2.21 (q, *J* = 6.3 Hz, 1H), 2.14 (dt, *J* = 9.3, 4.9 Hz, 1H), 1.99 (td, *J* = 11.6, 8.9, 5.3 Hz, 1H), 1.82 (d, *J* = 10.1 Hz, 1H), 1.76 - 1.68 (m, 1H), 0.60 (dq, *J* = 12.8, 7.9, 5.7 Hz, 1H), 0.29 (tt, *J* = 9.4, 4.8 Hz, 1H), -0.06 (dt, *J* = 9.8, 5.1 Hz, 1H), -0.23 (dp, *J* = 9.4, 4.7 Hz, 1H), -0.68 (dq, *J* = 9.6, 4.9 Hz, 1H).

### Example 77:

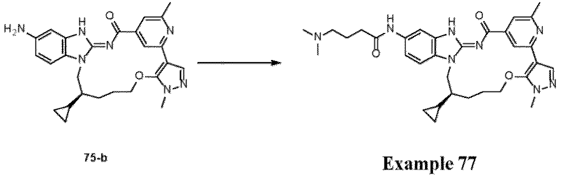

Compound **75-b** (30 mg) and 4-dimethylaminobutyric acid hydrochloride (10 mg) were dispersed in dichloromethane (5 mL), and HATU (29 mg) and triethylamine (19 mg) were sequentially added. The mixture was reacted at 25 °C for 2 h. After the reaction was completed, the reaction solution was washed with an aqueous sodium bicarbonate solution (1 M, 5 mL), dried over anhydrous sodium sulfate, concentrated until no liquid flowed out, and purified by preparative liquid phase chromatography to give **Example 77** (15 mg). ESI-MS: m/z = 585.35 [M+H] ⁺.

¹H NMR (500 MHz, DMSO-*d*₆) δ 12.67 (s, 1H), 10.03 (s, 1H), 8.35 (s, 1H), 7.93 (d, *J* = 1.9 Hz, 1H), 7.91 (s, 1H), 7.55 (d, *J* = 9.1 Hz, 2H), 7.45 (dd, *J* = 8.7, 2.0 Hz, 1H), 4.32 - 4.24 (m, 2H), 4.13 - 4.08 (m, 1H), 4.04 (dt, *J* = 8.7, 5.8 Hz, 1H), 3.73 (s, 3H), 2.55 (s, 3H), 2.46 (t, *J* = 7.4 Hz, 2H), 2.36 (t, *J* = 7.4 Hz, 2H), 2.31 (s, 6H), 2.21 (t, *J* = 6.4 Hz, 1H), 2.14 (d, *J* = 6.0 Hz, 1H), 2.03 - 1.96 (m, 1H), 1.82 (d, *J* = 10.1 Hz, 1H), 1.79 (p, *J* = 7.4 Hz, 2H), 1.74 - 1.67 (m, 1H), 0.60 (dt, *J* = 9.1, 5.0 Hz, 1H), 0.30 (tt, *J* = 9.3, 4.8 Hz, 1H), -0.06 (dq, *J* = 9.7, 4.9 Hz, 1H), -0.23 (tt, J = 9.5, 4.8 Hz, 1H), -0.67 (dq, *J* = 9.6, 4.8 Hz, 1H).

### Example 78:

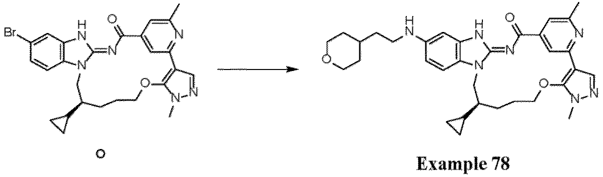

Compound **O** (75 mg), 4-(2-aminoethyl)tetrahydropyran (36 mg), Pd₂(dba)₃ (32 mg), 2-(di-*tert-*butylphosphine)biphenyl (21 mg), and potassium *tert*-butoxide (94 mg) were dispersed in *tert*-amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 78** (19 mg). ESI-MS: m/z = 584.44 [M+H] ⁺.

¹H NMR (500 MHz, chloroform-*d*) δ 11.97 (s, 1H), 8.39 (s, 1H), 8.15 (s, 1H), 7.66 (d, *J* = 1.2 Hz, 1H), 7.16 (d, *J* = 8.6 Hz, 1H), 6.58 (dd, *J* = 8.6, 2.2 Hz, 1H), 6.55 (d, *J* = 2.1 Hz, 1H), 4.35 (ddd, *J* = 11.4, 5.6, 3.0 Hz, 2H), 4.02 - 3.91 (m, 4H), 3.78 (s, 3H), 3.41 (td, *J* = 11.8, 1.9 Hz, 2H), 3.16 (t, *J* = 7.2 Hz, 2H), 2.64 (s, 3H), 2.33 - 2.24 (m, 1H), 2.23 - 2.13 (m, 2H), 1.80 - 1.77 (m, 1H), 1.68 - 1.61 (m, 8H), 0.56 - 0.49 (m, 1H), 0.44 (dq, *J* = 9.3, 4.8 Hz, 1H), 0.11 (tt, *J* = 9.1, 5.1 Hz, 1H), -0.03 (dd, *J* = 10.1, 5.3 Hz, 1H), -0.31 (dq, *J* = 10.0, 5.2 Hz, 1H).

### Example 79:

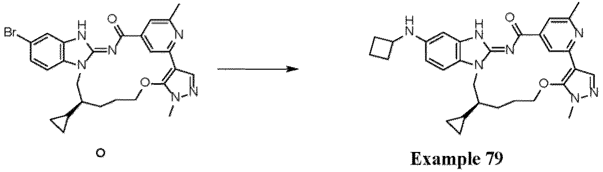

Compound **O** (75 mg), cyclobutyl amine (20 mg), Pd₂(dba)₃ (32 mg), 2-(di-*tert*-butylphosphine)biphenyl (21 mg), and potassium *tert*-butoxide (94 mg) were dispersed in *tert*-amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 79** (30 mg). ESI-MS: m/z = 526.39 [M+H] ⁺.

¹H NMR (500 MHz, chloroform-*d*) δ 12.25 (s, 1H), 8.40 (s, 1H), 8.30 (s, 1H), 7.91 (s, 1H), 7.57 (s, 1H), 7.17 (d, *J* = 8.6 Hz, 1H), 7.03 - 6.96 (m, 1H), 4.41 (d, *J* = 13.9 Hz, 1H), 4.23 (d, *J* = 7.7 Hz, 1H), 4.09 (d, *J* = 7.9 Hz, 1H), 3.94 (d, *J* = 8.0 Hz, 1H), 3.88 (s, 3H), 2.49 (s, 3H), 2.33 (d, *J* = 25.2 Hz, 4H), 2.30 - 2.21 (m, 3H), 1.94 (q, *J* = 10.2 Hz, 3H), 1.75 (dt, *J* = 16.3, 7.9 Hz, 2H), 0.54 - 0.46 (m, 1H), 0.35 (dd, *J* = 9.2, 4.6 Hz, 1H), -0.12 (d, *J* = 3.0 Hz, 1H), - 0.20 (dt, *J* = 9.8, 4.9 Hz, 1H), -0.65 (s, 1H).

### Example 80:

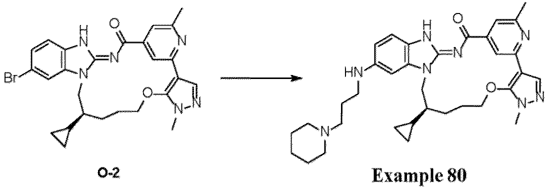

Compound **O-2** (50 mg), 1-(3-aminopropyl)piperidine (16 mg), Pd₂(dba)₃ (20 mg), 2-(di-*tert-*butylphosphine)biphenyl (13 mg), and potassium *tert*-butoxide (61 mg) were dispersed in *tert*-amyl alcohol (12 mL). The mixture was purged with nitrogen 3 times and reacted at 95 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 80** (25 mg).

ESI-MS: m/z = 597.45 [M+H] ⁺.

¹H NMR (500 MHz, DMSO-*d*₆) δ 12.37 (s, 1H), 8.34 (s, 1H), 7.91 (s, 1H), 7.54 (d, *J* = 1.2 Hz, 1H), 7.25 (d, *J* = 8.6 Hz, 1H), 6.68 (d, *J* = 2.1 Hz, 1H), 6.53 (dd, *J* = 8.6, 2.0 Hz, 1H), 4.28 (td, *J* = 8.6, 6.2 Hz, 1H), 4.22 (dd, *J* = 14.0, 3.6 Hz, 1H), 4.08 - 4.01 (m, 2H), 3.73 (s, 3H), 3.07 (t, *J* = 6.8 Hz, 2H), 2.54 (s, 3H), 2.36 (t, *J* = 7.1 Hz, 2H), 2.33 - 2.29 (m, 3H), 2.22 (p, *J* = 6.7, 6.3 Hz, 1H), 2.13 (tt, *J* = 8.6, 4.8 Hz, 1H), 2.00 (ddt, *J* = 13.7, 8.9, 3.9 Hz, 1H), 1.87 - 1.79 (m, 1H), 1.72 (p, *J* = 7.1 Hz, 3H), 1.50 (h, *J* = 5.1, 4.6 Hz, 4H), 1.39 (q, *J* = 5.9 Hz, 2H), 1.26 (dt, *J* = 17.5, 4.6 Hz, 1H), 0.65 - 0.55 (m, 1H), 0.32 (tt, *J* = 9.3, 4.6 Hz, 1H), -0.04 (dt, *J* = 9.4, 4.7 Hz, 1H), -0.11 (dp, *J* = 9.3, 4.5 Hz, 1H), -0.55 (dq, *J* = 9.6, 4.8 Hz, 1H).

### Example 81:

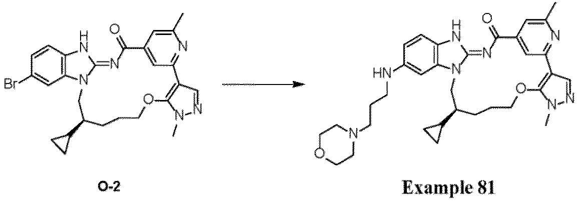

Compound **O-2** (70 mg), *N*-(3-aminopropyl)morpholine (22 mg), Pd₂(dba)₃ (30 mg), 2-(di-*tert-*butylphosphine)biphenyl (20 mg), and potassium *tert*-butoxide (88 mg) were dispersed in *tert*-amyl alcohol (5 mL). The mixture was purged with nitrogen 3 times and reacted at 95 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 81** (37 mg). ESI-MS: m/z = 599.42 [M+H] ⁺.

¹H NMR (500 MHz, DMSO-*d*₆) δ 12.30 (s, 1H), 7.91 (s, 1H), 7.54 (s, 1H), 7.25 (d, *J* = 8.5 Hz, 1H), 6.69 (d, *J* = 2.0 Hz, 1H), 6.53 (dd, *J* = 8.5, 2.1 Hz, 1H), 4.29 (q, *J* = 7.8 Hz, 1H), 4.22 (dd, *J* = 14.1, 3.6 Hz, 1H), 4.09 - 4.01 (m, 2H), 3.73 (s, 3H), 3.58 (t, *J* = 4.5 Hz, 4H), 3.09 (t, *J* = 6.8 Hz, 2H), 2.54 (s, 3H), 2.43 - 2.30 (m, 6H), 2.25 - 2.20 (m, 1H), 2.13 (dq, *J* = 14.5, 7.8, 6.2 Hz, 1H), 2.04 - 1.96 (m, 1H), 1.83 (d, *J* = 9.7 Hz, 1H), 1.73 (p, *J* = 7.3 Hz, 3H), 0.60 (tt, *J* = 8.5, 3.6 Hz, 1H), 0.32 (dp, *J* = 9.1, 4.6 Hz, 1H), -0.04 (dt, *J* = 9.8, 4.7 Hz, 1H), -0.12 (tt, *J* = 9.3, 4.6 Hz, 1H), - 0.56 (dq, *J* = 9.7, 4.8 Hz, 1H).

### Example 82:

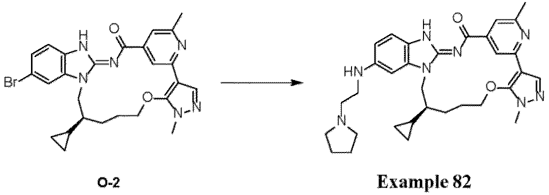

Compound **O-2** (75 mg), 1-(2-aminoethyl)pyrrolidine (32 mg), Pd₂(dba)₃ (32 mg), *2-(di-tert-*butylphosphine)biphenyl (21 mg), and potassium *tert*-butoxide (94 mg) were dispersed in tert-amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 82** (25 mg). ESI-MS: m/z = 569.40 [M+H] ⁺.

¹H NMR (500 MHz, DMSO-*d*₆) δ 12.44 (s, 1H), 8.39 (s, 1H), 8.30 (s, 1H), 7.96 (s, 1H), 7.59 (s, 1H), 7.31 (d, *J* = 8.6 Hz, 1H), 6.83 (s, 1H), 6.61 (dd, *J* = 8.5, 2.0 Hz, 1H), 4.34 (d, *J* = 7.8 Hz, 1H), 4.27 (dd, *J* = 13.9, 3.6 Hz, 1H), 4.13 - 4.07 (m, 2H), 3.78 (s, 3H), 3.29 (t, *J* = 6.7 Hz, 2H), 2.83 (t, *J* = 6.6 Hz, 2H), 2.71 (d, *J* = 5.9 Hz, 4H), 2.59 (s, 3H), 2.26 (q, *J* = 6.8 Hz, 1H), 2.18 (tt, *J* = 8.9, 4.8 Hz, 1H), 2.05 (ddd, *J* = 13.9, 9.2, 5.0 Hz, 1H), 1.91 - 1.86 (m, 1H), 1.80 (q, *J* = 3.4, 3.0 Hz, 4H), 1.75 (d, *J* = 14.2 Hz, 1H), 0.67 (tt, *J* = 8.5, 3.7 Hz, 1H), 0.37 (tt, *J* = 9.4, 4.9 Hz, 1H), 0.00 (dq, *J* = 9.7, 5.1 Hz, 1H), -0.08 (dq, *J* = 8.9, 4.6 Hz, 1H), -0.53 (dq, *J* = 9.8, 4.8 Hz, 1H).

### Example 83:

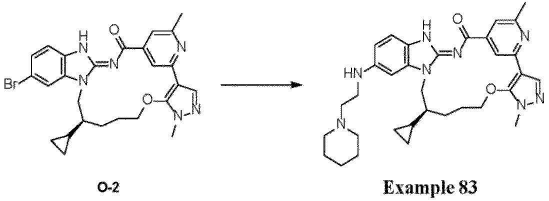

Compound **O-2** (60 mg), 1-(2-aminoethyl)piperidine (18 mg), Pd₂(dba)₃ (25mg), 2-(di-*tert*-butylphosphine)biphenyl (16 mg), and potassium *tert*-butoxide (74 mg) were dispersed in *tert*-amyl alcohol (15 mL). The mixture was purged with nitrogen 3 times and reacted at 95 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 83** (25 mg). ESI-MS: m/z = 583.41 [M+H] ⁺.

¹H NMR (500 MHz, DMSO-*d*₆) δ 12.38 (s, 1H), 8.34 (s, 1H), 7.91 (s, 1H), 7.54 (s, 1H), 7.26 (d, *J* = 8.6 Hz, 1H), 6.77 (d, *J* = 2.1 Hz, 1H), 6.56 (dd, *J* = 8.6, 2.0 Hz, 1H), 4.30 - 4.27 (m, 1H), 4.24 - 4.20 (m, 1H), 4.05 (dt, *J* = 9.1, 4.5 Hz, 2H), 3.73 (s, 3H), 3.20 (t, *J* = 6.7 Hz, 2H), 2.58 (t, *J* = 6.7 Hz, 2H), 2.54 (s, 3H), 2.28 (d, *J* = 6.6 Hz, 1H), 2.21 (q, *J* = 6.6, 6.1 Hz, 1H), 2.17 - 2.11 (m, 1H), 2.02 - 1.98 (m, 1H), 1.84 (q, *J* = 7.5 Hz, 1H), 1.72 (d, *J* = 13.5 Hz, 1H), 1.55 (p, *J=* 5.6 Hz, 4H), 1.44 - 1.39 (m, 3H), 1.24 (dd, *J* = 6.2, 3.6 Hz, 2H), 0.61 (qd, *J* = 10.4, 8.3, 5.1 Hz, 1H), 0.31 (dp, *J* = 8.7, 4.6 Hz, 1H), -0.03 - -0.07 (m, 1H), -0.13 (tt, *J* = 9.2, 4.5 Hz, 1H), -0.58 (dq, *J* = 9.6, 4.9 Hz, 1H).

### Example 84:

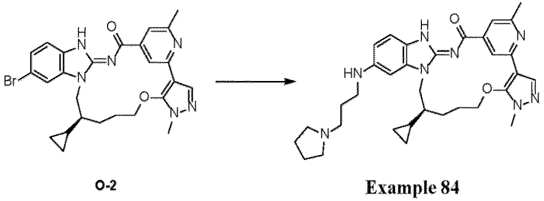

Compound **O-2** (50 mg), 1-(3-aminopropyl)pyrrolidine (14 mg), Pd₂(dba)₃ (20 mg), 2-(di-*tert-*butylphosphine)biphenyl (13 mg), and potassium *tert*-butoxide (61 mg) were dispersed in *tert-amyl* alcohol (12 mL). The mixture was purged with nitrogen 3 times and reacted at 95 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 84** (25 mg). ESI-MS: m/z = 583.41 [M+H] ⁺.

¹H NMR (500 MHz, DMSO-*d*₆) δ 12.40 (s, 1H), 8.36 (s, 1H), 8.25 (s, 1H), 7.99 (s, 1H), 7.59 (s, 1H), 7.36 (d, *J* = 8.6 Hz, 1H), 6.80 (s, 1H), 6.55 (dd, *J* = 8.5, 2.0 Hz, 1H), 4.37 (d, *J* = 7.8 Hz, 1H), 4.29 (dd, *J* = 13.9, 3.6 Hz, 1H), 4.17 - 4.01 (m, 2H), 3.77 (s, 3H), 3.25 (t, *J* = 6.7 Hz, 2H), 2.80 (t, *J* = 6.6 Hz, 2H), 2.71 (d, *J* = 5.9 Hz, 4H), 2.55 (s, 3H), 2.28 (q, *J* = 6.8 Hz, 1H), 2.20 (tt, *J* = 8.9, 4.8 Hz, 1H), 2.15 (q, *J* = 7.0 Hz, 2H), 2.03 (ddd, *J=* 13.9, 9.2, 5.0 Hz, 1H), 1.91 - 1.86 (m, 1H), 1.80 (q, *J* = 3.4, 3.0 Hz, 4H), 1.75 (d, *J* = 14.2 Hz, 1H), 0.67 (tt, *J* = 8.5, 3.7 Hz, 1H), 0.37 (tt, *J* = 9.4, 4.9 Hz, 1H), 0.00 (dq, *J* = 9.7, 5.1 Hz, 1H), -0.08 (dq, *J* = 8.9, 4.6 Hz, 1H), -0.53 (dq, *J* = 9.8, 4.8 Hz, 1H).

### Example 85:

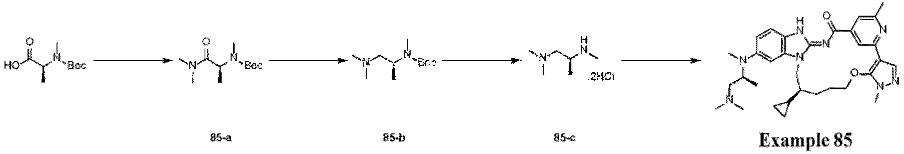

### (1) Preparation method for compound 85-a:

Compound BOC-N-methyl-L-alanine (10.0 g) was dispersed in dichloromethane (150 mL), and dimethylamine hydrochloride (6.0 g), HATU (20.6 g), and *N*,*N*-diisopropylethylamine (19.1 g) were sequentially added. The mixture was reacted at 25 °C for 16 h. After the reaction was completed as detected by TLC, the reaction solution was washed with an aqueous NaHCOs solution (1 M, 100 mL × 2). The organic phase was retained, dried, concentrated by rotary evaporation to dryness, and subjected to column chromatography to give compound **85-a** (9.3 g).

### (2) Preparation method for compound 85-b:

Compound **85-a** (9.3 g) was dispersed in tetrahydrofuran (120 mL), and the mixture was cooled to 0-5 °C. A solution of LiAlH₄ in tetrahydrofuran (1 M, 101 mL) was added dropwise with the temperature kept at this temperature. The mixture was reacted at 0-5 °C for 2 h. After the reaction was completed as detected by TLC, water (10 mL), an aqueous NaOH solution (15%, 10 mL), and water (30 mL) were sequentially added dropwise to the reaction solution, and the resulting mixture was filtered. The filtrate was dried, concentrated by rotary evaporation to dryness, and subjected to column chromatography to give compound **85-b** (4.6 g).

### (3) Preparation method for compound 85-c:

Compound **85-b** (4.6 g) was dispersed in a hydrogen chloride/1,4-dioxane solution (4 M, 53 mL), and the mixture was reacted at 25 °C for 16 h. After the reaction was completed as detected by TLC, the reaction solution was concentrated. Ethyl acetate (50 mL) was added, and the resulting mixture was stirred at 25 °C for 1 h and filtered. The filter cake was dried to give compound **85-c** (2.50 g).

### (4) Preparation method for Example 85:

Compound **O-2** in Example 8 (100 mg), compound **85-c** (25 mg), Pd₂(dba)₃ (43 mg), 2-(di-*tert-*butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 85** (10 mg). ESI-MS: m/z = 571.35 [M+H] ⁺.

¹H NMR (500 MHz, DMSO-*d*₆) δ 12.55 (s, 1H), 8.41 (s, 1H), 7.96 (s, 1H), 7.60 (s, 1H), 7.47 (d, *J* = 8.8 Hz, 1H), 7.25 (d, *J* = 2.3 Hz, 1H), 6.96 (dd, *J* = 8.9, 2.3 Hz, 1H), 4.53 - 4.44 (m, 1H), 4.37 (dt, *J* = 8.6, 4.4 Hz, 1H), 4.33 - 4.23 (m, 2H), 4.08 (dt, *J* = 8.9, 5.7 Hz, 1H), 3.78 (s, 3H), 3.54 (dd, *J* = 13.5, 10.7 Hz, 1H), 3.20 (dd, *J* = 13.7, 4.3 Hz, 1H), 2.86 (s, 6H), 2.77 (s, 3H), 2.60 (s, 3H), 2.33 - 2.25 (m, 1H), 2.20 (tq, *J* = 8.9, 4.5 Hz, 1H), 2.08 (ddd, *J* = 15.5, 10.2, 6.0 Hz, 1H), 1.90 (td, *J* = 9.2, 4.8 Hz, 1H), 1.83 - 1.73 (m, 1H), 1.06 (d, *J* = 6.5 Hz, 3H), 0.69 (tq, *J* = 8.4, 4.3, 3.4 Hz, 1H), 0.35 (tt, *J* = 9.2, 4.7 Hz, 1H), -0.00 (dq, *J* = 9.6, 5.0 Hz, 1H), -0.19 (tt, *J* = 9.3, 4.7 Hz, 1H), -0.61 (dq, *J* = 9.6, 4.9 Hz, 1H).

### Example 86:

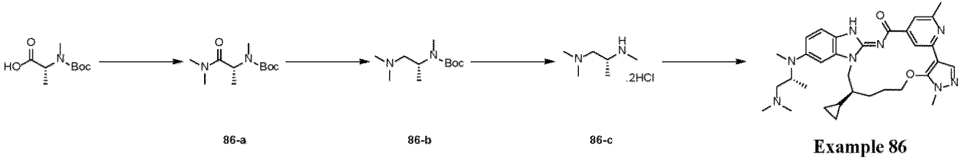

### (1) Preparation method for compound 86-a:

Compound BOC-*N*-methyl-D-alanine (10.0 g) was dispersed in dichloromethane (150 mL), and dimethylamine hydrochloride (6.0 g), HATU (20.6 g), and *N*,*N*-diisopropylethylamine (19.1 g) were sequentially added. The mixture was reacted at 25 °C for 16 h. After the reaction was completed as detected by TLC, the reaction solution was washed with an aqueous NaHCOs solution (1 M, 100 mL × 2). The organic phase was retained, dried, concentrated by rotary evaporation to dryness, and subjected to column chromatography to give compound **86-a** (8.9 g).

### (2) Preparation method for compound 86-b:

Compound **86-a** (8.9 g) was dispersed in tetrahydrofuran (100 mL), and the mixture was cooled to 0-5 °C. A solution of LiAlH₄ in tetrahydrofuran (1 M, 103 mL) was added dropwise with the temperature kept at this temperature. The mixture was reacted at 0-5 °C for 2 h. After the reaction was completed as detected by TLC, water (7 mL), an aqueous NaOH solution (15%, 7 mL), and water (21 mL) were sequentially added dropwise to the reaction solution, and the resulting mixture was filtered. The filtrate was dried, concentrated by rotary evaporation to dryness, and subjected to column chromatography to give compound **86-b** (4.2 g).

### (3) Preparation method for compound 86-c:

Compound **86-b** (4.2 g) was dispersed in a hydrogen chloride/1,4-dioxane solution (4 M, 52 mL), and the mixture was reacted at 25 °C for 16 h. After the reaction was completed as detected by TLC, the reaction solution was concentrated. Ethyl acetate (40 mL) was added, and the resulting mixture was stirred at 25 °C for 1 h and filtered. The filter cake was dried to give compound **86-c** (2.0 g).

### (4) Preparation method for Example 86:

Compound **O-2** (100 mg) in Example 8, compound **86-c** (25 mg), Pd₂(dba)₃ (43 mg), 2-(di-*tert-*butylphosphine)biphenyl (28 mg), and potassium *tert*-butoxide (126 mg) were dispersed in *tert-amyl* alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 86** (11 mg). ESI-MS: m/z = 571.35 [M+H] ⁺.

### Example 87:

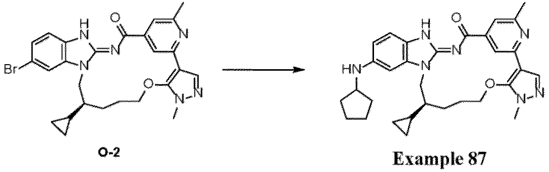

Compound **O-2** (60 mg), cyclopentylamine (12 mg), Pd₂(dba)₃ (25mg), 2-(di-*tert*-butylphosphine)biphenyl (16 mg), and potassium *tert*-butoxide (74 mg) were dispersed in *tert-amyl* alcohol (17 mL). The mixture was purged with nitrogen 3 times and reacted at 95 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 87** (25 mg). ESI-MS: m/z = 540.45 [M+H] ⁺.

¹H NMR (500 MHz, DMSO-*d*₆) δ 12.36 (s, 1H), 8.34 (d, *J* = 1.2 Hz, 1H), 7.90 (s, 1H), 7.54 (d, *J* = 1.2 Hz, 1H), 7.23 (d, *J* = 8.6 Hz, 1H), 6.68 (d, *J* = 2.0 Hz, 1H), 6.53 (dd, *J* = 8.6, 2.0 Hz, 1H), 5.57 (s, 1H), 4.28 (td, *J* = 8.5, 6.1 Hz, 1H), 4.23 (dd, *J* = 13.9, 3.6 Hz, 1H), 4.08 - 4.00 (m, 2H), 3.76 (t, *J* = 6.4 Hz, 1H), 3.73 (s, 3H), 2.54 (s, 3H), 2.27 - 2.19 (m, 1H), 2.13 (tt, *J* = 8.7, 4.9 Hz, 1H), 1.98 (dddd, *J* = 23.9, 12.4, 6.1, 1.9 Hz, 3H), 1.80 (dq, *J* = 9.1, 4.5 Hz, 1H), 1.70 (dp, *J* = 21.0, 7.3, 6.3 Hz, 3H), 1.57 (qd, *J* = 7.7, 3.5 Hz, 2H), 1.51 - 1.39 (m, 2H), 0.65 - 0.57 (m, 1H), 0.33 (tt, *J* = 9.1, 4.6 Hz, 1H), -0.04 (dt, *J* = 9.5, 4.7 Hz, 1H), -0.10 (td, *J* = 8.6, 4.3 Hz, 1H), -0.55 (dq, *J* = 9.5, 4.8 Hz, 1H).

### Example 88:

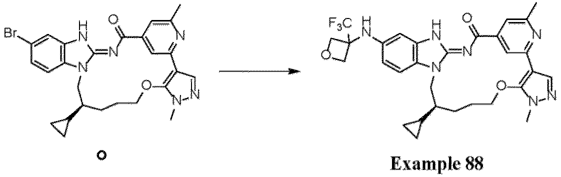

Compound **O** (75 mg), 3-(trifluoromethyl)oxetane-3-amine (50 mg), Pd₂(dba)₃ (32 mg), 2-(di-*tert-*butylphosphine)biphenyl (21 mg), and potassium *tert*-butoxide (94 mg) were dispersed in *tert-amyl* alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 88** (6 mg). ESI-MS: m/z = 596.20 [M+H] ⁺.

¹H NMR (500 MHz, chloroform-*d*) δ 12.01 (s, 1H), 8.39 (d, *J* = 1.4 Hz, 1H), 8.15 (s, 1H), 7.65 (d, *J* = 1.4 Hz, 1H), 7.20 (d, *J* = 8.5 Hz, 1H), 6.54 - 6.47 (m, 2H), 4.95 (dd, *J* = 7.1, 1.2 Hz, 2H), 4.83 (d, *J* = 7.0 Hz, 2H), 4.35 (tt, *J* = 13.5, 4.8 Hz, 2H), 3.96 (dt, *J* = 8.4, 5.8 Hz, 2H), 3.78 (s, 3H), 3.35 - 3.27 (m, 2H), 2.64 (s, 3H), 2.26 (dt, *J* = 14.0, 6.1 Hz, 1H), 2.18 (td, *J* = 11.9, 11.0, 4.7 Hz, 2H), 0.52 (dt, *J* = 12.7, 5.6 Hz, 1H), 0.46 (dq, *J* = 8.2, 4.4, 3.5 Hz, 1H), 0.15 - 0.09 (m, 1H), -0.01 (d, *J* = 8.2 Hz, 1H), -0.31 (dq, *J* = 9.9, 5.1 Hz, 1H).

### Example 89:

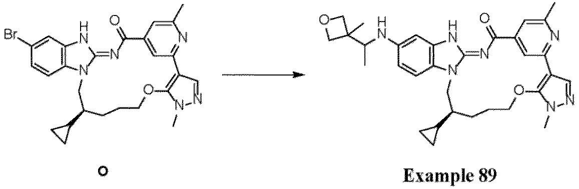

Compound **O** (75 mg), 1-(3-methyloxabutan-3-yl)ethan-1-amine hemioxalate (32 mg), Pd₂(dba)₃ (32 mg), 2-(di-*tert-*butylphosphine)biphenyl (21 mg), and potassium *tert*-butoxide (94 mg) were dispersed in *tert*-amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 89** (16 mg). ESI-MS: m/z = 570.44 [M+H] ⁺.

¹H NMR (500 MHz, chloroform-*d*) δ 11.95 (s, 1H), 8.40 (s, 1H), 8.16 (s, 1H), 7.67 (s, 1H), 7.17 (d, *J* = 8.5 Hz, 1H), 6.67 - 6.59 (m, 2H), 4.60 (dd, *J* = 6.2, 1.7 Hz, 1H), 4.55 (dd, *J* = 6.0, 2.6 Hz, 1H), 4.38 (dd, *J* = 6.2, 1.7 Hz, 2H), 4.36 - 4.33 (m, 1H), 3.95 (dt, *J* = 14.2, 7.5 Hz, 2H), 3.84 (q, *J* = 6.5 Hz, 1H), 3.78 (s, 3H), 3.72 (q, *J* = 7.0 Hz, 2H), 2.65 (s, 3H), 2.27 (q, *J* = 7.6 Hz, 1H), 2.19 (ddd, *J* = 12.3, 8.6, 5.3 Hz, 2H), 1.85 - 1.79 (m, 1H), 1.39 (s, 3H), 1.12 (dd, *J* = 6.4, 2.7 Hz, 3H), 0.58 - 0.49 (m, 1H), 0.45 (tt, *J* = 8.8, 4.2 Hz, 1H), 0.13 (tt, *J* = 8.9, 4.8 Hz, 1H), -0.00-0.04 (m, 1H), -0.29 (dt, *J* = 9.5, 4.9 Hz, 1H).

### Example 90:

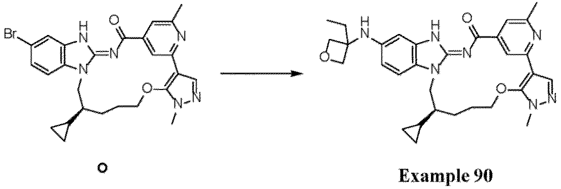

Compound **O** (75 mg), 3-ethyloxetane-3-amine hydrochloride (39 mg), Pd₂(dba)₃ (32 mg), 2-(di-*tert-*butylphosphine)biphenyl (21 mg), and potassium *tert*-butoxide (94 mg) were dispersed in *tert-amyl* alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 90** (10 mg). ESI-MS: m/z = 556.42 [M+H] ⁺.

¹H NMR (500 MHz, chloroform-*d*) δ 11.96 (s, 1H), 8.39 (s, 1H), 8.15 (s, 1H), 7.65 (s, 1H), 7.17 (d, *J* = 8.6 Hz, 1H), 6.45 (dd, *J*= 8.6, 2.2 Hz, 1H), 6.39 (d, *J* = 2.2 Hz, 1H), 4.77 (dd, *J* = 6.1, 2.7 Hz, 2H), 4.62 (d, *J* = 6.0 Hz, 2H), 4.34 (td, *J* = 13.0, 12.1, 5.3 Hz, 2H), 3.95 (dt, *J* = 18.1, 6.5 Hz, 2H), 3.78 (s, 3H), 2.64 (s, 3H), 2.28 (t, *J* = 6.9 Hz, 1H), 2.15 (dd, *J* = 13.9, 6.7 Hz, 3H), 1.82 (t, *J* = 7.5 Hz, 1H), 1.74 (s, 2H), 0.91 (t, *J* = 7.3 Hz, 3H), 0.52 (dq, *J* = 8.4, 4.2, 3.3 Hz, 1H), 0.46 (dq, *J* = 8.7, 4.4 Hz, 1H), 0.15 (tt, *J* = 9.4, 5.2 Hz, 1H), -0.02 (d, *J* = 5.2 Hz, 1H), -0.27 (dt, *J* = 9.6, 5.1 Hz, 1H).

### Example 91:

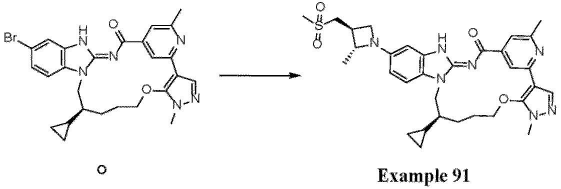

Compound **O** (75 mg), (2*R*,3*S*)-2-methyl-3-((methylsulfonyl)methyl)azetidine hydrochloride (56 mg), Pd₂(dba)₃ (32 mg), 2-(di-*tert*-butylphosphine)biphenyl (21 mg), and potassium *tert*-butoxide (94 mg) were dispersed in *tert*-amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 91** (31 mg). ESI-MS: m/z = 618.39 [M+H] ⁺.

¹H NMR (500 MHz, chloroform-*d*) δ 12.01 (s, 1H), 8.39 (s, 1H), 8.15 (s, 1H), 7.67 (s, 1H), 7.20 (d, *J* = 8.6 Hz, 1H), 6.47 (dt, *J* = 8.5, 2.5 Hz, 1H), 6.44 (q, *J* = 2.8 Hz, 1H), 4.35 (td, *J* = 11.9, 9.8, 5.1 Hz, 2H), 4.25 (q, *J* = 8.0 Hz, 1H), 3.93 (dq, *J* = 14.0, 5.3, 4.5 Hz, 3H), 3.78 (s, 3H), 3.46 (dt, *J* = 14.0, 7.2 Hz, 1H), 3.28 (dq, *J* = 13.7, 5.7, 4.5 Hz, 2H), 3.00 - 2.96 (m, 1H), 2.95 (s, 3H), 2.63 (s, 3H), 2.27 (q, *J* = 6.4 Hz, 1H), 2.17 (dd, *J* = 16.8, 11.7 Hz, 2H), 1.79 (d, *J* = 15.3 Hz, 2H), 1.59 (d, *J* = 6.0 Hz, 3H), 0.56 - 0.48 (m, 1H), 0.43 (tt, *J* = 9.0, 5.0 Hz, 1H), 0.09 (tt, *J* = 9.1, 5.1 Hz, 1H), -0.04 (dt, *J* = 9.9, 4.8 Hz, 1H), -0.34 (dq, *J* = 9.9, 5.1 Hz, 1H).

### Example 92:

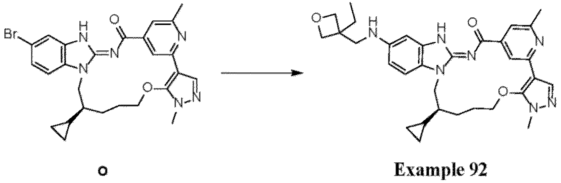

Compound **O** (75 mg), (3-ethyloxetan-3-yl)methylamine (32 mg), Pd₂(dba)₃ (32 mg), 2-(di-*tert-*butylphosphine)biphenyl (21 mg), and potassium *tert*-butoxide (94 mg) were dispersed in *tert*-amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 92** (10 mg). ESI-MS: m/z = 570.20 [M+H] ⁺.

¹H NMR (500 MHz, chloroform-*d*) δ 12.02 (s, 1H), 8.42 (s, 1H), 8.18 (s, 1H), 7.68 (s, 1H), 7.18 (d, *J* = 8.5 Hz, 1H), 6.65 (dd, *J* = 8.6, 2.2 Hz, 1H), 6.61 (d, *J* = 2.2 Hz, 1H), 4.51 (s, 4H), 4.35 (td, *J* = 12.0, 10.3, 5.2 Hz, 2H), 3.95 (dq, *J* = 9.3, 5.5 Hz, 2H), 3.79 (s, 3H), 3.31 (s, 2H), 2.65 (s, 3H), 2.21 (dtt, *J* = 30.7, 11.8, 5.6 Hz, 4H), 1.83 - 1.81 (m, 1H), 1.32 (d, *J* = 11.2 Hz, 1H), 0.95 (t, *J* = 7.4 Hz, 3H), 0.88 (t, *J* = 6.8 Hz, 1H), 0.52 (tt, *J* = 8.6, 3.9 Hz, 1H), 0.44 (tt, *J* = 9.2, 5.0 Hz, 1H), 0.11 (tt, *J* = 9.2, 5.1 Hz, 1H), -0.00 - -0.08 (m, 1H), -0.32 (dq, *J* = 10.1, 5.2 Hz, 1H).

### Example 93:

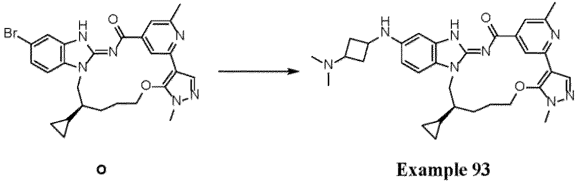

Compound **O** (75 mg), *N*¹,*N*¹-dimethylcyclobutane-1,3-diamine (32 mg), Pd₂(dba)₃ (32 mg), 2-(di-*tert-*butylphosphine)biphenyl (21 mg), and potassium *tert*-butoxide (94 mg) were dispersed in *tert*-amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 93** (25 mg). ESI-MS: m/z = 569.43 [M+H] ⁺.

¹H NMR (500 MHz, Methanol-*d*₄) δ 8.46 (s, 1H), 8.37 (d, *J* = 2.6 Hz, 1H), 8.02 (s, 1H), 7.57 (t, *J* = 8.5 Hz, 1H), 7.26 (t, *J* = 9.6 Hz, 1H), 6.67 - 6.57 (m, 2H), 4.31 (td, *J* = 18.0, 16.6, 11.1 Hz, 2H), 4.10 - 3.86 (m, 3H), 3.74 (s, 3H), 3.40 (t, *J* = 8.1 Hz, 1H), 2.94 - 2.84 (m, 1H), 2.76 (d, *J* = 10.0 Hz, 6H), 2.68 - 2.61 (m, 1H), 2.55 (s, 3H), 2.38 (dt, *J* = 14.3, 5.2 Hz, 1H), 2.20 (s, 2H), 2.05 (dt, *J* = 19.5, 10.0 Hz, 2H), 1.87 - 1.70 (m, 2H), 0.51 (d, *J* = 8.5 Hz, 1H), 0.34 (dq, *J* = 8.8, 4.7 Hz, 1H), -0.11 (dt, *J* = 18.2, 7.7 Hz, 2H), -0.58 (dt, *J* = 10.0, 5.3 Hz, 1H).

### Example 94:

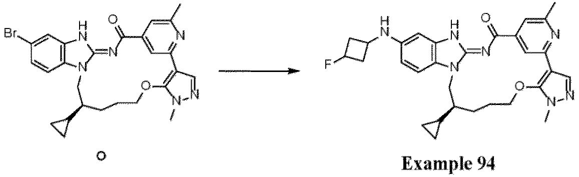

Compound **O** (75 mg), 3-fluorocyclobutylamine (25 mg), Pd₂(dba)₃ (32 mg), 2-(di-*tert*-butylphosphine)biphenyl (21 mg), and potassium *tert*-butoxide (94 mg) were dispersed in *tert-*amyl alcohol (5 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 94** (15 mg). ESI-MS: m/z = 544.36 [M+H] ⁺.

¹H NMR (500 MHz, Methanol-*d*₄) δ 8.46 (s, 1H), 8.15 (s, 1H), 7.70 (d, *J* = 4.5 Hz, 1H), 7.31 (d, *J* = 8.6 Hz, 1H), 6.71 (d, *J* = 8.3 Hz, 1H), 6.67 (s, 1H), 5.35 (ddt, *J* = 56.3, 6.6, 2.5 Hz, 1H), 4.47 - 4.30 (m, 2H), 4.19 - 4.05 (m, 2H), 3.95 (t, *J* = 11.1 Hz, 1H), 3.85 (s, 3H), 2.73 (ddd, *J* = 19.7, 10.0, 6.0 Hz, 2H), 2.66 (s, 3H), 2.51 - 2.39 (m, 2H), 2.34 - 2.24 (m, 2H), 2.15 - 2.03 (m, 1H), 1.85 (s, 2H), 0.67 - 0.53 (m, 1H), 0.44 (d, *J* = 8.5 Hz, 1H), 0.01 (q, *J* = 6.7, 6.1 Hz, 2H), -0.46 (q, *J* = 6.2, 5.6 Hz, 1H).

### Example 95:

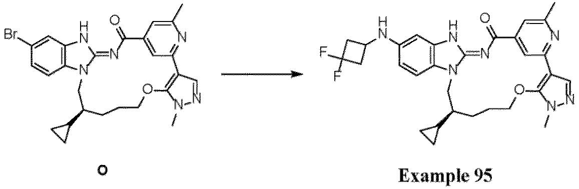

Compound **O** (100 mg), 3,3-difluorocyclobutylamine (55 mg), Pd₂(dba)₃ (43 mg), 2-(di-*tert-*butylphosphine)biphenyl (28 mg), and potassium *tert*-butoxide (126 mg) were dispersed in *tert*-amyl alcohol (3 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 95** (18 mg). ESI-MS: m/z =562.36 [M+H] ⁺.

¹H NMR (500 MHz, Methanol-*d*₄) δ 8.32 (s, 1H), 8.01 (s, 1H), 7.55 (s, 1H), 7.26 - 7.16 (m, 1H), 6.60 (d, *J* = 8.2 Hz, 2H), 4.33 - 4.26 (m, 1H), 4.22 (s, 1H), 3.95 (s, 1H), 3.88 - 3.74 (m, 2H), 3.71 (s, 3H), 3.01 (dt, *J* = 13.7, 7.1 Hz, 2H), 2.53 (s, 3H), 2.45 (d, *J* = 14.7 Hz, 2H), 2.22 - 2.09 (m, 2H), 1.96 (s, 1H), 1.72 (s, 2H), 0.46 (s, 1H), 0.31 (d, *J* = 8.8 Hz, 1H), -0.08 - -0.19 (m, 2H), -0.54 - -0.64 (m, 1H).

### Example 96:

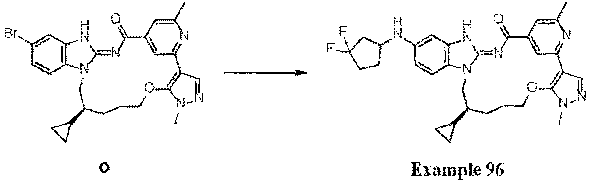

Compound **O** (100 mg), 3,3-difluorocyclopentylamine (46 mg), Pd₂(dba)₃ (43 mg), 2-(di-*tert-*butylphosphine)biphenyl (28 mg), and potassium *tert*-butoxide (126 mg) were dispersed in *tert*-amyl alcohol (3 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 96** (30 mg). ESI-MS: m/z = 576.38 [M+H] ⁺.

¹H NMR (500 MHz, Methanol-*d*₄) δ 8.35 (s, 1H), 8.02 (s, 1H), 7.58 (s, 1H), 7.20 (d, *J* = 8.4 Hz, 1H), 6.72 - 6.59 (m, 2H), 4.30 (q, *J* = 7.5, 6.9 Hz, 1H), 4.23 (d, *J* = 12.6 Hz, 1H), 4.02 - 3.89 (m, 2H), 3.85 (s, 1H), 3.72 (s, 3H), 2.59 (s, 1H), 2.54 (s, 3H), 2.32 - 2.22 (m, 2H), 2.21 - 2.06 (m, 3H), 1.98 (dt, *J* = 15.1, 7.3 Hz, 2H), 1.74 (dt, *J* = 24.0, 10.6 Hz, 3H), 0.47 (s, 1H), 0.32 (d, *J* = 9.3 Hz, 1H), -0.12 (t, *J* = 6.6 Hz, 2H), -0.58 (s, 1H).

### Example 97:

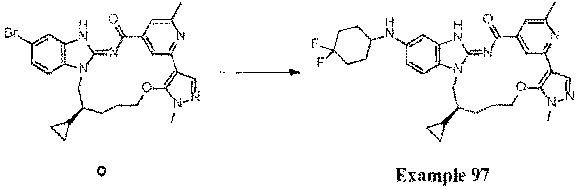

Compound **O** (100 mg), 4,4-difluorocyclohexylamine (51 mg), Pd₂(dba)₃ (35 mg), 2-(di-*tert-*butylphosphine)biphenyl (23 mg), and potassium *tert*-butoxide (126 mg) were dispersed in *tert-amyl* alcohol (3 mL). The mixture was purged with nitrogen 3 times and reacted at 90 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 97** (38 mg). ESI-MS: m/z = 590.42 [M+H] ⁺.

¹H NMR (500 MHz, Methanol-*d*₄) δ 8.47 (s, 1H), 8.15 (s, 1H), 7.70 (s, 1H), 7.30 (d, *J* = 8.6 Hz, 1H), 6.86 - 6.75 (m, 2H), 4.42 (q, *J* = 8.4, 7.4 Hz, 1H), 4.33 (d, *J* = 12.9 Hz, 1H), 4.09 (q, *J* = 6.4 Hz, 1H), 3.96 (t, *J* = 12.0 Hz, 1H), 3.84 (s, 3H), 3.48 (d, *J* = 11.6 Hz, 1H), 2.66 (s, 3H), 2.35 - 2.25 (m, 2H), 2.23 - 2.14 (m, 4H), 2.02 (dt, *J* = 42.9, 13.2 Hz, 3H), 1.84 (s, 2H), 1.69 (d, *J* = 12.0 Hz, 2H), 0.58 (d, *J* = 12.3 Hz, 1H), 0.44 (q, *J* = 7.3, 6.1 Hz, 1H), 0.04 - -0.05 (m, 2H), -0.45 (q, *J* = 6.6 Hz, 1H).

### Example 98:

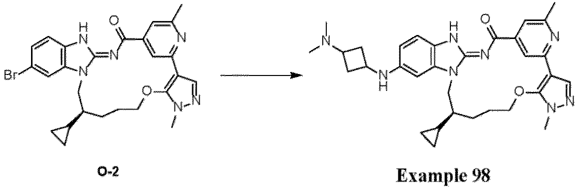

Compound **O-2** (100 mg), *N*¹,*N*¹-dimethylcyclobutane-1,3-diamine (25 mg), Pd₂(dba)₃ (18 mg), 2-(di-*tert-*butylphosphine)biphenyl (16 mg), and potassium *tert*-butoxide (126 mg) were dispersed in *tert*-amyl alcohol (4 mL). The mixture was purged with nitrogen 3 times and reacted at 95 °C for 3 h. After the reaction was completed, water was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 98** (13 mg). ESI-MS: m/z = 569.43 [M+H] ⁺.

¹H NMR (500 MHz, DMSO-*d*₆) δ 12.37 (s, 1H), 8.33 (s, 1H), 8.22 (s, 1H), 7.90 (s, 1H), 7.54 (s, 1H), 7.24 (d, *J* = 8.5 Hz, 1H), 6.64 (d, *J* = 2.1 Hz, 1H), 6.50 (dd, *J* = 8.6, 2.1 Hz, 1H), 4.29 (d, *J* = 7.1 Hz, 1H), 4.22 (dd, *J* = 14.0, 3.7 Hz, 1H), 4.04 (td, *J* = 9.1, 4.0 Hz, 2H), 3.73 (s, 3H), 2.63 - 2.56 (m, 2H), 2.54 (s, 3H), 2.43 - 2.39 (m, 1H), 2.22 (dd, *J* = 12.8, 6.6 Hz, 1H), 2.14 (q, *J* = 5.4, 4.8 Hz, 1H), 2.06 (s, 6H), 2.00 - 1.97 (m, 1H), 1.81 (d, *J* = 8.6 Hz, 1H), 1.77 - 1.69 (m, 1H), 1.66 - 1.55 (m, 2H), 1.46 (t, *J* = 7.3 Hz, 1H), 0.61 (tt, *J* = 8.4, 3.5 Hz, 1H), 0.33 (dq, *J* = 9.0, 4.8 Hz, 1H), -0.05 (dq, *J* = 9.4, 4.8 Hz, 1H), -0.14 (tt, *J* = 9.4, 4.6 Hz, 1H), -0.58 (dq, *J* = 9.7, 4.9 Hz, 1H).

### Example 99:

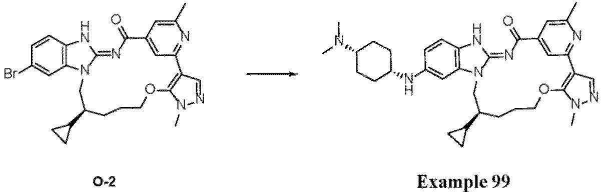

Compound **O-2** (100 mg), *cis*-*N*¹,*N*¹-dimethylcyclohexane-1,4-diamine dihydrochloride (48 mg), Pd₂(dba)₃ (43 mg), 2-(di-*tert*-butylphosphine)biphenyl (28 mg), and potassium *tert*-butoxide (126 mg) were dispersed in *tert-amyl* alcohol (4 mL). The mixture was purged with nitrogen 3 times and reacted at 95 °C for 3 h. After the reaction was completed, water was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 99** (15 mg). ESI-MS: m/z = 597.49 [M+H] ⁺. ¹H NMR (500 MHz, DMSO-*d*₆) δ 12.40 (s, 1H), 8.38 (s, 1H), 8.31 (s, 1H), 7.95 (s, 1H), 7.58 (d, *J* = 1.1 Hz, 1H), 7.28 (d, *J* = 8.6 Hz, 1H), 6.79 (d, *J* = 2.0 Hz, 1H), 6.66 (dd, *J* = 8.7, 2.0 Hz, 1H), 4.32 (dd, *J* = 8.9, 2.8 Hz, 1H), 4.26 (dd, *J* = 14.0, 3.7 Hz, 1H), 4.11 - 4.04 (m, 2H), 3.77 (s, 3H), 2.58 (s, 3H), 2.39 - 2.36 (m, 1H), 2.33 (s, 6H), 2.26 (q, *J* = 6.7, 6.2 Hz, 1H), 2.17 (dq, *J* = 9.1, 4.7, 3.9 Hz, 1H), 2.04 (dq, *J* = 14.9, 5.0, 4.3 Hz, 2H), 1.87 - 1.72 (m, 6H), 1.66 - 1.57 (m, 4H), 0.68 - 0.61 (m, 1H), 0.37 (tt, *J* = 9.2, 4.6 Hz, 1H), 0.00 (dq, *J* = 9.5, 4.9 Hz, 1H), -0.08 (tt, *J* = 9.4, 4.6 Hz, 1H), -0.50 (dq, *J* = 9.5, 4.8 Hz, 1H).

### Example 100:

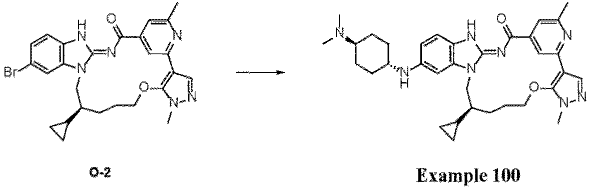

Compound **O-2** (100 mg), *trans*-*N*¹,*N*¹-dimethylcyclohexane-1,4-diamine hydrochloride (40 mg), Pd₂(dba)₃ (43 mg), 2-(di-*tert*-butylphosphine)biphenyl (28 mg), and potassium *tert*-butoxide (126 mg) were dispersed in *tert-amyl* alcohol (4 mL). The mixture was purged with nitrogen 3 times and reacted at 95 °C for 3 h. After the reaction was completed, water (30 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 100** (20 mg). ESI-MS: m/z = 597.49 [M+H] ⁺.

¹H NMR (500 MHz, DMSO-*d*₆) δ 12.40 (s, 1H), 8.37 (s, 1H), 8.31 (s, 1H), 7.94 (s, 1H), 7.58 (s, 1H), 7.26 (d, *J* = 8.6 Hz, 1H), 6.75 (d, *J* = 2.0 Hz, 1H), 6.56 (dd, *J* = 8.5, 2.0 Hz, 1H), 4.32 (td, *J* = 8.4, 5.8 Hz, 1H), 4.26 (dd, *J* = 14.0, 3.7 Hz, 1H), 4.08 (td, *J* = 9.0, 3.8 Hz, 2H), 3.77 (s, 3H), 2.58 (s, 3H), 2.48 - 2.39 (m, 1H), 2.35 (s, 6H), 2.26 (dt, *J* = 12.1, 5.9 Hz, 1H), 2.17 (dq, *J* = 8.8, 4.2, 3.5 Hz, 1H), 2.14 - 1.99 (m, 4H), 1.95 - 1.84 (m, 3H), 1.80 - 1.72 (m, 1H), 1.49 - 1.38 (m, 2H), 1.18 (ddt, *J* = 23.8, 13.0, 6.5 Hz, 2H), 0.68 - 0.58 (m, 1H), 0.37 (tt, *J* = 9.2, 4.6 Hz, 1H), -0.00 (dt, *J* = 9.5, 4.7 Hz, 1H), -0.08 (dp, *J* = 9.4, 4.7 Hz, 1H), -0.48 - -0.54 (m, 1H).

### Example 101:

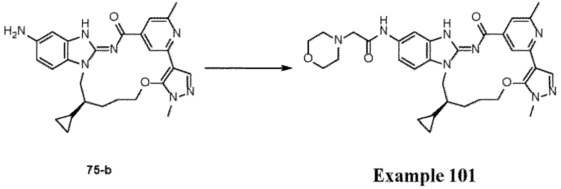

Compound **75-b** (30 mg) and morpholin-4-yl acetic acid (8 mg) were dispersed in dichloromethane (5 mL), and HATU (29 mg) and triethylamine (19 mg) were sequentially added. The mixture was reacted at 25 °C for 2 h. After the reaction was completed, the reaction solution was washed with an aqueous sodium bicarbonate solution (1 M, 5 mL), dried over anhydrous sodium sulfate, concentrated until no liquid flowed out, and purified by preparative liquid phase chromatography to give **Example 101** (12 mg). ESI-MS: m/z = 599.35 [M+H] ⁺.

¹H NMR (500 MHz, DMSO-*d*₆) δ 12.69 (s, 1H), 9.84 (s, 1H), 8.35 (s, 1H), 7.98 (d, *J* = 2.0 Hz, 1H), 7.91 (s, 1H), 7.57 (d, *J* = 9.8 Hz, 2H), 7.45 (dd, *J* = 8.8, 2.0 Hz, 1H), 4.33 - 4.24 (m, 2H), 4.17 - 4.09 (m, 1H), 4.04 (dt, *J* = 8.9, 5.8 Hz, 1H), 3.73 (s, 3H), 3.65 (t, *J* = 4.5 Hz, 4H), 3.15 (s, 2H), 2.55 (s, 3H), 2.53 (t, *J* = 4.3 Hz, 4H), 2.25 - 2.18 (m, 1H), 2.18 - 2.11 (m, 1H), 2.03 - 1.96 (m, 1H), 1.84 (t, *J* = 8.9 Hz, 1H), 1.70 (q, *J* = 5.9 Hz, 1H), 0.60 (dq, *J* = 8.5, 5.0, 3.4 Hz, 1H), 0.30 (tt, *J* = 9.2, 4.9 Hz, 1H), -0.06 (dq, *J* = 9.8, 5.0 Hz, 1H), -0.23 (tt, *J* = 9.3, 4.8 Hz, 1H), -0.68 (dq, *J* = 9.6, 4.8 Hz, 1H).

### Example 102:

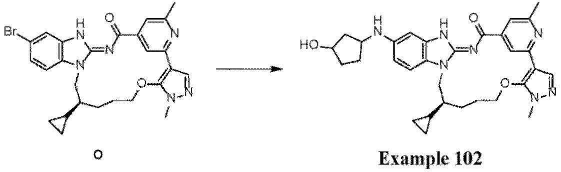

Compound O (70 mg), 3-aminocyclopentanol hydrochloride (22 mg), Pd₂(dba)₃ (30 mg), 2-(di-*tert-*butylphosphine)biphenyl (20 mg), and potassium *tert*-butoxide (88 mg) were dispersed in *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 102** (10 mg). ESI-MS: m/z = 556.36 [M+H] ⁺.

### Example 103:

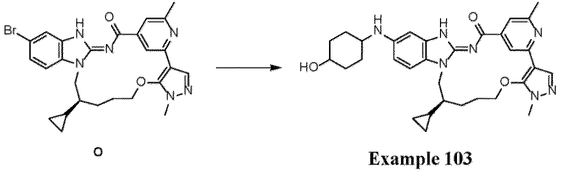

Compound **O** (70 mg), 4-aminocyclohexanol (18 mg), Pd₂(dba)₃ (30 mg), 2-(di-*tert*-butylphosphine)biphenyl (20 mg), and potassium *tert*-butoxide (88 mg) were dispersed in *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 103** (12 mg). ESI-MS: m/z = 570.35 [M+H] ⁺.

### Example 104:

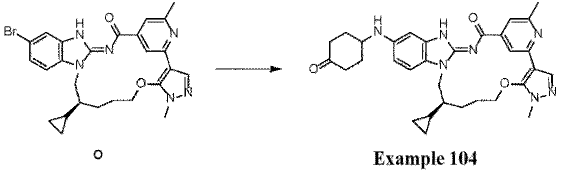

Compound **O** (70 mg), 4-aminocyclohexanone hydrochloride (23 mg), Pd₂(dba)₃ (30 mg), 2-(di-*tert-*butylphosphine)biphenyl (20 mg), and potassium *tert*-butoxide (88 mg) were dispersed in *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 104** (8 mg). ESI-MS: m/z = 568.35 [M+H] ⁺. ¹H NMR (500 MHz, chloroform-d) δ 12.11 (s, 1H), 8.61 (s, 1H), 8.40 (s, 1H), 8.17 (s, 1H), 7.71 (s, 1H), 7.15 (d, J = 8.5 Hz, 1H), 6.52 (d, J = 7.8 Hz, 2H), 4.45 - 4.37 (m, 2H), 3.98 (p, J = 5.9 Hz, 2H), 3.77 (s, 3H), 3.16 (d, J = 12.0 Hz, 1H), 2.92 (d, J = 12.4 Hz, 1H), 2.54 (s, 3H), 2.29 (ddd, *J* = 24.9, 15.6, 6.9 Hz, 2H), 2.17 - 2.09 (m, 2H), 2.02 (s, 2H), 1.83 - 1.74 (m, 2H), 1.48 (t, J = 10.5 Hz, 2H), 1.26 (t, J = 6.0 Hz, 2H), 0.47 - 0.39 (m, 1H), 0.28 (dq, J = 10.0, 5.1 Hz, 1H), 0.10 (dd, J = 10.2, 5.3 Hz, 1H), -0.07 (s, 1H), -0.51 (dq, J = 10.2, 5.0 Hz, 1H).

### Example 105:

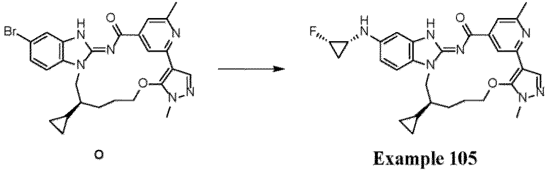

Compound **O** (70 mg), (1*R*,2*S*)-2-fluorocyclopropylamine *p*-toluenesulfonate (39 mg), Pd₂(dba)₃ (30 mg), 2-(di-*tert-*butylphosphine)biphenyl (20 mg), and potassium *tert*-butoxide (88 mg) were dispersed in *tert*-amyl alcohol (3 mL). The mixture was purged with nitrogen 3 times and reacted at 100 °C for 2 h. After the reaction was completed, 30 mL of water was added to the reaction solution, and the resulting mixture was extracted with ethyl acetate. The organic phases were combined, dried over anhydrous sodium sulfate, concentrated until no liquid flowed out, and purified by preparative liquid chromatography to give **Example 105** (8 mg). ESI-MS: m/z = 530.31 [M+H] ⁺.

### Example 106:

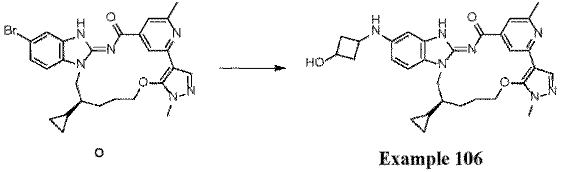

Compound **O** (100 mg), 3-aminocyclobutanol (20 mg), Pd₂(dba)₃ (43 mg), 2-(di-*tert*-butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 106** (15 mg). ESI-MS: m/z = 542.30 [M+H] ⁺.

### Example 107:

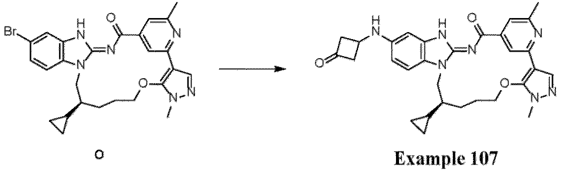

Compound **O** (100 mg), 3-aminocyclobutanone (20 mg), Pd₂(dba)₃ (43 mg), 2-(di-*tert*-butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 107** (10 mg). ESI-MS: m/z = 540.31 [M+H] ⁺.

### Example 108:

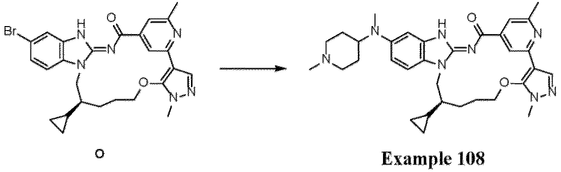

Compound **O** (100 mg), 1-methyl-4-(methylamino)piperidine (29 mg), Pd₂(dba)₃ (43 mg), 2-(di-*tert-*butylphosphine)biphenyl (28 mg), and potassium *tert*-butoxide (126 mg) were dispersed in *tert*-amyl alcohol (3 mL).

The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 108** (15 mg). ESI-MS: m/z = 583.40 [M+H] ⁺.

¹H NMR (500 MHz, chloroform-*d*) δ 12.18 (s, 1H), 8.41 (s, 1H), 8.15 (s, 1H), 7.69 (s, 1H), 7.22 (d, *J=* 9.1 Hz, 1H), 6.82 (dd, *J* = 6.5, 2.6 Hz, 2H), 4.42 - 4.32 (m, 2H), 3.95 (tt, *J* = 14.1, 6.4 Hz, 2H), 3.79 (s, 3H), 3.57 (td, *J* = 10.9, 4.7 Hz, 1H), 3.30 (d, *J* = 11.8 Hz, 2H), 2.79 (s, 3H), 2.62 (s, 3H), 2.58 (s, 3H), 2.52 (d, *J* = 12.0 Hz, 2H), 2.27 (q, *J* = 6.6 Hz, 1H), 2.22 - 2.08 (m, 4H), 1.82 (dd, *J* = 30.8, 9.5 Hz, 4H), 0.52 (dt, *J* = 12.6, 6.6 Hz, 1H), 0.44 (dq, *J* = 8.6, 4.7, 4.1 Hz, 1H), 0.10 (tt, *J* = 9.3, 5.2 Hz, 1H), -0.01 - -0.07 (m, 1H), -0.33 (dq, *J* = 9.9, 5.1 Hz, 1H).

### Example 109:

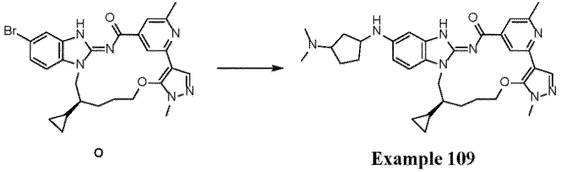

Compound **O** (100 mg), *N*¹,*N*¹-dimethylcyclopentane-1,3-diamine (29 mg), Pd₂(dba)₃ (43 mg), 2-(di-*tert-*butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 109** (23 mg). ESI-MS: m/z = 583.41 [M+H] ⁺.

### Example 110:

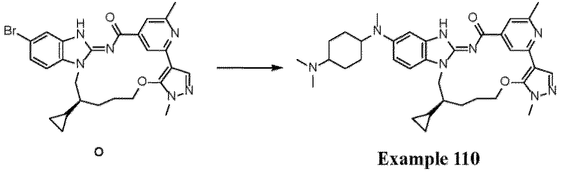

Compound **O** (100 mg), *N*¹,*N*¹,*N*⁴-trimethylcyclohexane-1,4-diamine (35 mg), Pd₂(dba)₃ (43 mg), 2-(di-*tert-*butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 110** (10 mg). ESI-MS: m/z = 611.43 [M+H] ⁺.

### Example 111:

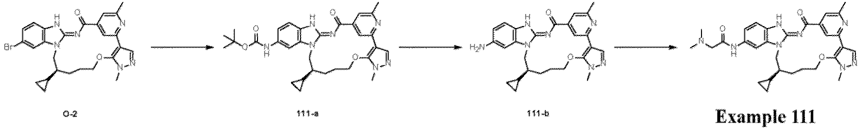

### (1) Preparation method for compound 111-a:

Compound **O-2** (1.0 g), *tert*-butyl carbamate (437 mg), Pd₂(dba)₃ (430 mg), 2-(di-*tert*-butylphosphine)biphenyl (280 mg), and potassium *tert-*butoxide (1.26 g) were dispersed in *tert*-amyl alcohol (20 mL). The mixture was purged with nitrogen 3 times and reacted at 100 °C for 2 h. After the reaction was completed, an aqueous sodium bicarbonate solution (1 M, 100 mL) was added to the reaction solution, and the resulting mixture was extracted with ethyl acetate. The organic phases were combined, dried over anhydrous sodium sulfate, concentrated until no liquid flowed out, and subjected to column chromatography to give compound **111-a** (420 mg). ESI-MS: m/z = 572.31 [M+H] ⁺.

### (2) Preparation method for compound 111-b:

Compound **111-a** (420 mg) was dispersed in dichloromethane (10 mL), and trifluoroacetic acid (5 mL) was added dropwise at 25 °C. After the dropwise addition, the mixture was reacted at 25 °C for 2 h. After the reaction was completed, the reaction solution was concentrated. An aqueous sodium bicarbonate solution (1 M, 20 mL) and ethyl acetate (30 mL) were added to the concentrate, and the resulting mixture was subjected to liquid separation. The organic phase was retained and concentrated until no liquid flowed out to give compound **111-b** (300 mg). ESI-MS: m/z = 472.32 [M+H] ⁺.

### (3) Preparation method for Example 111:

Compound **111-b** (30 mg) and *N,N-*dimethylglycine (7 mg) were dispersed in dichloromethane (5 mL), and HATU (29 mg) and triethylamine (19 mg) were sequentially added. The mixture was reacted at 25 °C for 2 h. After the reaction was completed, the reaction solution was washed with an aqueous sodium bicarbonate solution (1 M, 5 mL), dried over anhydrous sodium sulfate, concentrated until no liquid flowed out, and purified by preparative liquid phase chromatography to give **Example 111** (13 mg). ESI-MS: m/z = 557.34 [M+H] ⁺.

¹H NMR (500 MHz, DMSO-*d*₆) δ 12.66 (s, 1H), 9.85 (s, 1H), 8.34 (s, 1H), 8.07 (d, *J* = 1.9 Hz, 1H), 7.91 (s, 1H), 7.56 (d, *J* = 1.2 Hz, 1H), 7.45 (d, *J* = 8.5 Hz, 1H), 7.40 (dd, *J* = 8.6, 1.8 Hz, 1H), 4.32 - 4.26 (m, 2H), 4.07 - 4.04 (m, 1H), 4.00 (dd, *J* = 13.9, 8.6 Hz, 1H), 3.73 (s, 3H), 3.15 (d, *J* = 1.3 Hz, 2H), 2.55 (s, 3H), 2.34 (s, 6H), 2.23 (dt, *J* = 13.2, 6.8 Hz, 1H), 2.13 (q, *J* = 6.7, 5.4 Hz, 1H), 2.02 (dq, *J* = 13.9, 7.6, 6.3 Hz, 1H), 1.76 (ddt, *J* = 20.9, 13.0, 4.8 Hz, 2H), 0.58 (q, *J* = 8.1, 7.2 Hz, 1H), 0.35 (tt, *J* = 8.7, 4.5 Hz, 1H), 0.01 - -0.04 (m, 1H), -0.07 (dt, *J* = 8.8, 4.4 Hz, 1H), -0.52 (dq, *J* = 9.5, 4.7 Hz, 1H).

### Example 112:

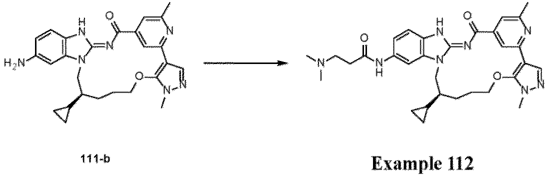

Compound **111-b** (30 mg) and 3-dimethylaminopropionic acid hydrochloride (9 mg) were dispersed in dichloromethane (5 mL), and HATU (29 mg) and triethylamine (19 mg) were sequentially added. The mixture was reacted at 25 °C for 2 h. After the reaction was completed, the reaction solution was washed with an aqueous sodium bicarbonate solution (1 M, 5 mL), dried over anhydrous sodium sulfate, concentrated until no liquid flowed out, and purified by preparative liquid phase chromatography to give **Example 112** (15 mg). ESI-MS: m/z = 571.36 [M+H] ⁺.

¹H NMR (500 MHz, DMSO-*d*₆) δ 12.67 (s, 1H), 10.19 (s, 1H), 8.34 (s, 1H), 8.07 (d, *J* = 1.9 Hz, 1H), 7.91 (s, 1H), 7.56 (d, *J* = 1.2 Hz, 1H), 7.45 (d, *J* = 8.5 Hz, 1H), 7.23 (dd, *J* = 8.6, 1.8 Hz, 1H), 4.28 (dt, *J* = 14.7, 5.4 Hz, 2H), 4.06 (dt, *J* = 7.3, 5.0 Hz, 1H), 3.98 (dd, *J* = 14.0, 8.4 Hz, 1H), 3.73 (s, 3H), 2.70 (t, *J* = 7.1 Hz, 2H), 2.55 (s, 3H), 2.52 (d, *J* = 6.9 Hz, 2H), 2.28 (s, 6H), 2.22 (h, *J* = 6.1, 5.3 Hz, 1H), 2.12 (td, *J* = 8.8, 4.3 Hz, 1H), 2.06 - 2.00 (m, 1H), 1.76 (qd, *J* = 14.0, 13.1, 8.5 Hz, 2H), 0.55 (qd, *J* = 8.0, 4.0 Hz, 1H), 0.36 (dp, *J* = 8.9, 4.4 Hz, 1H), -0.00 (q, *J* = 5.1, 4.5 Hz, 1H), -0.06 (tt, *J* = 9.8, 5.1 Hz, 1H), -0.51 (dq, *J* = 9.5, 4.7 Hz, 1H).

### Example 113:

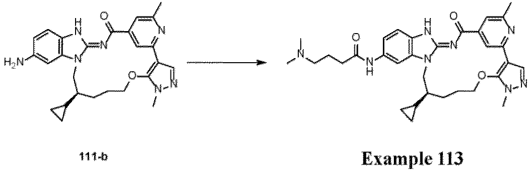

Compound **111-b** (30 mg) and 4-dimethylaminobutyric acid hydrochloride (10 mg) were dispersed in dichloromethane (5 mL), and HATU (29 mg) and triethylamine (19 mg) were sequentially added. The mixture was reacted at 25 °C for 2 h. After the reaction was completed, the reaction solution was washed with an aqueous sodium bicarbonate solution (1 M, 5 mL), dried over anhydrous sodium sulfate, concentrated until no liquid flowed out, and purified by preparative liquid phase chromatography to give **Example 113** (13 mg). ESI-MS: m/z = 585.39 [M+H] ⁺.

¹H NMR (500 MHz, DMSO-*d*₆) δ 12.82 - 12.47 (m, 1H), 10.05 (s, 1H), 8.34 (s, 1H), 8.08 (s, 1H), 7.91 (s, 1H), 7.56 (s, 1H), 7.44 (d, *J* = 8.6 Hz, 1H), 7.24 (d, *J* = 8.6 Hz, 1H), 4.28 (q, *J* = 8.7 Hz, 2H), 4.04 (d, *J* = 7.0 Hz, 1H), 3.98 (dd, *J* = 13.9, 8.3 Hz, 1H), 3.73 (s, 3H), 2.55 (s, 3H), 2.35 (dd, *J* = 16.8, 8.8 Hz, 4H), 2.20 (s, 6H), 2.13 (t, *J* = 7.5 Hz, 1H), 2.05 (s, 1H), 1.75 (d, *J* = 11.7 Hz, 5H), 0.56 (s, 1H), 0.39 - 0.33 (m, 1H), -0.01 (q, *J* = 4.9 Hz, 1H), -0.06 (s, 1H), -0.47 - -0.55 (m, 1H).

### Example 114:

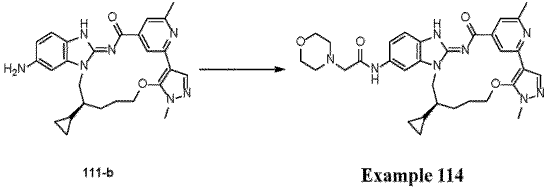

Compound **111-b** (30 mg) and morpholin-4-yl acetic acid (8 mg) were dispersed in dichloromethane (5 mL), and HATU (29 mg) and triethylamine (19 mg) were sequentially added. The mixture was reacted at 25 °C for 2 h. After the reaction was completed, the reaction solution was washed with an aqueous sodium bicarbonate solution (1 M, 5 mL), dried over anhydrous sodium sulfate, concentrated until no liquid flowed out, and purified by preparative liquid phase chromatography to give **Example 114** (15 mg). ESI-MS: m/z = 599.36 [M+H] ⁺.

¹H NMR (500 MHz, chloroform-*d*) δ 9.24 (s, 1H), 8.40 (d, *J* = 1.3 Hz, 1H), 8.30 (d, *J* = 1.9 Hz, 1H), 8.15 (s, 1H), 7.65 (d, *J* = 1.3 Hz, 1H), 7.29 (d, *J* = 8.4 Hz, 1H), 6.96 (dd, *J* = 8.5, 1.9 Hz, 1H), 4.41 (dd, *J* = 13.8, 3.2 Hz, 1H), 4.37 - 4.32 (m, 1H), 4.04 (dd, *J* = 13.9, 7.5 Hz, 1H), 3.98 - 3.94 (m, 1H), 3.81 (t, *J* = 4.6 Hz, 4H), 3.79 (s, 3H), 3.20 (d, *J* = 1.3 Hz, 2H), 2.67 (t, *J* = 4.7 Hz, 4H), 2.64 (s, 3H), 2.29 (dt, *J* = 13.3, 6.6 Hz, 1H), 2.21 - 2.16 (m, 2H), 1.84 - 1.78 (m, 2H), 0.58 (dq, *J* = 13.1, 7.3, 5.4 Hz, 1H), 0.44 (tt, *J* = 9.0, 4.9 Hz, 1H), 0.13 (tt, *J* = 9.0, 5.1 Hz, 1H), -0.04 (dt, *J* = 10.0, 4.9 Hz, 1H), -0.33 (dq, *J* = 9.9, 5.0 Hz, 1H).

### Example 115:

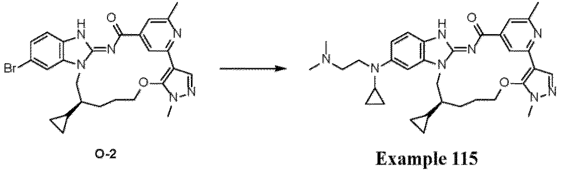

Compound **O-2** (100 mg), *N*¹-cyclopropyl-*N*²,*N*²-dimethylethane-1,2-diamine dihydrochloride (45 mg), Pd₂(dba)₃ (43 mg), 2-(di-*tert*-butylphosphine)biphenyl (28 mg), and potassium *tert*-butoxide (126 mg) were dispersed in *tert-*amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 115** (10 mg). ESI-MS: m/z = 583.40 [M+H] ⁺.

¹H NMR (500 MHz, DMSO-*d*₆) δ 12.46 (s, 1H), 8.38 (s, 1H), 7.94 (s, 1H), 7.59 (s, 1H), 7.39 (d, *J* = 8.9 Hz, 2H), 7.17 (s, 1H), 6.95 (d, *J* = 9.0 Hz, 1H), 4.30 (d, *J* = 23.1 Hz, 2H), 4.11 (d, *J* = 27.2 Hz, 2H), 3.76 (s, 3H), 2.58 (s, 3H), 2.43 (s, 4H), 2.17 (s, 6H), 2.05 (s, 2H), 1.88 - 1.73 (m, 3H), 0.90 (s, 3H), 0.66 (s, 1H), 0.63 - 0.57 (m, 1H), 0.49 (s, 1H), 0.35 (s, 1H), 0.00 (s, 1H), -0.06 - -0.14 (m, 1H), -0.56 (s, 1H).

### Example 116:

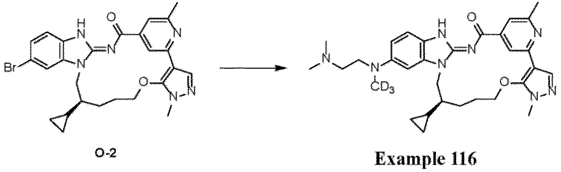

Compound **O-2** (100 mg), *N*¹,*N*¹-dimethyl-*N*²-(methyl-*d*₃)ethane-1,2-ethylenediamine dihydrochloride (34 mg), Pd₂(dba)₃ (43 mg), 2-(di-*tert*-butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 116** (22 mg). ESI-MS: m/z = 560.42 [M+H] ⁺.

¹H NMR (500 MHz, DMSO-*d*₆) δ 12.45 (s, 1H), 8.39 (s, 1H), 7.96 (s, 1H), 7.60 (s, 1H), 7.39 (d, *J* = 8.8 Hz, 1H), 6.93 (d, *J* = 2.3 Hz, 1H), 6.70 (dd, *J* = 8.8, 2.3 Hz, 1H), 4.34 (q, *J* = 7.9 Hz, 1H), 4.28 (dd, *J* = 14.0, 3.6 Hz, 1H), 4.18 (dd, *J* = 13.9, 9.2 Hz, 1H), 4.08 (dt, *J* = 8.7, 5.7 Hz, 1H), 3.78 (s, 3H), 3.57 (s, 1H), 3.47 (d, *J* = 2.3 Hz, 1H), 2.59 (s, 3H), 2.44 (ddd, *J* = 15.3, 10.5, 6.1 Hz, 2H), 2.26 (dd, *J* = 9.5, 4.6 Hz, 1H), 2.23 (s, 6H), 2.19 (td, *J* = 9.2, 8.3, 3.7 Hz, 1H), 2.06 (td, *J* = 12.6, 9.3, 5.4 Hz, 1H), 1.87 (d, *J* = 10.0 Hz, 1H), 1.81 - 1.72 (m, 1H), 0.68 (tt, *J* = 8.3, 3.3 Hz, 1H), 0.36 (tt, *J* = 9.2, 4.7 Hz, 1H), 0.00 (dq, *J* = 9.6, 4.9 Hz, 1H), -0.11 (dp, *J* = 9.5, 4.8 Hz, 1H), -0.55 (dq, *J* = 9.6, 4.9 Hz, 1H).

### Example 117:

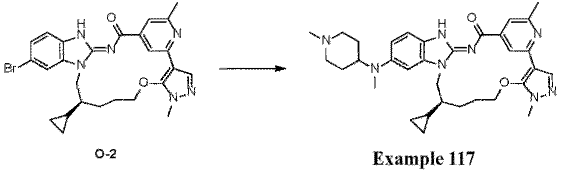

Compound **O-2** (100 mg), 1-methyl-4-(methylamino)piperidine (29 mg), Pd₂(dba)₃ (43 mg), 2-(di-*tert-*butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 117** (12 mg). ESI-MS: m/z = 583.40 [M+H] ⁺.

¹H NMR (500 MHz, DMSO-*d*₆) δ 12.47 (s, 1H), 8.39 (s, 1H), 8.27 (s, 1H), 7.95 (s, 1H), 7.59 (s, 1H), 7.39 (d, *J* = 8.7 Hz, 1H), 7.06 (s, 1H), 6.84 (d, *J* = 8.9 Hz, 1H), 4.33 (q, *J* = 7.8 Hz, 1H), 4.28 (dd, *J* = 14.0, 3.4 Hz, 1H), 4.20 (dd, *J* = 13.9, 8.9 Hz, 1H), 4.10 - 4.05 (m, 1H), 3.77 (s, 3H), 3.03 - 2.93 (m, 2H), 2.78 (s, 3H), 2.58 (s, 3H), 2.31 (s, 3H), 2.27 - 2.14 (m, 4H), 2.06 (dq, *J* = 13.0, 4.7, 3.9 Hz, 1H), 1.90 - 1.73 (m, 4H), 1.71 - 1.61 (m, 2H), 1.27 (d, *J* = 10.5 Hz, 1H), 0.67 (dq, *J* = 9.4, 5.2, 4.4 Hz, 1H), 0.35 (tt, *J* = 9.4, 4.8 Hz, 1H), 0.03 - -0.04 (m, 1H), -0.14 (tt, *J* = 9.2, 4.7 Hz, 1H), -0.54 (dq, *J* = 9.7, 4.8 Hz, 1H).

### Example 118:

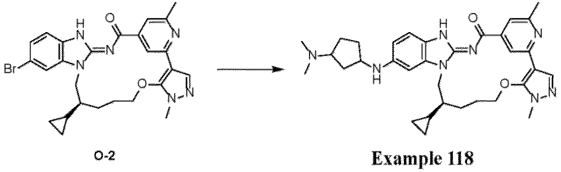

Compound **O-2** (100 mg), *N*¹,*N*¹-dimethylcyclopentane-1,3-diamine (29 mg), Pd₂(dba)₃ (43 mg), 2-(di*-tert-*butylphosphine)biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 118** (30 mg). ESI-MS: m/z = 583.41 [M+H] ⁺.

### Example 119:

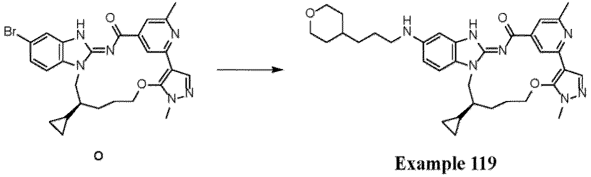

Compound **O** (70 mg), 3-(oxacyclohexen-4-yl)propan-1-amine (22 mg), Pd₂(dba)₃ (30 mg), 2-(di-*tert-*butylphosphine)biphenyl (20 mg), and potassium *tert*-butoxide (88 mg) were dispersed in *tert*-amyl alcohol (3 mL). The mixture was purged with nitrogen 3 times and reacted at 100 °C for 2 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid chromatography to give **Example 119** (10 mg). ESI-MS: m/z = 598.39 [M+H] ⁺.

### Example 120:

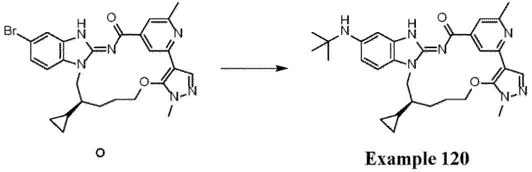

Compound **O** (70 mg), *tert*-butylamine (30 mg), Pd₂(dba)₃ (30 mg), 2-(di-*tert*-butylphosphine)biphenyl (20 mg), and potassium *tert*-butoxide (88 mg) were dispersed in *tert*-amyl alcohol (3 mL). The mixture was purged with nitrogen 3 times and reacted at 60 °C for 5 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 120** (8 mg). ESI-MS: m/z = 528.36 [M+H] ⁺.

### Example 121:

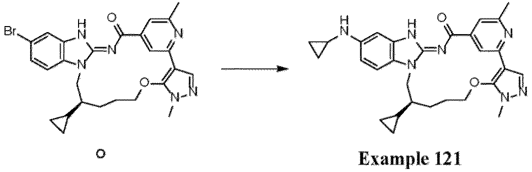

Compound **O** (70 mg), cyclopropylamine (25 mg), Pd₂(dba)₃ (30 mg), 2-(di-*tert*-butylphosphine)biphenyl (20 mg), and potassium *tert*-butoxide (88 mg) were dispersed in *tert*-amyl alcohol (3 mL). The mixture was purged with nitrogen 3 times and reacted at 60 °C for 5 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid chromatography to give **Example 121** (6 mg). ESI-MS: m/z = 512.34 [M+H] ⁺. Example 122:

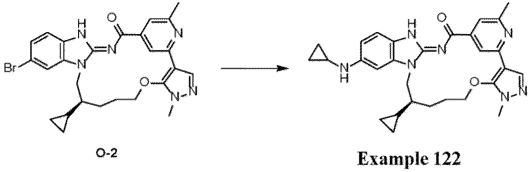

Compound **O-2** (70 mg), cyclopropylamine (25 mg), Pd₂(dba)₃ (30 mg), 2-(di-*tert*-butylphosphine)biphenyl (20 mg), and potassium *tert*-butoxide (88 mg) were dispersed in *tert*-amyl alcohol (3 mL). The mixture was purged with nitrogen 3 times and reacted at 60 °C for 5 h. After the reaction was completed, an aqueous sodium bicarbonate solution (1 M, 20 mL) was added to the reaction solution, and the resulting mixture was extracted with ethyl acetate. The organic phases were combined, dried over anhydrous sodium sulfate, concentrated until no liquid flowed out, and purified by preparative liquid phase chromatography to give **Example 122** (8 mg). ESI-MS: m/z = 512.34 [M+H] ⁺.

### Example 123:

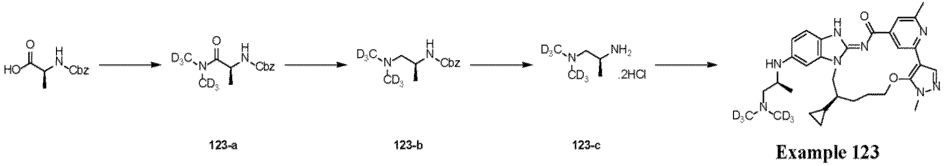

### (1) Preparation method for compound 123-a:

Compound *N*-benzyloxycarbonyl-L-alanine (3.82 g) was dispersed in dichloromethane (50 mL), and dimethyl-d6-amine hydrochloride (1.65 g), TBTU (6.05 g), and *N,N*-diisopropylethylamine (8.5 mL) were sequentially added at 0-5 °C. After the addition, the mixture was reacted at 25 °C for 16 h. After the reaction was completed as detected by TLC, the reaction solution was washed with sodium hydroxide solution (1 M, 100 mL × 2). The organic phase was retained, dried, concentrated by rotary evaporation to dryness, and subjected to column chromatography to give compound **123-a** (3.93 g).

### (2) Preparation method for compound 123-b:

Compound **123-a** (3.93 g) was dispersed in tetrahydrofuran (20 mL), and the mixture was cooled to 0-5 °C. Borane (1 M in THF, 46 mL) was added dropwise with the temperature kept at this temperature, and the mixture was reacted at 0-5 °C for 2 h. After the starting material was completely consumed as detected by TLC, hydrochloric acid (12 M, 3.0 mL) was slowly added dropwise to the reaction solution at 0-5 °C. After the dropwise addition, the mixture was heated at 60 °C for 2 h. After the intermediate disappeared as detected by TLC, the reaction solution was cooled to 20-25 °C and poured into water (50 mL), and the resulting mixture was adjusted to pH 9-10 with a saturated sodium bicarbonate solution and subjected to liquid separation. The aqueous phase was extracted with ethyl acetate (20 mL × 2). The organic phases were combined, dried, concentrated by rotary evaporation to dryness, and subjected to column chromatography to give compound **123-b** (1.52 g).

### (3) Preparation method for compound 123-c:

Compound **123-b** (1.52 g) was dispersed in methanol (40 mL), and hydrochloric acid (12 M, 3.8 mL) and Pd/C (10%, 0.15 g) were added. The mixture was purged with hydrogen three times, and heated to 50 °C and reacted for 5 h. After the reaction was completed as detected by TLC, the reaction solution was filtered through celite on a filter paper. The filtrate was concentrated by rotary evaporation to dryness to give **123-c** (0.83 g) as a colorless oily liquid.

### (4) Preparation method for Example 123:

**123-c** (68 mg) and potassium *tert*-butoxide (126 mg) were dispersed in *tert*-amyl alcohol (5 mL). The mixture was stirred at 20-25 °C for 5 min. Then compound **O-2** (100 mg) in Example 8, Pd₂(dba)₃ (26 mg), and 2-(dicyclohexylphosphine)-3,6-dimethoxy-2'-4'-6'-tri-I-propyl-11'-biphenyl (30 mg) were added, and the mixture was purged with nitrogen 3 times and reacted at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 123** (25 mg). ESI-MS: m/z = 563.55 [M+H] ⁺.

¹H NMR (500 MHz, DMSO-*d*₆) δ 12.43 (s, 1H), 8.38 (s, 1H), 7.95 (s, 1H), 7.58 (s, 1H), 7.30 (d, *J* = 8.5 Hz, 1H), 6.86 (s, 1H), 6.61 (d, *J* = 8.5 Hz, 1H), 4.37 - 4.31 (m, 1H), 4.30 - 4.24 (m, 1H), 4.09 (dd, *J* = 13.7, 8.8 Hz, 2H), 3.77 (s, 3H), 3.55 (s, 2H), 2.58 (s, 3H), 2.26 (d, *J* = 9.8 Hz, 1H), 2.18 (s, 1H), 2.04 (d, *J* = 7.1 Hz, 2H), 1.87 (s, 1H), 1.75 (d, *J* = 12.4 Hz, 1H), 1.20 (d, *J* = 6.2 Hz, 3H), 0.64 (s, 1H), 0.35 (d, *J* = 10.2 Hz, 1H), 0.00 (q, *J* = 4.9 Hz, 1H), -0.09 (s, 1H), -0.51 - -0.58 (m, 1H).

### Example 124:

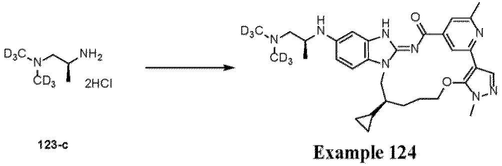

123-c (102 mg) and potassium *tert*-butoxide (189 mg) were dispersed in *tert*-amyl alcohol (5 mL). The mixture was stirred at 20-25 °C for 5 min. Then compound **O** (100 mg), Pd₂(dba)₃ (26 mg), and 2-(dicyclohexylphosphine)-3,6-dimethoxy-2'-4'-6'-tri-I-propyl-11'-biphenyl (30 mg) were added, and the mixture was purged with nitrogen 3 times and reacted in an oil bath at 100 °C for 2 h. After the mixture was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 124** (22 mg). ESI-MS: m/z = 563.48 [M+H] ⁺.

¹H NMR (500 MHz, DMSO-*d*₆) δ 12.41 (s, 1H), 8.34 (s, 1H), 7.90 (s, 1H), 7.54 (s, 1H), 7.34 (d, *J* = 8.8 Hz, 1H), 6.81 (s, 1H), 6.63 (d, *J* = 8.8 Hz, 1H), 4.29 (q, *J* = 7.8 Hz, 1H), 4.22 (dd, *J* = 14.2, 3.6 Hz, 1H), 4.04 (q, *J* = 6.2, 5.3 Hz, 2H), 3.73 (s, 3H), 3.50 (s, 2H), 2.54 (s, 3H), 2.20 (s, 1H), 2.14 (s, 1H), 2.00 (d, *J* = 13.8 Hz, 2H), 1.81 (s, 1H), 1.73 - 1.66 (m, 1H), 1.15 (d, *J* = 6.2 Hz, 3H), 0.59 (s, 1H), 0.30 (dq, *J* = 9.2, 4.6 Hz, 1H), -0.06 (dd, *J* = 9.5, 5.0 Hz, 1H), -0.16 (s, 1H), -0.59 (q, *J* = 5.1 Hz, 1H).

### Example 125:

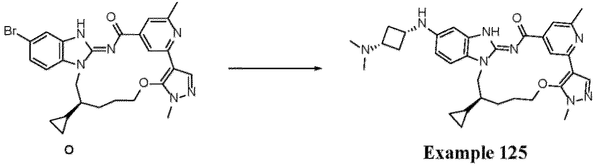

Compound **O** (120 mg), *cis-N*¹,*N*¹-dimethylcyclobutane-1,3-diamine (31 mg), Pd₂(dba)₃ (30 mg), 2-(dicyclohexylphosphine)-3,6-dimethoxy-2'-4'-6'-tri-I-propyl-11'-biphenyl (35 mg), and potassium *tert*-butoxide (148 mg) were dispersed in *tert*-amyl alcohol (5 mL). The mixture was purged with nitrogen 3 times and reacted in an oil bath at 100 °C for 3.0 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 125** (25 mg). ESI-MS: m/z = 569.44 [M+H] ⁺.

¹H NMR (500 MHz, DMSO-*d*₆) δ 12.38 (s, 1H), 8.40 (s, 1H), 7.97 (s, 1H), 7.61 (s, 1H), 7.39 (d, *J* = 8.7 Hz, 1H), 6.77 (s, 1H), 6.59 (d, *J* = 8.6 Hz, 1H), 4.35 (q, *J* = 7.8 Hz, 1H), 4.28 (dd, *J* = 14.0, 3.5 Hz, 1H), 4.09 (dq, *J* = 14.0, 4.6 Hz, 2H), 3.81 (d, *J* = 9.8 Hz, 1H), 3.79 (s, 3H), 2.91 (d, *J* = 7.5 Hz, 1H), 2.61 (s, 3H), 2.48 (t, *J* = 7.5 Hz, 1H), 2.30 - 2.24 (m, 1H), 2.20 (d, *J* = 13.1 Hz, 1H), 2.12 (s, 6H), 2.05 (d, *J* = 7.7 Hz, 2H), 1.87 (t, *J* = 8.5 Hz, 1H), 1.81 - 1.74 (m, 1H), 1.69 (q, *J* = 9.9, 9.1 Hz, 2H), 0.64 (h, *J* = 8.5, 7.3 Hz, 1H), 0.37 (tt, *J* = 9.1, 4.8 Hz, 1H), 0.00 (dq, *J* = 9.8, 5.0 Hz, 1H), -0.09 (dq, *J* = 8.8, 4.5 Hz, 1H), -0.53 (dt, *J* = 9.9, 5.1 Hz, 1H).

### Example 126:

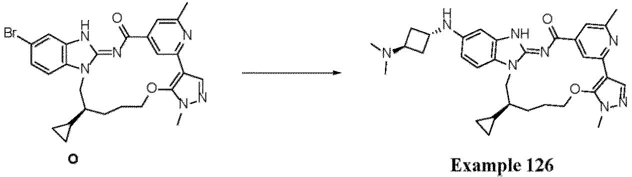

Compound **O** (120 mg), *trans*-*N*¹,*N*¹-dimethylcyclobutane-1,3-diamine (31 mg), Pd₂(dba)₃ (30 mg), 2-(dicyclohexylphosphine)-3,6-dimethoxy-2'-4'-6'-tri-I-propyl-11'-biphenyl (35 mg), and potassium *tert*-butoxide (148 mg) were dispersed in *tert*-amyl alcohol (5 mL). The mixture was purged with nitrogen 3 times and reacted in an oil bath at 100 °C for 3.0 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 126** (20 mg). ESI-MS: m/z = 569.46 [M+H] ⁺.

¹H NMR (500 MHz, DMSO-*d*₆) δ 12.42 (s, 1H), 8.40 (s, 1H), 7.97 (s, 1H), 7.61 (d, *J* = 1.2 Hz, 1H), 7.39 (d, *J* = 8.7 Hz, 1H), 6.72 (d, *J* = 2.1 Hz, 1H), 6.56 (dd, *J* = 8.6, 2.2 Hz, 1H), 6.01 (d, *J* = 5.3 Hz, 1H), 4.35 (q, *J* = 7.8 Hz, 1H), 4.28 (dd, *J* = 14.0, 3.5 Hz, 1H), 4.13 - 4.05 (m, 2H), 3.81 (d, *J* = 9.8 Hz, 1H), 3.79 (s, 3H), 2.85 (p, *J* = 6.6 Hz, 1H), 2.61 (s, 3H), 2.28 (ddd, *J* = 13.0, 7.5, 5.7 Hz, 3H), 2.20 (dq, *J* = 8.8, 4.3, 3.4 Hz, 1H), 2.14 (s, 6H), 2.07 - 1.99 (m, 3H), 1.88 (d, *J* = 9.3 Hz, 1H), 1.77 (dd, *J* = 11.3, 6.0 Hz, 1H), 0.65 (ddt, *J* = 13.0, 8.6, 4.6 Hz, 1H), 0.37 (tt, *J* = 9.3, 4.8 Hz, 1H), 0.00 (dq, *J* = 9.6, 5.0 Hz, 1H), -0.09 (dq, *J* = 9.4, 4.9 Hz, 1H), -0.53 (dq, *J* = 9.8, 5.0 Hz, 1H).

### Example 127:

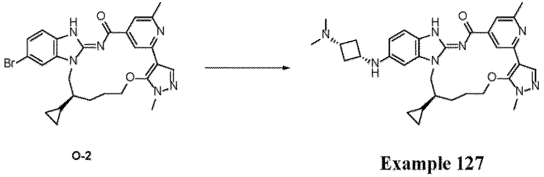

Compound **O-2** (60 mg), *cis-N*¹,*N*¹-dimethylcyclobutane-1,3-diamine (31 mg), Pd₂(dba)₃ (30 mg), 2-(dicyclohexylphosphine)-3,6-dimethoxy-2'-4'-6'-tri-I-propyl-11'-biphenyl (35 mg), and potassium *tert*-butoxide (148 mg) were dispersed in *tert-amyl* alcohol (5 mL). The mixture was purged with nitrogen 3 times and reacted in an oil bath at 100 °C for 3.0 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 127** (50 mg). ESI-MS: m/z = 569.46 [M+H] ⁺.

¹H NMR (500 MHz, DMSO-*d*₆) δ 12.38 (s, 1H), 8.40 (s, 1H), 8.30 (s, 1H), 7.97 (d, *J* = 1.2 Hz, 1H), 7.58 (d, *J* = 8.7 Hz, 1H), 7.32 (d, *J* = 8.5 Hz, 1H), 6.60 - 6.49 (m, 2H), 4.33 - 4.25 (m, 2H), 4.06 (d, *J* = 5.7 Hz, 2H), 3.81 (d, *J* = 9.8 Hz, 1H), 3.77 (s, 3H), 3.08 (p, *J* = 6.6 Hz, 1H), 2.61 (s, 3H), 2.28 (ddd, *J* = 13.0, 7.5, 5.7 Hz, 3H), 2.20 (dq, *J* = 8.8, 4.3, 3.4 Hz, 1H), 2.26 (s, 6H), 2.10 - 1.98 (m, 3H), 1.84 (q, *J* = 8.0, 7.6 Hz, 1H), 1.80 - 1.73 (m, 1H), 0.65 (dq, *J* = 17.8, 8.2, 6.6 Hz, 1H), 0.36 (tt, *J* = 9.3, 4.5 Hz, 1H), -0.01 (dq, *J* = 9.8, 5.0 Hz, 1H), -0.09 (dq, *J* = 9.1, 4.4 Hz, 1H), -0.55 (dt, *J* = 9.8, 4.8 Hz, 1H).

### Example 128:

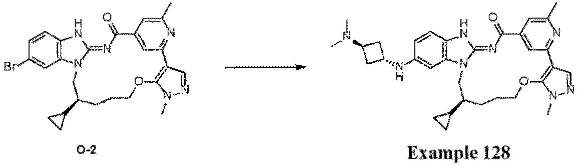

Compound **O-2** (120 mg), *trans-N*¹,*N*¹-dimethylcyclobutane-1,3-diamine (31 mg), Pd₂(dba)₃ (30 mg), 2-(dicyclohexylphosphine)-3,6-dimethoxy-2'-4'-6'-tri-I-propyl-11'-biphenyl (35 mg), and potassium *tert*-butoxide (148 mg) were dispersed in *tert*-amyl alcohol (5 mL). The mixture was purged with nitrogen 3 times and reacted in an oil bath at 100 °C for 3 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 128** (35 mg). ESI-MS: m/z = 569.49 [M+H] ⁺.

¹H NMR (500 MHz, DMSO-*d*₆) δ 12.42 (s, 1H), 8.38 (s, 1H), 8.32 (s, 1H), 7.95 (d, *J* = 1.2 Hz, 1H), 7.58 (d, *J* = 8.7 Hz, 1H), 7.32 (d, *J* = 8.5 Hz, 1H), 6.60 - 6.49 (m, 2H), 4.33 - 4.25 (m, 2H), 4.06 (d, *J* = 5.7 Hz, 2H), 3.81 (d, *J* = 9.8 Hz, 1H), 3.77 (s, 3H), 3.08 (p, *J* = 6.6 Hz, 1H), 2.61 (s, 3H), 2.28 (ddd, *J* = 13.0, 7.5, 5.7 Hz, 3H), 2.20 (dq, *J* = 8.8, 4.3, 3.4 Hz, 1H), 2.26 (s, 6H), 2.10 - 1.98 (m, 3H), 1.84 (q, *J* = 8.0, 7.6 Hz, 1H), 1.80 - 1.73 (m, 1H), 0.64 (dq, *J* = 17.8, 8.2, 6.6 Hz, 1H), 0.37 (tt, *J* = 9.3, 4.5 Hz, 1H), 0.00 (dq, *J* = 9.8, 5.0 Hz, 1H), -0.07 (dq, *J* = 9.1, 4.4 Hz, 1H), - 0.50 (dt, *J* = 9.8, 4.8 Hz, 1H).

### Example 129:

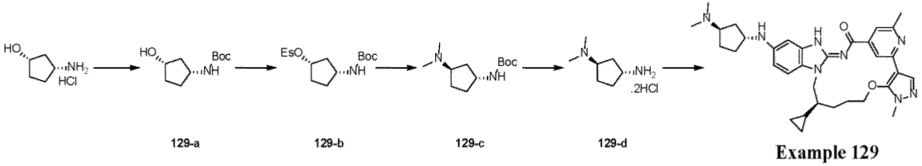

### (1) Preparation method for compound 129-a:

(1S,3R)-3-Aminocyclopentanol hydrochloride (500 mg) was dispersed in tetrahydrofuran (10 mL), and a solution of sodium hydroxide (363 mg) in water (3 mL) and di-tert-butyl dicarbonate (833 mg) were sequentially added. The mixture was reacted at 20-25 °C for 3 h. After the reaction was completed as detected by TLC, 20 mL of water was added to the reaction solution, and the resulting mixture was extracted with ethyl acetate (30 mL × 2). The organic phases were combined, dried over anhydrous sodium sulfate, and concentrated until no liquid flowed out to give compound **129-a** (731 mg), which was directly used in the next step without purification.

### (2) Preparation method for compound 129-b:

Compound **129-a** (731 mg) was dispersed in ethyl acetate (20 mL), and triethylamine (735 mg) and ethylsulfonyl chloride (513 mg) were sequentially added to the reaction solution with the temperature controlled at 5-15 °C. The mixture was reacted for 0.5 h with the temperature kept at 5-15 °C. After the reaction was completed as detected by TLC, the reaction solution was washed with water (20 mL × 2). The ethyl acetate phase was retained, dried over anhydrous sodium sulfate, and concentrated until no liquid flowed out to give compound **129-b** (1.06 g), which was directly used in the next step without purification.

### (3) Preparation method for compound 129-c:

Compound **129-b** (1.06 g) was dispersed in *N,N*-dimethylformamide (20 mL), and potassium carbonate (1.51 g), 4-dimethylaminopyridine (44 mg), and dimethylamine hydrochloride (5.92 g) were sequentially added. The mixture was reacted at 80 °C for 6 h. After the reaction was completed as monitored by TLC, 100 mL of water was added to the reaction solution, and the resulting mixture was extracted with ethyl acetate (50 mL × 2). The organic phases were combined, sequentially washed with water (50 mL) and saturated brine (50 mL), dried over anhydrous sodium sulfate, concentrated until no liquid flowed out, and subjected to column chromatography to give **129-c** (238 mg) as a liquid.

### (4) Preparation method for compound 129-d:

Compound **129-c** (238 mg) was dispersed in a hydrochloric acid methanol solution (4 M, 15 mL). The mixture was stirred at 20-25 °C for 1 h. After the reaction was completed as detected by TLC, the reaction solution was concentrated until no liquid flowed out, and the residue was redissolved in methanol (15 mL) and concentrated until no liquid flowed out to give compound **129-d** (150 mg).

### (5) Preparation method for Example 129:

Compound **O** (50 mg), **129-d** (25 mg), Pd₂(dba)₃ (29 mg), 2-di-*tert*-butylphosphino-2',4',6'-triisopropylbiphenyl (27 mg), and potassium *tert*-butoxide (141 mg) were dispersed in *tert*-amyl alcohol (5 mL). The mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 129** (22 mg). ESI-MS: m/z = 583.51 [M+H] ⁺.

¹H NMR (500 MHz, chloroform-*d*) δ 12.05 (s, 1H), 8.39 (s, 1H), 8.15 (s, 1H), 7.67 (s, 1H), 7.16 (d, *J* = 8.5 Hz, 1H), 6.55 (d, *J* = 9.4 Hz, 2H), 4.37 - 4.33 (m, 2H), 3.99 - 3.93 (m, 2H), 3.78 (s, 3H), 3.42 (t, *J* = 8.6 Hz, 1H), 2.73 (s, 1H), 2.71 - 2.66 (m, 6H), 2.63 (s, 3H), 2.38 (dt, *J* = 15.9, 7.9 Hz, 2H), 2.28 (dd, *J* = 13.9, 6.9 Hz, 1H), 2.21 - 2.12 (m, 3H), 2.02 - 1.93 (m, 2H), 1.82 - 1.73 (m, 2H), 1.59 (p, *J* = 6.8 Hz, 1H), 0.51 (d, *J* = 9.6 Hz, 1H), 0.43 (tt, *J* = 8.8, 4.7 Hz, 1H), 0.11 (d, *J* = 10.1 Hz, 1H), -0.01 - -0.07 (m, 1H), -0.32 (dt, *J* = 10.2, 5.2 Hz, 1H).

### Example 130:

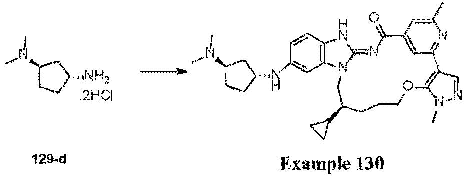

Compound **O-2** (50 mg) in Example 8, **129-d** (25 mg), Pd₂(dba)₃ (29 mg), 2-di-*tert*-butylphosphino-2',4',6'-triisopropylbiphenyl (27 mg), and potassium *tert*-butoxide (141 mg) were dispersed in *tert*-amyl alcohol (5 mL). The mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 130** (20 mg). ESI-MS: m/z = 583.53 [M+H] ⁺.

¹H NMR (500 MHz, chloroform-*d*) δ 8.39 (d, *J* = 4.4 Hz, 3H), 8.15 (s, 1H), 7.64 (s, 1H), 7.14 (d, *J* = 8.4 Hz, 1H), 6.59 (s, 1H), 6.51 (d, *J* = 8.5 Hz, 1H), 4.35 (tt, *J* = 8.7, 4.4 Hz, 2H), 4.05 (h, *J* = 6.1, 5.4 Hz, 1H), 4.00 - 3.91 (m, 2H), 3.78 (s, 3H), 3.46 (t, *J* = 8.5 Hz, 1H), 2.74 (d, *J* = 13.9 Hz, 6H), 2.63 (s, 3H), 2.42 (q, *J* = 9.2, 8.2 Hz, 2H), 2.30 - 2.11 (m, 4H), 2.08 - 1.94 (m, 2H), 1.86 - 1.75 (m, 2H), 1.65 - 1.55 (m, 1H), 0.55 (dq, *J* = 12.8, 6.6 Hz, 1H), 0.42 (tq, *J* = 9.0, 4.5 Hz, 1H), 0.06 (td, *J* = 8.6, 4.6 Hz, 1H), -0.05 (dq, *J* = 9.1, 4.7 Hz, 1H), -0.37 (ddt, *J* = 32.8, 9.7, 5.0 Hz, 1H).

### Example 131:

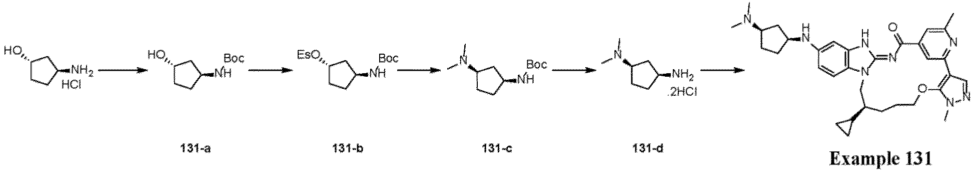

### (1) Preparation method for compound 131-a:

(1*S*,3*S*)-3-Aminocyclopentanol hydrochloride (500 mg) was dispersed in tetrahydrofuran (10 mL), and a solution of sodium hydroxide (363 mg) in water (3 mL) and di-*tert*-butyl dicarbonate (833 mg) were sequentially added. The mixture was reacted at 20-25 °C for 3 h. After the reaction was completed as detected by TLC, 20 mL of water was added to the reaction solution, and the resulting mixture was extracted with ethyl acetate (30 mL × 2). The organic phases were combined, dried over anhydrous sodium sulfate, and concentrated until no liquid flowed out to give compound **131-a** (720 mg), which was directly used in the next step without purification.

### (2) Preparation method for compound 131-b:

Compound **131-a** (720 mg) was dispersed in ethyl acetate (20 mL), and triethylamine (721 mg) and ethylsulfonyl chloride (505 mg) were sequentially added to the reaction solution with the temperature controlled at 5-15 °C. The mixture was reacted for 0.5 h with the temperature kept at 5-15 °C. After the reaction was completed as detected by TLC, the reaction solution was washed with water (20 mL × 2). The ethyl acetate phase was retained, dried over anhydrous sodium sulfate, and concentrated until no liquid flowed out to give compound **131-b** (1.01 g), which was directly used in the next step without purification.

### (3) Preparation method for compound 131-c:

Compound **131-b** (1.01 g) was dispersed in *N,N*-dimethylformamide (20 mL), and potassium carbonate (1.44 g), 4-dimethylaminopyridine (42 mg), and dimethylamine hydrochloride (5.64 g) were sequentially added. The mixture was reacted at 80 °C for 6 h. After the reaction was completed as monitored by TLC, 100 mL of water was added to the reaction solution, and the resulting mixture was extracted with ethyl acetate (50 mL × 2). The organic phases were combined, sequentially washed with water (50 mL) and saturated brine (50 mL), dried over anhydrous sodium sulfate, concentrated until no liquid flowed out, and subjected to column chromatography to give **131-c** (215 mg) as a liquid.

### (4) Preparation method for compound 131-d:

Compound **131-c** (215 mg) was dispersed in a hydrochloric acid methanol solution (4 M, 15 mL). The mixture was stirred at 20-25 °C for 1 h. After the reaction was completed as detected by TLC, the reaction solution was concentrated until no liquid flowed out, and the residue was redissolved in methanol (15 mL) and concentrated until no liquid flowed out to give compound **131-d** (140 mg).

### (5) Preparation method for Example 131:

Compound **O** (50 mg), **131-d** (25 mg), Pd₂(dba)₃ (29 mg), 2-di-*tert*-butylphosphino-2',4',6'-triisopropylbiphenyl (27 mg), and potassium *tert*-butoxide (141 mg) were dispersed in *tert*-amyl alcohol (5 mL). The mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 131** (15 mg). ESI-MS: m/z = 583.53 [M+H] ⁺.

¹H NMR (500 MHz, chloroform-*d*) δ 8.46 (s, 1H), 8.40 (d, *J* = 8.5 Hz, 1H), 8.15 (s, 1H), 7.70 (d, *J* = 24.2 Hz, 1H), 7.14 (dd, *J* = 18.5, 8.5 Hz, 1H), 6.65 - 6.44 (m, 2H), 4.35 (dt, *J* = 13.9, 4.1 Hz, 2H), 3.99 - 3.89 (m, 3H), 3.78 (s, 3H), 3.42 - 3.35 (m, 1H), 2.68 (d, *J* = 15.3 Hz, 6H), 2.64 (s, 3H), 2.40 - 2.31 (m, 1H), 2.27 (q, *J* = 6.6 Hz, 1H), 2.22 - 2.11 (m, 3H), 1.96 (dtd, *J* = 26.5, 18.1, 16.5, 10.2 Hz, 3H), 1.78 (q, *J* = 10.6, 9.1 Hz, 2H), 1.63 - 1.53 (m, 1H), 0.55 - 0.47 (m, 1H), 0.43 (dp, *J* = 9.1, 4.7 Hz, 1H), 0.10 (tt, *J* = 9.4, 4.9 Hz, 1H), -0.01 - -0.08 (m, 1H), -0.33 (dt, *J* = 8.8, 4.5 Hz, 1H).

### Example 132:

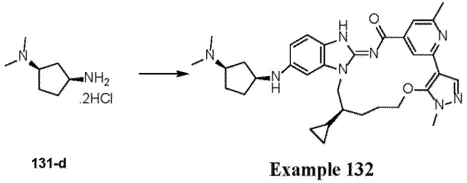

Compound **O-2** (50 mg) in Example 8, **131-d** (25 mg), Pd₂(dba)₃ (29 mg), 2-di-*tert*-butylphosphino-2',4',6'-triisopropylbiphenyl (27 mg), and potassium *tert*-butoxide (141 mg) were dispersed in *tert*-amyl alcohol (5 mL). The mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 132** (23 mg). ESI-MS: m/z = 583.51 [M+H] ⁺.

¹H NMR (500 MHz, chloroform-*d*) δ 11.93 (s, 1H), 8.39 (d, *J* = 4.5 Hz, 1H), 8.15 (s, 1H), 7.65 (s, 1H), 7.13 (dd, *J* = 8.5, 6.1 Hz, 1H), 6.63 - 6.47 (m, 2H), 4.35 (dd, *J* = 10.6, 4.2 Hz, 2H), 4.08 - 4.05 (m, 1H), 3.98 - 3.93 (m, 2H), 3.78 (s, 3H), 3.37 (q, *J* = 8.3 Hz, 1H), 2.71 (d, *J* = 34.9 Hz, 6H), 2.63 (s, 3H), 2.47 - 2.38 (m, 1H), 2.32 - 2.24 (m, 1H), 2.24 - 2.12 (m, 3H), 2.09 - 1.88 (m, 3H), 1.85 - 1.77 (m, 2H), 1.60 (dq, *J* = 13.7, 7.1 Hz, 1H), 0.55 (d, *J=* 14.4 Hz, 1H), 0.44 (tt, *J* = 9.0, 4.4 Hz, 1H), 0.12 (ddt, *J* = 39.2, 8.8, 4.2 Hz, 1H), -0.04 (q, *J* = 5.3, 4.6 Hz, 1H), -0.30 (ddq, *J* = 28.3, 9.8, 5.1 Hz, 1H).

### Example 133:

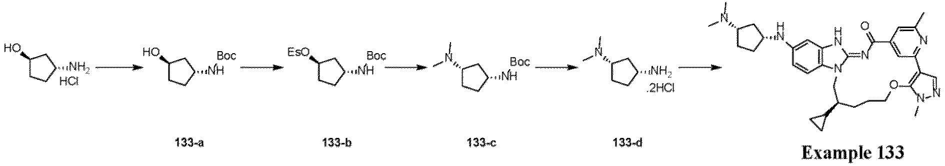

### (1) Preparation method for compound 133-a:

(1*R*,3*R*)-3-Aminocyclopentanol hydrochloride (500 mg) was dispersed in tetrahydrofuran (10 mL), and a solution of sodium hydroxide (363 mg) in water (3 mL) and di-*tert*-butyl dicarbonate (833 mg) were sequentially added. The mixture was reacted at 20-25 °C for 3 h. After the reaction was completed as detected by TLC, 20 mL of water was added to the reaction solution, and the resulting mixture was extracted with ethyl acetate (30 mL × 2). The organic phases were combined, dried over anhydrous sodium sulfate, and concentrated until no liquid flowed out to give compound **133-a** (730 mg), which was directly used in the next step without purification.

### (2) Preparation method for compound 133-b:

Compound **133-a** (730 mg) was dispersed in ethyl acetate (20 mL), and triethylamine (731 mg) and ethylsulfonyl chloride (512 mg) were sequentially added to the reaction solution with the temperature controlled at 5-15 °C. The mixture was reacted for 0.5 h with the temperature kept at 5-15 °C. After the reaction was completed as detected by TLC, the reaction solution was washed with water (20 mL × 2). The ethyl acetate phase was retained, dried over anhydrous sodium sulfate, and concentrated until no liquid flowed out to give compound **133-b** (1.05 g), which was directly used in the next step without purification.

### (3) Preparation method for compound 133-c:

Compound **133-b** (1.05 g) was dispersed in *N,N*-dimethylformamide (20 mL), and potassium carbonate (1.50 g), 4-dimethylaminopyridine (44 mg), and dimethylamine hydrochloride (5.86 g) were sequentially added. The mixture was reacted at 80 °C for 6 h. After the reaction was completed as monitored by TLC, 100 mL of water was added to the reaction solution, and the resulting mixture was extracted with ethyl acetate (50 mL × 2). The organic phases were combined, sequentially washed with water (50 mL) and saturated brine (50 mL), dried over anhydrous sodium sulfate, concentrated until no liquid flowed out, and subjected to column chromatography to give **133-c** (230 mg) as a liquid.

### (4) Preparation method for compound 133-d:

Compound **133-c** (230 mg) was dispersed in a hydrochloric acid methanol solution (4 M, 15 mL). The mixture was stirred at 20-25 °C for 1 h. After the reaction was completed as detected by TLC, the reaction solution was concentrated until no liquid flowed out, and the residue was redissolved in methanol (15 mL) and concentrated until no liquid flowed out to give compound **133-d** (155 mg).

### (5) Preparation method for Example 133:

Compound **O** (50 mg), **133-d** (25 mg), Pd₂(dba)₃ (29 mg), 2-di-*tert*-butylphosphino-2',4',6'-triisopropylbiphenyl (27 mg), and potassium *tert*-butoxide (141 mg) were dispersed in *tert*-amyl alcohol (5 mL). The mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 133** (18 mg). ESI-MS: m/z = 583.53 [M+H] ⁺.

¹H NMR (500 MHz, chloroform-*d*) δ 8.51 (s, 1H), 8.42 (d, *J* = 8.5 Hz, 1H), 8.13 (s, 1H), 7.71 (d, *J* = 24.2 Hz, 1H), 7.14 (dd, *J* = 18.5, 8.5 Hz, 1H), 6.62 - 6.49 (m, 2H), 4.35 (dt, *J* = 13.9, 4.1 Hz, 2H), 3.99 - 3.89 (m, 3H), 3.77 (s, 3H), 3.41 - 3.35 (m, 1H), 2.68 (d, *J* = 15.3 Hz, 6H), 2.61 (s, 3H), 2.38 - 2.31 (m, 1H), 2.28 (q, *J* = 6.6 Hz, 1H), 2.20 - 2.11 (m, 3H), 1.96 (dtd, *J* = 26.5, 18.1, 16.5, 10.2 Hz, 3H), 1.78 (q, *J* = 10.6, 9.1 Hz, 2H), 1.63 - 1.53 (m, 1H), 0.55 - 0.47 (m, 1H), 0.43 (dp, *J* = 9.1, 4.7 Hz, 1H), 0.10 (tt, *J* = 9.4, 4.9 Hz, 1H), -0.01 - -0.08 (m, 1H), -0.33 (dt, *J* = 8.8, 4.5 Hz, 1H).

### Example 134:

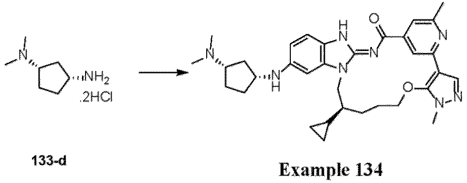

Compound **O-2** (50 mg) in Example 8, **133-d** (25 mg), Pd₂(dba)₃ (29 mg), 2-di-*tert*-butylphosphino-2',4',6'-triisopropylbiphenyl (27 mg), and potassium *tert*-butoxide (141 mg) were dispersed in *tert*-amyl alcohol (5 mL). The mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 134** (20 mg). ESI-MS: m/z = 583.51 [M+H] ⁺.

¹H NMR (500 MHz, chloroform-*d*) δ 8.42 (d, *J* = 4.4 Hz, 3H), 8.14 (s, 1H), 7.68 (s, 1H), 7.11 (d, *J* = 8.4 Hz, 1H), 6.59 (s, 1H), 6.50 (d, *J* = 8.5 Hz, 1H), 4.33 (tt, *J* = 8.7, 4.4 Hz, 2H), 4.09 (h, *J* = 6.1, 5.4 Hz, 1H), 4.00 - 3.92 (m, 2H), 3.78 (s, 3H), 3.45 (t, *J* = 8.5 Hz, 1H), 2.70 (d, *J* = 13.9 Hz, 6H), 2.61 (s, 3H), 2.41 (q, *J* = 9.2, 8.2 Hz, 2H), 2.30 - 2.13 (m, 4H), 2.04 - 1.92 (m, 2H), 1.84 - 1.74 (m, 2H), 1.65 - 1.55 (m, 1H), 0.56 (dq, *J* = 12.8, 6.6 Hz, 1H), 0.40 (tq, *J* = 9.0, 4.5 Hz, 1H), 0.04 (td, *J* = 8.6, 4.6 Hz, 1H), -0.05 (dq, *J* = 9.1, 4.7 Hz, 1H), -0.37 (ddt, *J* = 32.8, 9.7, 5.0 Hz, 1H).

### Example 135:

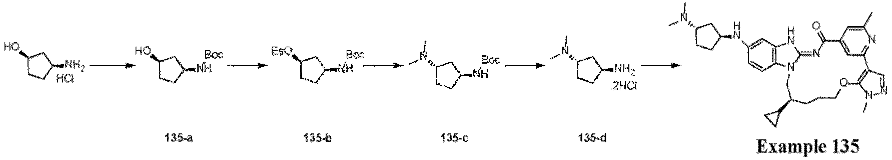

### (1) Preparation method for compound 135-a:

(1*R*,3*S*)-3-Aminocyclopentanol hydrochloride (500 mg) was dispersed in tetrahydrofuran (10 mL), and a solution of sodium hydroxide (363 mg) in water (3 mL) and di-*tert*-butyl dicarbonate (833 mg) were sequentially added. The mixture was reacted at 20-25 °C for 3 h. After the reaction was completed as detected by TLC, 20 mL of water was added to the reaction solution, and the resulting mixture was extracted with ethyl acetate (30 mL × 2). The organic phases were combined, dried over anhydrous sodium sulfate, and concentrated until no liquid flowed out to give compound **135-a** (700 mg), which was directly used in the next step without purification.

### (2) Preparation method for compound 135-b:

Compound **135-a** (700 mg) was dispersed in ethyl acetate (20 mL), and triethylamine (701 mg) and ethylsulfonyl chloride (491 mg) were sequentially added to the reaction solution with the temperature controlled at 5-15 °C. The mixture was reacted for 0.5 h with the temperature kept at 5-15 °C. After the reaction was completed as detected by TLC, the reaction solution was washed with water (20 mL × 2). The ethyl acetate phase was retained, dried over anhydrous sodium sulfate, and concentrated until no liquid flowed out to give compound **135-b** (1.00 g), which was directly used in the next step without purification.

### (3) Preparation method for compound 135-c:

Compound **135-b** (1.00 g) was dispersed in *N,N-*dimethylformatnide (20 mL), and potassium carbonate (1.43 g), 4-dimethylaminopyridine (42 mg), and dimethylamine hydrochloride (5.58 g) were sequentially added. The mixture was reacted at 80 °C for 6 h. After the reaction was completed as monitored by TLC, 100 mL of water was added to the reaction solution, and the resulting mixture was extracted with ethyl acetate (50 mL × 2). The organic phases were combined, sequentially washed with water (50 mL) and saturated brine (50 mL), dried over anhydrous sodium sulfate, concentrated until no liquid flowed out, and subjected to column chromatography to give **135-c** (210 mg) as a liquid.

### (4) Preparation method for compound 135-d:

Compound **135-c** (210 mg) was dispersed in a hydrochloric acid methanol solution (4 M, 15 mL). The mixture was stirred at 20-25 °C for 1 h. After the reaction was completed as detected by TLC, the reaction solution was concentrated until no liquid flowed out, and the residue was redissolved in methanol (15 mL) and concentrated until no liquid flowed out to give compound **135-d** (140 mg).

### (5) Preparation method for Example 135:

Compound **O** (50 mg), **135-d** (25 mg), Pd₂(dba)₃ (29 mg), 2-di-*tert*-butylphosphino-2',4',6'-triisopropylbiphenyl (27 mg), and potassium *tert*-butoxide (141 mg) were dispersed in *tert*-amyl alcohol (5 mL). The mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 135** (18 mg). ESI-MS: m/z = 583.53 [M+H] ⁺.

¹H NMR (500 MHz, chloroform-*d*) δ 12.06 (s, 1H), 8.39 (s, 1H), 8.15 (s, 1H), 7.67 (s, 1H), 7.16 (d, *J* = 8.5 Hz, 1H), 6.56 (d, *J* = 12.0 Hz, 2H), 4.38 - 4.32 (m, 2H), 4.02 - 3.88 (m, 3H), 3.78 (s, 3H), 3.44 (p, *J* = 8.4 Hz, 1H), 2.70 (s, 6H), 2.63 (s, 3H), 2.39 (dt, *J* = 14.0, 8.0 Hz, 2H), 2.26 (q, *J* = 6.6 Hz, 1H), 2.16 (ddd, *J* = 24.0, 16.9, 7.0 Hz, 3H), 1.97 (td, *J* = 14.1, 7.9 Hz, 2H), 1.83 - 1.73 (m, 2H), 1.64 - 1.53 (m, 1H), 0.50 (dd, *J* = 11.1, 6.2 Hz, 1H), 0.43 (tt, *J* = 9.1, 4.9 Hz, 1H), 0.10 (tt, *J* = 9.0, 5.1 Hz, 1H), -0.04 (q, *J* = 4.8 Hz, 1H), -0.33 (dq, *J* = 10.1, 5.0 Hz, 1H).

### Example 136:

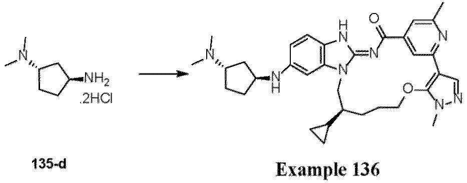

Compound **O-2** (50 mg) in Example 8, **135-d** (25 mg), Pd₂(dba)₃ (29 mg), 2-di-*tert*-butylphosphino-2',4',6'-triisopropylbiphenyl (27 mg), and potassium *tert*-butoxide (141 mg) were dispersed in *tert*-amyl alcohol (5 mL). The mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 136** (20 mg). ESI-MS: m/z = 583.51 [M+H] ⁺.

¹H NMR (500 MHz, chloroform-*d*) δ 8.44 (s, 1H), 8.39 (s, 1H), 8.15 (s, 1H), 7.64 (s, 1H), 7.14 (d, *J* = 8.5 Hz, 1H), 6.59 (d, *J* = 2.0 Hz, 1H), 6.55 - 6.47 (m, 1H), 4.35 (td, *J* = 8.4, 4.3 Hz, 2H), 4.08 - 4.04 (m, 1H), 3.95 (td, *J* = 8.4, 3.9 Hz, 2H), 3.78 (s, 3H), 3.39 (p, *J* = 8.2 Hz, 1H), 2.68 (s, 6H), 2.63 (s, 3H), 2.47 - 2.37 (m, 2H), 2.31 - 2.24 (m, 1H), 2.23 - 2.11 (m, 3H), 2.08 - 2.00 (m, 1H), 1.99 - 1.91 (m, 1H), 1.86 - 1.76 (m, 2H), 1.67 - 1.55 (m, 1H), 0.55 (tt, *J* = 9.0, 5.2 Hz, 1H), 0.42 (tt, *J* = 9.3, 5.1 Hz, 1H), 0.07 (tt, *J* = 9.0, 5.0 Hz, 1H), -0.03 (p, *J* = 5.0 Hz, 1H), -0.34 (dq, *J* = 10.2, 5.1 Hz, 1H).

### Example 137:

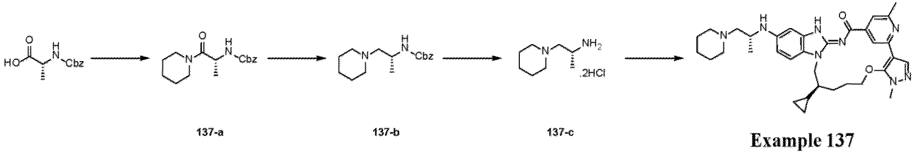

### (1) Preparation method for compound 137-a:

*N*-benzyloxycarbonyl-D-alanine (5.0 g) was dispersed in dichloromethane (75 mL), and piperidine hydrochloride (3.3 g), HATU (12.8 g), and *N,N*-diisopropylethylamine (8.7 g) were sequentially added. The mixture was reacted at 25 °C for 16 h. After the reaction was completed as detected by TLC, the reaction solution was washed with 1 M NaHCOs (50 mL × 2). The organic phase was retained, dried, concentrated by rotary evaporation to dryness, and subjected to column chromatography to give compound **137-a** (5.5 g).

### (2) Preparation method for compound 137-b:

Compound **137-a** (5.5 g) was dispersed in tetrahydrofuran (50 mL), and the mixture was cooled to 0-5 °C. Borane (1 M in THF, 38 mL) was added dropwise with the temperature kept at this temperature, and the mixture was reacted at 0-5 °C for 2 h. After the starting material was completely consumed as detected by TLC, hydrochloric acid (12 M, 5 mL) was slowly added dropwise to the reaction solution at 0-5 °C. After the dropwise addition, the mixture was heated at 60 °C for 2 h. After the intermediate disappeared as detected by TLC, the reaction solution was cooled to 20-25 °C and poured into water (200 mL), and the resulting mixture was adjusted to pH 9-10 with a saturated sodium bicarbonate solution and subjected to liquid separation. The aqueous phase was extracted with ethyl acetate (50 mL × 2). The organic phases were combined, dried, concentrated by rotary evaporation to dryness, and subjected to column chromatography to give compound **137-b** (2.9 g).

### (3) Preparation method for compound 137-c:

Compound **137-b** (2.9 g) was dispersed in methanol (30 mL), and hydrochloric acid (12 M, 3.0 mL) and Pd/C (10%, 0.3 g) were added. The mixture was purged with hydrogen three times, and heated to 50 °C and reacted for 5 h. After the reaction was completed as detected by TLC, the reaction solution was filtered through celite on a filter paper and concentrated. Ethyl acetate (40 mL) was added, and the resulting mixture was stirred at 25 °C for 1 h and filtered. The filter cake was dried to give **137-c** (1.8 g).

### (4) Preparation method for Example 137:

Compound **O** (100 mg), compound **137-c** (48 mg), Pd₂(dba)₃ (43 mg), 2-(dicyclohexylphosphine)-3,6-dimethoxy-2'-4'-6'-tri-I-propyl-11'-biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give Example 137 (32 mg). ESI-MS: m/z = 597.48 [M+H] ⁺.

¹H NMR (500 MHz, DMSO-*d*₆) δ 12.38 (s, 1H), 8.34 (d, *J* = 1.3 Hz, 1H), 8.18 (s, 1H), 7.91 (s, 1H), 7.54 (d, *J* = 1.2 Hz, 1H), 7.32 (d, *J* = 8.7 Hz, 1H), 6.79 (d, *J* = 2.2 Hz, 1H), 6.60 (dd, *J* = 8.7, 2.2 Hz, 1H), 4.32 - 4.26 (m, 1H), 4.22 (dd, *J* = 13.9, 3.5 Hz, 1H), 4.02 (ddd, *J =* 17.0, 12.9, 7.6 Hz, 2H), 3.73 (s, 3H), 3.53 - 3.49 (m, 1H), 2.54 (s, 3H), 2.46 (dt, *J* = 12.4, 6.7 Hz, 2H), 2.42 - 2.36 (m, 2H), 2.22 (dt, *J* = 14.5, 7.3 Hz, 2H), 2.14 (dt, *J* = 10.9, 5.5 Hz, 1H), 2.03 - 1.94 (m, 1H), 1.80 (s, 1H), 1.74 - 1.66 (m, 1H), 1.56 - 1.49 (m, 4H), 1.39 (t, *J* = 5.9 Hz, 2H), 1.25 (d, *J* = 4.7 Hz, 1H), 1.15 (d, *J* = 6.2 Hz, 3H), 0.63 - 0.55 (m, 1H), 0.31 (tt, *J* = 9.2, 4.6 Hz, 1H), -0.05 (dq, *J* = 9.5, 4.8 Hz, 1H), -0.14 (tt, *J* = 9.4, 4.7 Hz, 1H), -0.57 (dq, *J* = 9.6, 4.8 Hz, 1H).

### Example 138:

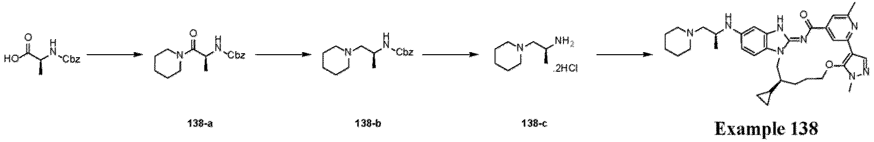

### (1) Preparation method for compound 138-a:

*N*-benzyloxycarbonyl-L-alanine (5.0 g) was dispersed in dichloromethane (75 mL), and piperidine hydrochloride (3.3 g), HATU (12.8 g), and *N,N-*diisopropylethylatnine (8.7 g) were sequentially added. The mixture was reacted at 25 °C for 16 h. After the reaction was completed as detected by TLC, the reaction solution was washed with 1 M NaHCOs (50 mL × 2). The organic phase was retained, dried, concentrated by rotary evaporation to dryness, and subjected to column chromatography to give compound **138-a** (5.4 g).

### (2) Preparation method for compound 138-b:

Compound **138-a** (5.4 g) was dispersed in tetrahydrofuran (50 mL), and the mixture was cooled to 0-5 °C. Borane (1 M in THF, 37 mL) was added dropwise with the temperature kept at this temperature, and the mixture was reacted at 0-5 °C for 2 h. After the starting material was completely consumed as detected by TLC, hydrochloric acid (12 M, 5 mL) was slowly added dropwise to the reaction solution at 0-5 °C. After the dropwise addition, the mixture was heated at 60 °C for 2 h. After the intermediate disappeared as detected by TLC, the reaction solution was cooled to 20-25 °C and poured into water (200 mL), and the resulting mixture was adjusted to pH 9-10 with a saturated sodium bicarbonate solution and subjected to liquid separation. The aqueous phase was extracted with ethyl acetate (50 mL × 2). The organic phases were combined, dried, concentrated by rotary evaporation to dryness, and subjected to column chromatography to give compound **138-b** (2.9 g).

### (3) Preparation method for compound 138-c:

Compound **138-b** (2.9 g) was dispersed in methanol (30 mL), and hydrochloric acid (12 M, 3.0 mL) and Pd/C (10%, 0.3 g) were added. The mixture was purged with hydrogen three times, and heated to 50 °C and reacted for 5 h. After the reaction was completed as detected by TLC, the reaction solution was filtered through celite on a filter paper and concentrated. Ethyl acetate (40 mL) was added, and the resulting mixture was stirred at 25 °C for 1 h and filtered. The filter cake was dried to give **137-c** (1.7 g).

### (4) Preparation method for Example 138:

Compound **O** (100 mg), compound **138-c** (48 mg), Pd₂(dba)₃ (43 mg), 2-(dicyclohexylphosphine)-3,6-dimethoxy-2'-4'-6'-tri-I-propyl-11'-biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 138** (28 mg). ESI-MS: m/z = 597.50 [M+H] ⁺.

¹H NMR (500 MHz, DMSO-*d*₆) δ 12.41 (s, 1H), 8.34 (s, 1H), 7.91 (d, *J* = 2.7 Hz, 1H), 7.54 (s, 1H), 7.35 (d, *J* = 8.8 Hz, 1H), 6.82 (d, *J* = 2.1 Hz, 1H), 6.64 (d, *J* = 8.8 Hz, 1H), 4.33 - 4.26 (m, 1H), 4.22 (dd, *J* = 13.9, 3.5 Hz, 1H), 4.02 (dd, *J* = 14.9, 8.8 Hz, 2H), 3.73 (s, 3H), 3.53 - 3.50 (m, 1H), 2.81 - 2.59 (m, 3H), 2.54 (s, 3H), 2.24 - 2.10 (m, 2H), 1.99 (dq, *J* = 15.2, 6.3, 5.1 Hz, 1H), 1.81 (t, *J* = 8.2 Hz, 1H), 1.69 (q, *J* = 6.6, 5.7 Hz, 2H), 1.61 (s, 4H), 1.44 (d, *J* = 7.5 Hz, 2H), 1.24 (d, *J* = 6.2 Hz, 2H), 1.16 (d, *J* = 6.2 Hz, 3H), 0.58 (tq, *J* = 8.9, 5.1, 4.5 Hz, 1H), 0.30 (tt, *J* = 9.4, 4.8 Hz, 1H), -0.06 (dq, *J* = 9.6, 4.8 Hz, 1H), -0.15 (dq, *J* = 9.0, 4.7 Hz, 1H), -0.59 (dq, *J* = 9.6, 4.9 Hz, 1H).

### Example 139:

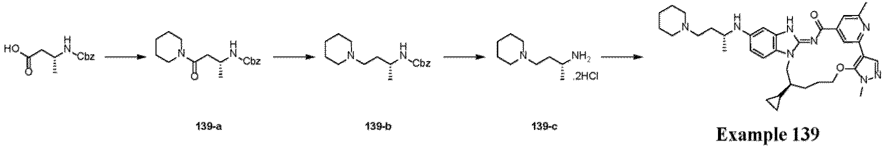

### (1) Preparation method for compound 139-a:

CBZ-R-3-aminobutyric acid (5.0 g) was dispersed in dichloromethane (75 mL), and piperidine hydrochloride (3.1 g), HATU (12.0 g), and *N,N*-diisopropylethylamine (8.2 g) were sequentially added. The mixture was reacted at 25 °C for 16 h. After the reaction was completed as detected by TLC, the reaction solution was washed with 1 M NaHCOs (50 mL × 2). The organic phase was retained, dried, concentrated by rotary evaporation to dryness, and subjected to column chromatography to give compound **139-a** (5.1 g).

### (2) Preparation method for compound 139-b:

Compound **139-a** (5.1 g) was dispersed in tetrahydrofuran (50 mL), and the mixture was cooled to 0-5 °C. Borane (1 M in THF, 34 mL) was added dropwise with the temperature kept at this temperature, and the mixture was reacted at 0-5 °C for 2 h. After the starting material was completely consumed as detected by TLC, hydrochloric acid (12 M, 5 mL) was slowly added dropwise to the reaction solution at 0-5 °C. After the dropwise addition, the mixture was heated at 60 °C for 2 h. After the intermediate disappeared as detected by TLC, the reaction solution was cooled to 20-25 °C and poured into water (200 mL), and the resulting mixture was adjusted to pH 9-10 with a saturated sodium bicarbonate solution and subjected to liquid separation. The aqueous phase was extracted with ethyl acetate (50 mL × 2). The organic phases were combined, dried, concentrated by rotary evaporation to dryness, and subjected to column chromatography to give compound **139-b** (2.5 g).

### (3) Preparation method for compound 139-c:

Compound **139-b** (2.5 g) was dispersed in methanol (30 mL), and hydrochloric acid (12 M, 2.5 mL) and Pd/C (10%, 0.3 g) were added. The mixture was purged with hydrogen three times, and heated to 50 °C and reacted for 5 h. After the reaction was completed as detected by TLC, the reaction solution was filtered through celite on a filter paper and concentrated. Ethyl acetate (40 mL) was added, and the resulting mixture was stirred at 25 °C for 1 h and filtered. The filter cake was dried to give **139-c** (1.5 g).

### (4) Preparation method for Example 139:

Compound **O** (100 mg), compound **139-c** (51 mg), Pd₂(dba)₃ (43 mg), 2-(dicyclohexylphosphine)-3,6-dimethoxy-2'-4'-6'-tri-I-propyl-11'-biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 139** (22 mg). ESI-MS: m/z = 611.55 [M+H] ⁺.

¹H NMR (500 MHz, chloroform-*d*) δ 12.07 (s, 1H), 8.39 (s, 1H), 8.15 (s, 1H), 7.67 (s, 1H), 7.13 (d, *J* = 8.5 Hz, 1H), 6.60 - 6.51 (m, 2H), 4.34 (dt, *J* = 14.0, 4.6 Hz, 2H), 3.94 (tt, *J* = 14.3, 6.4 Hz, 2H), 3.78 (s, 3H), 3.51 (p, *J* = 6.1 Hz, 1H), 2.64 (s, 3H), 2.46 (ddd, *J* = 32.5, 16.3, 9.9 Hz, 6H), 2.27 (q, *J* = 7.1 Hz, 1H), 2.17 (ddd, *J* = 15.7, 11.2, 6.7 Hz, 2H), 1.73 (tq, *J* = 19.8, 6.9, 5.7 Hz, 4H), 1.62 (p, *J* = 5.6 Hz, 4H), 1.48 (s, 2H), 1.21 (d, *J* = 6.2 Hz, 3H), 0.53 (dp, *J* = 12.7, 4.5, 3.8 Hz, 1H), 0.44 (tt, *J* = 9.1, 4.9 Hz, 1H), 0.14 (tt, *J* = 9.3, 5.2 Hz, 1H), -0.03 (t, *J* = 4.7 Hz, 1H), -0.28 (dq, *J* = 10.0, 5.1 Hz, 1H).

### Example 140:

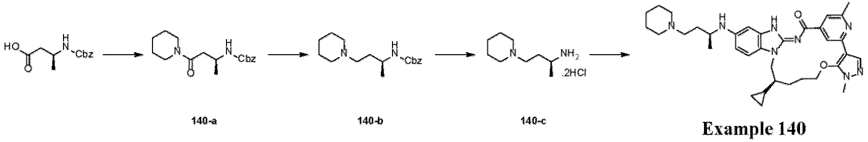

### (1) Preparation method for compound 140-a:

CBZ-S-3-aminobutyric acid (5.0 g) was dispersed in dichloromethane (75 mL), and piperidine hydrochloride (3.1 g), HATU (12.0 g), and *N,N*-diisopropylethylamine (8.2 g) were sequentially added. The mixture was reacted at 25 °C for 16 h. After the reaction was completed as detected by TLC, the reaction solution was washed with 1 M NaHCOs (50 mL × 2). The organic phase was retained, dried, concentrated by rotary evaporation to dryness, and subjected to column chromatography to give compound **140-a** (5.3 g).

### (2) Preparation method for compound 140-b:

Compound **140-a** (5.3 g) was dispersed in tetrahydrofuran (50 mL), and the mixture was cooled to 0-5 °C. Borane (1 M in THF, 35 mL) was added dropwise with the temperature kept at this temperature, and the mixture was reacted at 0-5 °C for 2 h. After the starting material was completely consumed as detected by TLC, hydrochloric acid (12 M, 5 mL) was slowly added dropwise to the reaction solution at 0-5 °C. After the dropwise addition, the mixture was heated at 60 °C for 2 h. After the intermediate disappeared as detected by TLC, the reaction solution was cooled to 20-25 °C and poured into water (200 mL), and the resulting mixture was adjusted to pH 9-10 with a saturated sodium bicarbonate solution and subjected to liquid separation. The aqueous phase was extracted with ethyl acetate (50 mL × 2). The organic phases were combined, dried, concentrated by rotary evaporation to dryness, and subjected to column chromatography to give compound **140-b** (2.8 g).

### (3) Preparation method for compound 140-c:

Compound **140-b** (2.8 g) was dispersed in methanol (30 mL), and hydrochloric acid (12 M, 2.8 mL) and Pd/C (10%, 0.3 g) were added. The mixture was purged with hydrogen three times, and heated to 50 °C and reacted for 5 h. After the reaction was completed as detected by TLC, the reaction solution was filtered through celite on a filter paper and concentrated. Ethyl acetate (40 mL) was added, and the resulting mixture was stirred at 25 °C for 1 h and filtered. The filter cake was dried to give **140-c** (1.7 g).

### (4) Preparation method for Example 140:

Compound **O** (100 mg), compound **140-c** (51 mg), Pd₂(dba)₃ (43 mg), 2-(dicyclohexylphosphine)-3,6-dimethoxy-2'-4'-6'-tri-I-propyl-11'-biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 140** (35 mg). ESI-MS: m/z = 611.53 [M+H] ⁺.

¹H NMR (500 MHz, chloroform-*d*) δ 12.09 (s, 1H), 8.40 (s, 1H), 8.16 (s, 1H), 7.73 (d, *J* = 1.3 Hz, 1H), 7.11 (d, *J* = 8.6 Hz, 1H), 6.61 (dd, *J* = 8.6, 2.1 Hz, 1H), 6.47 (d, *J* = 2.2 Hz, 1H), 4.40 - 4.32 (m, 2H), 3.95 (dt, *J* = 8.9, 5.9 Hz, 1H), 3.90 (dd, *J* = 13.9, 7.5 Hz, 1H), 3.78 (s, 3H), 3.27 (q, *J* = 6.3 Hz, 1H), 3.00 (d, *J* = 9.0 Hz, 1H), 2.80 (s, 4H), 2.68 - 2.65 (m, 1H), 2.64 (s, 3H), 2.27 (dt, *J* = 13.4, 6.8 Hz, 1H), 2.22 - 2.17 (m, 1H), 2.13 (dd, *J* = 12.7, 5.5 Hz, 1H), 1.89 (ddd, *J* = 13.8, 9.1, 4.1 Hz, 2H), 1.84 - 1.75 (m, 6H), 1.57 (s, 2H), 1.18 (d, *J* = 6.2 Hz, 3H), 0.55 -0.47 (m, 1H), 0.42 (tt, *J* = 8.9, 4.8 Hz, 1H), 0.11 (tt, *J* = 9.1, 5.1 Hz, 1H), -0.04 (h, *J* = 4.9 Hz, 1H), -0.32 (dq, *J* = 9.9, 5.1 Hz, 1H).

### Example 141:

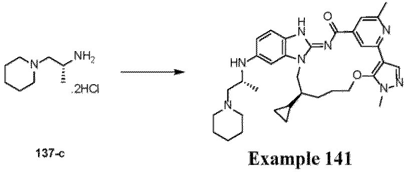

Compound **O-2** (100 mg) in Example 8, compound **137-c** (48 mg), Pd₂(dba)₃ (43 mg), 2-(dicyclohexylphosphine)-3,6-dimethoxy-2'-4'-6'-tri-I-propyl-11'-biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 141** (25 mg). ESI-MS: m/z = 597.50 [M+H] ⁺. ¹H NMR (500 MHz, DMSO-*d*₆) δ 12.38 (s, 1H), 8.34 (s, 1H), 7.91 (s, 1H), 7.54 (d, *J* = 1.2 Hz, 1H), 7.25 (d, *J* = 8.5 Hz, 1H), 6.80 (s, 1H), 6.56 (dd, *J* = 8.6, 2.0 Hz, 1H), 4.29 (td, *J* = 8.6, 6.2 Hz, 1H), 4.23 (dd, *J* = 13.9, 3.6 Hz, 1H), 4.04 (dq, *J* = 8.9, 6.7, 5.9 Hz, 2H), 3.73 (s, 3H), 3.55 - 3.48 (m, 1H), 2.72 - 2.59 (m, 2H), 2.54 (s, 3H), 2.49 - 2.31 (m, 3H), 2.23 (p, *J* = 6.6 Hz, 1H), 2.13 (tt, *J* = 8.7, 4.7 Hz, 1H), 1.99 (dq, *J* = 8.6, 5.5, 4.2 Hz, 1H), 1.86 - 1.79 (m, 1H), 1.76 - 1.68 (m, 1H), 1.54 (s, 4H), 1.41 (s, 2H), 1.24 (d, *J* = 6.0 Hz, 1H), 1.11 (d, *J* = 6.1 Hz, 3H), 0.62 (qd, *J* = 8.1, 6.4, 3.0 Hz, 1H), 0.33 (tt, *J* = 9.3, 4.6 Hz, 1H), -0.04 (dt, *J* = 9.4, 4.7 Hz, 1H), -0.13 (tt, *J* = 9.1, 4.7 Hz, 1H), -0.57 (dq, *J* = 9.8, 4.9 Hz, 1H).

### Example 142:

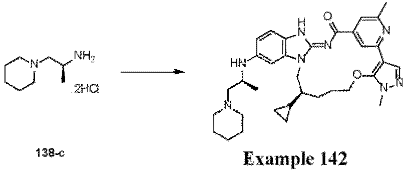

Compound **O-2** (100 mg) in Example 8, compound **138-c** (48 mg), Pd₂(dba)₃ (43 mg), 2-(dicyclohexylphosphine)-3,6-dimethoxy-2'-4'-6'-tri-I-propyl-11'-biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 142** (18 mg). ESI-MS: m/z = 597.51 [M+H] ⁺.

¹H NMR (500 MHz, DMSO-*d*₆) δ 12.38 (s, 1H), 8.34 (s, 1H), 7.91 (s, 1H), 7.54 (d, *J* = 1.2 Hz, 1H), 7.25 (d, *J* = 8.5 Hz, 1H), 6.81 (s, 1H), 6.56 (d, *J* = 8.6 Hz, 1H), 4.29 (td, *J* = 8.6, 6.1 Hz, 1H), 4.22 (dd, *J* = 14.1, 3.6 Hz, 1H), 4.09 - 4.00 (m, 2H), 3.73 (s, 3H), 3.51 (q, *J* = 1.9 Hz, 1H), 2.74 - 2.62 (m, 2H), 2.54 (s, 3H), 2.49 - 2.29 (m, 3H), 2.25 - 2.20 (m, 1H), 2.13 (dq, *J* = 9.4, 4.5 Hz, 1H), 2.00 (td, *J* = 14.5, 13.7, 5.0 Hz, 1H), 1.82 (d, *J* = 10.2 Hz, 1H), 1.75 - 1.68 (m, 1H), 1.53 (s, 4H), 1.38 (dd, J= 14.2, 7.2 Hz, 2H), 1.24 (d, *J* = 9.1 Hz, 1H), 1.16 (d, *J* = 6.3 Hz, 3H), 0.61 (dp, *J* = 8.5, 4.5, 3.6 Hz, 1H), 0.31 (tt, *J* = 9.4, 4.7 Hz, 1H), -0.04 (dt, *J* = 9.5, 4.7 Hz, 1H), -0.12 (d, *J* = 10.6 Hz, 1H), -0.57 (dq, *J* = 9.6, 4.9 Hz, 1H).

### Example 143:

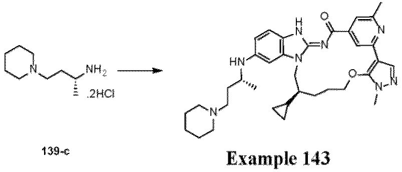

Compound **O-2** (100 mg) in Example 8, compound **139-c** (51 mg), Pd₂(dba)₃ (43 mg), 2-(dicyclohexylphosphine)-3,6-dimethoxy-2'-4'-6'-tri-I-propyl-11'-biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 143** (27 mg). ESI-MS: m/z = 611.52 [M+H] ⁺.

¹H NMR (500 MHz, chloroform-*d*) δ 11.90 (s, 1H), 8.39 (d, *J* = 1.4 Hz, 1H), 8.15 (s, 1H), 7.65 (d, *J* = 1.3 Hz, 1H), 7.11 (d, *J* = 8.5 Hz, 1H), 6.53 (d, *J* = 9.9 Hz, 2H), 4.37 (d, *J* = 14.7 Hz, 2H), 3.94 (tt, *J* = 14.3, 6.4 Hz, 2H), 3.78 (s, 3H), 3.56 (h, *J* = 6.3 Hz, 1H), 2.64 (s, 3H), 2.46 (d, *J* = 32.2 Hz, 4H), 2.29 (q, *J* = 10.4, 8.7 Hz, 1H), 2.20 - 2.13 (m, 2H), 2.05 - 1.98 (m, 1H), 1.78 (d, *J* = 19.1 Hz, 4H), 1.63 (p, *J* = 5.7 Hz, 4H), 1.52 - 1.45 (m, 3H), 1.21 (d, *J* = 6.3 Hz, 3H), 0.56 - 0.49 (m, 1H), 0.45 (tt, *J* = 9.1, 4.9 Hz, 1H), 0.16 (tt, *J* = 9.1, 4.9 Hz, 1H), 0.07 - 0.01 (m, 1H), -0.28 (dq,J = 10.0, 5.1 Hz, 1H).

### Example 144:

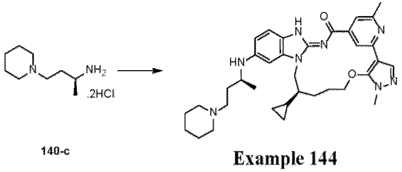

Compound **O-2** (100 mg) in Example 8, compound **140-c** (51 mg), Pd₂(dba)₃ (43 mg), 2-(dicyclohexylphosphine)-3,6-dimethoxy-2'-4'-6'-tri-I-propyl-11'-biphenyl (28 mg), and potassium *tert*-butoxide (126 mg) were dispersed in *tert*-amyl alcohol (3 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and purified by preparative liquid phase chromatography to give **Example 144** (23 mg). ESI-MS: m/z = 611.52 [M+H] ⁺.

¹H NMR (500 MHz, chloroform-*d*) δ 11.92 (s, 1H), 8.39 (d, *J* = 1.4 Hz, 1H), 8.15 (s, 1H), 7.65 (d, *J* = 1.3 Hz, 1H), 7.15 - 7.09 (m, 1H), 6.53 (d, *J* = 7.2 Hz, 2H), 4.35 (tt, *J* = 13.2, 4.6 Hz, 2H), 4.00 - 3.95 (m, 1H), 3.92 (dd, *J* = 13.9, 7.4 Hz, 1H), 3.78 (s, 3H), 3.53 (h, *J* = 6.3 Hz, 1H), 2.63 (s, 3H), 2.61 - 2.52 (m, 4H), 2.50 (s, 2H), 2.29 (q, *J* = 10.4, 8.7 Hz, 1H), 2.21 - 2.14 (m, 2H), 1.86 - 1.74 (m, 4H), 1.66 (p, *J* = 5.7 Hz, 4H), 1.49 (s, 2H), 1.25 (d, *J* = 6.3 Hz, 3H), 0.59 - 0.52 (m, 1H), 0.45 (tt, *J* = 9.4, 4.8 Hz, 1H), 0.17 (tt, *J* = 9.1, 4.9 Hz, 1H), 0.04 - 0.00 (m, 1H), -0.26 (dq, *J* = 9.9, 5.1 Hz, 1H).

### Example 145:

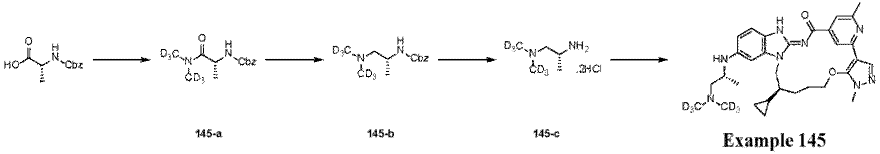

### (1) Preparation method for compound 145-a:

Compound *N*-benzyloxycarbonyl-D-alanine (3.82 g) was dispersed in dichloromethane (50 mL), and dimethyl-d6-amine hydrochloride (1.65 g), TBTU (6.05 g), and *N,N*-diisopropylethylamine (8.5 mL) were sequentially added at 0-5 °C. After the addition, the mixture was reacted at 25 °C for 16 h. After the reaction was completed as detected by TLC, the reaction solution was washed with sodium hydroxide solution (1 M, 100 mL × 2). The organic phase was retained, dried, concentrated by rotary evaporation to dryness, and subjected to column chromatography to give compound **145-a** (3.95 g).

### (2) Preparation method for compound 145-b:

Compound **145-a** (3.95 g) was dispersed in tetrahydrofuran (20 mL), and the mixture was cooled to 0-5 °C. Borane (1 M in THF, 46 mL) was added dropwise with the temperature kept at this temperature, and the mixture was reacted at 0-5 °C for 2 h. After the starting material was completely consumed as detected by TLC, hydrochloric acid (12 M, 3.0 mL) was slowly added dropwise to the reaction solution at 0-5 °C. After the dropwise addition, the mixture was heated at 60 °C for 2 h. After the intermediate disappeared as detected by TLC, the reaction solution was cooled to 20-25 °C and poured into water (50 mL), and the resulting mixture was adjusted to pH 9-10 with a saturated sodium bicarbonate solution and subjected to liquid separation. The aqueous phase was extracted with ethyl acetate (20 mL × 2). The organic phases were combined, dried, concentrated by rotary evaporation to dryness, and subjected to column chromatography to give compound **145-b** (1.65 g).

### (3) Preparation method for compound 145-c:

Compound **145-b** (1.65 g) was dispersed in methanol (40 mL), and hydrochloric acid (12 M, 4.2 mL) and Pd/C (10%, 0.15 g) were added. The mixture was purged with hydrogen three times, and heated to 50 °C and reacted for 5 h. After the reaction was completed as detected by TLC, the reaction solution was filtered through celite on a filter paper. The filtrate was concentrated by rotary evaporation to dryness to give **145-c** (0.86 g) as a colorless oily liquid.

### (4) Preparation method for Example 145:

**145-c** (68 mg) and potassium *tert*-butoxide (126 mg) were dispersed in *tert*-amyl alcohol (5 mL). The mixture was stirred at 20-25 °C for 5 min. Then compound **O-2** (100 mg) in Example 8, Pd₂(dba)₃ (26 mg), and 2-(dicyclohexylphosphine)-3,6-dimethoxy-2'-4'-6'-tri-I-propyl-11'-biphenyl (30 mg) were added, and the mixture was purged with nitrogen 3 times and reacted at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 145** (22 mg). ESI-MS: m/z = 563.55 [M+H] ⁺.

¹H NMR (500 MHz, DMSO-*d*₆) δ 12.42 (s, 1H), 8.34 (s, 1H), 7.91 (s, 1H), 7.54 (d, *J* = 1.2 Hz, 1H), 7.29 (d, *J* = 8.5 Hz, 1H), 6.92 (s, 1H), 6.61 (dd, *J* = 8.6, 2.0 Hz, 1H), 4.30 (td, *J* = 8.6, 6.1 Hz, 1H), 4.24 (dd, *J* = 14.0, 3.5 Hz, 1H), 4.12 - 4.02 (m, 2H), 3.73 (s, 3H), 2.54 (s, 3H), 2.22 (dt, *J* = 13.0, 7.1 Hz, 1H), 2.14 (ddd, *J* = 14.2, 8.7, 4.4 Hz, 1H), 2.00 (ddd, *J* = 14.4, 8.2, 4.1 Hz, 2H), 1.84 (q, *J* = 11.9, 7.7 Hz, 1H), 1.75 - 1.67 (m, 1H), 1.29 (d, *J* = 7.3 Hz, 2H), 1.10 (d, *J* = 6.2 Hz, 3H), 0.63 (qd, *J* = 8.8, 4.0 Hz, 1H), 0.33 (dp, *J* = 9.7, 4.6 Hz, 1H), -0.04 (dt, *J* = 9.7, 4.7 Hz, 1H), -0.16 (tt, *J* = 9.4, 4.7 Hz, 1H), -0.60 (dq, *J* = 9.5, 4.9 Hz, 1H).

### Example 146:

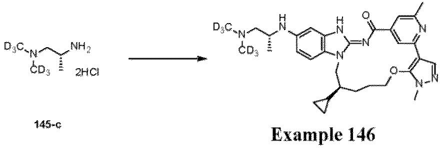

**145-c** (102 mg) and potassium *tert*-butoxide (189 mg) were dispersed in *tert*-amyl alcohol (5 mL). The mixture was stirred at 20-25 °C for 5 min. Then compound **O** (100 mg), Pd₂(dba)₃ (26 mg), and 2-(dicyclohexylphosphine)-3,6-dimethoxy-2'-4'-6'-tri-I-propyl-11'-biphenyl (30 mg) were added, and the mixture was purged with nitrogen 3 times and reacted in an oil bath at 100 °C for 2 h. After the reaction was completed as detected by LC-MS, 30 mL of water was added to the reaction solution, and the resulting mixture was extracted with ethyl acetate (20 mL × 2). The organic phases were combined, dried over anhydrous sodium sulfate, concentrated until no liquid flowed out, and purified by preparative liquid phase chromatography to give **Example 146** (28 mg). ESI-MS: m/z = 563.48 [M+H] ⁺.

¹H NMR (500 MHz, DMSO-*d*₆) δ 12.45 (s, 1H), 8.40 (d, *J* = 11.5 Hz, 1H), 7.96 (s, 1H), 7.62 (d, *J* = 22.0 Hz, 1H), 7.39 (d, *J* = 8.6 Hz, 1H), 6.86 (s, 1H), 6.67 (d, *J* = 8.6 Hz, 1H), 4.40 - 4.31 (m, 1H), 4.28 (d, *J* = 14.4 Hz, 1H), 4.08 (d, *J* = 12.2 Hz, 2H), 3.78 (s, 3H), 2.60 (s, 3H), 2.22 (d, *J* = 35.8 Hz, 2H), 2.05 (d, *J* = 16.1 Hz, 2H), 1.86 (s, 1H), 1.75 (s, 1H), 1.40 (d, *J* = 6.6 Hz, 2H), 1.20 (d, *J* = 6.1 Hz, 3H), 0.64 (qd, *J* = 8.8, 4.0 Hz, 1H), 0.36 (dp, *J* = 9.7, 4.6 Hz, 1H), -0.01 (dt, *J* = 9.7, 4.7 Hz, 1H), -0.09 (tt, *J* = 9.4, 4.7 Hz, 1H), -0.52 (dq, *J* = 9.5, 4.9 Hz, 1H).

### Example 147/148:

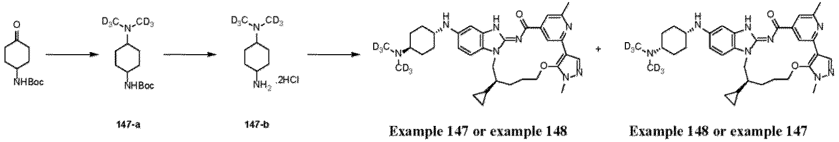

### (1) Preparation method for compound 147-a:

4-*N*-Boc-aminocyclohexanone (1.0 g) was dispersed in methanol (10 mL) and tetrahydrofuran (10 mL), and dimethyl-d6-amine hydrochloride (1.02 g) and triethylamine (1.12 g) were sequentially added at 20-25 °C, followed by sodium cyanoborohydride (750 mg) after 30 min. After the addition, the mixture was reacted at 25 °C for 16 h. After the reaction was completed as detected by TLC, 30 mL of water was added to the reaction solution, and the resulting mixture was extracted with ethyl acetate (20 mL × 2). The organic phases were combined, dried over anhydrous sodium sulfate, and concentrated until no liquid flowed out to give compound **147-a** (1.2 g), which was directly used in the next step without purification.

### (2) Preparation of compound 147-b:

Compound **147-a** (1.2 g) was dispersed in ethyl acetate (10 mL), and a solution of hydrogen chloride in ethyl acetate (2 M, 25 mL) was slowly added dropwise at 20-25 °C. After the addition, the mixture was reacted at 25 °C for 16 h. After the reaction was completed as detected by TLC, the reaction solution was concentrated. Ethyl acetate (20 mL) was added, and the resulting mixture was stirred at 25 °C for 1 h and filtered. The filter cake was dried to give compound **147-b** (0.9 g), which was directly used in the next step without purification.

### (3) Preparation method for Examples 147 and 148:

Compound O (200 mg), compound 147-b (100 mg), Pd₂(dba)₃ (53 mg), 2-di-*tert*-butylphosphino-2',4',6'-triisopropylbiphenyl (48 mg), and potassium *tert*-butoxide (250 mg) were dispersed in *tert*-amyl alcohol (5 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, concentrated until no liquid flowed out, and subjected to preparative chromatography (liquid phase chromatography conditions: column: Novasep C18 250 ×50 mm, 10 µm; mobile phase: A: 0.05% formic acid-water, C: acetonitrile, gradient: 10% C-30% C (0-60 min); flow rate: 50 mL/min; wavelength: 254 nm) to give **Example 147** (22 mg, retention time: 38.5 min), ESI-MS: m/z = 602.62 [M+H] ⁺, and **Example 148** (25 mg, retention time: 47.5 min.), ESI-MS: m/z = 602.63 [M+H] ⁺. Example 147: ¹H NMR (500 MHz, DMSO-*d*₆) δ 12.39 (s, 1H), 8.40 (s, 1H), 7.97 (s, 1H), 7.61 (s, 1H), 7.37 (d, *J* = 8.6 Hz, 1H), 6.87 - 6.79 (m, 1H), 6.64 (d, *J* = 8.6 Hz, 1H), 4.35 (d, *J* = 7.9 Hz, 1H), 4.29 - 4.25 (m, 1H), 4.08 (d, *J* = 9.7 Hz, 2H), 3.79 (s, 3H), 3.20 - 3.13 (m, 1H), 2.71 - 2.64 (m, 1H), 2.60 (s, 3H), 2.31 - 2.23 (m, 1H), 2.21 (s, 1H), 2.15 (d, *J* = 10.9 Hz, 2H), 2.06 (d, *J* = 6.9 Hz, 1H), 2.01 (d, *J* = 12.8 Hz, 2H), 1.86 (s, 1H), 1.75 (h, *J* = 6.8 Hz, 1H), 1.48 (q, *J* = 12.4 Hz, 2H), 1.26 (dd, *J* = 22.7, 11.6 Hz, 2H), 0.69 - 0.60 (m, 1H), 0.37 (tt, *J* = 9.2, 4.6 Hz, 1H), 0.00 (dq, *J* = 9.7, 4.9 Hz, 1H), -0.09 (dp, *J* = 9.9, 5.1 Hz, 1H), -0.52 (dq, *J* = 9.8, 4.8 Hz, 1H). Example 148: ¹H NMR (500 MHz, DMSO-*d*₆) δ 12.40 (s, 1H), 8.40 (s, 1H), 7.97 (s, 1H), 7.60 (s, 1H), 7.38 (d, *J* = 8.7 Hz, 1H), 6.85 (d, *J* = 2.2 Hz, 1H), 6.71 (dd, *J* = 8.7, 2.2 Hz, 1H), 4.35 (d, *J* = 8.0 Hz, 1H), 4.29 - 4.25 (m, 1H), 4.08 (td, *J* = 8.8, 8.3, 4.5 Hz, 2H), 3.79 (s, 3H), 3.48 - 3.46 (m, 1H), 2.60 (s, 3H), 2.41 (d, *J* = 9.7 Hz, 1H), 2.27 (dt, *J* = 12.8, 6.5 Hz, 1H), 2.19 (dq, *J* = 13.3, 6.6, 5.7 Hz, 1H), 2.05 (dd, *J* = 11.5, 6.6 Hz, 1H), 1.90 - 1.83 (m, 3H), 1.82 - 1.73 (m, 3H), 1.65 (tt, *J* = 12.2, 5.3 Hz, 4H), 0.64 (qt, *J* = 8.8, 5.0 Hz, 1H), 0.37 (dp, *J* = 9.2, 4.6 Hz, 1H), 0.01 (dt, *J* = 9.6, 5.0 Hz, 1H), -0.08 (dp, *J* = 9.5, 4.7 Hz, 1H), -0.51 (dt, *J* = 9.6, 4.9 Hz, 1H).

### Example 149:

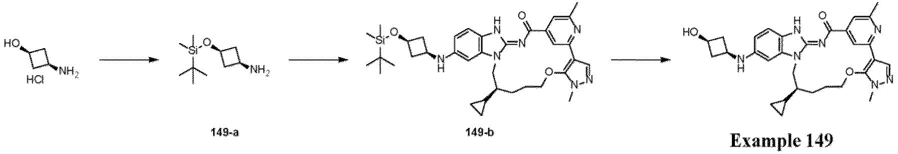

### (1) Preparation method for compound 149-a:

*Cis*-3-aminocyclobutanol hydrochloride (200 mg) was dispersed in dichloromethane (5 mL), and imidazole (320 mg) and *tert*-butyldimethylsilyl chloride (320 mg) were sequentially added at 20-25 °C. After the addition, the reaction was reacted at 25 °C for 16 h. After the reaction was completed as detected by TLC, the reaction solution was washed with a 1 M aqueous sodium bicarbonate solution (5 mL × 2). The organic phase was retained, dried over anhydrous sodium sulfate, and concentrated until no liquid flowed out to give compound **149-a** (220 mg), which was directly used in the next step without purification.

### (2) Preparation method for compound 149-b:

Compound **O-2** (100 mg) in Example 8, compound **149-a** (53 mg), Pd₂(dba)₃ (26 mg), 2-(dicyclohexylphosphine)-3,6-dimethoxy-2'-4'-6'-tri-I-propyl-11'-biphenyl (26 mg), and potassium *tert*-butoxide (126 mg) were dispersed in *tert*-amyl alcohol (5 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, and concentrated until no liquid flowed out to give compound **149-b** (80 mg).

### (3) Preparation method for Example 149:

Compound **149-b** (80 mg) was dispersed in a solution of tetrabutylammonium fluoride in tetrahydrofuran (1 M, 5 mL). The mixture was reacted at 25 °C for 2 h. After the reaction was completed as detected by TLC, the reaction solution was concentrated until no liquid flowed out and purified by preparative liquid phase chromatography to give **Example 149** (10 mg). ESI-MS: m/z = 542.43 [M+H] ⁺.

¹H NMR (500 MHz, DMSO-*d*₆) δ 12.42 (s, 1H), 8.38 (s, 1H), 8.32 (s, 1H), 7.95 (d, *J* = 1.2 Hz, 1H), 7.58 (d, *J* = 8.7 Hz, 1H), 7.32 (d, *J* = 8.5 Hz, 1H), 6.77 (s, 1H), 6.61 (d, *J* = 8.7 Hz, 1H), 520 (s, 1H), 4.35 - 4.27 (m, 2H), 4.08 (d, *J* = 5.7 Hz, 2H), 3.81 (d, *J* = 9.8 Hz, 1H), 3.77 (s, 3H), 3.01 (p, *J* = 6.6 Hz, 1H), 2.58 (s, 3H), 2.28 (ddd, *J* = 13.0, 7.5, 5.7 Hz, 3H), 2.20 (dq, *J* = 8.8, 4.3, 3.4 Hz, 1H), 2.10 - 1.98 (m, 3H), 1.84 (q, *J* = 8.0, 7.6 Hz, 1H), 1.80 - 1.73 (m, 1H), 0.64 (dq, *J* = 17.8, 8.2, 6.6 Hz, 1H), 0.37 (tt, *J* = 9.3, 4.5 Hz, 1H), 0.00 (dq, *J* = 9.8, 5.0 Hz, 1H), -0.07 (dq, *J* = 9.1, 4.4 Hz, 1H), -0.50 (dt, *J* = 9.8, 4.8 Hz, 1H).

### Example 150:

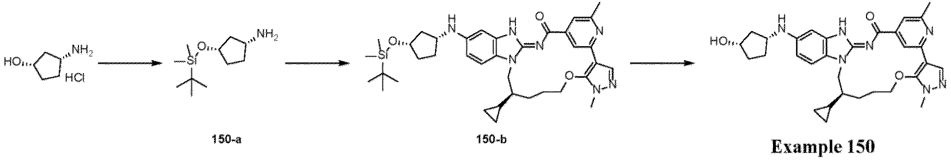

### (1) Preparation method for compound 150-a:

(1*S*,3*R*)-3-aminocyclopentanol hydrochloride (180 mg) was dispersed in dichloromethane (5 mL), and imidazole (300 mg) and *tert*-butyldimethylsilyl chloride (300 mg) were sequentially added at 20-25 °C. After the addition, the reaction was reacted at 25 °C for 16 h. After the reaction was completed as detected by TLC, the reaction solution was washed with a 1 M aqueous sodium bicarbonate solution (5 mL × 2). The organic phase was retained, dried over anhydrous sodium sulfate, and concentrated until no liquid flowed out to give compound **150-a** (200 mg), which was directly used in the next step without purification.

### (2) Preparation method for compound 150-b:

Compound **O** (100 mg), compound 150-a (53 mg), Pd₂(dba)₃ (26 mg), 2-(dicyclohexylphosphine)-3,6-dimethoxy-2'-4'-6'-tri-I-propyl-11'-biphenyl (26 mg), and potassium *tert*-butoxide (126 mg) were dispersed in *tert*-amyl alcohol (5 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, and concentrated until no liquid flowed out to give compound **150-b** (90 mg).

### (3) Preparation method for Example 150:

Compound **150-b** (90 mg) was dispersed in a solution of tetrabutylammonium fluoride in tetrahydrofuran (1 M, 5 mL). The mixture was reacted at 25 °C for 2 h. After the reaction was completed as detected by TLC, the reaction solution was concentrated until no liquid flowed out and purified by preparative liquid phase chromatography to give **Example 150** (10 mg). ESI-MS: m/z = 556.41 [M+H] ⁺.

¹H NMR (500 MHz, DMSO-*d*₆) δ 12.33 (s, 1H), 8.39 (s, 1H), 7.96 (s, 1H), 7.60 (s, 1H), 7.37 (d, *J* = 8.6 Hz, 1H), 6.79 (s, 1H), 6.62 (d, *J* = 8.7 Hz, 1H), 4.67 (s, 1H), 4.34 (d, *J* = 8.0 Hz, 1H), 4.27 (d, *J* = 13.6 Hz, 1H), 4.18 (s, 1H), 4.11 - 4.04 (m, 2H), 3.78 (s, 3H), 3.71 - 3.64 (m, 1H), 2.60 (s, 3H), 2.29 (ddd, *J* = 24.9, 15.6, 6.9 Hz, 2H), 2.19 (s, 1H), 2.02 (s, 2H), 1.86 (s, 1H), 1.81 - 1.72 (m, 2H), 1.66 (d, *J* = 7.6 Hz, 2H), 1.45 (dd, *J* = 12.5, 6.0 Hz, 1H), 0.64 (s, 1H), 0.36 (d, *J* = 8.9 Hz, 1H), 0.03 - -0.03 (m, 1H), -0.09 (s, 1H), -0.49 - -0.57 (m, 1H).

### Example 151:

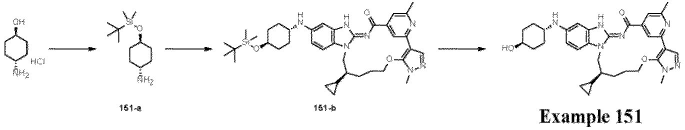

### (1) Preparation method for compound 151-a:

*Trans*-4-aminocyclohexanol hydrochloride (180 mg) was dispersed in dichloromethane (5 mL), and imidazole (260 mg) and *tert*-butyldimethylsilyl chloride (260 mg) were sequentially added at 20-25 °C. After the addition, the reaction was reacted at 25 °C for 16 h. After the reaction was completed as detected by TLC, the reaction solution was washed with a 1 M aqueous sodium bicarbonate solution (5 mL × 2). The organic phase was retained, dried over anhydrous sodium sulfate, and concentrated until no liquid flowed out to give compound **151-a** (208 mg), which was directly used in the next step without purification.

### (2) Preparation method for compound 151-b:

Compound **O** (100 mg), compound 151-a (53 mg), Pd₂(dba)₃ (26 mg), 2-(dicyclohexylphosphine)-3,6-dimethoxy-2'-4'-6'-tri-I-propyl-11'-biphenyl (26 mg), and potassium *tert*-butoxide (126 mg) were dispersed in *tert*-amyl alcohol (5 mL). The mixture was purged with nitrogen 3 times and reacted at 100 °C for 2 h. After the reaction was completed, a 1 M aqueous sodium bicarbonate solution (20 mL) was added to the reaction solution, and the resulting mixture was extracted with ethyl acetate (10 mL × 3). The organic phases were combined, dried over anhydrous sodium sulfate, and concentrated until no liquid flowed out to give compound **151-b** (80 mg).

### (3) Preparation method for Example 151:

Compound **151-b** (80 mg) was dispersed in a solution of tetrabutylammonium fluoride in tetrahydrofuran (1 M, 5 mL). The mixture was reacted at 25 °C for 2 h. After the reaction was completed as detected by TLC, the reaction solution was concentrated until no liquid flowed out and purified by preparative liquid phase chromatography to give **Example 151** (15 mg). ESI-MS: m/z = 570.45 [M+H] ⁺.

¹H NMR (500 MHz, chloroform-d) δ 11.98 (s, 1H), 8.56 (s, 1H), 8.40 (s, 1H), 8.15 (s, 1H), 7.64 (s, 1H), 7.18 (d, J = 8.6 Hz, 1H), 6.78 (dd, J = 8.7, 2.1 Hz, 1H), 6.60 (d, J = 2.1 Hz, 1H), 4.79 (s, 1H), 4.39 - 4.32 (m, 2H), 4.14 (s, 1H), 4.07 - 4.00 (m, 2H), 3.94 (dq, J = 13.9, 7.4, 6.2 Hz, 2H), 3.77 (s, 3H), 3.69 - 3.64 (m, 1H), 2.83 (d, J = 11.1 Hz, 1H), 2.61 (s, 3H), 2.31 - 2.25 (m, 1H), 2.22 - 2.15 (m, 2H), 2.12 - 2.06 (m, 2H), 1.86 (s, 1H), 1.82 - 1.73 (m, 2H), 0.51 (d, J = 12.0 Hz, 1H), 0.44 (dq, J = 8.9, 4.5, 4.1 Hz, 1H), 0.17 - 0.09 (m, 1H), -0.01 (q, J = 4.8 Hz, 1H), -0.41 (dd, J = 9.8, 5.3 Hz, 1H).

### Example 152:

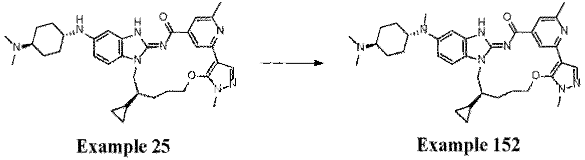

Compound **Example 25** (100 mg) was dispersed in methanol (3 mL) and acetic acid (0.3 mL), and polyoxymethylene (20 mg) was added at 20-25 °C. Sodium cyanoborohydride (32 mg) was added after 1 h, and after the addition the mixture was reacted at 20-25 °C for 12 h. After the reaction was completed as detected by LC-MS, the reaction solution was concentrated until no liquid flowed out and purified by preparative liquid phase chromatography to give **Example 152** (50 mg). ESI-MS: m/z = 611.53 [M+H] ⁺.

¹H NMR (500 MHz, DMSO-*d*₆) δ 12.41 (s, 1H), 8.40 (s, 1H), 8.32 (s, 1H), 7.97 (s, 1H), 7.61 (s, 1H), 7.48 (d, *J* = 8.9 Hz, 1H), 7.06 (d, *J* = 2.3 Hz, 1H), 6.89 (dd, *J* = 9.1, 2.4 Hz, 1H), 4.36 - 4.33 (m, 1H), 4.29 (d, *J* = 10.5 Hz, 1H), 4.11 - 4.08 (m, 2H), 3.78 (s, 3H), 3.61 (ddt, *J* = 11.5, 8.0, 3.6 Hz, 1H), 2.78 (s, 3H), 2.71 - 2.65 (m, 1H), 2.60 (s, 3H), 2.56 (q, *J* = 2.0 Hz, 3H), 2.47 (s, 3H), 2.25 (s, 1H), 2.19 (td, *J* = 9.7, 4.7 Hz, 1H), 2.08 - 2.00 (m, 3H), 1.90 - 1.80 (m, 3H), 1.78 -1.71 (m, 1H), 1.63 (q, *J* = 12.3 Hz, 2H), 1.52 (qd, *J* = 11.9, 3.1 Hz, 2H), 0.64 (tt, *J* = 8.5, 3.5 Hz, 1H), 0.35 (tt, *J* = 9.2, 4.7 Hz, 1H), -0.00 (dq, *J* = 9.7, 4.9 Hz, 1H), -0.12 (dq, *J* = 8.9, 4.6 Hz, 1H), -0.55 (dq, *J* = 9.6, 4.9 Hz, 1H).

### Experimental Example 1: In Vitro Kinase Activity

### 1.1 Screening for inhibitory activity for EGFR (WT) kinase

A 50 ng/µL EGFR (WT, Carna) stock solution was diluted with a kinase buffer (50 mM HEPES, 10 mM MgCl₂, 2 mM DTT, 1 mM EGTA, 0.01% Tween 20), and 6 µL of 1.67× working solution at 0.005 ng/µL (final concentration: 0.003 ng/µL) was added to each well. Different compounds dissolved in DMSO were added to the wells using a nanoliter pipettor, resulting in the final concentrations of the compounds of 100 nM to 0.0244 nM (4-fold gradient for 7 concentrations in total), and blank control wells (without enzyme) and negative control wells (with enzyme, plus vehicle DMSO) were set, 2 replicate wells for each concentration. After enzyme was reacted with the compound or vehicle for 30 min, 5× ATP at 25 µM (final concentration: 5 µM, Sigma) prepared with a kinase buffer was mixed with 5× substrate at 0.5 µM (final concentration: 0.1 µM, ULight-poly GT, PerkinElmer) at a ratio of 1:1 (v:v), and the mixture was added to each well at 4 µL/well. After being sealed with a film, the plate was incubated at room temperature for 2 h, and then 5 µL of 4× EDTA at 40 mM (final concentration: 10 mM) was added to each well. After the plate was incubated for 5 min at room temperature, 5 µL of 4× detection reagent at 8 nM (final concentration: 2 nM, Eu-anti-phospho-tyrosine antibody, PerkinElmer) was added to each well. After being incubated at room temperature for 1 h, the plate was read using a PerkinElmer Envision multi-mode microplate reader (excitation: 320 nm, emission: 665 nm), and IC₅₀ was calculated by four-parameter fitting.

### 1.2 Screening for inhibitory activity for EGFR (L858R/T790M) kinase

A 50 ng/µL EGFR (L858R/T790M, Carna) stock solution was diluted with a kinase buffer (50 mM HEPES, 10 mM MgCl₂, 2 mM DTT, 1 mM EGTA, 0.01% Tween 20), and 6 µL of 1.67× working solution at 0.004175 ng/µL (final concentration: 0.0025 ng/µL) was added to each well. Different compounds dissolved in DMSO were added to wells using a nanoliter pipettor, resulting in the final concentrations of the compounds of 10 nM to 0.0024 nM (4-fold gradient, 7 concentrations in total), and blank control wells (without enzyme) and negative control wells (with enzyme, plus vehicle DMSO) were set, 2 replicate wells for each concentration. After enzyme was reacted with the compound or vehicle for 30 min, 5× ATP at 25 µM (final concentration: 5 µM, Sigma) prepared with a kinase buffer was mixed with 5× substrate at 0.5 µM (final concentration: 0.1 µM, ULight-poly GT, PerkinElmer) at a ratio of 1:1 (v:v), and the mixture was added to each well at 4 µL/well. After being sealed with a film, the plate was incubated at room temperature for 2 h, and then 5 µL of 4× EDTA at 40 mM (final concentration: 10 mM) was added to each well. After the plate was incubated for 5 min at room temperature, 5 µL of 4× detection reagent at 8 nM (final concentration: 2 nM, Eu-anti-phospho-tyrosine antibody, PerkinElmer) was added to each well. After being incubated at room temperature for 1 h, the plate was read using a PerkinElmer Envision multi-mode microplate reader (excitation: 320 nm, emission: 665 nm), and IC₅₀ was calculated by four-parameter fitting.

### 1.3 Screening for inhibitory activity for EGFR (d746-750 (d19)/T790M) kinase

A 50 ng/µL EGFR (d746-750/T790M, Carna) stock solution was diluted with a kinase buffer (50 mM HEPES, 10 mM MgCl₂, 2 mM DTT, 1 mM EGTA, 0.01% Tween 20), and 6 µL of 1.67× working solution at 0.005 ng/µL (final concentration: 0.003 ng/µL) was added to each well. Different compounds dissolved in DMSO were added to the wells using a nanoliter pipettor, resulting in the final concentrations of the compounds of 10 nM to 0.0024 nM (4-fold gradient for 7 concentrations in total), and blank control wells (without enzyme) and negative control wells (with enzyme, plus vehicle DMSO) were set, 2 replicate wells for each concentration. After enzyme was reacted with the compound or vehicle for 30 min, 5× ATP at 25 µM (final concentration: 5 µM, Sigma) prepared with a kinase buffer was mixed with 5× substrate at 0.5 µM (final concentration: 0.1 µM, ULight-poly GT, PerkinElmer) at a ratio of 1:1 (v:v), and the mixture was added to each well at 4 µL/well. After being sealed with a film, the plate was incubated at room temperature for 2 h, and then 5 µL of 4× EDTA at 40 mM (final concentration: 10 mM) was added to each well. After the plate was incubated for 5 min at room temperature, 5 µL of 4× detection reagent at 8 nM (final concentration: 2 nM, Eu-anti-phospho-tyrosine antibody, PerkinElmer) was added to each well. After being incubated at room temperature for 1 h, the plate was read using a PerkinElmer Envision multi-mode microplate reader (excitation: 320 nm, emission: 665 nm), and IC₅₀ was calculated by four-parameter fitting.

### 1.4 Screening for inhibitory activity for EGFR (L858R/T790M/C797S) kinase

A 50 ng/µL EGFR (L858R/T790M/C797S, BPS) stock solution was diluted with a kinase buffer (50 mM HEPES, 10 mM MgCl₂, 2 mM DTT, 1 mM EGTA, 0.01% Tween 20), and 6 µL of 1.67× working solution at 0.00167 ng/µL (final concentration: 0.001 ng/µL) was added to each well. Different compounds dissolved in DMSO were added to wells using a nanoliter pipettor, resulting in the final concentrations of the compounds of 10 nM to 0.0024 nM (4-fold gradient, 7 concentrations in total), and blank control wells (without enzyme) and negative control wells (with enzyme, plus vehicle DMSO) were set, 2 replicate wells for each concentration. After enzyme was reacted with the compound or vehicle for 30 min, 5× ATP at 25 µM (final concentration: 5 µM, Sigma) prepared with a kinase buffer was mixed with 5× substrate at 0.5 µM (final concentration: 0.1 µM, ULight-poly GT, PerkinElmer) at a ratio of 1:1 (v:v), and the mixture was added to each well at 4 µL/well. After being sealed with a film, the plate was incubated at room temperature for 2 h, and then 5 µL of 4× EDTA at 40 mM (final concentration: 10 mM) was added to each well. After the plate was incubated for 5 min at room temperature, 5 µL of 4× detection reagent at 8 nM (final concentration: 2 nM, Eu-anti-phospho-tyrosine antibody, PerkinElmer) was added to each well. After being incubated at room temperature for 1 h, the plate was read using a PerkinElmer Envision multi-mode microplate reader (excitation: 320 nm, emission: 665 nm), and IC₅₀ was calculated by four-parameter fitting.

### 1.5 Screening for inhibitory activity for EGFR (d746-750/T790M/C797S) kinase

A 50 ng/µL EGFR (d746-750/T790M/C797S, BPS) stock solution was diluted with a kinase buffer (50 mM HEPES, 10 mM MgCl₂, 2 mM DTT, 1 mM EGTA, 0.01% Tween 20), and 6 µL of 1.67× working solution at 0.05 ng/µL (final concentration: 0.03 ng/µL) was added to each well. Different compounds dissolved in DMSO were added to the wells using a nanoliter pipettor, resulting in the final concentrations of the compounds of 10 nM to 0.0024 nM (4-fold gradient for 7 concentrations in total), and blank control wells (without enzyme) and negative control wells (with enzyme, plus vehicle DMSO) were set, 2 replicate wells for each concentration. After enzyme was reacted with the compound or vehicle for 30 min, 5× ATP at 25 µM (final concentration: 5 µM, Sigma) prepared with a kinase buffer was mixed with 5× substrate at 0.5 µM (final concentration: 0.1 µM, ULight-poly GT, PerkinElmer) at a ratio of 1:1 (v:v), and the mixture was added to each well at 4 µL/well. After being sealed with a film, the plate was incubated at room temperature for 2 h, and then 5 µL of 4× EDTA at 40 mM (final concentration: 10 mM) was added to each well. After the plate was incubated for 5 min at room temperature, 5 µL of 4× detection reagent at 8 nM (final concentration: 2 nM, Eu-anti-phospho-tyrosine antibody, PerkinElmer) was added to each well. After being incubated at room temperature for 1 h, the plate was read using a PerkinElmer Envision multi-mode microplate reader (excitation: 320 nm, emission: 665 nm), and IC₅₀ was calculated by four-parameter fitting. The results are shown in Table 1.

### 1.6 Screening for inhibitory activity for EGFR (L858R) and EGFR (d19) kinases

A 50 ng/µL EGFR (L858R, BPS) or EGFR (d19, BPS) stock solution was diluted with a kinase buffer (50 mM HEPES, 10 mM MgCl₂, 2 mM DTT, 1 mM EGTA, 0.01% Tween 20), and 6 µL of 1.67× working solution at 0.05 ng/µL (final concentration: 0.03 ng/µL) was added to each well. Different compounds dissolved in DMSO were added to the wells using a nanoliter pipettor, resulting in the final concentrations of the compounds of 10 nM to 0.0024 nM (4-fold gradient for 7 concentrations in total), and blank control wells (without enzyme) and negative control wells (with enzyme, plus vehicle DMSO) were set, 2 replicate wells for each concentration. After enzyme was reacted with the compound or vehicle for 30 min, 5× ATP at 25 µM (final concentration: 5 µM or 1 mM, Sigma) prepared with a kinase buffer was mixed with 5× substrate at 0.5 µM (final concentration: 0.1 µM, ULight-poly GT, PerkinElmer) at a ratio of 1:1 (v:v), and the mixture was added to each well at 4 µL/well. After being sealed with a film, the plate was incubated at room temperature for 2 h, and then 5 µL of 4× EDTA at 40 mM (final concentration: 10 mM) was added to each well. After the plate was incubated for 5 min at room temperature, 5 µL of 4× detection reagent at 8 nM (final concentration: 2 nM, Eu-anti-phospho-tyrosine antibody, PerkinElmer) was added to each well. After being incubated at room temperature for 1 h, the plate was read using a PerkinElmer Envision multi-mode microplate reader (excitation: 320 nm, emission: 665 nm), and IC₅₀ was calculated by four-parameter fitting.

**Table 1. Results for in vitro kinase activity**

| **Compound No.** | EGFR(WT) | EGFR(L858R/T 790M) | EGFR(d19/T790 M) | EGFR(L858R/T7 90M/C797S) | EGFR(d19/T790 M/C797S) |
|---|---|---|---|---|---|
| | (IC₅₀, nM) | | | | |
| | | (IC₅₀, nM) | (IC₅₀, nM) | (IC₅₀, nM) | (IC₅₀, nM) |
| Example 1 | 0.933 | 0.022 | 0.011 | 0.018 | 0.022 |
| Example 3 | 0.864 | 0.027 | 0.014 | 0.022 | 0.028 |
| Example 4 | 2.8 | 0.068 | 0.037 | 0.081 | 0.062 |
| Example 5 | 3.1 | 0.091 | 0.05 | 0.076 | 0.074 |
| Example 8 | 2.5 | 0.029 | 0.016 | 0.031 | 0.020 |
| Example 9 | 0.56 | 0.45 | 0.28 | 0.50 | 0.45 |
| Example 10 | 0.12 | 0.020 | 0.014 | 0.019 | 0.023 |
| Example 11 | 1.9 | 0.044 | 0.025 | 0.055 | 0.024 |
| Example 12 | 2.6 | 0.19 | 0.15 | 0.20 | 0.17 |
| Example 13 | 2.0 | 0.088 | 0.058 | 0.095 | 0.11 |
| Example 14 | 4.0 | 0.11 | 0.068 | 0.10 | 0.10 |
| Example 15 | 0.59 | 0.027 | 0.019 | 0.026 | 0.019 |
| Example 16 | 3.4 | 0.12 | 0.060 | 0.12 | 0.063 |
| Example 17 | 0.7 | 0.011 | 0.012 | 0.0079 | 0.014 |
| Example 18 | 1.7 | 0.040 | 0.020 | 0.051 | 0.042 |
| Example 19 | 1.8 | 0.028 | 0.015 | 0.035 | 0.028 |
| Example 20 | 0.20 | 0.016 | 0.012 | 0.020 | 0.020 |
| Example 21 | 2.4 | 0.060 | 0.032 | 0.075 | 0.059 |
| Example 22 | 2.0 | 0.040 | 0.018 | 0.052 | 0.030 |
| Example 23 | 2.2 | 0.029 | 0.014 | 0.020 | 0.019 |
| Example 24 | 1.1 | 0.031 | 0.016 | 0.020 | 0.021 |
| Example 25 | 0.049 | 0.021 | 0.011 | 0.014 | 0.014 |
| Example 26 | 0.24 | 0.025 | 0.011 | 0.017 | 0.017 |
| Example 27 | 0.78 | 0.030 | 0.019 | 0.033 | 0.027 |
| Example 28 | 0.45 | 0.011 | 0.0072 | 0.011 | 0.011 |
| Example 29 | 1.4 | 0.019 | 0.0089 | 0.021 | 0.015 |
| Example 30 | 2.8 | 0.13 | 0.030 | 0.12 | 0.046 |
| Example 31 | 2.2 | 0.038 | 0.018 | 0.042 | 0.031 |
| Example 32 | 0.34 | 0.018 | 0.010 | 0.029 | 0.013 |
| Example 33 | 0.47 | 0.015 | 0.0098 | 0.012 | 0.015 |
| Example 34 | 0.40 | 0.028 | 0.014 | 0.028 | 0.016 |
| Example 35 | 0.55 | 0.030 | 0.020 | 0.035 | 0.023 |
| Example 36 | 1.5 | 0.033 | 0.015 | 0.035 | 0.022 |
| Example 37 | 1.4 | 0.027 | 0.013 | 0.037 | 0.022 |
| Example 38 | 2.1 | 0.066 | 0.014 | 0.062 | 0.026 |
| Example 39 | 0.61 | 0.040 | 0.012 | 0.027 | 0.024 |
| Example 40 | 1.6 | 0.027 | 0.014 | 0.030 | 0.021 |
| Example 41 | 1.1 | 0.018 | 0.0089 | 0.021 | 0.016 |
| Example 42 | 0.68 | 0.013 | 0.0076 | 0.011 | 0.012 |
| Example 43 | 1.2 | 0.026 | 0.015 | 0.039 | 0.026 |
| Example 44 | 0.36 | 0.010 | 0.0081 | 0.0073 | 0.012 |
| Example 45 | 0.95 | 0.020 | 0.013 | 0.024 | 0.018 |
| Example 46 | 1.6 | 0.017 | 0.013 | 0.021 | 0.020 |
| Example 47 | 0.55 | 0.023 | 0.017 | 0.028 | 0.027 |
| Example 48 | 0.51 | 0.023 | 0.017 | 0.028 | 0.027 |
| Example 49 | 2.2 | 0.048 | 0.030 | 0.051 | 0.039 |
| Example 50 | 1.3 | 0.054 | 0.038 | 0.066 | 0.053 |
| Example 51 | 1.8 | 0.017 | 0.013 | 0.021 | 0.020 |
| Example 52 | 0.57 | 0.026 | 0.021 | 0.035 | 0.028 |
| Example 53 | 0.72 | 0.011 | 0.0098 | 0.014 | 0.013 |
| Example 54 | 0.39 | 0.017 | 0.013 | 0.025 | 0.021 |
| Example 55 | 0.50 | 0.017 | 0.014 | 0.022 | 0.020 |
| Example 56 | 0.64 | 0.016 | 0.014 | 0.022 | 0.021 |
| Example 57 | 0.64 | 0.011 | 0.0092 | 0.015 | 0.016 |
| Example 58 | 0.51 | 0.012 | 0.0094 | 0.015 | 0.015 |
| Example 59 | 5.9 | 0.081 | 0.040 | 0.085 | 0.074 |
| Example 60 | 8.0 | 0.090 | 0.039 | 0.081 | 0.070 |
| Example 61 | >10 | 0.51 | 0.047 | 0.28 | 0.056 |
| Example 62 | 0.77 | 0.031 | 0.013 | 0.016 | 0.022 |
| Example 63 | 0.33 | 0.009 | 0.008 | 0.0082 | 0.013 |
| Example 67 | | 0.14 | | 0.49 | 0.083 |
| Example 70 | 0.99 | 0.015 | 0.013 | 0.029 | 0.040 |
| Example 72 | 0.93 | 0.038 | 0.011 | 0.021 | 0.013 |
| Example 79 | 6.2 | 0.0070 | 0.028 | 0.123 | 0.153 |
| Example 80 | 0.56 | 0.031 | 0.010 | 0.019 | 0.035 |
| Example 81 | 0.68 | 0.044 | 0.011 | 0.027 | 0.037 |
| Example 82 | 0.58 | 0.020 | 0.0085 | 0.014 | 0.020 |
| Example 83 | 0.79 | 0.040 | 0.013 | 0.023 | 0.017 |
| Example 84 | 1.2 | 0.10 | 0.018 | 0.043 | 0.042 |
| Example 87 | 13 | 0.042 | 0.058 | 0.226 | 0.318 |
| Example 88 | 6.0 | 0.010 | 0.044 | 0.177 | 0.181 |
| Example 89 | | 0.80 | 0.51 | 0.60 | 0.36 |
| Example 93 | | 0.61 | 0.11 | 0.5 | |
| Example 94 | | | 0.75 | | |
| Example 98 | | 0.26 | 0.087 | 0.28 | |
| Example 99 | | 0.21 | 0.091 | 0.21 | |
| Example 100 | | 0.23 | 0.092 | 0.24 | |
| Example 111 | | 0.26 | | | 0.073 |
| Example 112 | | 0.14 | | 0.12 | 0.063 |
| Example 113 | | 0.15 | | 0.12 | 0.066 |
| Example 117 | | 0.77 | 0.12 | 0.45 | |
| Example 123 | | 0.16 | | 0.10 | 0.10 |
| Example 124 | | 0.22 | | 0.16 | 0.12 |
| Example 125 | | 0.17 | | 0.094 | 0.080 |
| Example 126 | | 0.12 | | 0.076 | 0.073 |
| Example 127 | | 0.41 | 0.12 | 0.35 | |
| Example 128 | | 0.63 | 0.13 | 0.46 | |
| Example 129 | | 0.30 | | 0.39 | 0.065 |
| Example 130 | | 0.30 | | 0.36 | 0.056 |
| Example 131 | | 0.094 | | 0.078 | 0.080 |
| Example 132 | | 0.082 | | 0.063 | 0.066 |
| Example 133 | | 0.49 | | 0.66 | 0.097 |
| Example 134 | | 0.55 | | 0.48 | 0.069 |
| Example 135 | | 0.32 | | 0.43 | 0.062 |
| Example 136 | | 0.71 | 0.15 | 0.34 | |
| Example 137 | | 0.13 | | 0.52 | 0.085 |
| Example 138 | | 0.13 | | 0.35 | 0.087 |
| Example 139 | | 0.087 | | 0.21 | 0.076 |
| Example 140 | | 0.11 | | 0.30 | 0.077 |
| Example 141 | | 0.084 | | 0.24 | 0.072 |
| Example 142 | | 0.14 | | 0.35 | 0.079 |
| Example 143 | | 0.11 | | 0.40 | 0.079 |
| Example 144 | | 0.080 | | 0.17 | 0.068 |
| Example 146 | | 0.52 | | 0.75 | 0.074 |
| Example 147 | | 0.081 | | 0.12 | 0.076 |
| Example 148 | | 0.090 | | 0.22 | 0.079 |
| Example 151 | | 0.23 | | 0.25 | 0.12 |

### Experimental Example 2: Inhibitory Activity for Cell Proliferation

### 2.1 Inhibitory effect of compounds on proliferation of BAF3-EGFR (del19/T790M) cells

A flask of BAF3-EGFR (del19/T790M) cells in the logarithmic growth phase and good conditions was taken, and the cells were collected and added to a centrifuge tube. 20 µL of cells were taken and counted, and the required number of cells (mL) was pipetted and centrifuged at 1200 rpm for 3 min. The supernatant was discarded, and an appropriate amount of plating medium (RPMI medium + 10% FBS) was added to adjust the cell density to 1.0 × 10⁵ cells/mL. The cells were seeded on a 96-well plate at 100 µL/well using a multi-channel pipette and cultured in a cell incubator at 37 °C, 5% CO₂ with saturated humidity. After overnight incubation, compounds were loaded using a nanoliter pipettor, 2 duplicate wells were set for each concentration, and cells without compound added were used as negative controls. After 72 h, CCK-8 was added at 10 µL/well for incubation for 2 h, then absorbance was measured at 450 nm with an Envision plate reader, and inhibition rate was calculated.

### 2.2 Inhibitory effect of compounds on proliferation of BAF3-EGFR (L858R/T790M/C797S) cells

A flask of EGFR (L858R/T790M/C797S) cells in the logarithmic growth phase and good conditions was taken, and the cells were collected and added to a centrifuge tube. 20 µL of cells were taken and counted, and the required number of cells (mL) was pipetted and centrifuged at 1200 rpm for 3 min. The supernatant was discarded, and an appropriate amount of plating medium (RPMI medium + 10% FBS) was added to adjust the cell density to 1.5 × 10⁵ cells/mL. The cells were seeded on a 96-well plate at 100 µL/well using a multi-channel pipette and cultured in a cell incubator at 37 °C, 5% CO₂ with saturated humidity. After overnight incubation, compounds were loaded using a nanoliter pipettor, 2 duplicate wells were set for each concentration, and cells without compound added were used as negative controls. After 72 h, CCK-8 was added at 10 µL/well for incubation for 2 h, then absorbance was measured at 450 nm with an Envision plate reader, and inhibition rate was calculated.

### 2.3 Inhibitory effect of compounds on proliferation of BAF3-EGFR (del19/T790M/C797S) cells

A flask of BAF3-EGFR (del19/T790M/C797S) cells in the logarithmic growth phase and good conditions was taken, and the cells were collected and added to a centrifuge tube. 20 µL of cells were taken and counted, and the required number of cells (mL) was pipetted and centrifuged at 1200 rpm for 3 min. The supernatant was discarded, and an appropriate amount of plating medium (RPMI medium + 10% FBS) was added to adjust the cell density to 1.0 × 10⁵ cells/mL. The cells were seeded on a 96-well plate at 100 µL/well using a multi-channel pipette and cultured in a cell incubator at 37 °C, 5% CO₂ with saturated humidity. After overnight incubation, compounds were loaded using a nanoliter pipettor, 2 duplicate wells were set for each concentration, and cells without compound added were used as negative controls. After 72 h, CCK-8 was added at 10 µL/well for incubation for 2 h, then absorbance was measured at 450 nm with an Envision plate reader, and inhibition rate was calculated.

### 2.4 Inhibitory effect of compounds on proliferation of PC-9-EGFR (Del19 T790M C797S) cells

PC-9 EGFR-Del19-T790M-C797S cells in the logarithmic growth phase and good conditions were taken, washed with PBS, digested with pancreatin, terminated with a complete medium, collected and added to a centrifuge tube, adjusted to a cell density of 2 × 10⁴ cells/mL, and seeded in a 96-well plate (100 µL/well). Meanwhile, compounds were loaded using a nanoliter pipettor resulting in the final concentrations of the compounds of 1000 nM-0.06 nM, and a control was set, replicate wells for each concentration. After 72 h of incubation in the cell incubator, the assay reagent CCK-8 (manufacturer: Dojindo Laboratories, Beijing; 10 µL/well) was added. After 2 h of incubation in the cell incubator, absorbance was measured at 450 nm on a PerkinElmer Envision microplate reader, and the inhibition rate was calculated.

Inhibition rate (%) = (mean value of negative control group - mean value of experimental group) / (mean value of negative control group - mean value of blank group) × 100%. A dose-response curve was fitted by four-parameter analysis, with the logarithm of compound concentration serving as abscissa and inhibition rate serving as ordinate, such that IC₅₀ was calculated. The results for the cell inhibitory activity of related compounds are shown in Table 2.

**Table 2 Results for cell inhibitory activity (IC₅₀, nM)**

| **Compound No.** | BAF3(EGFR T790M L858R C797S) | BAF3(EGFR T790M Del19 C797S) | PC-9-EGFR (Del19 T790M C797S) |
|---|---|---|---|
| Example 8 | <5 | <1 | |
| Example 10 | <5 | | <50 |
| Example 11 | <5 | <1 | |
| Example 15 | <5 | <1 | |
| Example 17 | <5 | <1 | |
| Example 22 | <5 | <1 | |
| Example 24 | <5 | | |
| Example 25 | <5 | <1 | <50 |
| Example 26 | <5 | <1 | <50 |
| Example 27 | <5 | <1 | |
| Example 28 | <5 | <1 | <50 |
| Example 29 | <5 | <1 | <50 |
| Example 35 | <5 | <1 | |
| Example 36 | <5 | <1 | |
| Example 37 | <5 | <1 | <50 |
| Example 38 | <5 | <1 | <50 |
| Example 39 | <5 | <1 | <50 |
| Example 40 | <5 | <1 | <50 |
| Example 41 | <5 | <1 | <50 |
| Example 42 | <5 | <1 | |
| Example 44 | <5 | <1 | <50 |
| Example 45 | <5 | <1 | <50 |
| Example 46 | <5 | <1 | |
| Example 47 | <5 | <1 | <50 |
| Example 48 | <5 | <1 | <50 |
| Example 51 | <5 | | |
| Example 52 | <5 | | |
| Example 53 | <5 | | |
| Example 54 | <5 | <1 | <50 |
| Example 55 | <5 | <1 | <50 |
| Example 57 | <5 | <1 | |
| Example 58 | <5 | <1 | |
| Example 62 | <5 | <1 | |
| Example 63 | <5 | <1 | <50 |
| Example 65 | <5 | <1 | |
| Example 66 | <5 | <1 | <50 |
| Example 67 | <5 | | |
| Example 70 | <5 | | |
| Example 72 | <5 | <1 | |
| Example 73 | <5 | | |
| Example 74 | <5 | <1 | <50 |
| Example 78 | <5 | | |
| Example 80 | <5 | <1 | <50 |
| Example 81 | <5 | <1 | <50 |
| Example 82 | <5 | <1 | <50 |
| Example 83 | <5 | <1 | |
| Example 85 | | | <50 |
| Example 88 | <5 | | |
| Example 89 | <5 | <1 | <50 |
| Example 90 | <5 | <1 | |
| Example 92 | <5 | <1 | |
| Example 98 | <5 | | <50 |
| Example 99 | <5 | | <50 |
| Example 100 | <5 | | <50 |
| Example 111 | <5 | | |
| Example 112 | <5 | | <50 |
| Example 116 | | | <50 |
| Example 117 | | | <50 |
| Example 123 | <5 | | <50 |
| Example 126 | <5 | | <50 |
| Example 127 | | | <50 |
| Example 128 | | | <50 |
| Example 130 | <5 | | |
| Example 131 | <5 | | <50 |
| Example 132 | <5 | | <50 |
| Example 133 | <5 | | |
| Example 134 | <5 | | |
| Example 135 | <5 | | <50 |
| Example 136 | | | <50 |
| Example 137 | <5 | | |
| Example 138 | <5 | | |
| Example 139 | <5 | | <50 |
| Example 140 | <5 | | |
| Example 141 | <5 | | <50 |
| Example 142 | <5 | | |
| Example 143 | <5 | | <50 |
| Example 144 | <5 | | <50 |
| Example 145 | <5 | | |
| Example 146 | <5 | | <50 |
| Example 147 | <5 | | <50 |
| Example 148 | <5 | | <50 |
| Example 150 | <5 | | |
| Example 151 | <5 | | <50 |

### Experimental Example 3: Evaluation on In Vitro Stability in Liver Microsomes

300 µL of the final incubation system contains 30 µL of liver microsomes (protein concentration: 0.15 mg/mL), 30 µL of NADPH + MgCl₂, 3 µL of the test compound (in acetonitrile), and 237 µL of PBS buffer (pH 7.4). The proportion of the organic solvent (acetonitrile) was 1%. Samples were prepared in duplicate of 0.3 mL for each species (e.g., human or mouse liver microsomes). Tubes each containing 270 µL of a mixed solution of substrate and enzyme and NADPH were pre-incubated at 37 °C for 5 min. 30 µL of NADPH + MgCl₂ was added and the mixture was mixed. 50 µL of the mixture was taken at 0 min, 15 min, 30 min, and 60 min, and 300 µL of glacial acetonitrile containing an internal standard was added to terminate the reaction. 50 µL of the incubated sample was added with 300 µL of glacial acetonitrile containing an internal standard (20 ng/mL diazepam) for precipitation. The mixture was vortexed for 5 min, and centrifuged (12,000 rpm, 4 °C) for 10 min. 75 µL of supernatant was taken and diluted with 75 µL of ultrapure water. After being homogeneously mixed, 0.5 µL of the resulting sample was injected for analysis. The parameters for the elimination of related compounds in human liver microsomes are shown in Table 3.

**Table 3. Data for stability in liver microsomes (residual%, T = 60 min)**

| **Compound No.** | **Human liver microsome** | **Compound No.** | **Human liver microsome** |
|---|---|---|---|
| Example 9 | 59.54% | Example 99 | 84.54% |
| Example 10 | 94.30% | Example 100 | 82.55% |
| Example 15 | 86.29% | Example 112 | 71.24% |
| Example 20 | 73.66% | Example 124 | 77.10% |
| Example 25 | 103.07% | Example 125 | 93.95% |
| Example 26 | 98.30% | Example 126 | 83.66% |
| Example 33 | 83.39% | Example 127 | 75.00% |
| Example 35 | 85.02% | Example 129 | 92.24% |
| Example 44 | 80.24% | Example 130 | 56.81% |
| Example 47 | 96.11% | Example 131 | 95.64% |
| Example 48 | 95.68% | Example 132 | 63.76% |
| Example 68 | 78.57% | Example 133 | 95.34% |
| Example 70 | 81.11% | Example 134 | 65.19% |
| Example 71 | 82.82% | Example 135 | 97.30% |
| Example 72 | 64.73% | | |

### Experimental Example 4: Evaluation on In Vivo Pharmacokinetics

ICR mice weighing 18-20 g were randomly grouped with 9 mice in each group after 3-5 days of acclimatization, and then intragastrically given the corresponding compound at a dose of 10 mg/kg.

The animals to be tested (ICR mice) were fasted for 12 h before administration and food was provided 4 h after administration, and water was freely drunk before and after the experiment and during the experiment.

After intragastric administration, about 0.1 mL of blood was collected from the orbit at 0 min, 15 min, 30 min, 1 h, 2 h, 4 h, 6 h, 8 h, 10 h, and 24 h. After anticoagulation with EDTA-K2, blood samples were transferred to a centrifuge within 30 min, and centrifuged at 4000 rpm for 10 min at 4 °C to separate the plasma. All the plasma samples were collected and immediately stored at -20 °C for testing.

300 µL of acetonitrile solution containing an internal standard (20 mg/mL diazepam) was added to 20 µL of the plasma sample to be tested and a standard curve sample. The mixture was shaken and homogeneously mixed for 5 min, and centrifuged at 12,000 rpm for 10 min. 70 µL of supernatant was taken and diluted with 70 µL of ultrapure water. After being mixed well, 2 µL of the resulting sample was subjected to LC/MS/MS, and a chromatogram was recorded. The test results are shown in Table 4.

**Table 4. Data for in vivo pharmacokinetics**

| Example | Route of administration | AUC(0-t) (h*ng/ml) | AUC(0-∞) (h*ng/ml) | MRT(0-t) (h) | T1/2 (h) | Tmax (h) | Cmax (ng/ml) | CLz (L/h/kg) | F (%) |
|---|---|---|---|---|---|---|---|---|---|
| 28 | PO | 938 | 965 | 7.72 | 4.29 | 6.00 | 74.5 | -- | 27.91% |
| | IV | 336 | 341 | 3.96 | 4.64 | -- | -- | 2.93 | |
| 29 | PO | 3625 | 3636 | 5.99 | 3.06 | 0.50 | 553 | -- | 42.88% |
| | IV | 845 | 850 | 1.10 | 1.65 | -**-** | **--** | 1.18 | |
| 39 | PO | 3093 | 3096 | 3.84 | 233 | 0.50 | 488 | -- | 118.35% |
| | IV | 261 | 262 | 0.61 | 1.74 | -**-** | **--** | 3.81 | |
| 66 | PO | 2040 | 2202 | 826 | 5.98 | 600 | 143 | -**-** | -- |
| 68 | PO | 1948 | 1961 | 7.54 | 304 | 4.00 | 148 | -**-** | |
| 71 | PO | 1623 | 1681 | 7.85 | 450 | 600 | 138 | -**-** | -- |
| 74 | PO | 824 | 830 | 6.42 | 3.18 | 0.50 | 97.6 | -**-** | 35.36% |
| | IV | 233 | 236 | 1.35 | 1.84 | -**-** | -**-** | 4.23 | |
| 90 | PO | 3012 | 3018 | 6.47 | 2.46 | 0.50 | 352 | -**-** | 68.38% |
| | IV | 441 | 441 | 1.65 | 1.58 | -**-** | -**-** | 2.27 | |
| 117 | PO | 826 | 848 | 6.79 | 4.17 | 6.00 | 77.1 | -**-** | 29.09% |
| | IV | 284 | 286 | 330 | 2.18 | -**-** | -**-** | 3.49 | |
| 128 | PO | 654 | 662 | 6.81 | 3.46 | 6.00 | 57.8 | **-**- | 30.51% |
| | IV | 214 | 221 | 1.65 | 2.17 | -**-** | -**-** | 4.52 | |

Experiments showed that the compounds of the present application had good pharmacokinetic properties *in vivo,* relatively high oral exposure, exposure concentration, and bioavailability, and relatively long half-life.

### Experimental Example 5: Evaluation on In Vivo Pharmacodynamics

Evaluation on drug effect on nude mouse subcutaneous xenograft tumor model of PC9-EGFR (del19-T790M-C797S) tumor mass: PC9-EGFR (del19-T790M-C797S) cells were inoculated subcutaneously in the right axilla of SPF-grade female nude mice (source: Laboratory Animal Operation Department, Shanghai Institute for Biomedical and Pharmaceutical Technologies) at 1 × 10⁷ cells/mouse for the F1 generation. The nude mouse tumor tissue of the F1 generation was taken out, and the necrotic tissue was removed. Then the rest of the tumor tissue was cut into tumor masses of about 1.5-2 mm and inoculated subcutaneously in the right axilla of the nude mice. When the mean tumor volume reached about 200 mm³, the animals were grouped.

Evaluation on drug effect on nude mouse subcutaneous xenograft tumor model of NCI-H1975 cells: NCI-H1975 cells were inoculated subcutaneously in the right axilla of SPF-grade female nude mice (source: Jiangsu Huachuang Sino Pharmaceutical Technology Co., Ltd.) at 1 × 10⁶ cells/mouse. When the mean tumor volume reached about 200 mm³, the animals were grouped.

The day of grouping was defined as day 0, and intragastric administration was started on day 0 and performed once a day. The tumor volume was measured 2-3 times a week, meanwhile, the mice were weighed and the data were recorded; the general behavior of the mice was observed and recorded every day. After the experiment was completed, the tumors were removed, weighed, and photographed.

The detection index and the calculation formula are as follows:
Tumor volume TV (mm³) = 1/2 × (a × b²), wherein a represents the long diameter of the tumor, and b represents the short diameter of the tumor;
Relative tumor volume RTV = TVₜ/TV₀, wherein TV₀ represents the tumor volume on day 0, and TVₜ represents the tumor volume at each measurement;
Relative tumor proliferation rate T/C (%) = (T_{RTV}/C_{RTV}) × 100%, wherein T_{RTV} represents RTV of the treatment group, and C_{RTV} represents RTV of the vehicle control group;
Tumor growth inhibition rate TGI (%) = (1 - TW/TW₀) × 100%; wherein TW represents the tumor weight of the treatment group, and TWo represents the tumor weight of the vehicle control group;
Weight change rate WCR (%) = (Wtₜ - Wt₀)/Wt₀ × 100%, wherein Wto represents the body weight of the mouse on day 0, and Wtt represents the body weight of the mouse at each measurement.

The test results are shown in Tables 5-7.

**Table 5**

| Model | NCI-H1975(L858R T790M) | | | |
|---|---|---|---|---|
| Days of administration | 17 days | | | |
| Compound No. | Example 29 | | Example 25 | |
| Dose of administration | 5mpk (i.g) | 10mpk (i.g) | 5mpk (i.g) | 10mpk (i.g) |
| Tumor growth inhibition% | 40.0% (volume, d15) | 75.0% (volume, d15) | 22.5% (volume, d15) | 72.5% (volume, d15) |
| | 41.7% (tumor weight, d18) | 83.9% (tumor weight, d18) | 31.4% (tumor weight, d18) | 75.8% (tumor weight, d18) |

**Table 6**

| Model | NCI-H1975(L858R T790M) | | | | |
|---|---|---|---|---|---|
| Days of administration | 14 days | | | | |
| Compound No. | Example 38 | Example 39 | Example 45 | Example 66 | |
| Dose of administration | 10mpk (i.g) | 10mpk (i.g) | 10mpk (i.g) | 5mpk (i.g) | 10mpk (i.g) |
| Tumor growth inhibition% | 76.6% (volume, d12) | 70.2% (volume, d12) | 76.6% (volume, d12) | 71.3% (volume, d12) | 86.2% (volume, d12) |
| | 77.3% (tumor weight, d14) | 65.1% (tumor weight, d14) | 76.2% (tumor weight, d14) | 73.5% (tumor weight, d14) | 87.5% (tumor weight, d14) |

**Table 7**

| Model | PC9-EGFR (del19-T790M -C797S) | |
|---|---|---|
| Days of administration | 29 days | 4 days |
| Compound No. | Example 66 | |
| Dose of administration | 15mpk*4+10mpk*25 (i.g) | 30mpk*4 (i.g) |
| Tumor growth inhibition% | 94.3% (volume, d27) | 60.0% (volume, d27) |
| | 95.8% (tumor weight, d29) | 56.5% (tumor weight, d29) |

Test results showed that the compounds of the present application had a good *in vivo* drug effect, and specifically, they could effectively reduce the volume and the weight of tumors *in vivo.*

## Claims

1. A compound of formula I", a stereoisomer thereof or a pharmaceutically acceptable salt thereof, 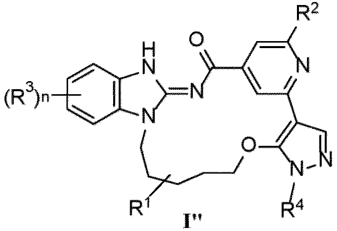 wherein,
R¹ is selected from the group consisting of C₃₋₅ cycloalkyl, C₃₋₅ cycloalkyl-C₁₋₃ alkyl-, C₁₋₆ haloalkyl, and C₁₋₆ alkyl;
R² is selected from the group consisting of hydrogen, halogen, OH, NH₂, C₁₋₈ alkyl, and C₁₋₈ alkoxy, wherein the C₁₋₈ alkyl or C₁₋₈ alkoxy is optionally substituted with one or more R';
R' is selected from the group consisting of halogen, OH, cyano, NH₂, and nitro;
R³ is selected from the group consisting of halogen, cyano, nitro, NH₂, OH, C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, 5- to 10-membered heteroaryl, -NHR^{b}, -NR^{a}R^{b}, -OR^{a}, - SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, -N(C₁₋₄ alkyl)C(O)OR^{a}, and - NHC(O)NHR^{a}, wherein the C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, or 5- to 10-membered heteroaryl is optionally substituted with one or more R^{c};
R^{c} is selected from the group consisting of oxo, OH, NH₂, halogen, cyano, nitro, C₁₋₈ alkyl, C₁₋₈ alkoxy, -N(C₁₋₈ alkyl)₂, -NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, - NHS(O)₂R^{a}, -C₁₋₆ alkyl -S(O)₂-C₁₋₆ alkyl, and 3- to 8-membered heterocycloalkyl optionally substituted with one or more groups independently selected from the group consisting of OH, halogen, and C₁₋₆ alkyl;
R^{a} and R^{b} are each independently selected from the group consisting of the following groups optionally substituted with one or more R": C₁₋₈ alkyl, C₂₋₈ alkenyl, C₂₋₈ alkynyl, C₃₋₁₀ cycloalkyl, 3- to 10-membered heterocycloalkyl, C₆₋₁₀ aryl, and 5- to 10-membered heteroaryl;
R" are each independently selected from the group consisting of halogen, NH₂, OH, oxo, deuterium, cyano, nitro, C₁₋₈ alkyl, C₁₋₈ alkoxy, C₁₋₈ haloalkyl, -N(C₁₋₈ alkyl)₂, -NH(C₁₋₈ alkyl), C₃₋₆ cycloalkyl, and 3- to 10-membered heterocycloalkyl optionally substituted with C₁₋₈ alkyl;
n is selected from the group consisting of 0, 1, 2, 3, and 4;
R⁴ is selected from the group consisting of C₁₋₆ alkyl and C₃₋₅ cycloalkyl,
provided that when R¹ is selected from C₁₋₆ alkyl, R⁴ is selected from C₃₋₅ cycloalkyl.

2. The compound of formula I", the stereoisomer thereof or the pharmaceutically acceptable salt thereof according to claim 1, being selected from a compound of formula I', a stereoisomer thereof or a pharmaceutically acceptable salt thereof, 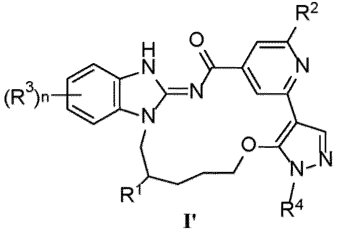 wherein,
R¹ is selected from the group consisting of C₃₋₅ cycloalkyl, C₃₋₅ cycloalkyl-C₁₋₃ alkyl-, C₁₋₆ haloalkyl, and C₁₋₆ alkyl;
R² is selected from the group consisting of hydrogen, C₁₋₈ alkyl, and C₁₋₈ alkoxy, wherein the C₁₋₈ alkyl or C₁₋₈ alkoxy is optionally substituted with one or more R';
R' is selected from the group consisting of halogen, hydroxy, cyano, amino, and nitro;
R³ is selected from the group consisting of halogen, cyano, nitro, NH₂, OH, C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, 5- to 10-membered heteroaryl, -NHRb, -NR^{a}R^{b}, -OR^{a}, - SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, and -N(C₁₋₄ alkyl)C(O)OR^{a}, wherein the C₁₋₈ alkyl, C₂₋₈ alkenyl, C₂₋₈ alkynyl, C₃₋₁₀ cycloalkyl, 3- to 10-membered heterocyclyl, C₆₋₁₀ aryl, or 5- to 10-membered heteroaryl is optionally substituted with one or more R^{c};
R^{c} is selected from the group consisting of oxo, halogen, cyano, nitro, C₁₋₈ alkoxy, -N(C₁₋₈ alkyl)₂, -NH(C₁₋₈ alkyl), - C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, and 3- to 8-membered heterocycloalkyl optionally substituted with one or more groups independently selected from the group consisting of hydroxy, halogen, and C₁₋₆ alkyl;
R^{a} and R^{b} are each independently selected from the group consisting of the following groups optionally substituted with one or more R": C₁₋₈ alkyl, C₂₋₈ alkenyl, C₂₋₈ alkynyl, C₃₋₁₀ cycloalkyl, 3- to 10-membered heterocycloalkyl, C₆₋₁₀ aryl, and 5- to 10-membered heteroaryl;
R" are each independently selected from the group consisting of halogen, NH₂, OH, cyano, nitro, C₁₋₈ alkyl, C₁₋₈ alkoxy, -N(C₁₋₈ alkyl)₂, -NH(C₁₋₈ alkyl), C₃₋₆ cycloalkyl, and 3- to 10-membered heterocycloalkyl;
n is selected from the group consisting of 0, 1, 2, 3, and 4;
R⁴ is selected from the group consisting of C₁₋₆ alkyl and C₃₋₅ cycloalkyl,
provided that when R¹ is selected from C₁₋₆ alkyl, R⁴ is selected from C₃₋₅ cycloalkyl.

3. The compound, the stereoisomer thereof or the pharmaceutically acceptable salt thereof according to any one of claims 1-2, wherein R¹ is selected from the group consisting of C₃₋₄ cycloalkyl, C₃₋₄ cycloalkyl-C₁₋₂ alkyl-, C₁₋₃ haloalkyl, and C₁₋₃ alkyl;
optionally, R¹ is selected from the group consisting of cyclopropyl, cyclopropyl-CH₂-, halomethyl, and methyl;
optionally, R¹ is selected from the group consisting of cyclopropyl, cyclopropyl-CH₂-, methyl substituted with one or more fluoro, and methyl.

4. The compound, the stereoisomer thereof or the pharmaceutically acceptable salt thereof according to any one of claims 1-3, wherein R² is selected from the group consisting of hydrogen, halogen, OH, NH₂, C₁₋₆ alkyl, and C₁₋₆ alkoxy, wherein the C₁₋₆ alkyl or C₁₋₆ alkoxy is optionally substituted with one or more R';
optionally, R² is selected from the group consisting of hydrogen, halogen, OH, NH₂, C₁₋₃ alkyl, and C₁₋₃ alkoxy,
wherein the C₁₋₃ alkyl or C₁₋₃ alkoxy is optionally substituted with one or more R';
optionally, R² is selected from the group consisting of hydrogen, fluoro, chloro, bromo, C₁₋₃ alkyl, and C₁₋₃ alkoxy;
optionally, R² is selected from the group consisting of fluoro, chloro, bromo, and C₁₋₃ alkyl;
optionally, R² is selected from the group consisting of fluoro and methyl;
optionally, R² is selected from the group consisting of hydrogen, C₁₋₆ alkyl, and C₁₋₆ alkoxy, wherein the C₁₋₆ alkyl or C₁₋₆ alkoxy is optionally substituted with one or more R';
optionally, R² is selected from the group consisting of hydrogen, C₁₋₃ alkyl, and C₁₋₃ alkoxy, wherein the C₁₋₃ alkyl or C₁₋₃ alkoxy is optionally substituted with one or more R';
optionally, R² is selected from the group consisting of hydrogen, C₁₋₃ alkyl, and C₁₋₃ alkoxy;
optionally, R² is selected from C₁₋₃ alkyl;
optionally, R² is selected from methyl.

5. The compound, the stereoisomer thereof or the pharmaceutically acceptable salt thereof according to any one of claims 1-4, wherein R' is selected from halogen.

6. The compound, the stereoisomer thereof or the pharmaceutically acceptable salt thereof according to any one of claims 1-5, R³ is selected from the group consisting of halogen, cyano, nitro, NH₂, OH, C₁₋₆ alkyl, C₂₋₆ alkenyl, C₂₋₆ alkynyl, C₃₋₈ cycloalkyl, 3- to 8-membered heterocyclyl, C₆₋₁₀ aryl, 5- to 6-membered heteroaryl, -NHR^{b}, -NR^{a}R^{b}, - OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, -N(C₁₋₄ alkyl)C(O)OR^{a}, and -NHC(O)NHR^{a}, wherein the C₁₋₆ alkyl, C₂₋₆ alkenyl, C₂₋₆ alkynyl, C₃₋₈ cycloalkyl, 3- to 8-membered heterocyclyl, C₆₋₁₀ aryl, or 5- to 6-membered heteroaryl is optionally substituted with one or more R^{c};
optionally, R³ is selected from the group consisting of halogen, cyano, nitro, NH₂, OH, C₁₋₄ alkyl, C₂₋₄ alkenyl, C₂₋₄ alkynyl, C₃₋₆ cycloalkyl, 3- to 6-membered heterocycloalkyl, phenyl, 5- to 6-membered heteroaryl, -NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, -N(C₁₋₄ alkyl)C(O)OR^{a}, and -NHC(O)NHR^{a}, wherein the C₁₋₄ alkyl, C₂₋₄ alkenyl, C₂₋₄ alkynyl, C₃₋₆ cycloalkyl, 3- to 6-membered heterocycloalkyl, phenyl, or 5- to 6-membered heteroaryl is optionally substituted with one or more R^{c};
optionally, R³ is selected from the group consisting of halogen, cyano, NH₂, OH, C₁₋₆ alkyl, C₂₋₆ alkenyl, C₂₋₆ alkynyl, C₃₋₈ cycloalkyl, 3- to 8-membered heterocyclyl, -NHR^{b}, -NR^{a}R^{b}, -OR^{a}, -SR^{a}, -OC(O)R^{a}, -NHC(O)R^{a}, -N(C₁₋₄ alkyl)C(O)R^{a}, -NHS(O)₂R^{a}, -NHC(O)OR^{a}, -N(C₁₋₄ alkyl)C(O)OR^{a}, and -NHC(O)NHR^{a}, wherein the C₁₋₆ alkyl, C₂₋₆ alkenyl, C₂₋₆ alkynyl, C₃₋₈ cycloalkyl, or 3- to 8-membered heterocyclyl is optionally substituted with one or more R^{c};
optionally, R³ is selected from the group consisting of fluoro, chloro, bromo, C₁₋₄ alkyl, C₂₋₄ alkenyl, 4- to 6-membered heterocyclyl, -NHR^{b}, -NR^{a}R^{b}, -OR^{a}, -SR^{a}, -OC(O)R^{a}, -NHC(O)R^{a}, -NHC(O)OR^{a}, -N(C₁₋₄ alkyl)C(O)OR^{a}, and -NHC(O)NHR^{a}; wherein the C₁₋₄ alkyl, C₂₋₄ alkenyl, or 4- to 6-membered heterocyclyl is optionally substituted with one or more R^{c};
optionally, R³ is selected from the group consisting of fluoro, chloro, bromo, C₁₋₃ alkyl, C₂₋₄ alkenyl, 4- to 5-membered azacycloalkyl, -NHR^{b}, -NR^{a}R^{b}, -OR^{a}, -SR^{a}, -OC(O)R^{a}, -NHC(O)R^{a}, -NHC(O)OR^{a}, -N(C₁₋₄ alkyl)C(O)OR^{a}, and -NHC(O)NHR^{a}; the C₁₋₃ alkyl, C₂₋₄ alkenyl, or 4- to 5-membered azacycloalkyl is optionally substituted with one or more R^{c};
optionally, R³ is selected from the group consisting of bromo, methyl, butenyl, azetidinyl, pyrrolidinyl, -NHR^{b}, - NR^{a}R^{b}, -OR^{a}, -NHC(O)R^{a}, and -NHC(O)NHR^{a}; the methyl, butenyl, azetidinyl, or pyrrolidinyl is optionally substituted with one or more R^{c};
optionally, R³ is selected from the group consisting of bromo, 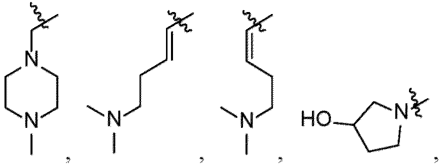 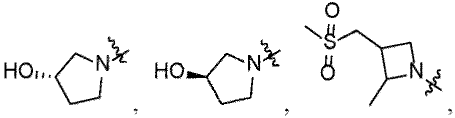 -NHR^{b}, -NR^{a}R^{b}, -OR^{a}, -NHC(O)R^{a}, and -NHC(O)NHR^{a};
optionally, R³ is selected from the group consisting of -NHRb, -NR^{a}R^{b}, -OR^{a}, -NHC(O)R^{a}, and -NHC(O)NHR^{a};
optionally, R³ is selected from the group consisting of -NHR^{b} and -NR^{a}R^{b};
optionally, R³ is selected from -OR^{a};
optionally, R³ is selected from the group consisting of -NHC(O)R^{a} and -NHC(O)NHR^{a};
or R³ is selected from the group consisting of halogen, cyano, nitro, NH₂, OH, C₁₋₆ alkyl, C₂₋₆ alkenyl, C₂₋₆ alkynyl, C₃₋₈ cycloalkyl, 3- to 8-membered heterocyclyl, C₆₋₁₀ aryl, 5- to 6-membered heteroaryl, -NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, - S(O)₂H, -S(O)₂R^{a}, -S(O)₂NH₂, -S(O)₂NR^{a}R^{b}, -NHS(O)₂R^{a}, -NHC(O)OR^{a}, and -N(C₁₋₄ alkyl)C(O)OR^{a}, wherein the C₁₋₆ alkyl, C₂₋₆ alkenyl, C₂₋₆ alkynyl, C₃₋₈ cycloalkyl, 3- to 8-membered heterocyclyl, C₆₋₁₀ aryl, or 5- to 6-membered heteroaryl is optionally substituted with one or more R^{c};
optionally, R³ is selected from the group consisting of halogen, cyano, nitro, NH₂, OH, C₁₋₄ alkyl, C₂₋₄ alkenyl, C₂₋₄ alkynyl, C₃₋₆ cycloalkyl, 3- to 6-membered heterocycloalkyl, phenyl, 5- to 6-membered heteroaryl, -NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₄ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, and -N(C₁₋₄ alkyl)C(O)OR^{a}, wherein the C₁₋₄ alkyl, C₂₋₄ alkenyl, C₂₋₄ alkynyl, C₃₋₆ cycloalkyl, 3- to 6-membered heterocycloalkyl, phenyl, or 5- to 6-membered heteroaryl is optionally substituted with one or more R^{c};
optionally, R³ is selected from the group consisting of halogen, cyano, nitro, NH₂, OH, C₁₋₃ alkyl, C₂₋₃ alkenyl, C₂₋₃ alkynyl, C₃₋₆ cycloalkyl, 3- to 6-membered heterocycloalkyl, phenyl, 5- to 6-membered heteroaryl, -NHR^{b}, -NR^{a}R^{b}, -OR^{a}, -SR^{a}, -C(O)H, -C(O)R^{a}, COOH, -C(O)OR^{a}, -C(O)NH₂, -C(O)NR^{a}R^{b}, -OC(O)R^{a}, -NHC(O)R^{a}, -N(C₁₋₃ alkyl)C(O)R^{a}, -S(O)₂H, -S(O)₂R^{a}, -S(O)₂NH₂, -S(O)₂NR^{a}R^{b}, -NHS(O)₂R^{a}, -NHC(O)OR^{a}, and -N(C₁₋₃ alkyl)C(O)OR^{a}, wherein the C₁₋₃ alkyl, C₂₋₃ alkenyl, C₂₋₃ alkynyl, C₃₋₆ cycloalkyl, 3- to 6-membered heterocycloalkyl, phenyl, or 5- to 6-membered heteroaryl is optionally substituted with one or more R^{c};
optionally, R³ is selected from the group consisting of halogen, cyano, NH₂, OH, and -NHR^{b};
optionally, R³ is selected from the group consisting of fluoro, chloro, bromo, and -NHR^{b};
optionally, R³ is selected from the group consisting of bromo, 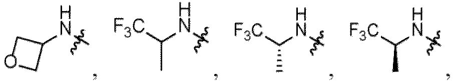 and

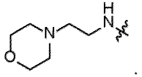

7. The compound, the stereoisomer thereof or the pharmaceutically acceptable salt thereof according to any one of claims 1-6, wherein R^{c} is selected from the group consisting of OH, NH₂, oxo, halogen, cyano, nitro, C₁₋₆ alkyl, C₁₋₆ alkoxy, -N(C₁₋₆ alkyl)₂, -NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, - S(O)₂NR^{a}R^{b}, -NHS(O)₂R^{a}, -C₁₋₆ alkyl-S(O)₂-C₁₋₆ alkyl, and 3- to 6-membered heterocycloalkyl optionally substituted with one or more groups independently selected from the group consisting of OH, halogen, and C₁₋₄ alkyl; optionally, R^{c} is selected from the group consisting of OH, NH₂, oxo, halogen, cyano, nitro, C₁₋₄ alkyl, C₁₋₄ alkoxy, - N(C₁₋₄ alkyl)₂, -NH(C₁₋₄ alkyl), -C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, -C₁₋₄ alkyl-S(O)₂-C₁₋₄ alkyl, and 4- to 6-membered heterocycloalkyl optionally substituted with one or more groups independently selected from the group consisting of OH, halogen, and C₁₋₃ alkyl;
optionally, R^{c} is selected from the group consisting of OH, NH₂, halogen, cyano, C₁₋₄ alkyl, C₁₋₄ alkoxy, -N(C₁₋₄ alkyl)₂, -NH(C₁₋₄ alkyl), -C₁₋₄ alkyl-S(O)₂-C₁₋₄ alkyl, and 4- to 6-membered heterocycloalkyl optionally substituted with one or more groups independently selected from the group consisting of OH, halogen, and C₁₋₃ alkyl;
optionally, R^{c} is selected from the group consisting of OH, C₁₋₃ alkyl, -N(C₁₋₃ alkyl)₂, -C₁₋₃ alkyl-S(O)₂-C₁₋₃ alkyl, and 6-membered heterocycloalkyl optionally substituted with one or more groups independently selected from the group consisting of OH, halogen, and C₁₋₃ alkyl;
optionally, R^{c} is selected from the group consisting of OH, C₁₋₃ alkyl, -N(C₁₋₃ alkyl)₂, -C₁₋₃ alkyl-S(O)₂-C₁₋₃ alkyl, and piperazinyl optionally substituted with one or more C₁₋₃ alkyl;
optionally, R^{c} is selected from the group consisting of OH, methyl, 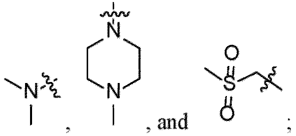
or R^{c} is selected from the group consisting of oxo, OH, NH₂, halogen, cyano, nitro, C₁₋₈ alkoxy, -N(C₁₋₈ alkyl)₂, - NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, and 3- to 8-membered heterocycloalkyl optionally substituted with one or more groups independently selected from the group consisting of OH, halogen, and C₁₋₆ alkyl;
optionally, R^{c} is selected from the group consisting of oxo, halogen, cyano, nitro, C₁₋₆ alkoxy, -N(C₁₋₆ alkyl)₂, -NH(C₁₋₈ alkyl), -C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, and 3- to 6-membered heterocycloalkyl optionally substituted with one or more groups independently selected from the group consisting of hydroxy, halogen, and C₁₋₄ alkyl;
optionally, R^{c} is selected from the group consisting of oxo, halogen, cyano, nitro, C₁₋₄ alkoxy, -N(C₁₋₄ alkyl)₂, -NH(C₁₋₄ alkyl), -C(O)R^{a}, -C(O)OR^{a}, -C(O)NR^{a}R^{b}, -OC(O)R^{a}, -NHC(O)R^{a}, -S(O)₂R^{a}, -S(O)₂NR^{a}R^{b}, -NHS(O)₂R^{a}, and 4- to 6-membered heterocycloalkyl optionally substituted with one or more groups independently selected from the group consisting of hydroxy, halogen, and C₁₋₃ alkyl;
optionally, R^{c} is selected from the group consisting of oxo, halogen, cyano, C₁₋₄ alkoxy, -N(C₁₋₄ alkyl)₂, -NH(C₁₋₄ alkyl), and 4- to 6-membered heterocycloalkyl optionally substituted with one or more groups independently selected from the group consisting of hydroxy, halogen, and C₁₋₃ alkyl.

8. The compound, the stereoisomer thereof or the pharmaceutically acceptable salt thereof according to any one of claims 1-7, wherein R^{a} and R^{b} are each independently selected from the group consisting of the following groups optionally substituted with one or more R": C₁₋₆ alkyl, C₂₋₆ alkenyl, C₂₋₆ alkynyl, C₃₋₆ cycloalkyl, 3- to 8-membered heterocycloalkyl, C₆₋₁₀ aryl, and 5- to 6-membered heteroaryl;
optionally, R^{a} and R^{b} are each independently selected from the group consisting of the following groups optionally substituted with one or more R": C₁₋₈ alkyl, C₂₋₈ alkenyl, C₂₋₈ alkynyl, C₃₋₈ cycloalkyl, and 3- to 8-membered heterocycloalkyl;
optionally, R^{a} and R^{b} are each independently selected from the group consisting of the following groups optionally substituted with one or more R": C₁₋₈ alkyl, C₃₋₈ cycloalkyl, and 3- to 8-membered heterocycloalkyl;
optionally, R^{a} and R^{b} are each independently selected from the group consisting of the following groups optionally substituted with one or more R": C₁₋₅ alkyl, C₃₋₆ cycloalkyl, and 4- to 6-membered heterocycloalkyl;
optionally, R^{a} and R^{b} are each independently selected from the group consisting of the following groups optionally substituted with one or more R": methyl, ethyl, propyl, butyl, pentyl, cyclopropyl, cyclobutyl, cyclopentyl, cyclohexyl, oxetanyl, azetidinyl, tetrahydrofuranyl, tetrahydropyranyl, and piperidinyl;
or R^{a} and R^{b} are each independently selected from the following groups optionally substituted with one or more R": C₁₋₄ alkyl, C₂₋₄ alkenyl, C₂₋₄ alkynyl, C₃₋₆ cycloalkyl, 3- to 6-membered heterocycloalkyl, phenyl, and 5- to 6-membered heteroaryl;
optionally, R^{a} and R^{b} are each independently selected from the group consisting of the following groups optionally substituted with one or more R": C₃₋₆ cycloalkyl and 4- to 6-membered heterocycloalkyl.

9. The compound, the stereoisomer thereof or the pharmaceutically acceptable salt thereof according to any one of claims 1-8, wherein R" are each independently selected from the group consisting of halogen, oxo, deuterium, NH₂, OH, cyano, nitro, C₁₋₈ alkyl, C₁₋₈ haloalkyl, C₁₋₈ alkoxy, -N(C₁₋₈ alkyl)₂, -N(C₁₋₈ deuteroalkyl)₂, -NH(C₁₋₈ alkyl), C₃₋₆ cycloalkyl, and 3- to 10-membered heterocycloalkyl optionally substituted with C₁₋₈ alkyl;
optionally, R" are each independently selected from the group consisting of halogen, oxo, deuterium, NH₂, OH, cyano, nitro, C₁₋₆ haloalkyl, C₁₋₆ alkyl, C₁₋₆ alkoxy, -N(C₁₋₆ alkyl)₂, -N(C₁₋₆ deutetoalkyl)₂, -NH(C₁₋₆ alkyl), C₃₋₆ cycloalkyl, and 3- to 6-membered heterocycloalkyl optionally substituted with C₁₋₆ alkyl;
optionally, R" are each independently selected from the group consisting of halogen, oxo, deuterium, NH₂, OH, cyano, nitro, C₁₋₃ alkyl, C₁₋₃ haloalkyl, C₁₋₃ alkoxy, -N(C₁₋₃ alkyl)₂, -N(C₁₋₃ deuteroalkyl)₂, -NH(C₁₋₃ alkyl), C₄₋₆ cycloalkyl, and 3- to 6-membered heterocycloalkyl optionally substituted with C₁₋₃ alkyl;
optionally, R" are each independently selected from the group consisting of fluoro, chloro, bromo, oxo, deuterium, NH₂, OH, cyano, C₁₋₄ alkyl, C₁₋₄ fluoroalkyl, C₁₋₄ alkoxy, -N(C₁₋₄ alkyl)₂, -N(C₁₋₄ deuteroalkyl)₂, -NH(C₁₋₄ alkyl), C₅₋₆ cycloalkyl, and 4- to 6-membered heterocycloalkyl optionally substituted with C₁₋₄ alkyl;
optionally, R" are each independently selected from the group consisting of fluoro, OH, oxo, deuterium, -NH₂, methyl, ethyl, propyl, trifluoromethyl, -N(CH₃)₂, -N(CD₃)₂, -N(CH₂CH₃)₂, -NHCH₃, morpholinyl, pyrrolidinyl, piperidinyl, tetrahydropyranyl, 1,4-dioxanyl, and oxetanyl optionally substituted with methyl or ethyl;
or R" are each independently selected from the group consisting of halogen and 3- to 6-membered heterocycloalkyl; optionally, R" are each independently selected from the group consisting of fluoro, chloro, bromo, and 6-membered heterocycloalkyl;
optionally, R" are each independently selected from the group consisting of fluoro and morpholinyl.

10. The compound, the stereoisomer thereof or the pharmaceutically acceptable salt thereof according to any one of claims 1-9, wherein n is selected from the group consisting of 0, 1, 2, and 3;
optionally, n is selected from the group consisting of 0, 1, and 2;
optionally, n is selected from the group consisting of 1 and 2;
optionally, n is selected from 1.

11. The compound, the stereoisomer thereof or the pharmaceutically acceptable salt thereof according to any one of claims 1-10, R⁴ is selected from the group consisting of C₁₋₃ alkyl and C₃₋₄ cycloalkyl;
optionally, R⁴ is selected from the group consisting of methyl, ethyl, cyclopropyl, and cyclobutyl;
optionally, R⁴ is selected from the group consisting of methyl and cyclopropyl;
optionally, R⁴ is selected from cyclopropyl;
optionally, R⁴ is selected from methyl.

12. The compound, the stereoisomer thereof or the pharmaceutically acceptable salt thereof according to any one of claims 1-11, wherein the compound of formula I" is selected from the group consisting of a compound of formula I'A, a compound of formula I'B, a compound of formula I, a compound of formula IA, a compound of formula IB, a compound of formula I-1, a compound of formula I-1A, a compound of formula I-1B, a compound of formula I-2, a compound of formula I-2A, a compound of formula I-2B, a compound of formula II, a compound of formula IIA, a compound of formula IIB, a compound of formula II-1, a compound of formula II-1A, a compound of formula II-1B, a compound of formula II-2, a compound of formula II-2A, a compound of formula II-2B, a compound of formula III-1, a compound of formula III-1A, a compound of formula III-1B, A compound of formula III-2, a compound of formula III-2A, a compound of formula III-2B, a compound of formula IV, a compound of formula IVA, a compound of formula IVB, a compound of formula IV-1, a compound of formula IV-1A, a compound of formula IV-1B, a compound of formula IV-2, a compound of formula IV-2A, or a compound of formula IV-2B,

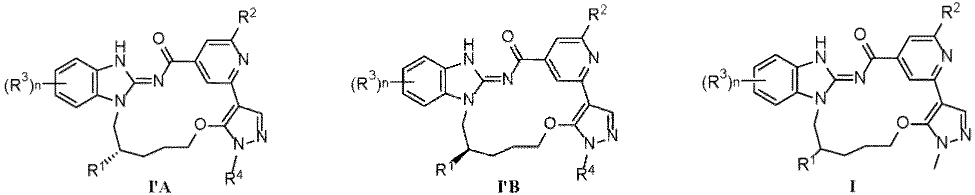

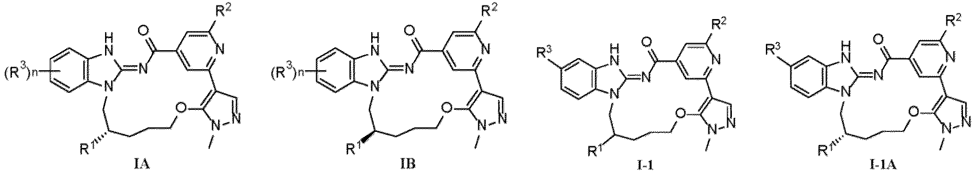

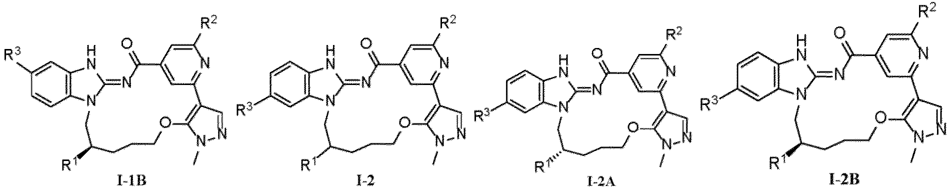

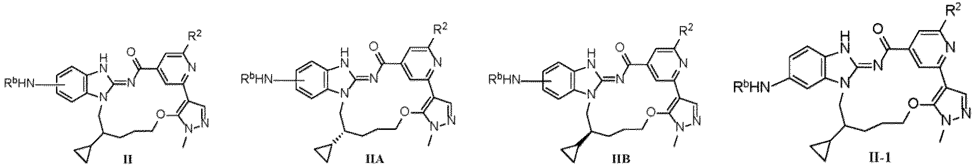

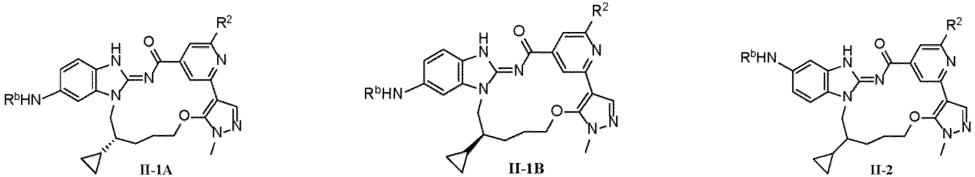

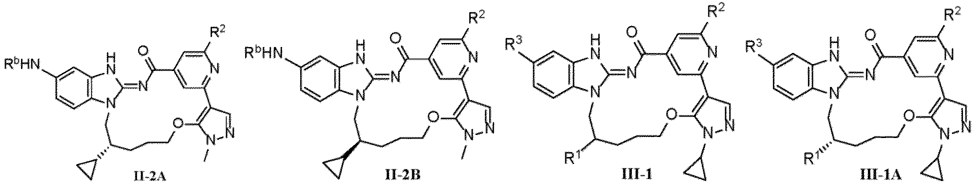

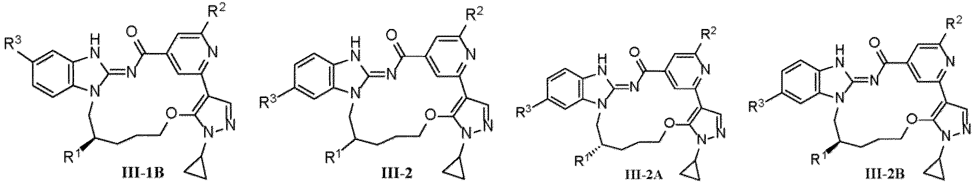

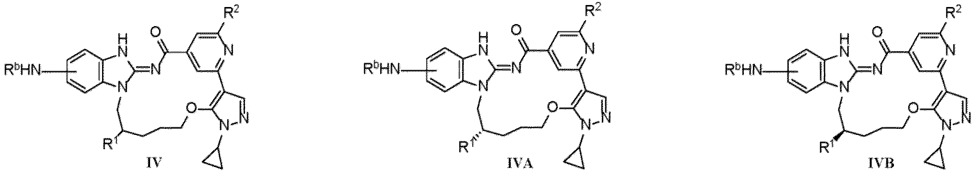

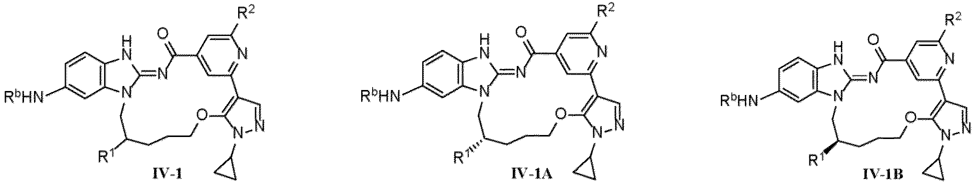

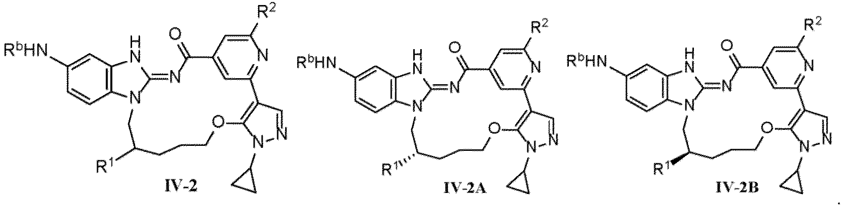

13. A compound, a stereoisomer thereof or a pharmaceutically acceptable salt thereof, selected from the group consisting of:

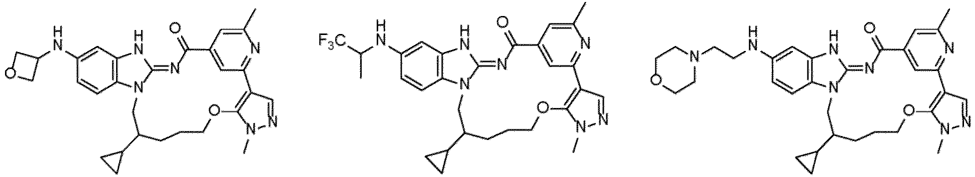

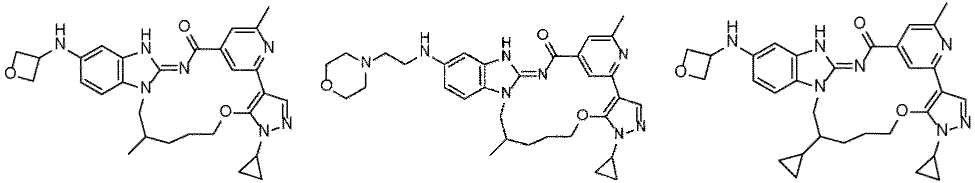

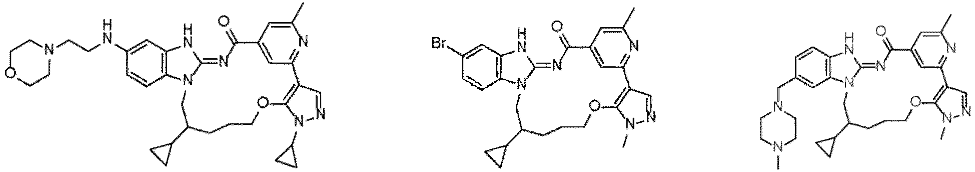

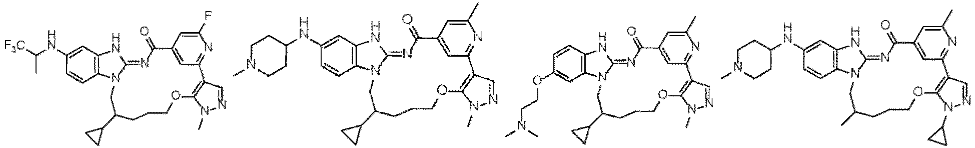

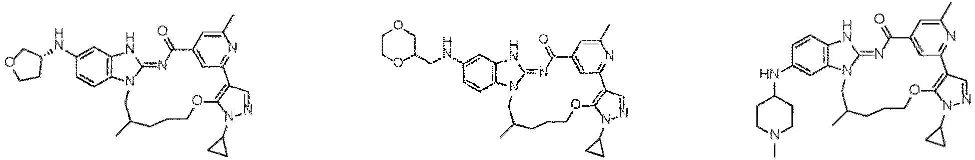

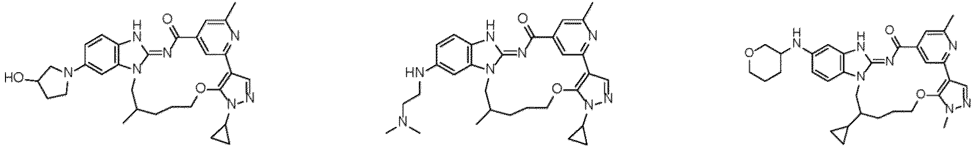

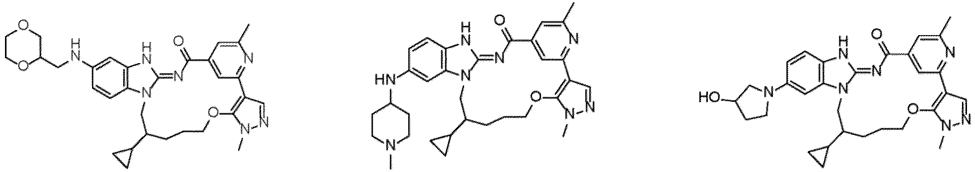

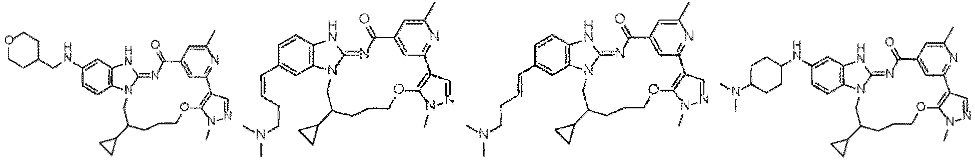

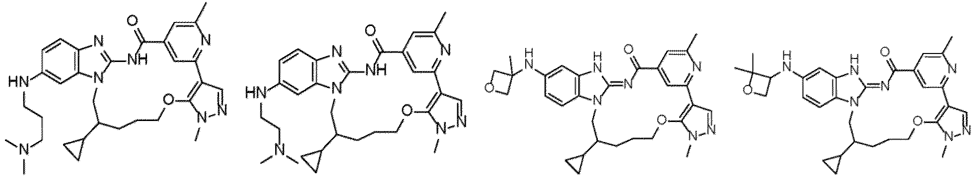

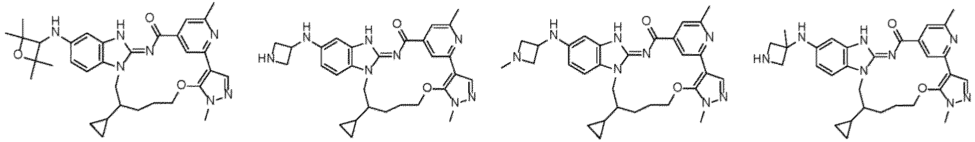

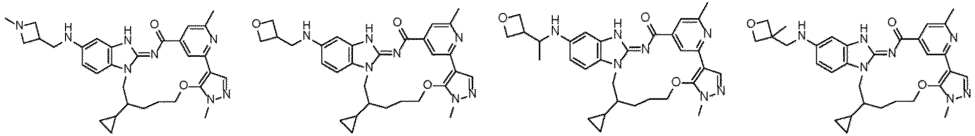

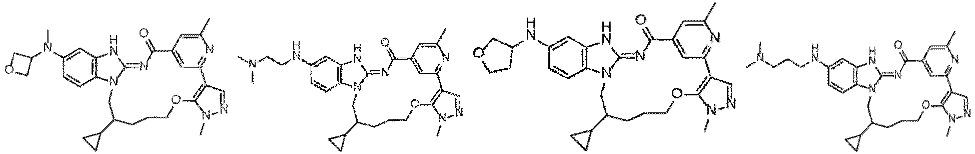

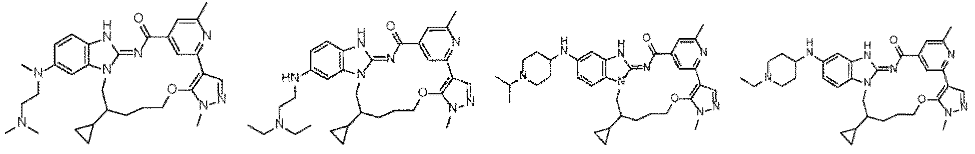

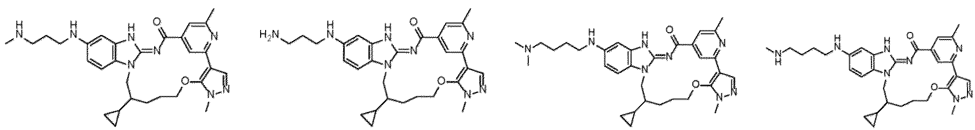

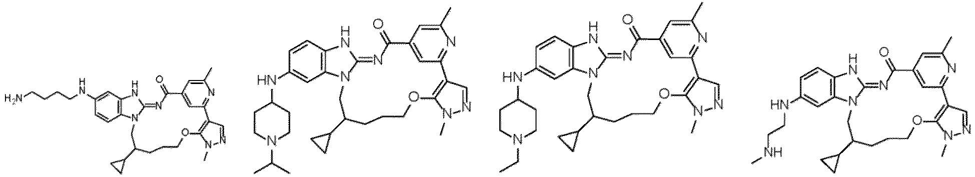

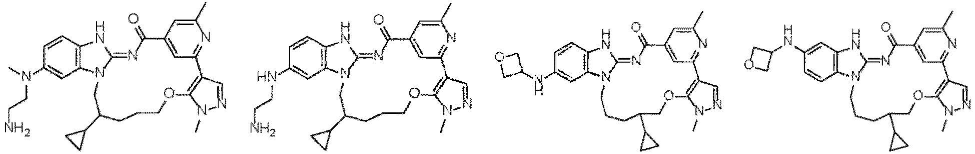

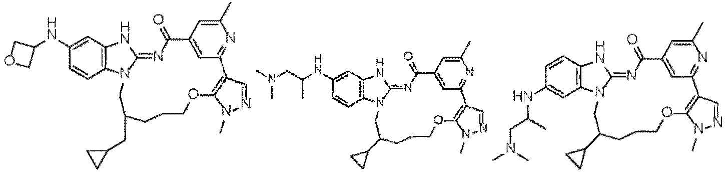

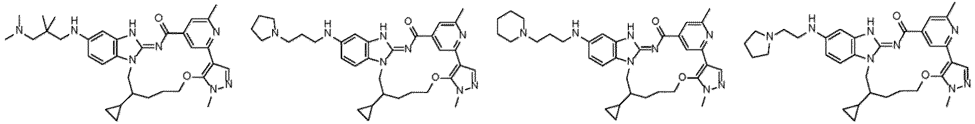

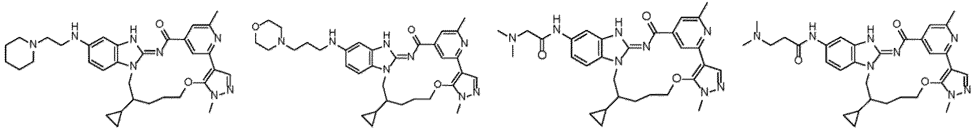

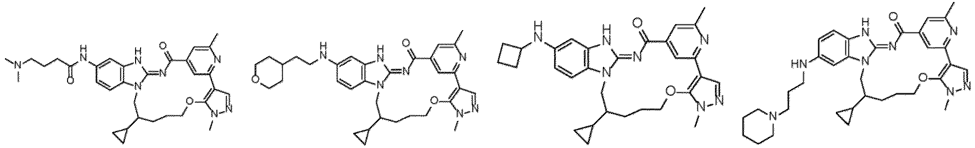

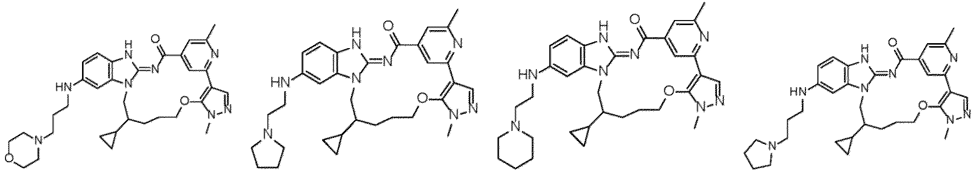

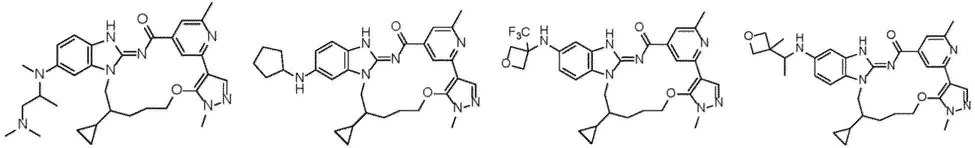

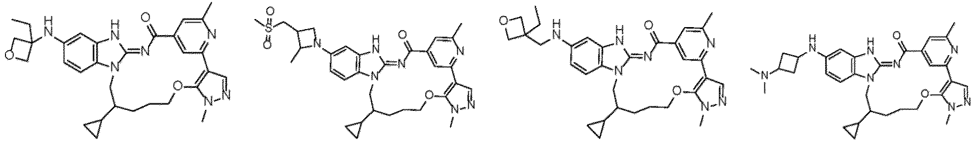

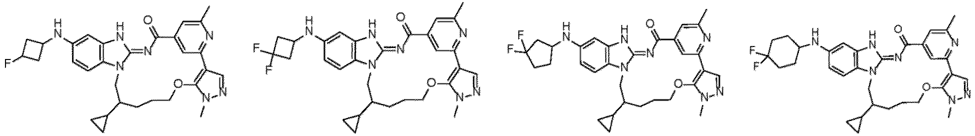

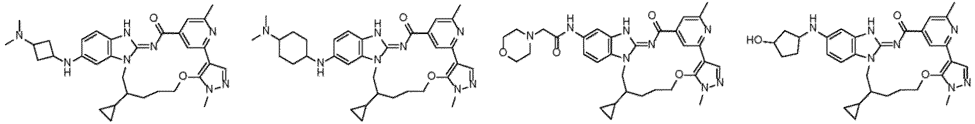

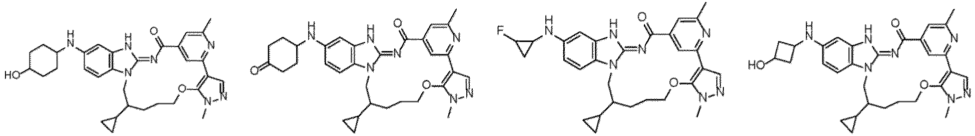

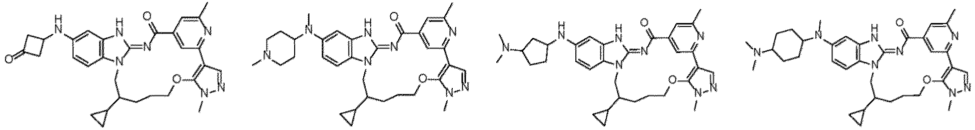

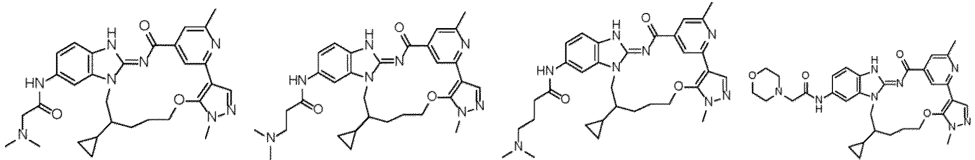

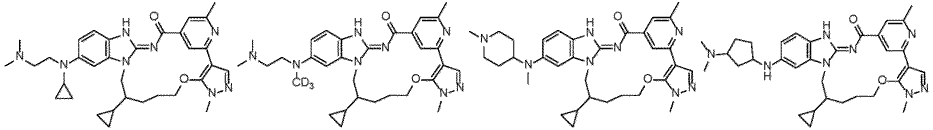

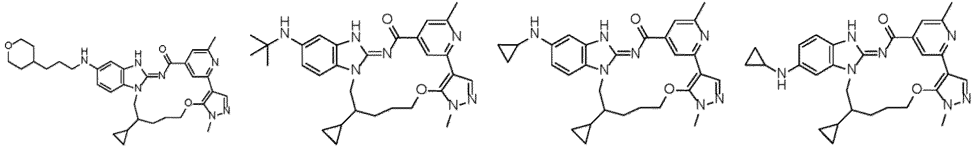

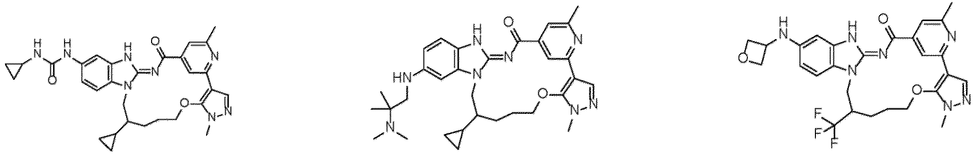

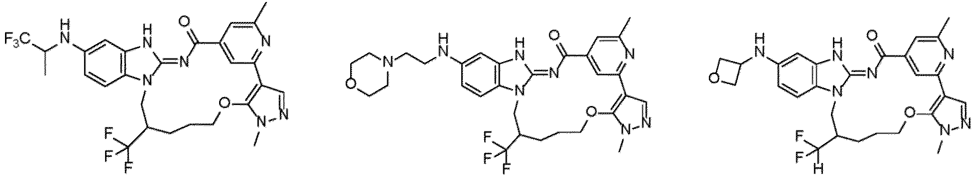

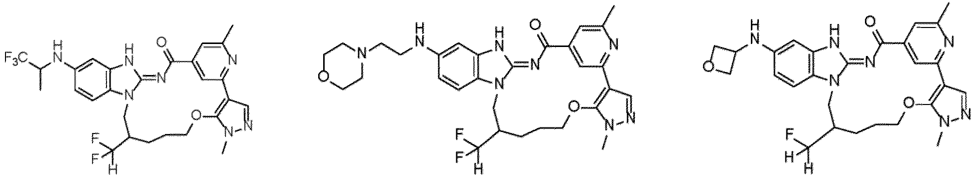

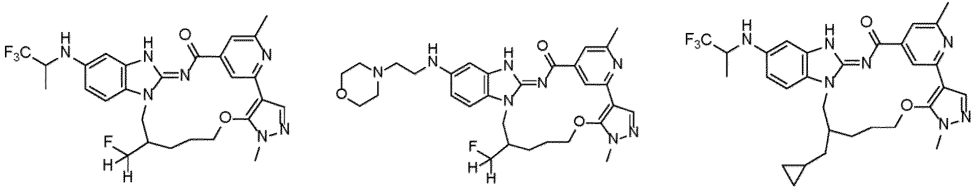

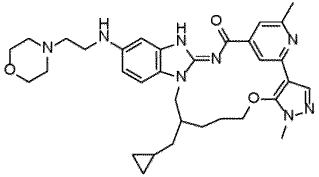

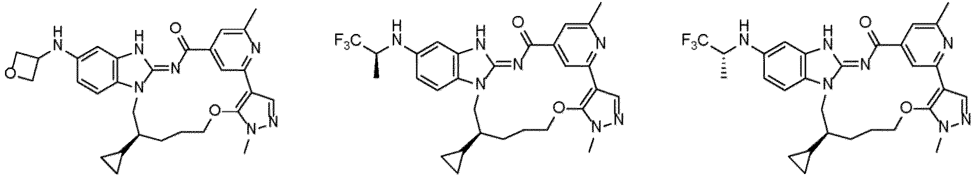

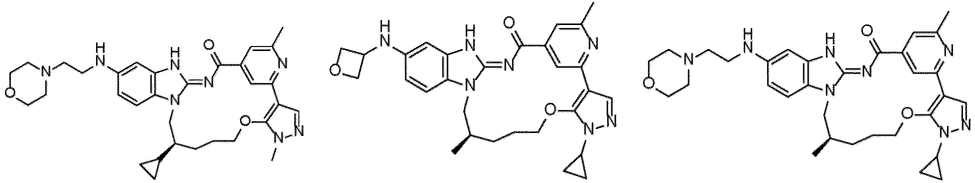

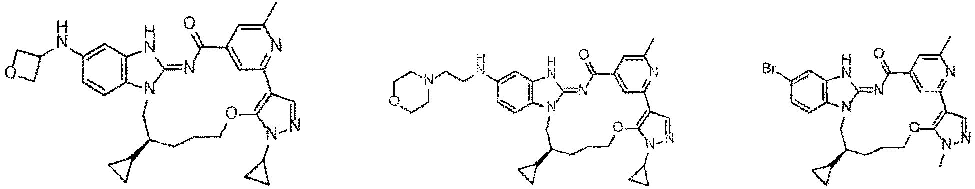

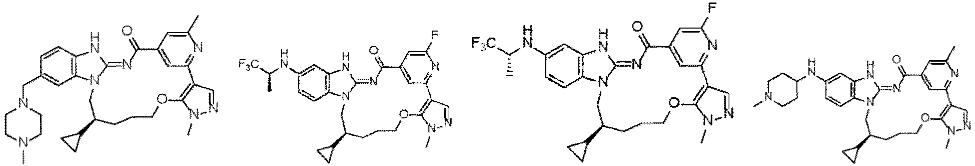

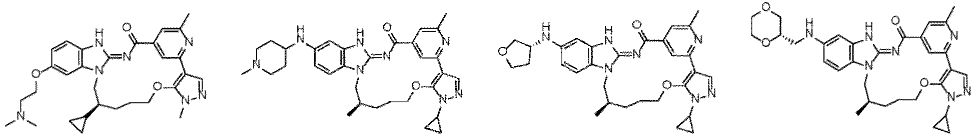

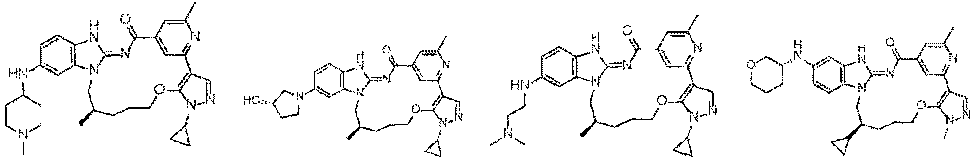

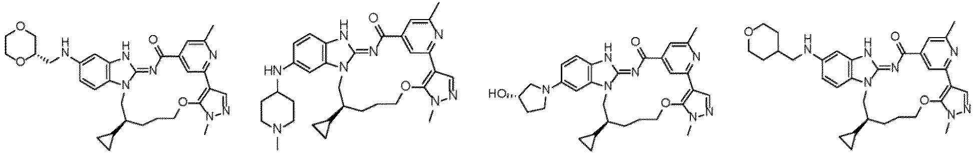

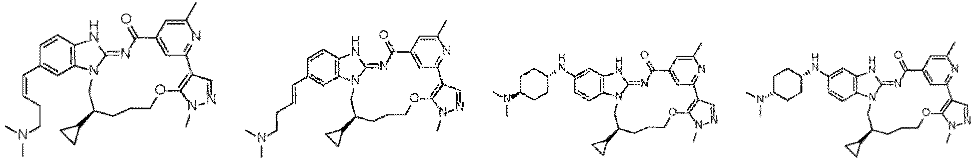

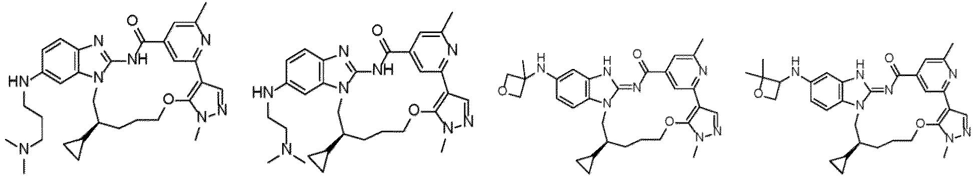

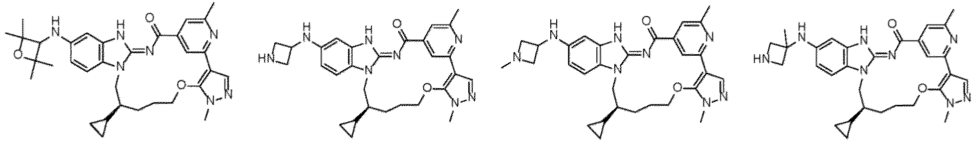

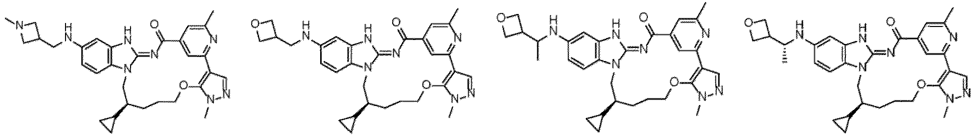

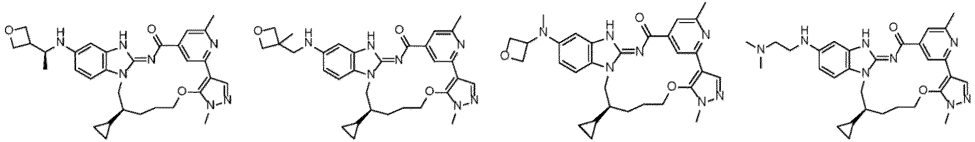

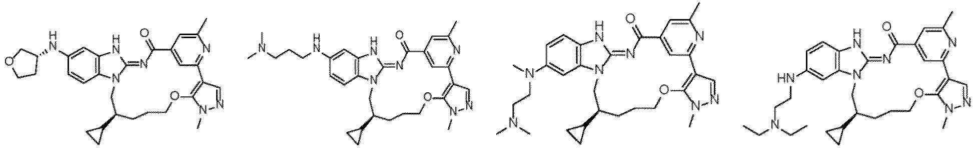

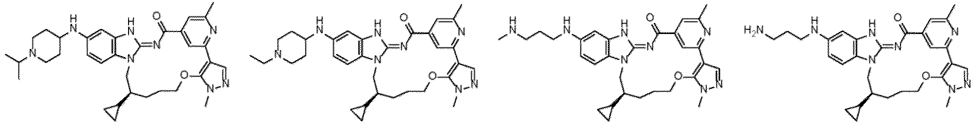

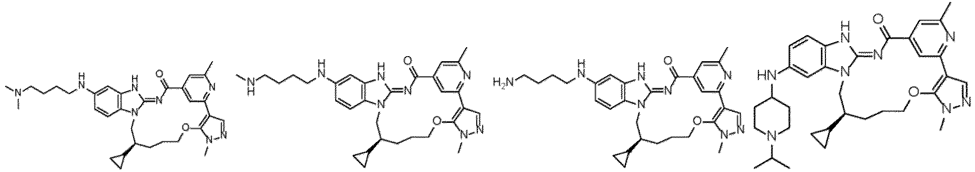

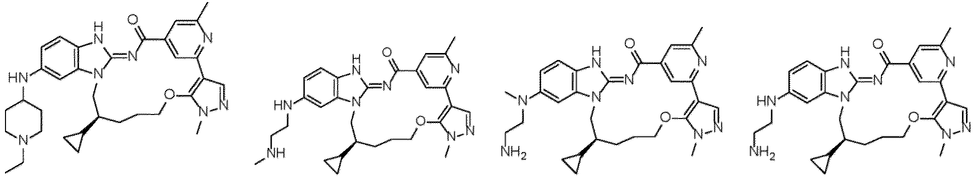

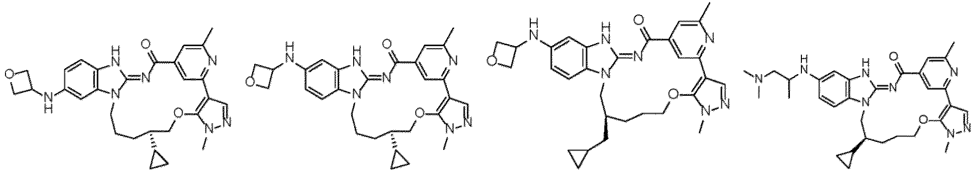

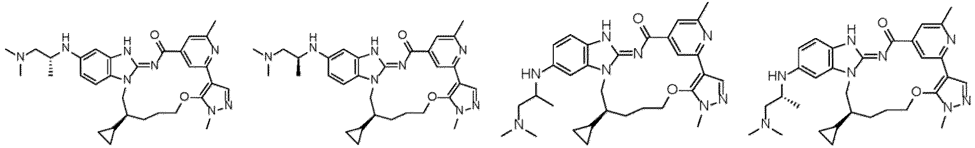

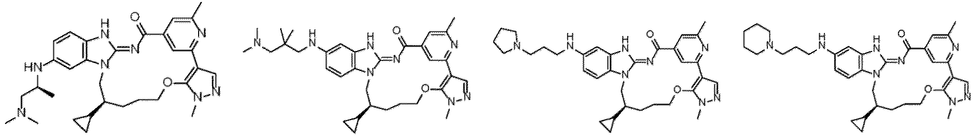

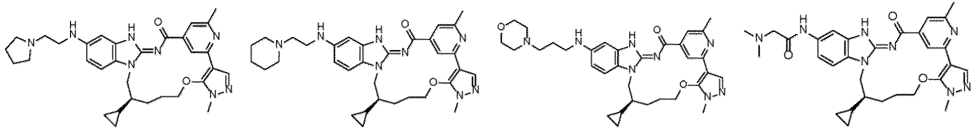

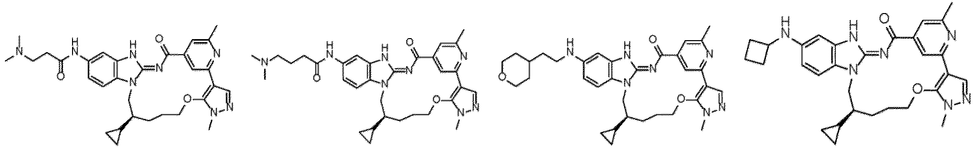

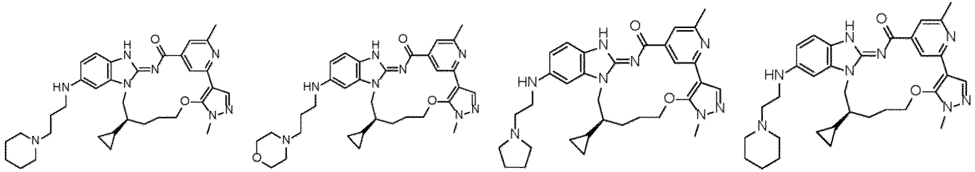

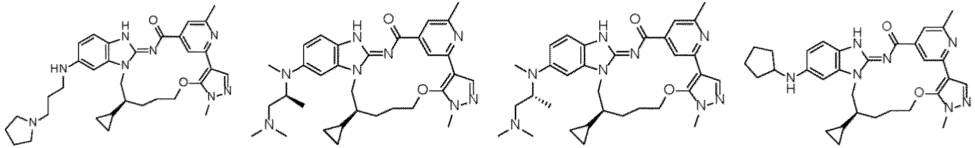

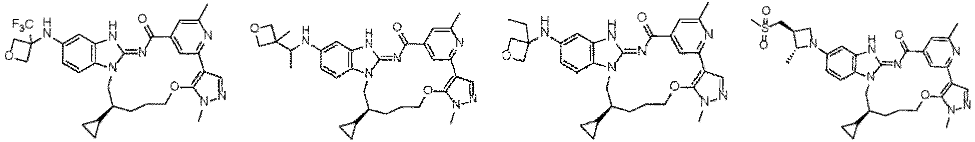

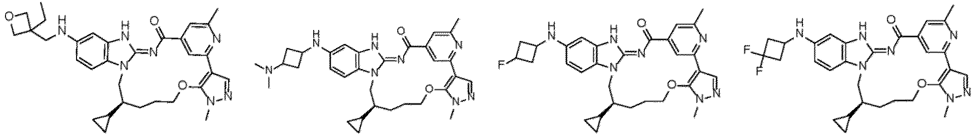

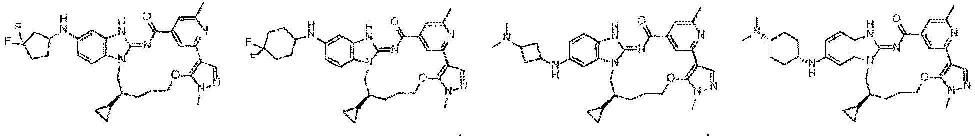

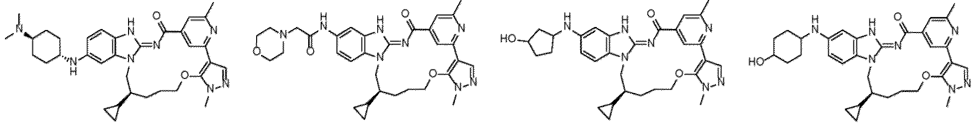

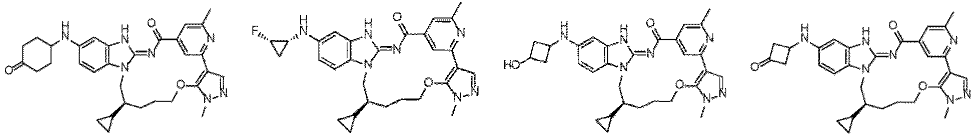

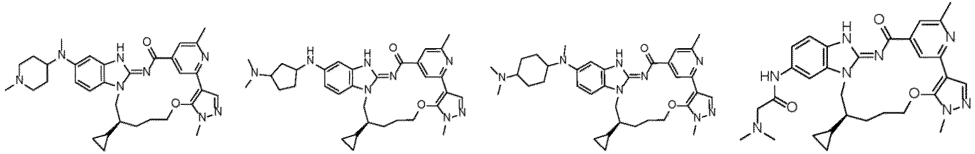

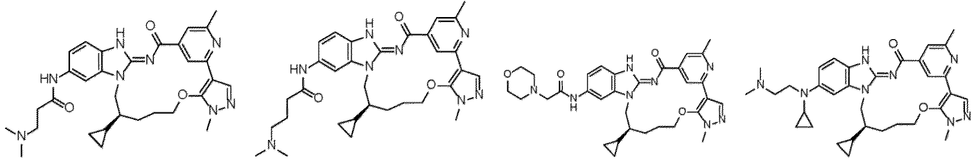

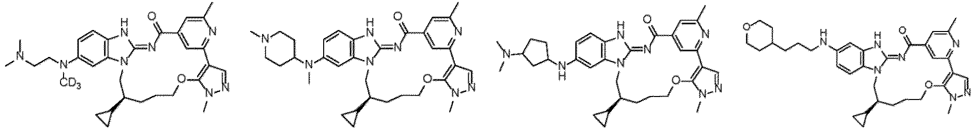

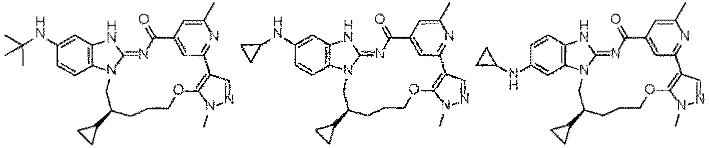

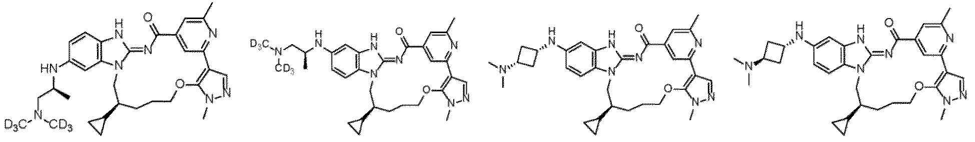

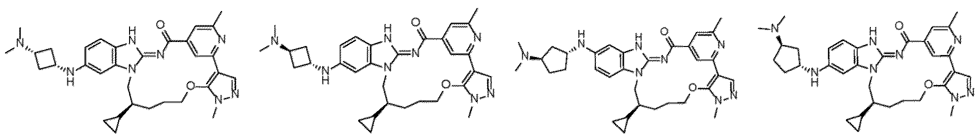

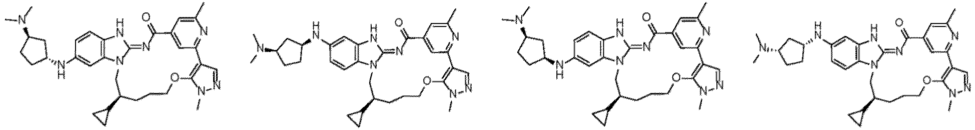

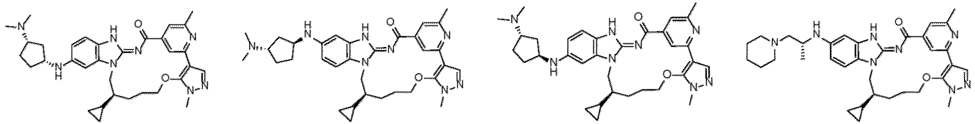

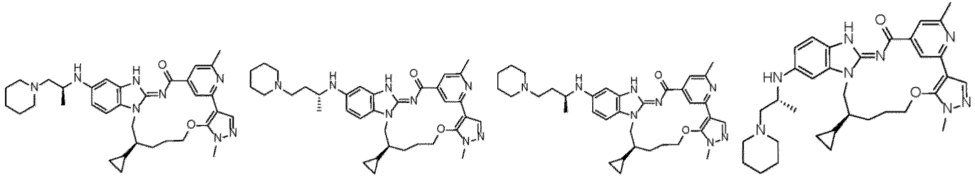

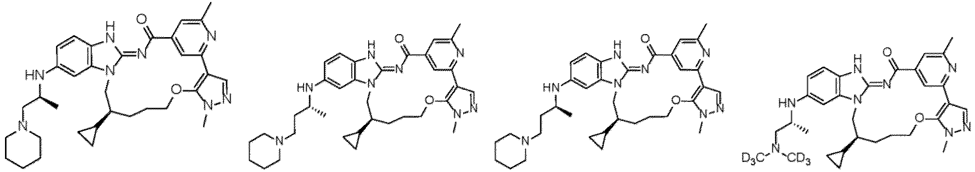

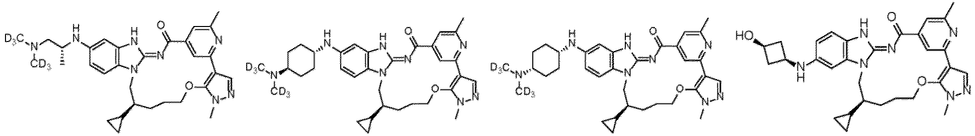

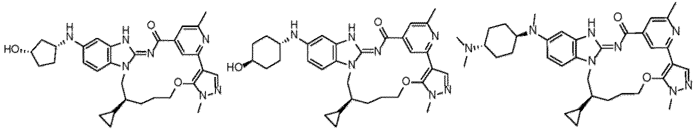

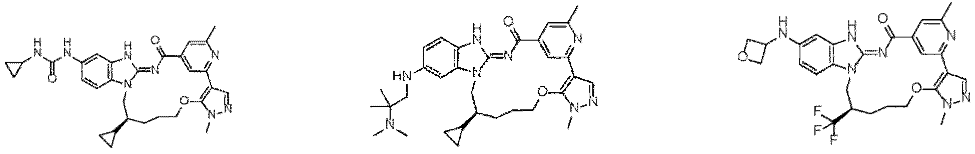

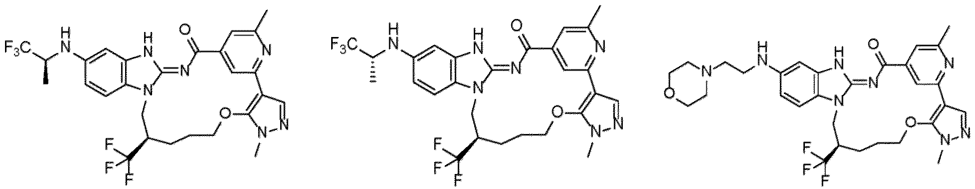

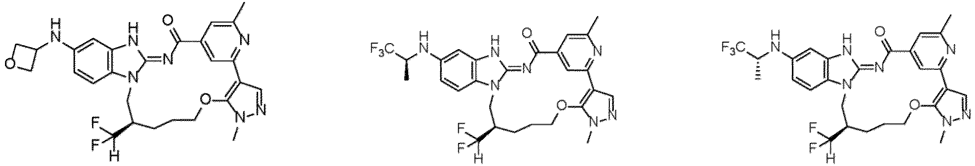

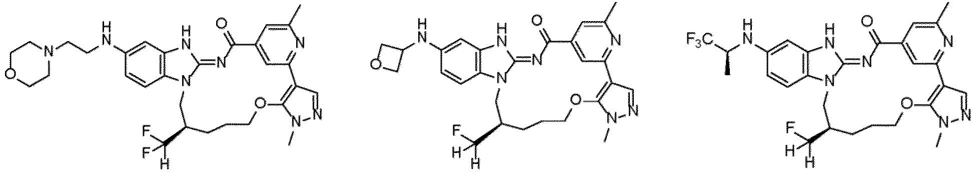

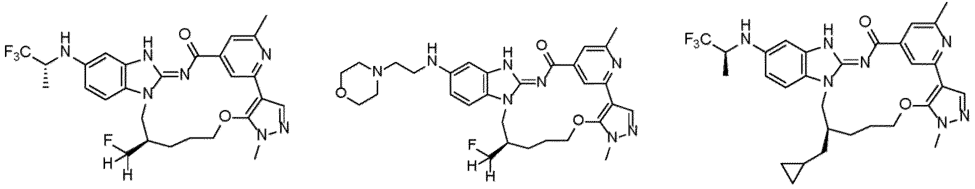

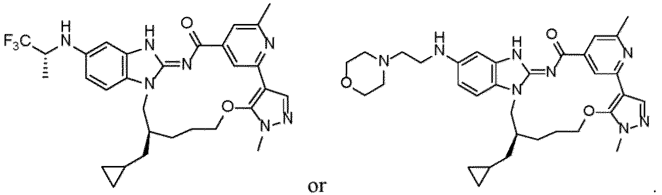

14. A pharmaceutical composition comprising the compound, the stereoisomer thereof or the pharmaceutically acceptable salt thereof according to any one of claims 1-13.

15. Use of the compound, the stereoisomer thereof or the pharmaceutically acceptable salt thereof according to any one of claims 1-13, or the pharmaceutical composition according to claim 14 for preparing a medicament for preventing or treating a cancer;
optionally, the cancer is selected from lung cancer;
optionally, the cancer is selected from non-small cell lung cancer.
